(19) Europäisches Patentamt / European Patent Office / Office européen des brevets

(11) **EP 4 117 716 B1**

(12) **EUROPEAN PATENT SPECIFICATION**

(45) Date of publication and mention
of the grant of the patent:
**06.05.2026 Bulletin 2026/19**

(51) International Patent Classification (IPC):
*A61K 39/00* (2006.01)        *A61K 35/17* (2025.01)
*A61P 35/00* (2006.01)        *C12N 5/10* (2006.01)

(21) Application number: **21716011.8**

(52) Cooperative Patent Classification (CPC):
**C12N 5/0646; A61K 40/15; A61K 40/31;
A61K 40/35; A61K 40/4201; A61K 40/4211;**
A61K 2239/17; A61K 2239/21; A61K 2239/38;
A61K 2239/48; C12N 2501/2312; C12N 2501/2315;
C12N 2501/2318; C12N 2510/00

(22) Date of filing: **10.03.2021**

(86) International application number:
**PCT/US2021/021758**

(87) International publication number:
**WO 2021/183675 (16.09.2021 Gazette 2021/37)**

(54) **METHODS FOR GENERATING ENGINEERED MEMORY-LIKE NK CELLS AND COMPOSITIONS THEREOF**

VERFAHREN ZUR ERZEUGUNG MANIPULIERTER GEDÄCHTNISÄHNLICHER NK-ZELLEN UND ZUSAMMENSETZUNGEN DAVON

PROCÉDÉS DE GÉNÉRATION DE CELLULES NK DE TYPE MÉMOIRE MODIFIÉES ET COMPOSITIONS DE CELLES-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.03.2020   US 202062987612 P
01.12.2020   US 202063119959 P
03.12.2020   US 202063121127 P
08.01.2021   US 202117144834**

(43) Date of publication of application:
**18.01.2023 Bulletin 2023/03**

(73) Proprietors:
• **Massachusetts Institute of Technology
Cambridge, MA 02139 (US)**
• **Dana-Farber Cancer Institute, Inc.
Boston, MA 02215-5450 (US)**

(72) Inventors:
• **CHEN, Jianzhu
Lexington, MA 02420 (US)**
• **RITZ, Jerome
Lincoln, MA 01773-3906 (US)**
• **ROMEE, Rizwan
Wellesley, MA 02782 (US)**
• **DONG, Han
Boston, MA 02215 (US)**
• **XIE, Guozhu
Cambridge, MA 02142 (US)**
• **HAM, James, Dongjoo
Cambridge, MA 02139 (US)**

(74) Representative: **Hoffmann Eitle
Patent- und Rechtsanwälte PartmbB
Arabellastraße 30
81925 München (DE)**

(56) References cited:
• **ROMEE R ET AL: "Cytokine-induced memory-like natural killer cells exhibit enhanced responses against myeloid leukemia.", SCIENCE TRANSLATIONAL MEDICINE, vol. 8, no. 357, 21 September 2016 (2016-09-21), pages 357ra123, XP055815094, ISSN: 1946-6242**

- ROMEE R ET AL: "Supplemental Material", SCIENCE TRANSLATIONAL MEDICINE, vol. 8, no. 357, 357RA123, 21 September 2016 (2016-09-21), XP055815100, ISSN: 1946-6234, DOI: 10.1126/scitranslmed.aaf2341
- VAN DER LEE D I ET AL: "Mutated nucleophosmin 1 as immunotherapy target in acute myeloid leukemia.", JOURNAL OF CLINICAL INVESTIGATION, vol. 129, no. 2, 1 February 2019 (2019-02-01), pages 774 - 785, XP055815085, ISSN: 1558-8238
- GREINER J ET AL: "Mutated regions of nucleophosmin 1 elicit both CD4+ and CD8+ T-cell responses in patients with acute myeloid leukemia", BLOOD, vol. 120, no. 6, 9 August 2012 (2012-08-09), pages 1282 - 1289, XP055426172, ISSN: 0006-4971, DOI: 10.1182/blood-2011-11-394395
- YANG X & WANG J: "Precision therapy for acute myeloid leukemia", JOURNAL OF HEMATOLOGY AND ONCOLOGY, vol. 11, no. 1, 3, 5 January 2018 (2018-01-05), XP055815106, ISSN: 1756-8722
- COLAMARTINO A B L ET AL: "Efficient and Robust NK-Cell Transduction With Baboon Envelope Pseudotyped Lentivector", FRONTIERS IN IMMUNOLOGY, vol. 10, 2873, 16 December 2019 (2019-12-16), XP055815121, ISSN: 1664-3224
- BERRIEN-ELLIOTT M M ET AL: "Chimeric Antigen Receptor Modified Memory-like (CAR-ML) NK Cells Exhibit Potent Responses to NK-Resistant Tumors", BLOOD, vol. 134, no. Suppl. 1, 869, 13 November 2019 (2019-11-13), & 61st Annual Meeting and Exposition of the American Society of Hematology (ASH); Orlando, FL, USA; 7-10 December 2019, XP086672024, ISSN: 0006-4971, DOI: 10.1182/blood-2019-127546
- DONG H ET AL: "Engineered Memory-like NK Cars Targeting a Neoepitope Derived from Intracellular NPM1c Exhibit Potent Activity and Specificity Against Acute Myeloid Leukemia", BLOOD, vol. 136, no. Suppl. 1, 703, 5 November 2020 (2020-11-05), & 62nd American Society of Hematology Annual Meeting; 5-8 December 2020, XP055815141, ISSN: 0006-4971, DOI: 10.1182/blood-2020-134148

**Description**

**BACKGROUND**

Cell-based immunotherapies are in development for treatment of cancer. Approaches using adoptive cell transfer of T cells, monocyte-derived cells (e.g., macrophages, dendritic cells) and natural killer (NK) cells are being explored as cancer treatments (see, e.g., Andreesen, R. et al (1990) Cancer Res 50:7450-7456; Ruggeri, L. et al (2002) Science 295:2097-2100; Rezvani, K. (2019) Bone Marrow Transplantation 54:785-788). Specifically, adoptive cell therapy (ACT), in which ex vivo activated/expanded NK cells are administered to patients, is one of the cancer treatments currently being tested. These approaches involve the use of autologous NK cells taken from patients that are activated/expanded ex vivo and then reinfused to combat tumors, such as metastatic tumors. Strategies that enhance the persistence, in vivo expansion, and effector functions of ACT NK is needed.

Chimeric antigen receptor (CAR) T cell therapy has emerged as one of the strategies for the treatment of cancer. Chimeric antigen receptors (CARs) are genetically-engineered, artificial transmembrane receptors that confer a defined specificity for an antigen (e.g., ligand) onto an immune effector cell (e.g., a T cell, natural killer cell or other immune cell), which results in activation of the effector cell upon recognition and binding to the antigen. However, current CARs targeting lineage-restricted or tumor-associated antigens (TAAs) can be accompanied by severe toxicity due to low antigen expression in normal tissues (see Coulie et al., NAT REV CANCER 14: 135 (2014); Srivastava & Riddell, J IMMUNOL 200: 459 (2018)). Furthermore, because TAAs are not required for tumor cell survival, loss of TAA expression is the major cause of development of tumor resistance to CAR-T therapies (see Srivastava & Riddell, J IMMUNOL 200: 459 (2018)). Accordingly, more effective CAR therapy strategies are needed.

**SUMMARY OF THE INVENTION**

The present invention is set out in the claims and provides an engineered cytokine-induced memory-like human NK cell or a population of said cells, wherein the engineered NK cell or population of said cells expresses a chimeric antigen receptor (CAR) polypeptide comprising an intracellular domain, a transmembrane domain and an extracellular binding domain, wherein the extracellular binding domain specifically binds to an antigen comprising an NPM1c neoepitope in complex with a class I major histocompatibility complex (MHC class I) protein.

In some aspects, the disclosure provides an engineered cytokine-induced memory-like (ML) human NK cell or a population of said cells, wherein the engineered NK cell or population of said cells expresses a chimeric antigen receptor (CAR) polypeptide comprising an intracellular domain, a transmembrane domain and an extracellular binding domain, wherein the extracellular binding domain specifically binds to an antigen comprising an NPM1c neoepitope in complex with a class I major histocompatibility complex (MHC class I) protein.

In some aspects, the engineered NK cell of the disclosure comprises a CAR comprising an extracellular binding domain that does not bind to, or substantially does not bind to: (a) the MHC class I protein alone, and/or (b) a control peptide in complex with the MHC class I protein, optionally wherein the control peptide is an NY-ESO-1 epitope or influenza virus M1 epitope.

In some aspects, the engineered NK cell of the disclosure comprises a CAR comprising an extracellular binding domain that binds to an antigen comprising an NPM1c neoepitope in complex with MHC class I protein, wherein the NPM1c neoepitope comprises an amino acid sequence $X_1X_2X_3X_4X_5X_6X_7X_8X_9$, wherein $X_1$ is selected from A, V, L or I, wherein $X_2$ is selected from A, T, S, V, L, I, M or Q, wherein $X_3$ is selected from Q or N, wherein $X_4$ is selected from D or E, wherein $X_5$ is selected from L, I, V, M, A or F, wherein $X_6$ is selected from C, S, or A, wherein $X_7$ is selected from L, I, V, M, A, or F, wherein $X_8$ is selected from A, V, L or I, and wherein $X_9$ is selected from L, I, V, M or A. In some aspects, $X_1$ is selected from A or V, wherein $X_2$ is selected from V, I, or L, wherein $X_3$ is selected from Q or N, wherein $X_4$ is selected from D or E, wherein $X_5$ is selected from L or I, wherein $X_6$ is selected from C or S, wherein $X_7$ is selected from V, L or I, wherein $X_8$ is selected from A or V, and wherein $X_9$ is selected from V, I, or L. In some aspects, $X_1$ is A, wherein $X_2$ is selected from V, I, or L, wherein $X_3$ is Q, wherein $X_4$ is D, wherein $X_5$ is L, wherein $X_6$ is C, wherein $X_7$ is L, wherein $X_8$ is A, and wherein $X_9$ is selected from V, I, or L.

In any of the foregoing or related aspects, the NPM1c neoepitope comprises an amino acid sequence selected from: AIQDLCLAV (SEQ ID NO:1) or AIQDLCVAV (SEQ ID NO: 71). In some aspects, the NPM1c neoepitope comprises an amino acid sequence selected from: CLAVEEVSL (SEQ ID NO:72), VEEVSLRK (SEQ ID NO:73), AVEEVSLR (SEQ ID NO:74), AVEEVSLRK (SEQ ID NO:75), CLAVEEVSLRK (SEQ ID NO:76). In some aspects, the neoepitope comprises the amino acid sequence AIQDLCLAV (SEQ ID NO:1).

In any of the foregoing or related aspects, the neoepitope is 7, 8, 9, 10, 11, or 12 amino acid residues in length.

In any of the foregoing or related aspects, the MHC class I protein is an HLA-A*02 protein or is encoded by the HLA-A*02 allele group. In some aspects, the MHC class I protein is encoded by the HLA-A*02:01 allele.

In some aspects, the engineered NK cell of the disclosure comprises a CAR comprising an extracellular domain comprising:

> (i) a heavy chain variable region (VH) comprising VH complementarity determining region (CDR)1, VH CDR2 and VH CDR3, said VH CDR1, VH CDR2 and VH CDR3 being the CDRs of a VH that has an amino acid sequence of SEQ ID NO: 5, and/or
> (ii) a light chain variable region (VL) comprising VL complementarity determining region (CDR)1, VL CDR2 and VL CDR3, said VL CDR1, VL CDR2 and VL CDR3 being the CDRs of a VL that has an amino acid sequence of SEQ ID NO: 3.

In some aspects, the engineered NK cell of the disclosure comprises a CAR comprising an extracellular domain comprising a VH comprising VH CDR1, VH CDR2 and VH CDR3, wherein the VH CDR1 has the amino acid sequence GFTFSSYA (SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11). In some aspects, the extracellular domain comprises a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8).

In some aspects, the engineered NK cell of the disclosure comprises a CAR comprising an extracellular domain comprises a VH and a VL, wherein the VH comprises an amino acid sequence which is at least 90% identical, or at least 95% identical, to the amino acid sequence of SEQ ID NO: 5, and/or wherein the VL comprises an amino acid sequence which is at least 90% identical, or at least 95% identical, to the amino acid sequence of SEQ ID NO: 3.

In some aspects, the engineered NK cell of the disclosure comprises a CAR comprising an extracellular domain comprises a VH and a VL, wherein the VH comprises the amino acid sequence of SEQ ID NO: 5, and/or wherein the VL comprises the amino acid sequence of SEQ ID NO: 3.

In some aspects, the engineered NK cell of the disclosure comprises a CAR comprising an extracellular domain which is a scFv. In some aspects, the scFv is a human scFv. In some aspects, the scFv comprises a VH comprising VH CDR1, VH CDR2 and VH CDR3, wherein the VH CDR1 has the amino acid sequence GFTFSSYA (SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11). In some aspects, the scFv comprises a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8). In some aspects the scFv comprises a VH and a VL, wherein the VH comprises an amino acid sequence which is at least 90% identical, or at least 95% identical, to the amino acid sequence of SEQ ID NO: 5, and/or wherein the VL comprises an amino acid sequence which is at least 90% identical, or at least 95% identical, to the amino acid sequence of SEQ ID NO: 3. In some aspects, the scFv comprises a VH and a VL, wherein the VH comprises the amino acid sequence of SEQ ID NO: 5, and/or wherein the VL comprises the amino acid sequence of SEQ ID NO: 3.

In some aspects, the scFv comprises a linker. In some aspects, the linker is a peptide linker. In some aspects, the peptide linker is a Gly-Ser linker. In some aspects, the Gly-Ser linker is selected from the group consisting of (Gly4Ser) (SEQ ID NO:58), (Gly4Ser)2 (SEQ ID NO:59), (Gly4Ser)3 (SEQ ID NO:60), and (Gly4Ser)4 (SEQ ID NO:61). In some aspects, the Gly-Ser linker comprises the amino acid sequence SGSSGGSSSG (SEQ ID NO:4).

In any of the foregoing or related aspects, the scFv has an amino acid sequence which is at least 80% identical, at least 85% identical, at least 90% identical, or at least 95% identical, to the amino acid sequence of SEQ ID NO: 2; optionally wherein the scFv comprises: (a) a VH comprising VH CDR1, VH CDR2 and VH CDR3, wherein the VH CDR1 has the amino acid sequence GFTFSSYA ( SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11); and/or (b) a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8). In some aspects, the scFv comprises the amino acid sequence of SEQ ID NO: 2.

In any of the foregoing or related aspects, the antigen is on the surface of a cancer cell. In some aspects, the cancer is Acute Myeloid Leukemia (AML).

In any of the foregoing or related aspects, the extracellular domain binds to the antigen with an equilibrium dissociation constant (Kd) of 100 nM or less, 50 nM or less, 20 nM or less, 10 nM or less, from 0.5 nM to 100 nM, or from 1 nM to 15 nM.

In some aspects, the engineered NK of the disclosure comprises a CAR comprising a transmembrane domain, wherein the transmembrane domain is selected from a transmembrane domain of CD3-zeta, CD8, CD28, DAP12, 2B4, NKG2D, CD16, NKp44 or NKp46. In some aspects, the engineered NK of the disclosure comprises a CAR comprising an intracellular domain, wherein the intracellular domain comprises one or more costimulatory domains of one or more costimulatory molecules selected from the group consisting of: CD27, CD28, 4-1BB, OX40, CD30, CD40, PD-1, ICOS, 2B4, DAP10, CD137 and DAP12. In some aspects, the intracellular domain comprises a CD3-zeta signaling domain and a 4-1BB costimulatory domain; wherein the transmembrane domain comprises a CD8 transmembrane domain, and wherein the CAR polypeptide further comprises a CD8 hinge region. In some aspects, the intracellular domain comprises a CD3-zeta signaling domain comprising the amino acid sequence set forth in SEQ ID NO: 27, and a 4-1BB costimulatory domain comprising the amino acid sequence set forth in SEQ ID NO: 26; wherein the CAR polypeptide comprises a CD8 transmembrane domain and a CD8 hinge region, wherein the CD8 transmembrane domain and the CD8 hinge region comprise the amino acid sequence set forth in SEQ ID NO: 25; and wherein the extracellular binding domain comprises the antibody, or antigen binding fragment thereof, and a leading sequence comprising the amino acid sequence set forth in SEQ ID NO: 23.

In any of the foregoing or related aspects, the extracellular binding domain is an scFv comprising the amino acid sequence set forth in SEQ ID NO: 24, or an amino acid sequence which is at least 70% identical, at least 75% identical, at least 80% identical, at least 85% identical, at least 90% identical, or at least 95% identical, to the amino acid sequence of SEQ ID NO: 24.

In any of the foregoing or related aspects, the intracellular domain further comprises a self-cleaving peptide sequence and a cytokine, wherein cleavage of the self-cleaving peptide releases the cytokine. In some aspects, the cytokine is IL-12, IL-7, IL-13, IL-15, TNF-α, IFN-γ, or CCL19.

In any of the foregoing or related aspects, the engineered NK cell of the disclosure comprises a CAR polypeptide comprising the amino acid sequence set forth in SEQ ID NO: 22, or an amino acid sequence which is at least 70% identical, at least 75% identical, at least 80% identical, at least 85% identical, at least 90% identical, or at least 95% identical, to the amino acid sequence of SEQ ID NO:22.

In any of the foregoing or related aspects, the engineered NK cell or population of cells of the disclosure is activated following exposure of a primary human NK cell or population of primary human NK cells to one or more or any combination of the following cytokines: IL-2, IL-12, IL-7, IL-15, IL-21, and IL-18, optionally wherein the cytokine is a recombinant human cytokine. In some aspects, the engineered NK cell or population of cells is activated following exposure to a combination of IL-12 and IL-15; IL-12 and IL-18; IL-15 and IL-18; or IL-12, IL-15 and IL-18. In some aspects, the NK cell or population of said cells is exposed to: IL-12 in a concentration range from 1-20 ng/mL; IL-15 in a concentration range from 1-50 ng/mL and IL-18 in a concentration range from 10-100 ng/mL, optionally wherein the NK cell or population of said cells is exposed to about 10 ng/mL IL-12, about 1 ng/mL IL-15, and about 50 ng/mL IL-18, for a period of about 3-48 hours, about 7-24 hours, about 16-20 hours, about 12-24 hours or about 14-16 hours, optionally for a period of about 16 hours.

In any of the foregoing or related aspects, the CAR polypeptide is introduced following exposure of the primary human NK cell or population of said cells to the one or more cytokines.

In any of the foregoing or related aspects, the engineered NK cell or population of cells of the disclosure:

(i) produces increased IFNγ in the presence of one or more cytokines and/or tumor targets relative to a control human NK cell or human NK cell line;
(ii) has enhanced antibody-dependent cellular cytotoxicity relative to a control human NK cell or human NK cell line; and/or
(iii) has enhanced anti-tumor efficacy relative to a control human NK cell or human NK cell line,

optionally wherein the control NK cell is a human NK cell activated in the presence of IL-15 alone, or a human NK cell line activated in the presence of IL-15 alone.

In any of the foregoing or related aspects, expression of one or more of the following polypeptides is increased in the engineered NK cell or population of said cells of the disclosure relative to a control human NK cell or human NK cell line: CD94/NKG2A, NKp30, NKp44, NKG2D, and CD25, optionally wherein the control human NK cell is a human NK cell activated in the presence of IL-15 alone, or the control human NK cell line is a human NK cell line activated in the presence of IL-15 alone.

In any of the foregoing or related aspects, one or more of the following polypeptides is relatively unchanged in the engineered NK cell or population of said cells of the disclosure relative to a control human NK cell or human NK cell line: KIR, CD57, NKG2C, DNAM-1 and CD137, optionally wherein the control human NK cell is a human NK cell activated in the presence of IL-15 alone, or the control human NK cell line is a human NK cell line activated in the presence of IL-15 alone.

In any of the foregoing or related aspects, expression of CD16 and/or CD11b is decreased in the engineered NK cell or population of said cells of the disclosure relative to a control human NK cell or human NK cell line, optionally wherein the control human NK cell is a human NK cell activated in the presence of IL-15 alone, or the control human NK cell line is a human NK cell line activated in the presence of IL-15 alone.

In any of the foregoing or related aspects, the engineered NK cell or population of said cells of the disclosure is CD25+NKG2A+NKp30+NKp44+.

In any of the foregoing or related aspects, the engineered NK cell or population said cells of the disclosure expresses an IL-15 polypeptide, optionally a human IL-15 polypeptide. In some aspects, the IL-15 polypeptide is a secreted IL-15 polypeptide or a membrane bound IL-15 polypeptide. In some aspects, the membrane bound IL-15 polypeptide is a fusion of IL-15 to a heterologous transmembrane domain.

In any of the foregoing or related aspects, the engineered NK cell or population of cells of the disclosure expands 1.5-fold, 2-fold, 3-fold, or 4-fold following *in vivo* administration.

In any of the foregoing or related aspects, the engineered NK cell or population of cells of the disclosure exhibits an enhanced response to cytokine or activating receptor re-stimulation following *in vivo* administration. In some aspects, the enhanced response is maintained for weeks to months, optionally for a period of 2 weeks to 3 months, a period of 3 weeks to 2 months, or about one month.

In any of the foregoing or related aspects, the engineered NK cell or population of said cells when contacted with a target cell presenting the NPM1c neoepitope in complex with a MHC class I protein (i) has increased expression of IFN-gamma relative to a control population of NK cells; (ii) has increased expression of granzyme B relative to a control population of NK cells; (iii) has increased expression of one or more activation markers relative to a control population of NK cells, wherein the one or more activation markers are selected from: CD25, CD69, ICOS, CD226, CD107a, and CD62L; (iv) has increased expression of one or more activating receptors relative to a control population of NK cells, wherein the one or more activating receptors are selected from: NKp30, NKG2D, NKp44; (v) has increased expression of one or more maturation markers relative to a control population of NK cells, wherein the one or more maturation markers are selected from: CD56 and NKG2A; (vi) has decreased expression of CD57 relative to a control population of NK cells; (vii) has increased expression of TIGIT relative to a control population of NK cells; and/or (viii) has decreased expression of TRAIL relative to a control population of NK cells; optionally wherein the control population of NK cells of any one of (i)-(viii) is an untransduced population of cytokine-induced ML NK cells. In some aspects, the engineered NK cell or population of said cells when contacted with a target cell presenting the NPM1c neoepitope in complex with a MHC class I protein has increased expression of IFN-gamma. In some aspects, the engineered NK cell or population of said cells when contacted with a target cell presenting the NPM1c neoepitope in complex with a MHC class I protein has increased expression of one or more activation markers. In some aspects, the activation marker is CD107a. In some aspects, the activation marker is CD62L. In some aspects, the engineered NK cell or population of said cells when contacted with a target cell presenting the NPM1c neoepitope in complex with a MHC class I protein has increased expression of one or more activating receptors. In some aspects, the activating receptor is NKG2D. In some aspects, the engineered NK cell or population of said cells when contacted with a target cell presenting the NPM1c neoepitope in complex with a MHC class I protein has decreased expression of CD57. In some aspects, the engineered NK cell or population of said cells when contacted with a target cell presenting the NPM1c neoepitope in complex with a MHC class I protein has increased expression of CD56 and NKG2A. In some aspects, the engineered NK cell or population of said cells when contacted with a target cell presenting the NPM1c neoepitope in complex with a MHC class I protein has increased expression of TIGIT. In some aspects, the engineered NK cell or population of said cells when contacted with a target cell presenting the NPM1c neoepitope in complex with a MHC class I protein has decreased expression of TRAIL.

In some aspects, the disclosure provides a pharmaceutical composition comprising an engineered NK cell or population of cells described herein and a pharmaceutically acceptable carrier.

In some aspects, the disclosure provides, a method for producing the engineered NK cell or population of cells of the disclosure, wherein the method comprises:

(i) obtaining a primary human NK cell or population of said cells;
(ii) contacting the NK cell or population of said cells with an amount of IL-12, IL-15, IL-18, or any combination thereof, for a period of time sufficient to obtain a cytokine-induced memory-like NK cell or population of said cells;
(iii) contacting the NK cell or population of said cells of step (ii) with a lentiviral vector encoding the CAR polypeptide under conditions to transduce the NK cell or population of said cells;
(iv) isolating the NK cell expressing the CAR polypeptide or population of said cells; and
(v) optionally, expanding the isolated cell.

In some aspects, the primary human NK cell or population of said cells is derived from an iPSC, cord blood, or PBMCs. In some aspects, the primary human NK cell or population of said cells is autologous or allogeneic.

In some aspects, the period of time in step (ii) is about 12-16 hours, optionally about 14-16 hours, optionally about 16 hours. In some aspects, step (iii) further comprises resting the NK cell or population of said cells for a period of about 24-72 hours prior to (iv).

In some aspects, the lentiviral vector is a baboon envelope glycoprotein (BaEV-gp) pseudotyped lentivirus. In some aspects the lentiviral vector is a pseudotyped lentiviral vector comprising a BaEV-gp, e.g., wherein the BaEV-gp comprises the amino acid sequence of SEQ ID NO: 107.

In any of the foregoing or related aspects, the cytokine-induced memory-like NK cell or population of said cells produces increased levels of IFNγ relative to a control human NK cell or NK cell line, optionally wherein the control human NK cell is a human NK cell activated in the presence of IL-15 alone, or the control human NK cell line is a human NK cell line activated in the presence of IL-15 alone.

In some aspects, the disclosure provides a method of producing a population of engineered cytokine-induced memory-like (ML) NK cells expressing a heterologous polypeptide, the method comprising: contacting a population of cytokine-induced ML NK cells expressing ASCT-2 with a pseudotyped lentiviral vector encoding the heterologous polypeptide under conditions to transduce the population, wherein the pseudotyped lentiviral vector comprises a glycoprotein that binds to ASCT-2, thereby producing the population of engineered cytokine-induced ML NK cells expressing the heterologous polypeptide. In some aspects, the cytokine-induced ML NK cells are obtained from a population of primary human NK cells. In some aspects, the population of primary human NK cells is derived from iPSCs, cord blood, or PBMCs. In some aspects, the population of primary human NK cells is autologous or allogeneic. In some aspects, expression of ASCT-2 is increased in the population of cytokine-induced ML NK cells relative to a control population of human NK cells by about 1.3-fold, 1.4-fold, 1.5-fold, 1.6-fold, 1.7-fold, 1.8-fold, 2-fold, 2.5-fold, 3-fold, 3.5-fold, or 4-fold, optionally wherein the control population of human NK cells is activated in the presence of IL-15 alone. In some aspects, the population of cytokine-induced ML NK cells is pre-activated by exposure to IL-12, IL-18, and IL-15. In some aspects, the population of cytokine-induced ML NK cells is pre-activated by exposure to IL-12 and IL-18. In some aspects, the population of cytokine-induced ML NK cells is pre-activated for a period of time of about 8-24 hours, optionally 12-20 hours, optionally about 12-16 hours, optionally about 14-16 hours, optionally about 16 hours. In some aspects, the population of cytokine-induced ML NK cells is pre-activated by exposure to about 10 ng/mL IL-12, about 50 ng/mL IL-15, and about 50 ng/mL IL-18 for a period of time of about 3-48 hours, about 7-24 hours, about 16-20 hours, about 12-24 hours, about 14-16 hours, or about 16 hours. In some aspects, the population of cytokine-induced ML NK cells is pre-activated by exposure to about 10 ng/mL IL-12 and about 50 ng/mL IL-18 for a period of time of about 3-48 hours, about 7-24 hours, about 16-20 hours, about 12-24 hours, about 14-16 hours, or about 16 hours. In some aspects, the population of cytokine-induced ML NK cells is rested for a period of time following the exposure to one or more cytokines and prior to the contacting, wherein the period of time is about 24-72 hours. In some aspects, at least about 10% of the population of cytokine-induced ML NK cells is transduced. In some aspects, about 10-90% of the population of cytokine-induced ML NK cells is transduced, optionally about 35-80%, optionally about 40-60%, optionally about 60%. In some aspects, the glycoprotein is a retroviral glycoprotein. In some aspects, the retroviral glycoprotein is a baboon envelope (BaEV) glycoprotein. In some aspects, the BaEV glycoprotein comprises the amino acid sequence set forth by SEQ ID NO: 107.

In some aspects, the disclosure provides a method of producing a population of engineered cytokine-induced memory-like (ML) NK cells expressing a heterologous polypeptide, the method comprising: (i) obtaining a population of primary human NK cells; (ii) contacting the population of primary human NK cells with IL-12, IL-18, and IL-15 for a period of time sufficient to obtain a population of cytokine-induced ML NK cells expressing ASCT-2; and (iii) contacting the population of (ii) with a pseudotyped lentiviral vector encoding the heterologous polypeptide under conditions to transduce the population of cytokine-induced ML NK cells; wherein the pseudotyped lentiviral vector comprises a glycoprotein that binds to ASCT-2; thereby producing a population of engineered cytokine-induced ML NK cells expressing the heterologous polypeptide.

In some aspects, the disclosure provides a method of producing a population of engineered cytokine-induced memory-like (ML) NK cells expressing a heterologous polypeptide, the method comprising: (i) obtaining a population of primary human NK cells; (ii) contacting the population of primary human NK cells with IL-12 and IL-18 for a period of time sufficient to obtain a population of cytokine-induced ML NK cells expressing ASCT-2; and (iii) contacting the population of (ii) with a pseudotyped lentiviral vector encoding the heterologous polypeptide under conditions to transduce the population of cytokine-induced ML NK cells; wherein the pseudotyped lentiviral vector comprises a glycoprotein that binds to ASCT-2; thereby producing a population of engineered cytokine-induced ML NK cells expressing the heterologous polypeptide.

In any of the foregoing or related aspects, the glycoprotein is a retroviral glycoprotein. In some aspects, the retroviral glycoprotein is a baboon envelope (BaEV) glycoprotein. In some aspects, the BaEV glycoprotein comprises the amino acid sequence set forth by SEQ ID NO: 107. In some aspects, the population of primary human NK cells is derived from iPSCs, cord blood, or PBMCs. In some aspects, the population of primary human NK cells is autologous or allogeneic. In some aspects, the period of time in step (ii) is about 12-16 hours, optionally about 14-16 hours, optionally about 16 hours. In some aspects, the proportion of the population of cytokine-induced memory-like NK cells that is transduced as a result of (iii) is at least 10%. In some aspects, about 10-90% of the population of cytokine-induced memory-like NK cells is transduced, optionally about 35-80%, optionally about 40-60%, optionally about 60%.

In any of the foregoing or related aspects, the population of cytokine-induced ML NK cells (i) produces increased IFN$\gamma$ in the presence of one or more cytokines and/or tumor targets relative to a control human NK cell line; (ii) has enhanced antibody-dependent cellular cytotoxicity relative to a control human NK cell line; and/or (iii) has enhanced anti-tumor efficacy relative to a control human NK cell line, optionally wherein the control human NK cell line is a human NK cell line activated in the presence of IL-15 alone. In some aspects, expression of one or more of the following polypeptides is increased in the population of cytokine-induced NK cells relative to a control human NK cell line: CD94/NKG2A, NKp30, NKp44, NKG2D, and CD25. In some aspects, CD94 is increased in the population of cytokine-induced NK cells relative to a control human NK cell line. In some aspects, NKp30 is increased in the population of cytokine-induced NK cells relative to a control human NK cell line. In some aspects, NKp44 is increased in the population of cytokine-induced NK cells relative to a control human NK cell line. In some aspects NKG2D is increased in the population of cytokine-induced NK cells relative to a control human NK cell line. In some aspects, CD25 is increased in the population of cytokine-induced NK cells relative to a control human NK cell line. In some aspects, one or more of the following polypeptides is relatively unchanged in the population of cytokine-induced NK cells relative to a control human NK cell line: KIR, CD57, NKG2C, DNAM-1 and CD137. In some aspects, KIR is relatively unchanged in the population of cytokine-induced NK cells relative to a control human NK cell line. In some aspects, CD57 is relatively unchanged in the population of cytokine-induced NK cells relative to a control human NK cell line. In some aspects, NKG2C is relatively unchanged in the population of cytokine-induced NK cells relative to a control human NK cell line. In some aspects, DNAM-1 is relatively unchanged in the population of cytokine-induced NK cells relative to a control human NK cell line. In some aspects, CD137 is relatively unchanged in the population of cytokine-induced NK cells relative to a control human NK cell line. In some aspects, expression of CD16 and/or CD11b is decreased in the population of cytokine-induced ML NK cells relative to a control human NK cell line. In some aspects, a plurality of the population of cytokine-induced ML NK cells are CD25+NKG2A+NKp30+NKp44+. In some aspects, the population of cytokine-induced ML NK cells expands 1.5-fold, 2-fold, 3-fold, or 4-fold following *in vivo* administration. In some aspects, the population of cytokine-induced ML NK cells exhibits an enhanced response to cytokine or activating receptor re-stimulation following *in vivo* administration. In some aspects, the enhanced response is maintained for weeks to months, optionally for a period of 2 weeks to 3 months, a period of 3 weeks to 2 months, or about one month.

In any of the foregoing or related aspects, the pseudotyped lentiviral vector further encodes an IL-15 polypeptide, optionally a human IL-15 polypeptide. In some aspects, the IL-15 polypeptide is a secreted IL-15 polypeptide or a membrane bound IL-15 polypeptide. In some aspects, the membrane bound IL-15 polypeptide is fused to a heterologous transmembrane domain, optionally a CD8 transmembrane domain. In some aspects, expansion of the population of cytokine-induced ML NK cells is increased by about 1.5-fold, 2-fold, 3-fold, or 4-fold following the transducing relative to a control ML NK population transduced without the IL-15 polypeptide.

In any of the foregoing or related aspects, the method further comprises isolating the population of cytokine-induced ML NK cells; and optionally, expanding the population of cells.

In any of the foregoing or related aspects, the heterologous polypeptide is a CAR. In some aspects, the CAR comprises an intracellular domain, a transmembrane domain and an extracellular binding domain, wherein the extracellular binding domain specifically binds to an antigen comprising an NPM1c neoepitope in complex with a class I major histocompatibility complex (MHC class I) protein. In some aspects, the extracellular binding domain does not bind to, or substantially does not bind to: (a) the MHC class I protein alone, and/or (b) a control peptide in complex with the MHC class I protein, optionally wherein the control peptide is an NY-ESO-1 epitope or influenza virus M1 epitope. In some aspects, the NPM1c neoepitope comprises an amino acid sequence $X_1X_2X_3X_4X_5X_6X_7X_8X_9$, wherein $X_1$ is selected from A, V, L or I, wherein $X_2$ is selected from A, T, S, V, L, I, M or Q, wherein $X_3$ is selected from Q or N, wherein $X_4$ is selected from D or E, wherein $X_5$ is selected from L, I, V, M, A or F, wherein $X_6$ is selected from C, S, or A, wherein $X_7$ is selected from L, I, V, M, A, or F, wherein $X_8$ is selected from A, V, L or I, and wherein $X_9$ is selected from L, I, V, M or A. In some aspects, $X_1$ is selected from A or V, $X_2$ is selected from V, I, or L, $X_3$ is selected from Q or N, $X_4$ is selected from D or E, $X_5$ is selected from L or I, $X_6$ is selected from C or S, $X_7$ is selected from V, L or I, $X_8$ is selected from A or V, and $X_9$ is selected from V, I, or L. In some aspects, $X_1$ is A, $X_2$ is selected from V, I, or L, $X_3$ is Q, $X_4$ is D, $X_8$ is L, $X_6$ is C, $X_7$ is L, $X_8$ is A, and $X_9$ is selected from V, I, or L. In some aspects, the NPM1c neoepitope comprises an amino acid sequence selected from: AIQDLCLAV (SEQ ID NO:1) or AIQDLCVAV (SEQ ID NO: 71). In some aspects, the neoepitope comprises the amino acid sequence AIQDLCLAV (SEQ ID NO:1). In some aspects, the neoepitope is 7, 8, 9, 10, 11, or 12 amino acid residues in length. In some aspects, the MHC class I protein is an HLA-A*02 protein or is encoded by the HLA-A*02 allele group. In some aspects, the MHC class I protein is encoded by the HLA-A*02:01 allele.

In some aspects, the NPM1c neoepitope comprises an amino acid sequence selected from: CLAVEEVSL (SEQ ID NO:72), VEEVSLRK (SEQ ID NO:73), AVEEVSLR (SEQ ID NO:74), AVEEVSLRK (SEQ ID NO:75), CLAVEEVSLRK (SEQ ID NO:76). In some aspects, the neoepitope is 7, 8, 9, 10, 11, or 12 amino acid residues in length. In some aspects, the MHC class I protein is an HLA-A*02 protein or is encoded by the HLA-A*02 allele group. In some aspects, the MHC class I protein is encoded by the HLA-A*02:01 allele.

In any of the foregoing or related aspects, the extracellular domain of the CAR comprises: (i) a heavy chain variable region (VH) comprising VH complementarity determining region (CDR)1, VH CDR2 and VH CDR3, said VH CDR1, VH CDR2 and VH CDR3 being the CDRs of a VH that has an amino acid sequence of SEQ ID NO: 5, and/or (ii) a light chain variable region (VL) comprising VL complementarity determining region (CDR)1, VL CDR2 and VL CDR3, said VL CDR1, VL CDR2 and VL CDR3 being the CDRs of a VL that has an amino acid sequence of SEQ ID NO: 3. In some aspects, the extracellular domain comprises a VH comprising VH CDR1, VH CDR2 and VH CDR3, wherein the VH CDR1 has the amino acid sequence GFTFSSYA (SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11). In some aspects, the extracellular domain comprises a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8). In some aspects, the extracellular domain comprises a VH and a VL, wherein the VH comprises an amino acid sequence which is at least 90% identical, or at least 95% identical, to the amino acid sequence of SEQ ID NO: 5, and/or wherein the VL comprises an amino acid sequence which is at least 90% identical, or at least 95% identical, to the amino acid sequence of SEQ ID NO: 3. In some aspects, the extracellular domain comprises a VH and a VL, wherein the VH comprises the amino acid sequence of SEQ ID NO: 5, and/or wherein the VL comprises the amino acid sequence of SEQ ID NO: 3.

In any of the foregoing or related aspects, the extracellular domain of the CAR comprises an scFv. In some aspects, the scFv is a human scFv. In some aspects, the scFv comprises a VH comprising VH CDR1, VH CDR2 and VH CDR3, wherein the VH CDR1 has the amino acid sequence GFTFSSYA (SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11). In some aspects, the scFv comprises a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8). In some aspects the scFv comprises a VH and a VL, wherein the VH comprises an amino acid sequence which is at least 90% identical, or at least 95% identical, to the amino acid sequence of SEQ ID NO: 5, and/or wherein the VL comprises an amino acid sequence which is at least 90% identical, or at least 95% identical, to the amino acid sequence of SEQ ID NO: 3. In some aspects, the scFv comprises a VH and a VL, wherein the VH comprises the amino acid sequence of SEQ ID NO: 5, and/or wherein the VL comprises the amino acid sequence of SEQ ID NO: 3. In some aspects, the scFv comprises a linker. In some aspects, the linker is a peptide linker. In some aspects, the peptide linker is a Gly-Ser linker. In some aspects, the Gly-Ser linker is selected from the group consisting of (Gly4Ser) (SEQ ID NO:58), (Gly4Ser)2 (SEQ ID NO:59), (Gly4Ser)3 (SEQ ID NO:60), and (Gly4Ser)4 (SEQ ID NO:61). In some aspects, the Gly-Ser linker comprises the amino acid sequence SGSSGGSSSG (SEQ ID NO:4).

In any of the foregoing or related aspects, the scFv has an amino acid sequence which is at least 80% identical, at least 85% identical, at least 90% identical, or at least 95% identical, to the amino acid sequence of SEQ ID NO: 2; optionally wherein the scFv comprises: (a) a VH comprising VH CDR1, VH CDR2 and VH CDR3, wherein the VH CDR1 has the amino acid sequence GFTFSSYA ( SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11); and/or (b) a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8). In some aspects, the scFv comprises the amino acid sequence of SEQ ID NO: 2.

In any of the foregoing or related aspects, the extracellular binding domain of the CAR specifically binds to an antigen comprising an NPM1c neoepitope in complex with a class I major histocompatibility complex (MHC class I) protein, wherein the antigen is on the surface of a cancer cell. In some aspects, the cancer is Acute Myeloid Leukemia (AML). In some aspects, the extracellular domain binds to the antigen with an equilibrium dissociation constant (Kd) of 100 nM or less, 50 nM or less, 20 nM or less, 10 nM or less, from 0.5 nM to 100 nM, or from 1 nM to 15 nM.

In some aspects, the CAR comprises a transmembrane domain, wherein the transmembrane domain is selected from a transmembrane domain of CD3-zeta, CD8, CD28, DAP12, 2B4, NKG2D, CD16, NKp44 or NKp46. In some aspects, the CAR comprises an intracellular domain, wherein the intracellular domain comprises one or more costimulatory domains of one or more costimulatory molecules selected from the group consisting of: CD27, CD28, 4-1BB, OX40, CD30, CD40, PD-1, ICOS, 2B4, DAP10, CD137 and DAP12. In some aspects, the intracellular domain comprises a CD3-zeta signaling domain and a 4-1BB costimulatory domain; wherein the transmembrane domain comprises a CD8 transmembrane domain, and wherein the CAR polypeptide further comprises a CD8 hinge region. In some aspects, the intracellular domain comprises a CD3-zeta signaling domain comprising the amino acid sequence set forth in SEQ ID NO: 27, and a 4-1BB costimulatory domain comprising the amino acid sequence set forth in SEQ ID NO: 26; wherein the CAR polypeptide comprises a CD8 transmembrane domain and a CD8 hinge region, wherein the CD8 transmembrane domain and the CD8 hinge region comprise the amino acid sequence set forth in SEQ ID NO: 25; and wherein the extracellular binding domain comprises the antibody, or antigen binding fragment thereof, and a leading sequence comprising the amino acid sequence set forth in SEQ ID NO: 23.

In any of the foregoing or related aspects, the extracellular binding domain of the CAR is an scFv comprising the amino acid sequence set forth in SEQ ID NO: 24, or an amino acid sequence which is at least 70% identical, at least 75% identical, at least 80% identical, at least 85% identical, at least 90% identical, or at least 95% identical, to the amino acid sequence of SEQ ID NO: 24.

In any of the foregoing or related aspects, the CAR polypeptide comprises the amino acid sequence set forth in SEQ ID NO: 22, or an amino acid sequence which is at least 70% identical, at least 75% identical, at least 80% identical, at least 85% identical, at least 90% identical, or at least 95% identical, to the amino acid sequence of SEQ ID NO:22.

In any of the foregoing or related aspects, the intracellular domain of the CAR further comprises a self-cleaving peptide sequence and a cytokine, wherein cleavage of the self-cleaving peptide releases the cytokine. In some aspects, the cytokine is IL-12, IL-7, IL-13, IL-15, TNF-α, IFN-γ, or CCL19. In some aspects, the cytokine is an IL-15 polypeptide. In some aspects, the IL-15 polypeptide is secreted following expression. In some aspects, the CAR polypeptide comprises the amino acid sequence set forth in SEQ ID NO: 102, or an amino acid sequence which is at least 70% identical, at least 75% identical, at least 80% identical, at least 85% identical, at least 90% identical, or at least 95% identical, to the amino acid sequence of SEQ ID NO: 102. In some aspects, the IL-15 polypeptide is fused to a heterologous transmembrane domain, optionally wherein the heterologous transmembrane domain is a CD8 transmembrane domain. In some aspects, the IL-15 polypeptide is expressed as a membrane-bound IL-15 polypeptide. In some aspects, the CAR polypeptide comprises the amino acid sequence set forth in SEQ ID NO: 100, or an amino acid sequence which is at least 70% identical, at least 75% identical, at least 80% identical, at least 85% identical, at least 90% identical, or at least 95% identical, to the amino acid sequence of SEQ ID NO: 100.

In some aspects, the disclosure provides a population of engineered cytokine-induced ML NK cells expressing a CAR comprising an extracellular binding domain that specifically binds to an antigen comprising an NPM1c neoepitope in complex with a class I major histocompatibility complex (MHC class I) protein, the population prepared according to a method described herein. In some aspects, the population of engineered ML NK cells in the presence of a target cell presenting the NPM1c neoepitope in complex with a MHC class I protein, (i) has increased expression of IFNgamma; (ii) has increased expression of granzyme B relative to a control population of NK cells; (iii) has increased expression of one or more activation markers relative to a control population of NK cells, wherein the one or more activation markers are selected from: CD25, CD107a, CD69, ICOS, CD226, and CD62L; (iv) has increased expression of one or more activating receptors relative to a control population of NK cells, wherein the one or more activating receptors are selected from: NKp30, NKG2D, NKp44; (v) has increased expression of one or more maturation markers relative to a control population of NK cells, wherein the one or more maturation markers are selected from: CD56 and NKG2A; (vi) has decreased expression of CD57 relative to a control population of NK cells; (vii) has increased expression of TIGIT relative to a control population of NK cells; and/or (viii) has decreased expression of TRAIL relative to a control population of NK cells; optionally wherein the control population of NK cells is an untransduced population of cytokine-induced ML NK cells. In some aspects, the engineered NK cell or population of said cells when contacted with a target cell presenting the NPM1c neoepitope in complex with a MHC class I protein has increased expression of IFN-gamma. In some aspects, the engineered NK cell or population of said cells when contacted with a target cell presenting the NPM1c neoepitope in complex with a MHC class I protein has increased expression of one or more activation markers. In some aspects, the activation marker is CD107a. In some aspects, the activation marker is CD62L. In some aspects, the engineered NK cell or population of said cells when contacted with a target cell presenting the NPM1c neoepitope in complex with a MHC class I protein has increased expression of one or more activating receptors. In some aspects, the activating receptor is NKG2D. In some aspects, the engineered NK cell or population of said cells when contacted with a target cell presenting the NPM1c neoepitope in complex with a MHC class I protein has decreased expression of CD57. In some aspects, the engineered NK cell or population of said cells when contacted with a target cell presenting the NPM1c neoepitope in complex with a MHC class I protein has increased expression of CD56 and NKG2A. In some aspects, the engineered NK cell or population of said cells when contacted with a target cell presenting the NPM1c neoepitope in complex with a MHC class I protein has increased expression of TIGIT. In some aspects, the engineered NK cell or population of said cells when contacted with a target cell presenting the NPM1c neoepitope in complex with a MHC class I protein has decreased expression of TRAIL.

In some aspects, the disclosure provides a method of treating a cancer in a subject in need thereof, wherein the cell surface of cells comprising the cancer displays an NPM1c neoepitope in complex with a MHC class I protein, the method comprising administering to the subject an engineered NK cell or population of cells, or a pharmaceutical composition, described herein, in an amount sufficient to treat the cancer. In some aspects, the cancer is AML. In some aspects, the method of treating cancer is a method of reducing cancer burden or a method of increasing survival in the subject.

In other aspects, the disclosure provides a method of treating AML in a subject in need thereof, the method comprising administering to the subject an engineered NK cell or population of cells, or a pharmaceutical composition, as described herein, in an amount sufficient to treat AML.

In any of the foregoing or related aspects, the AML is a relapsed AML or a refractory AML.

In other aspects, the disclosure provides a method of preventing relapse of AML in a subject in remission from AML, the method comprising administering to an engineered NK cell or population of cells, or a pharmaceutical composition, as described herein.

In any of the foregoing or related aspects, any of the methods described herein comprise before the administering step, detecting whether the subject expresses NPM1c or whether the subject has an *NPM1c* mutation in the *NPM1* gene, and if the subject expresses NPM1c or has an *NPM1c* mutation proceeding with the administering step.

In any of the foregoing or related aspects, the engineered NK cell or population of cells of the disclosure exhibits enhanced expansion following *in vivo* administration.

In any of the foregoing or related aspects, any of the methods described herein further comprises administering one or more additional therapeutic agents or procedures.

In some aspects the disclosure provides use of an engineered NK cell or population of cells, or a pharmaceutical composition, as described herein, in the manufacture of a medicament for treating a cancer in a subject, wherein the cell surface of cells comprising the cancer displays an NPM1c neoepitope in complex with a MHC class I protein; optionally wherein the use is in combination with one or more additional therapeutic agents or procedures.

In any of the foregoing or related aspects, the subject is a human.

In other aspects, the disclosure provides a kit comprising one or more containers comprising: (i) an engineered NK cell or population of cells, or a pharmaceutical composition, described herein; (ii) optionally, one or more additional therapeutic agents, and (iii) instructions for use in treating cancer in a subject.

## BRIEF DESCRIPTION OF THE DRAWINGS

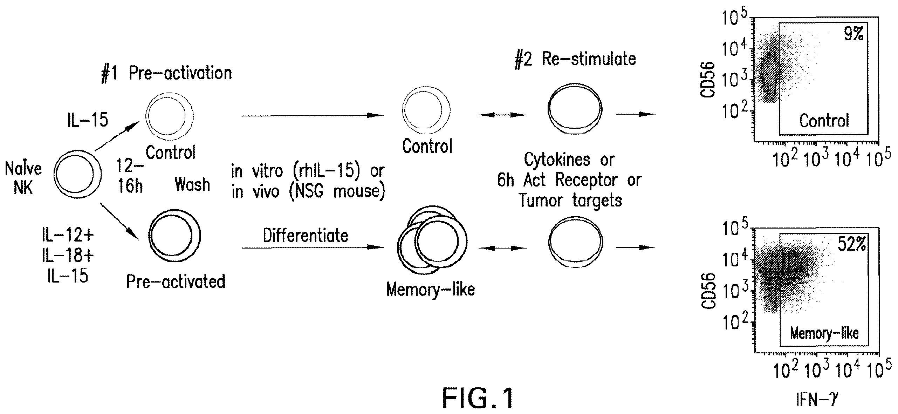

**FIG. 1** shows a summary of human cytokine-induced memory-like (interchangeably referred to herein as "memory-like", "CIML", "ML") NK cell differentiation. Conventional, naive NK cells are pre-activated for 12-16 hours with a combination of IL-12, IL-15, and IL-18 (or IL-15 alone as a control), washed and allowed to differentiate in vitro (with low dose IL-15 for survival) or in vivo (in NSG mice with rh IL-2 or rhIL-15 for survival). IL-12/15/18 pre-activation resulted in extensive proliferation, and differentiation into memory-like NK cells. Upon a second (re-) stimulation, memory-like NK cells exhibit enhanced responses, compared to control or naive NK cells. In this example, IFN-γ production is shown as a prototype NK cell function readout, in response to leukemia target cells.

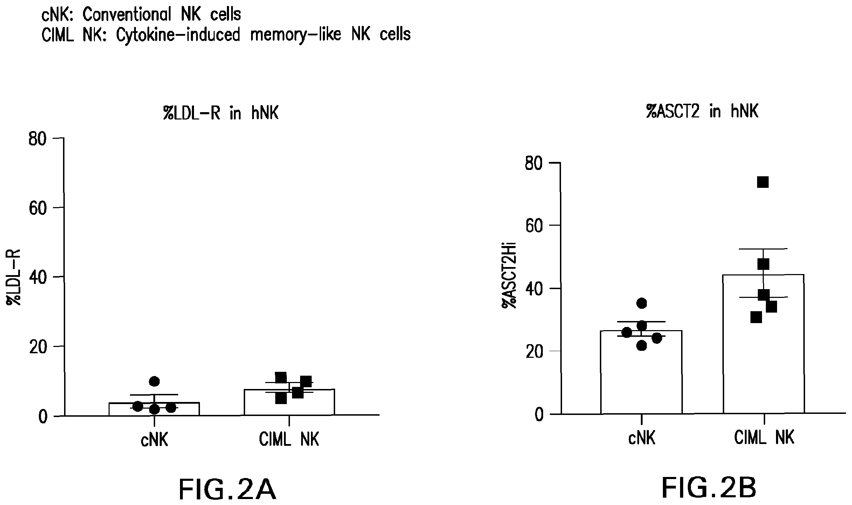

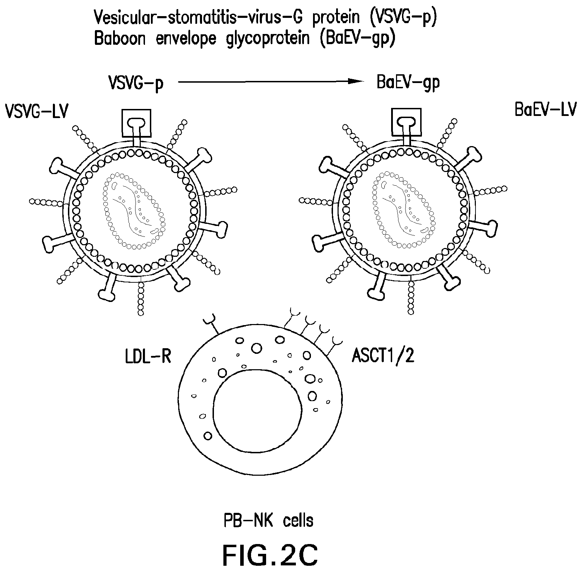

**FIGS. 2A-2C** shows expression of LSL-R and ASCT2 in primary conventional and CIML NK cells. **FIG. 2A** is a graph demonstrating the percent (%) LDL-R expression in human NK (hNK) cells that were conventional primary human NK cells (cNK) or cytokine-induced memory-like NK cells (CIML-NK). **FIG. 2B** is a graph demonstrating the percent (%) ASCT2 expression in cNK or CIML-NK cells. **FIG. 2C** is a schematic depicting relative expression on NK cells of ASCT1/2 targeted by lentivirus pseudotyped with baboon envelope glycoprotein (BaEV-LV) versus LDL-R targeted by lentivirus pseudotyped with vesicular-stomatitis-virus-G protein (VSVG-LV).

**FIG. 3A** shows a schematic of an anti-NPM1c CAR expressed on NK cells and its recognition of AIQ (AIQDLCLAV; SEQ ID NO: 1) presented by HLA-A2 complex (AIQ-HLA-A2 complex) on AML cells. Also shown is a schematic of the anti-NPM1c-CAR vector. **FIG. 3B** provides flow cytometry data showing anti-NPM1c CAR expressed on T cells recognizes the AIQ-HLA-A2 complex.

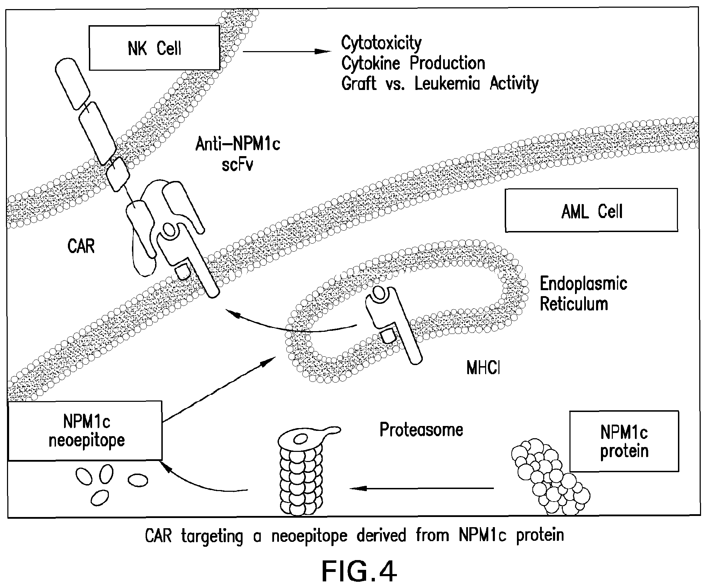

**FIG. 4** shows NPM1 is mutated in AML. Schematic adapted from Xie, et al. Nat Biomed Eng (2021) 5:124.

**FIG. 5** shows optimized CAR-editing of CIML NK cells using an unconventional lentiviral transduction approach (lentivirus pseudotyped with BaEV). Shown is a flow plot quantifying CAR scFv and GFP expression in anti-NPM1c NK cells and untransduced (UT) cells.

**FIG. 6A** is a schematic demonstrating the method used to generate cytokine induce memory-like NK cells and transduction with a CAR construct using lentivirus (e.g., pseudotyped with BaEV) to generate a CAR-expressing CIML NK cell.

**FIG. 6B** is a graph demonstrating percent (%) ASCT2 expression in NK cells stimulated with combinations of 50ng/mL IL-15, 10ng/mL IL-12, and 50ng/mL IL-18 for 16 hours.

**FIGs. 6C-6E** provide graphs quantifying fold change in expression of ASCT mRNA as quantified by qPCR in hNK cells stimulated with combinations of IL-12 and IL-18 **(FIGs. 6C-6D)** or IL-12, IL-18, and IL-15 **(FIG. 6E).** Control cells were untreated or treated with IL-15 alone, IL-12 alone, or IL-18 alone.

**FIG. 6F** is a histogram demonstrating transduction efficiency with the lentivirus pseudotyped with baboon envelope glycoprotein (BaEV-LV) encoding an anti-NPM1c CAR in ML NK cells pre-activated with different combinations of cytokines.

**FIG. 7A** shows graphs of transduction rates for ML-NK cells obtained from different human donors transfected with BaEV-LV encoding an anti-NPM1c CAR as determined by measuring CAR expression using flow cytometry (represented as % Protein L binding).

**FIG. 7B** shows efficient gene expression in primary human and mouse CIML NK cells transduced with BaEV-LV encoding GFP. Primary hNK cells from PBMCs were pre-activated overnight with recombinant human IL-12, IL-18, and IL-15 to generate the human CIML NK cells. Mouse NK cells from spleens were pre-activated overnight with recombinant mouse IL-12, IL-18, and IL-15 to generate the mouse CIML NK cells.

**FIG. 7C** provides phenotypic markers characteristic of hNK cells based on stage of maturation and development.

**FIG. 7D** provides a strategy for gating on live, CD56$^+$CD3$^-$ cells to distinguish hNK cell subsets based on stage of maturation from less mature (NK1 = CD56$^{bright}$CD16$^-$), intermediate maturity (NK2 = CD56$^{dim}$CD16$^+$KIRs$^-$ or NK3 = CD56$^{dim}$CD16$^+$KIRs$^+$CD57$^-$), or fully matured (NK4 = CD56$^{dim}$CD16$^+$KIRs$^+$CD57$^+$) by flow cytometry analysis.

**FIG. 7E** provides a bar graph quantifying surface expression of ASCT2 for hNK cell subsets NK1-NK4 distinguished according to the gating strategy depicted in **FIG. 7D.** hNK cells were obtained from four different human donors.

**FIG. 7F** provides histograms to quantify ASCT2 surface expression for hNK cell subsets NK1-NK4 distinguished according to the gating strategy depicted in **FIG. 7D.** Shown is data for hNK cells obtained from one human donor.

**FIG. 8A** shows potent anti-AML function of CAR ML-NK cells. Shown is a flow plot (left panel) and corresponding bar graph (right panel) quantifying IFN$\gamma$ and CD107a expression in untransduced (UT) cells and anti-NPM1c-CAR ML NK cells following co-culture with NPM1c$^+$HLA-A2$^+$ target cells.

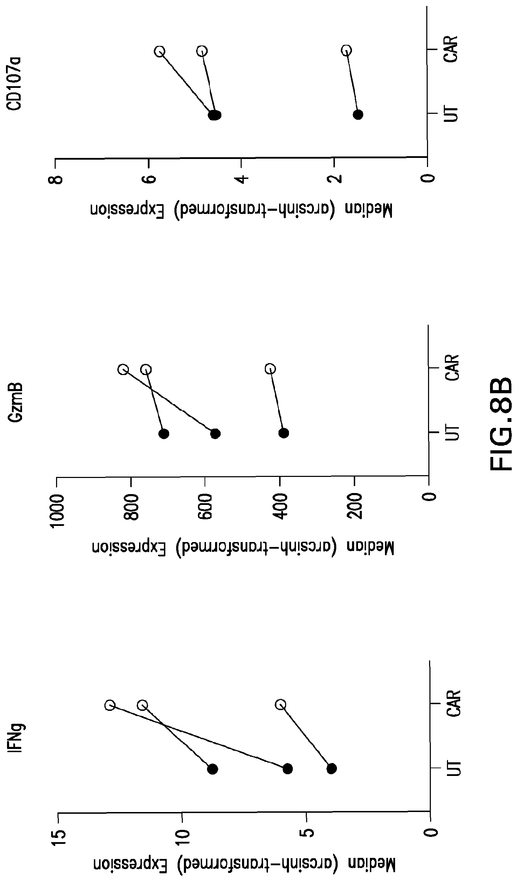

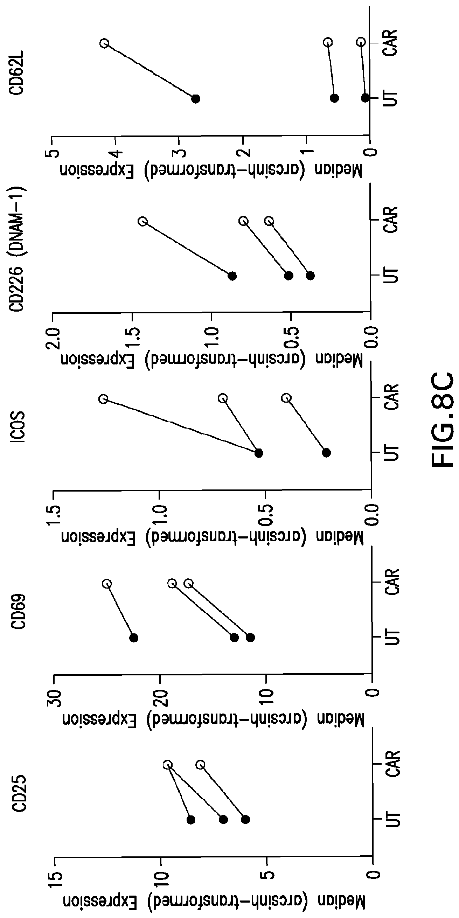

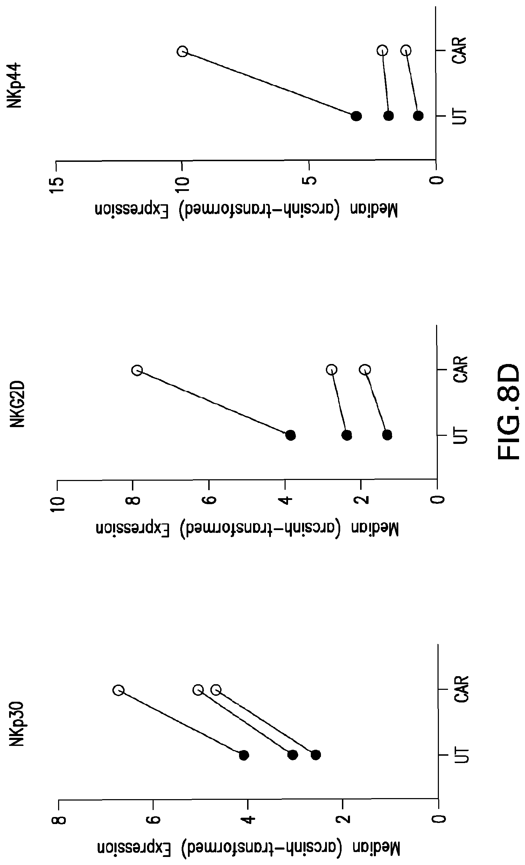

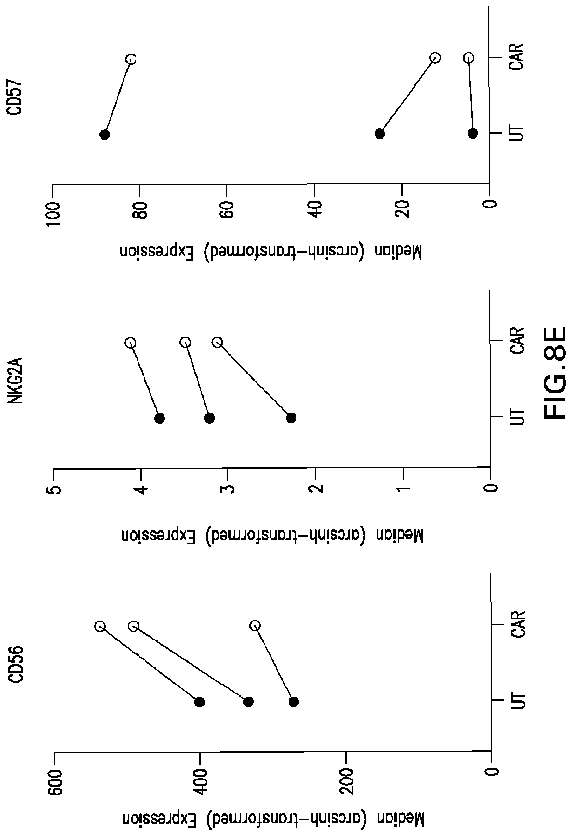

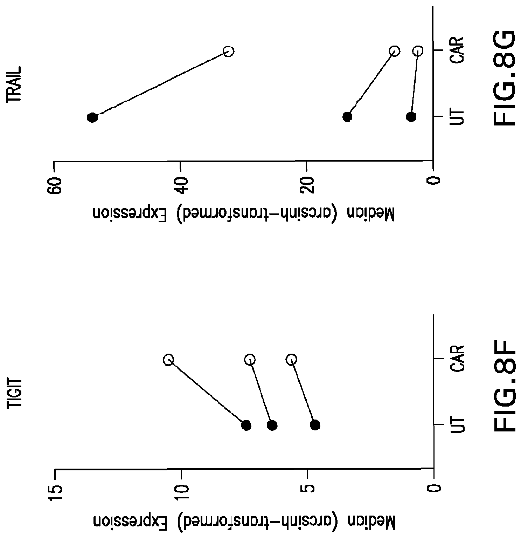

**FIGS. 8B-8G** show analysis of phenotypic markers on CIML-NK cells transduced with anti-NPM1c CAR using a BaEV-LV as compared to untransduced NK cells following co-culture with OCI-AML3 (NPM1c+HLA-A2+) cells. Quantification is based on mass cytometry data collected by CyTOF. **FIG. 8B** provides quantification of CD107a, cytokines (IFN$\gamma$), and cytotoxicity markers (granzyme B); **FIG. 8C** provides quantification of activation markers (CD25, CD69, ICOS, CD226, CD62L); **FIG. 8D** provides quantification of activating receptors (NKp30, NGD2D, NKp44); **FIG. 8E** provides quantification of maturation markers (CD56, NKG2A, CD57); **FIG. 8F** provides quantification of exhaustion marker TIGIT; and **FIG. 8G** provides quantification of apoptosis marker TRAIL.

**FIG. 9A** is a graph measuring cell expansion in *in vitro* culture of untransduced (UT) ML NK cells, CAR-s15 (anti-NPM1c CAR ML NK cells expressing secreted IL15), or CAR-m15 (anti-NPM1c CAR ML NK cells expressing membrane-bound IL15) over a 15 day period.

**FIGS. 9B-9C** provide a bar graph **(FIG. 9B)** and corresponding flow charts **(FIG. 9C)** demonstrating CAR expression was maintained following transduction. CAR expression was measured at day 4 and day 23 post transduction in untransduced (UT) cells, anti-NPM1c CAR only ML NK cells, CAR-s15 ML NK cells, and CAR-m15 ML NK cells. CAR expression is shown as % Protein L binding in viable NK cells.

**FIG. 10A** provides bar graphs demonstrating CAR-s15 ML NK cells and CAR-m15 ML NK cells express IFN$\gamma$ when co-cultured with NPM1c:HLA-A2-expressing target cells. The NK cells were co-cultured at an effector:target ratio of 1:1 using target cells that were either OCI-AML3 (NPM1c+, HLA-A2+, Luc+; top graph) or OCI-AML2 (NPM1c-, HLA-A2+, Luc+; bottom graph). Control cells were untransduced ML NK cells. Cells were co-cultured for 5 hours and analyzed for IFN$\gamma$ expression using flow cytometry.

**FIG. 10B** provides line graphs demonstrating CAR-s15 ML NK cells and CAR-m15 ML NK cells induce apoptosis of NPM1c:HLA-A2-expressing target cells, as measured by the percent (%) of apoptotic target cells. Target cells were either OCI-AML3 (NPM1c+, HLA-A2+, Luc+; top graph) or OCI-AML2 (NPM1c-, HLA-A2+, Luc+; bottom graph). Cells were co-cultured at the indicated E:T ratios for 4 hours with untransduced ML-NK cells, CAR-s15 ML-NK cells, or CAR m15 ML-NK cells, then analyzed for Annexin V staining using flow cytometry.

**FIG. 10C** provides line graphs demonstrating cell survival in target cells (OCI-AML3 (NPM1c+, HLA-A2+, Luc+; left graph) or OCI-AML2 (NPM1c-, HLA-A2+, Luc+; right graph) after 24 hour culture with untransduced ML-NK cells, anti-CAR NPM1c-only ML-NK cells, CAR-s15 ML-NK cells, or CAR-m15 ML-NK cells at different E:T ratios.

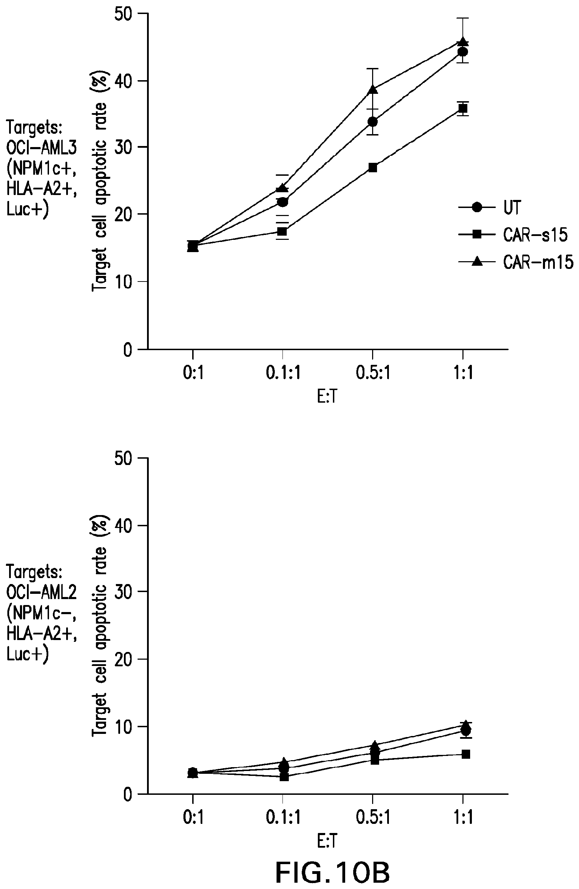

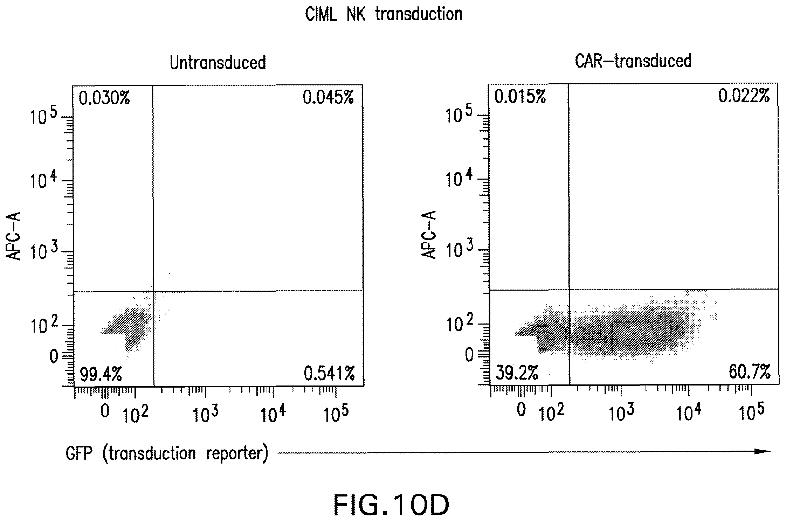

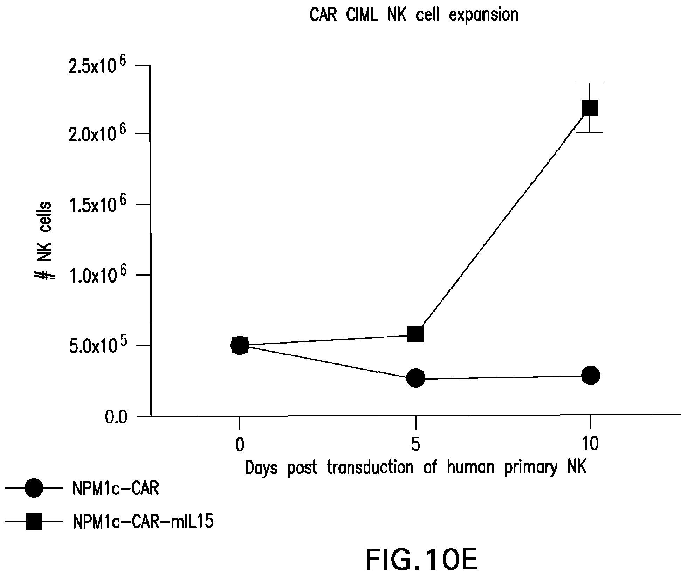

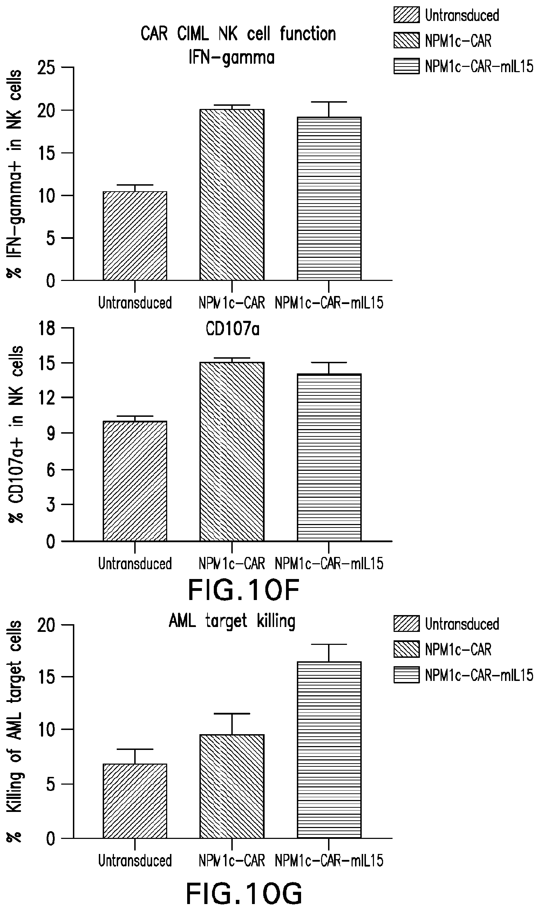

**FIGS. 10D-10G** show engineered memory-like NK CARs targeting a neoepitope derived from intracellular NPM1c exhibit potent activity and specificity against acute myeloid leukemia (AML). **FIG. 10D** shows efficient lentiviral-mediated (BaEV-LV) CAR editing in primary human CIML NK cells at 72 hour post transduction. **FIG. 10E** shows co-transduction with anti-NPM1c CAR and membrane-bound IL-15 facilitates CAR-CIML NK cell expansion *in vitro.* **FIG. 10F** shows IFN-gamma and CD107a expression in CIML NK cells transduced anti-NPM1c CAR alone or co-transduced with anti-NPM1c CAR and mIL-15 and stimulated by OCI-AML3 target cells (NPM1c+ HLA-2A+) for 4 hours before flow cytometric analysis. **FIG. 10G** shows killing of OCI-AML3 (NPM1c+ HLA-2A+) target cells that were co-cultured for 4 hours with CIML NK cells transduced with anti-NPM1c CAR alone or co-transduced with anti-NPM1c CAR and mIL-15.

**FIG. 11A** provides a timeline of generating anti-NPM1c-CAR-ML NK cells and generation of mice containing OCI-AML3-Luc cells and subsequent treatment with the CAR cells and monitoring of tumor burden.

**FIG. 11B** provides an image of luciferase expression in mice involved in the study outlined in **FIG. 11A.** Luciferase was measured at day 3, day 10, and day 13 in mice that received untransduced cells or anti-NPM1c CAR ML NK cells expressing secreted IL-15 (s15), membrane bound IL-15 (m15) or CAR only (no IL-15).

**FIG. 11C** provides a line graph demonstrating overall (Total) luciferase expression (as imaged in **FIG. 11B)** in mice bearing tumor OCI-AML3 cells.

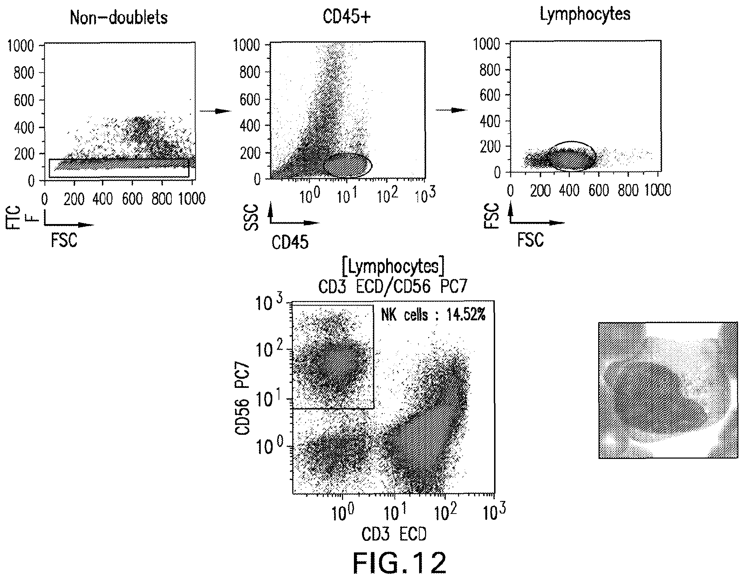

**FIG. 12** shows cell sorting of Natural Killer (NK) cells.

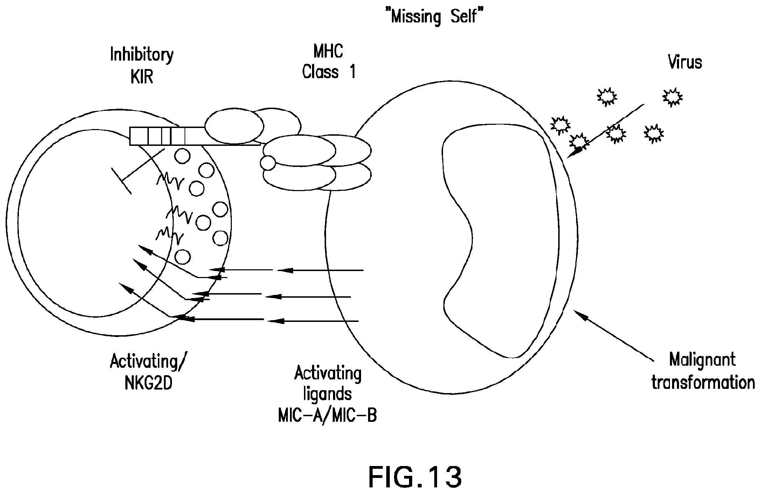

**FIG. 13** is a schematic showing NK cell responses are dictated by net balance of activating and inhibitory signals.

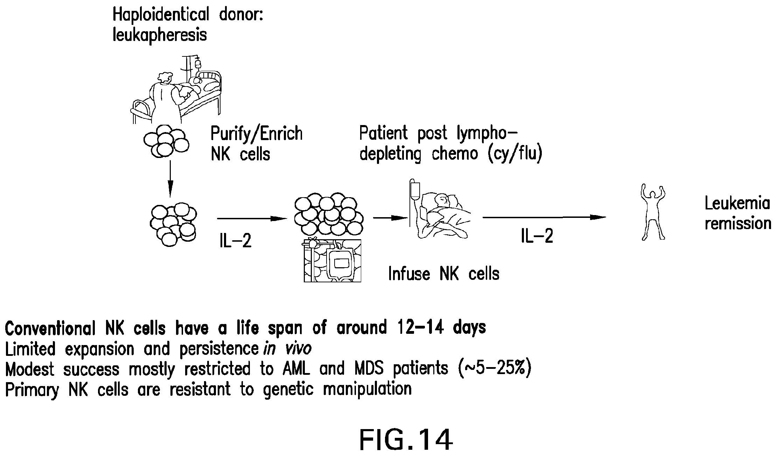

**FIG. 14** is a schematic showing strategies of adoptive transfer of NK cells.

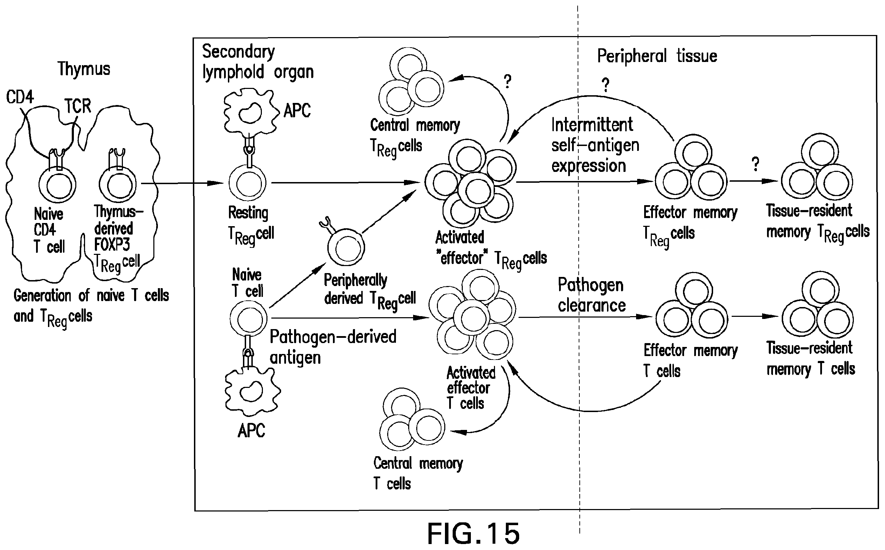

**FIG. 15** is a schematic showing memory in immune system. *Adapted from Rosenblum et al, 2016.*

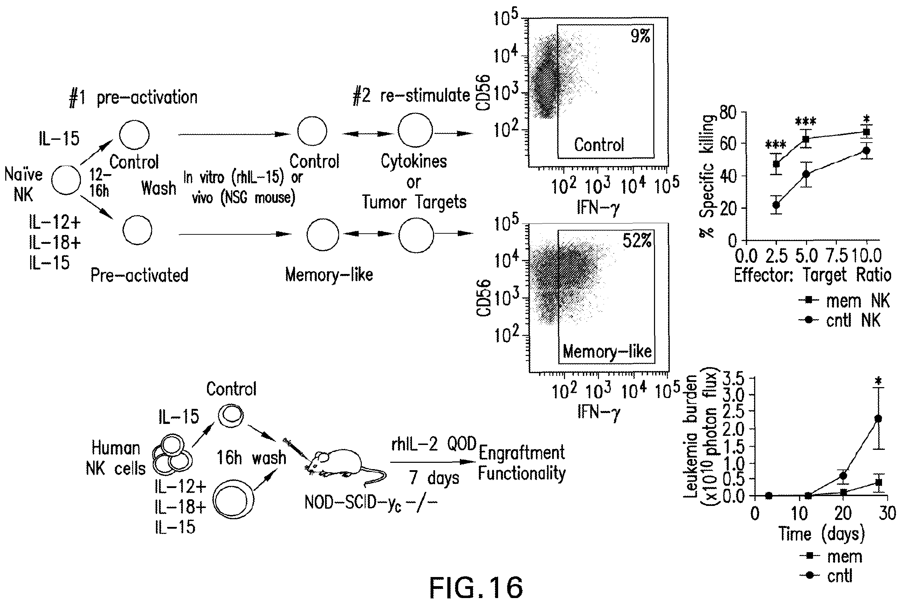

**FIG. 16** shows activation of convention NK cells with IL-12 plus IL-18 induces differentiation into Memory-like NK cells. *See,* Romee et al, Blood, 2012*;* Romee, et al, Science TM, 2016.

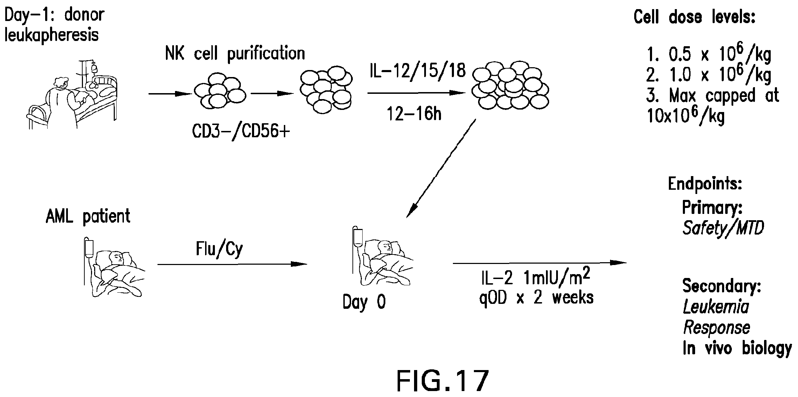

**FIG. 17** shows first-in-human Phase 1 study of memory-like NK cells in relapsed/refractory AML.

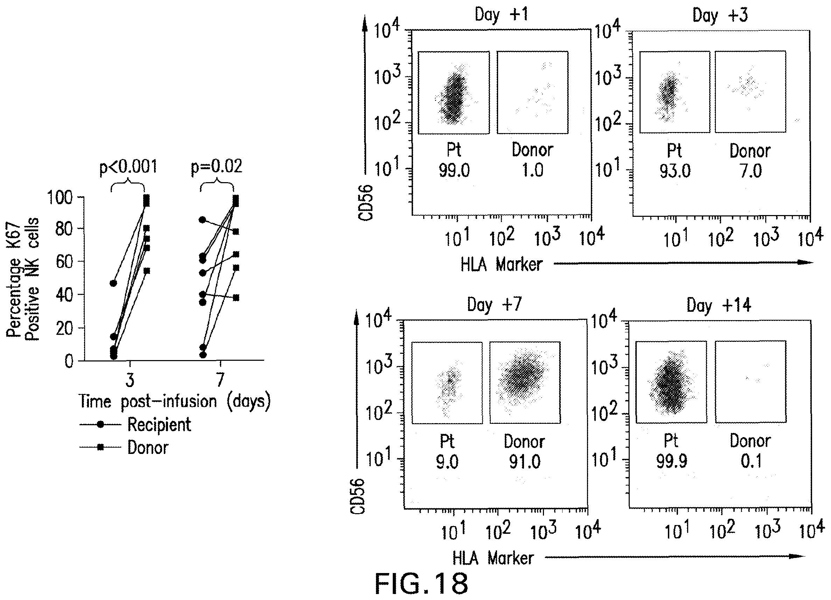

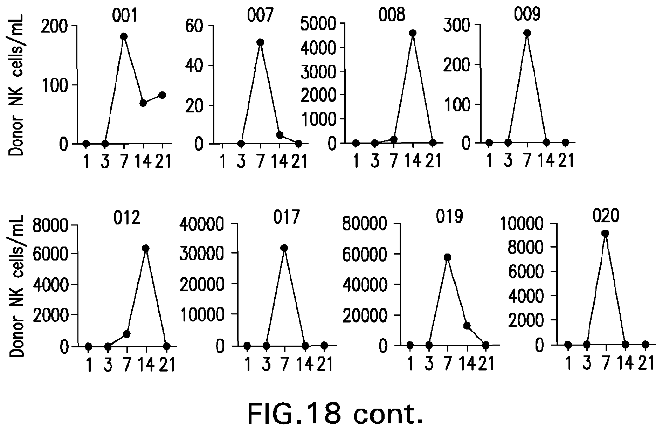

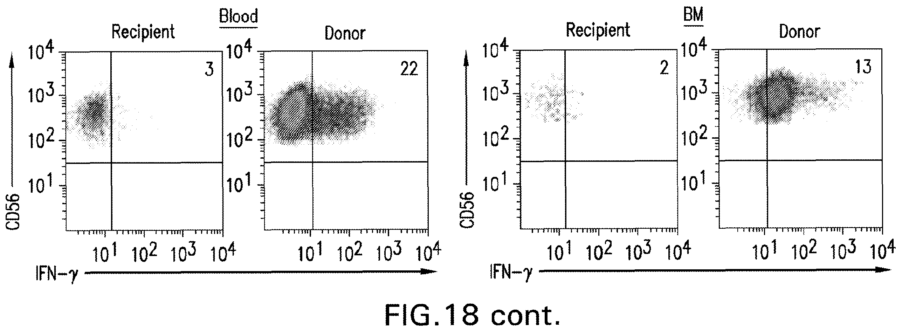

**FIG. 18** shows CIML NK cells proliferate and expand after adoptive transfer. *Adapted from* Romee, et al. Science TM, 2016.

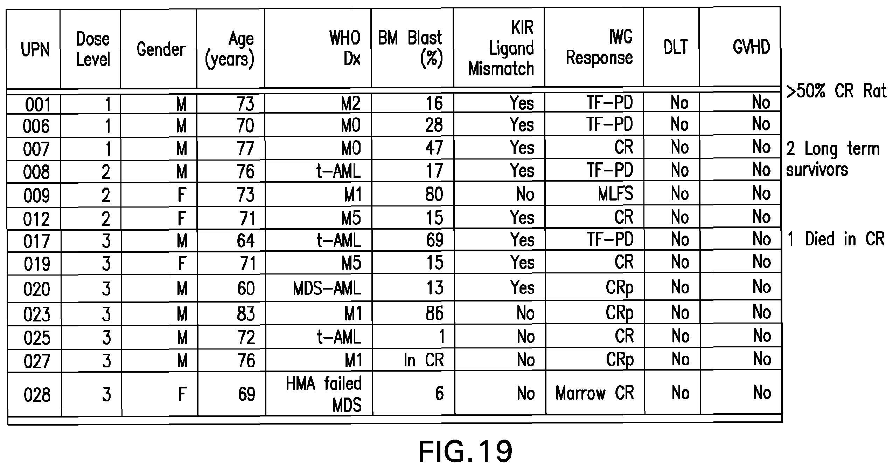

**FIG. 19** shows memory-like NK cells were safe and with promising clinical activity. *See,* Romee et al, Science TM, 2016.

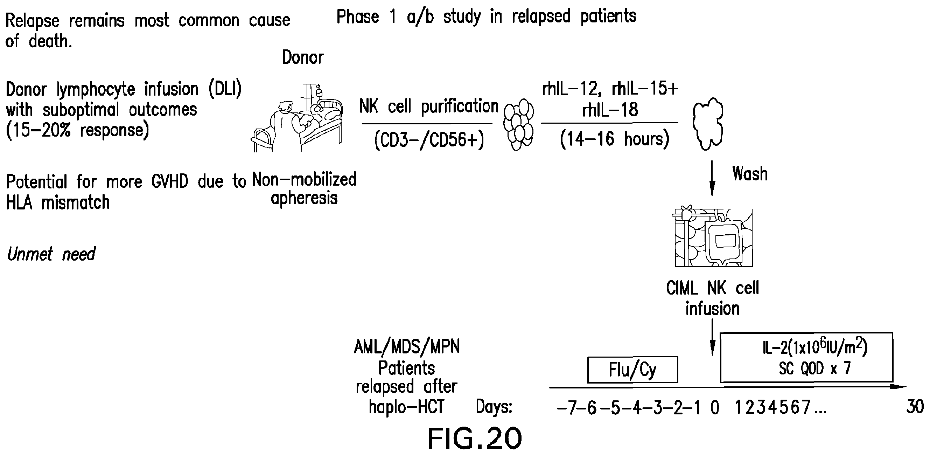

**FIG. 20** is a schematic outlining a Phase 1a/b study in patients with relapse after stem cell transplantation. Based on NK cell defects in haplos as well no effective and safe treatment option for patients who relapse after haplo HCt, a phase 1 study of Memory Like NK cells was initiated. Post transplant relapse is associated with very poor outcomes as shown on the left panel, and HLA mismatch can be a dangerous situation for using DLI with increased risk of GVHD. Using NK cells and generating memory-like NK cells from the same donor as original stem cell graft, immune compatible. Then they get 7 doses of low dose IL-2.

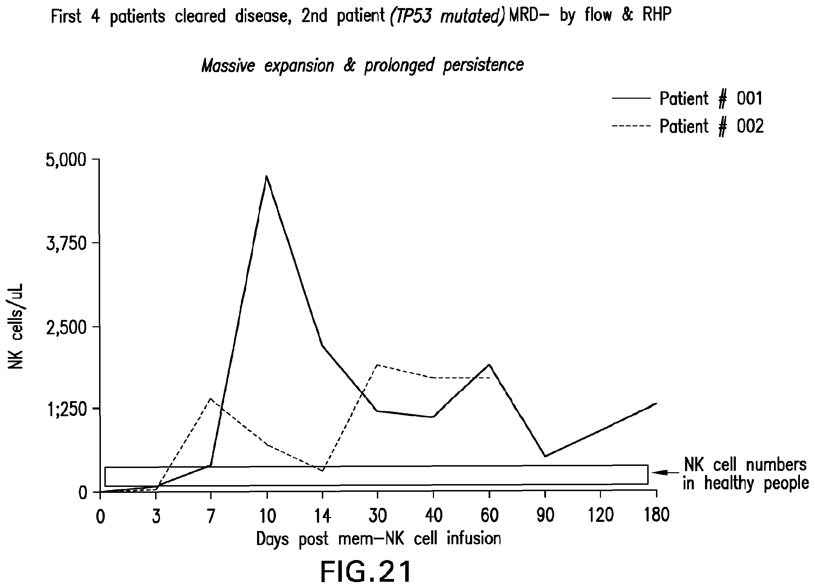

**FIG. 21** shows massive *in vivo* expansion and prolonged/sustained persistence of memory-like NK ("Mem-NK") cells. Roughly 500-fold expansion is shown. CIML NK cells were detectable for at least 65 days, mimicking what was observed in mice and also confirming long half life of these cells in humans. Long term persistence makes them quite attractive as a platform for NK cell-based immunotherapy approaches.

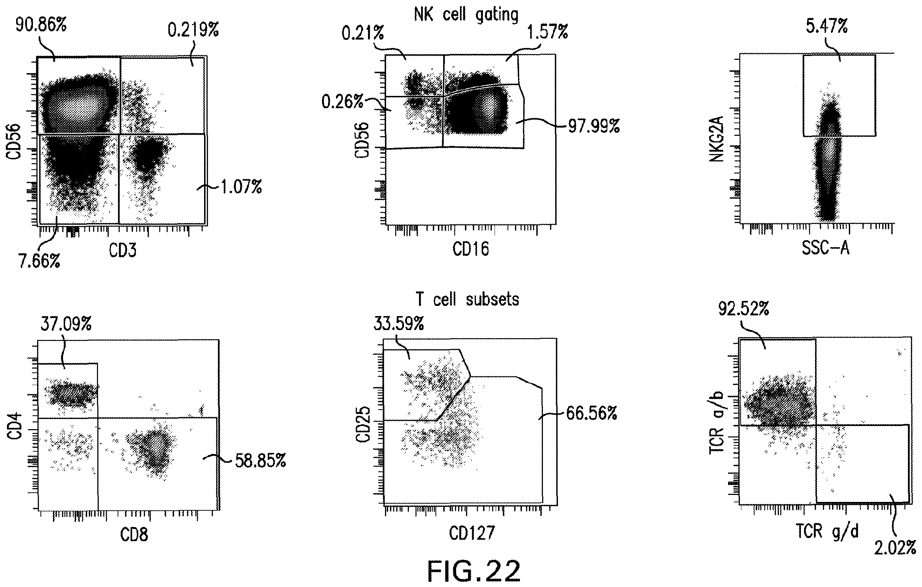

**FIG. 22** shows expansion of mature NK cells with minimal expansion of Tregs at day 28.

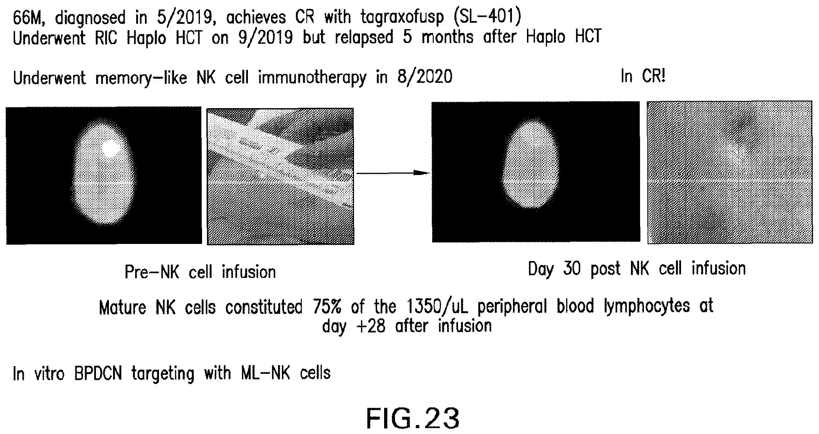

**FIG. 23** shows patient with blastic plasmacytoid DC neoplasm (BPDCN) achieves remission with memory-like NK cell infusion.

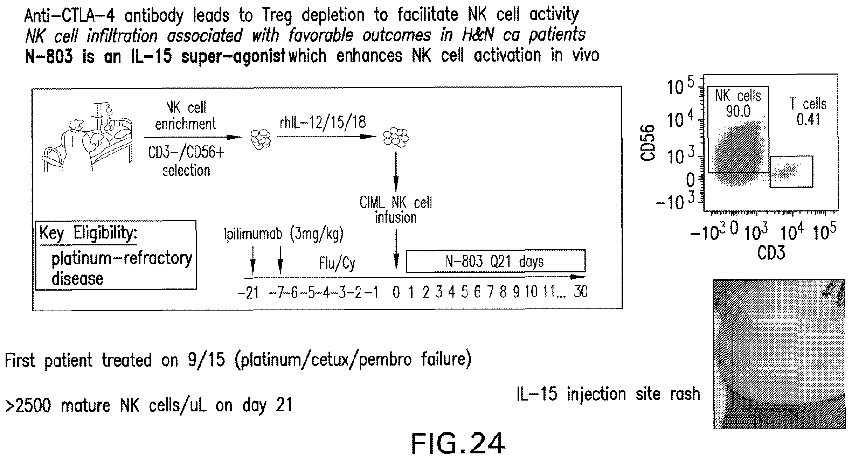

**FIG. 24** shows CTLA-4 inhibition plus memory-like NK cell immune cell therapy in advanced head and neck cancer. There is data showing NK infiltration predicts PFS in H&N cancer patients. Also, CTAL4 blockade ipi induces intra tumoral Treg depletion. For example, a phase 1 trial combining memory-like NK cells with Ipi and then also give long acting IL-15 super agonist.

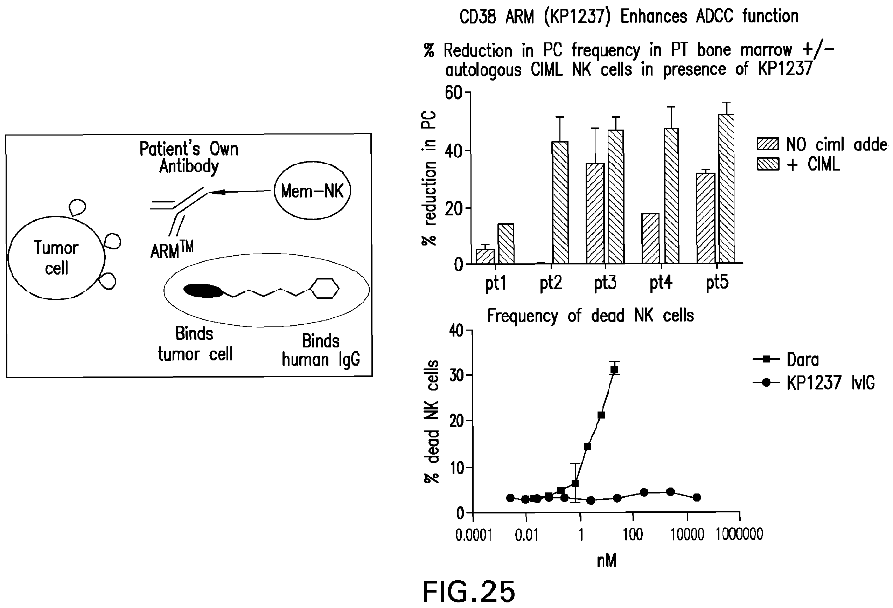

**FIG. 25** shows antibody recruiting molecules (ARMs) are new bispecific NK cell engagers.

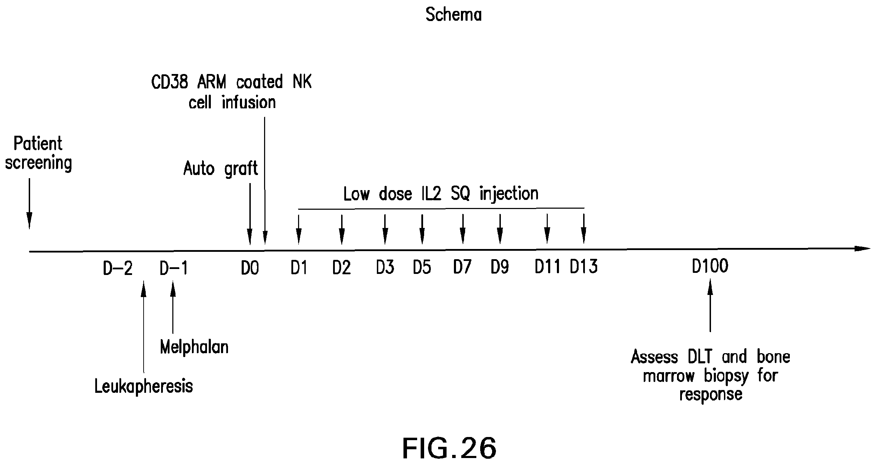

**FIG. 26** shows Phase I/II trial of CD38 ARM plus Mem-Like NK cells in MRD$^+$ MM with auto-HCT.

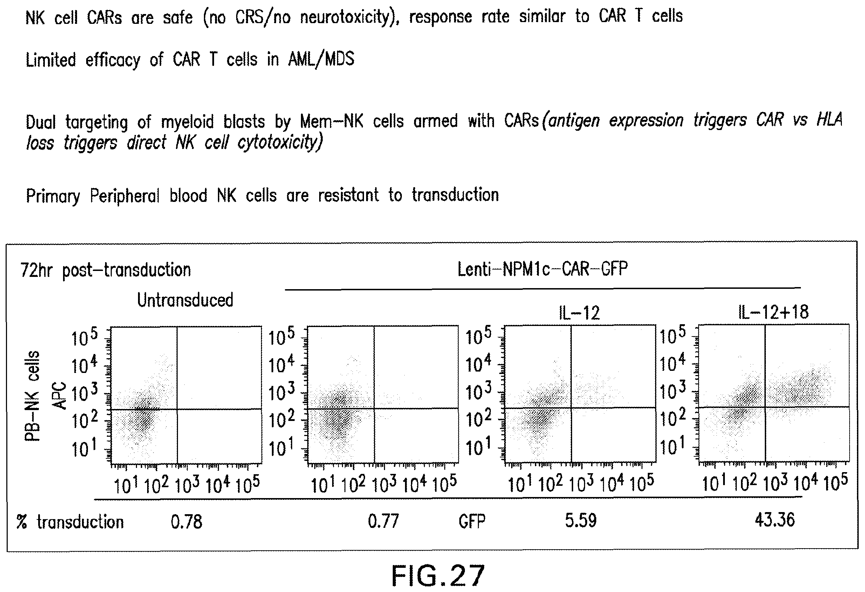

**FIG. 27** shows development of memory-like NK cell platform for CAR development.

**FIG. 28** shows the vector map of pHIV-CAR-CD19hscFv-P2A-GFP (8606 bp).

## DETAILED DESCRIPTION

The present disclosure provides methods for producing a population of cytokine-induced memory-like natural killer (NK) cells expressing a heterologous polypeptide (e.g., a CAR described herein). As described herein, methods of the disclosure provide increased transduction efficiency in a population of cytokine-induced memory like NK cells relative to a conventional method of cellular transduction, e.g., methods using a canonical pseudotyped lentiviral vector that integrates the vesicular stomatitis virus G (VSVG) glycoprotein. As demonstrated herein, the LDL receptor that binds to the VSVG glycoprotein is poorly expressed by NK cells (e.g., either conventional NK cells or cytokine-induced memory-like NK cells). Without being bound by theory, poor expression of the LDL receptor prevents lentiviral vector pseudotyped with VSVG glycoprotein from undergoing effective uptake in NK cells.

In contrast, it was discovered that cytokine-induced memory-like NK cells express increased levels of the alanine, serine, cysteine transporter 2 (ASCT2). As demonstrated herein, cytokine-induced memory-like NK cells pre-activated with (i) IL-12 and IL-18, or (ii) IL-12, IL-18, and IL-15 express levels of ASCT2 that are increased by about 1.5-2-fold relative to control NK cells (e.g., conventional NK cells activated with IL-15 only). In some aspects, the levels of ASCT was increased by about 1.5-3-fold relative to control NK cells (e.g., conventional NK cells activated with IL-15 only). Moreover, it was discovered that a lentiviral vector pseudotyped with a glycoprotein that binds ASCT2 resulted in high levels of transduction (e.g., at least 10%, 20%, 30%, 40%, 50%, 60%, 70%, 80%, or 90%) in cytokine-induced NK cells. In some aspects, the retroviral glycoprotein is a baboon envelope (BaEV) glycoprotein that binds to ASCT2. Furthermore, it was demonstrated that transduction in cytokine-induced memory-like NK cells that express high levels of ASCT2 was increased relative to control NK cells (e.g., conventional NK cells activated with IL-15 only) that express low levels of ASCT2. Without being bound by theory, the increased expression of ASCT by cytokine-induced memory-like NK cells enables efficient binding and uptake of pseudotyped lentiviral vector comprising an ASCT2-binding glycoprotein (e.g., BaEV).

Accordingly, in some aspects, the disclosure provides methods for producing a population of cytokine-induced memory-like NK cells expressing a heterologous polypeptide (e.g., a CAR polypeptide described herein), the method comprising contacting a population of cytokine-induced memory-like NK cell that express ASCT2 with a pseudotyped lentiviral vector comprising a glycoprotein that binds to ASCT (e.g., BaEV). In some aspects, providing the population of cytokine-induced memory-like NK cells expressing ASCT2 comprises contacting a population of primary human NK cells with IL-12, IL-18, and IL-15. In some aspects, providing the population of cytokine-induced memory-like NK cells expressing ASCT2 comprises contacting a population of primary human NK cells with IL-12 and IL-18. In some aspects, the population of primary human NK cells is contacted for a period of time sufficient to obtain a population of cytokine-induced memory-like NK cells, e.g., a population of cytokine-induced memory-like NK cells having one or more optimal properties relative to control NK cells (e.g., conventional NK cells contacted with IL-15 only).

Additionally, the present disclosure is based, at least in part, on the discovery that expression of ASCT2 in the population of human primary NK cells correlates with the stage of NK cell maturation and/or development. As demonstrated herein, the subset of the population of human primary NK cells expressing markers characteristic of a less mature, less developed, "stem-cell like", and/or proliferative phenotype (e.g., CD56$^{bright}$CD16$^{low/-}$ NKG2A$^+$CD57$^-$KIRs$^-$) has increased levels of ASCT2 expression relative to the subset of the population expressing phenotypic markers characteristic of a more mature and/or more developed phenotype (e.g., CD56$^{dim}$CD16$^+$NKG2A$^{+/-}$KIRs$^+$CD57$^+$). Without being bound by theory, it is believed higher expression of ASCT2 by less mature human primary NK cells results in increased transduction efficiency using a lentivirus pseudotyped with a glycoprotein that binds ASCT (e.g., BaEV) relative to human primary NK cells having a more mature phenotype and lower ASCT expression.

Furthermore, the present disclosure provides engineered cytokine-induced memory-like natural killer (NK) cells expressing a novel chimeric antigen receptor (CAR) targeting a neoepitope derived from an intracellular neoantigen resulting from a tumor-specific oncogenic driver gene mutation identified in NPM1c, a four-nucleotide duplication in nucleophosmin, a driver of oncogene mutation in ~35% of AML. The mutation creates a neoepitope that is presented by the most common HLA-A2 allele.

The anti-NPM1c CAR expressing cytokine-induced memory-like NK cells ("ML NK" cells) of the disclosure have been shown to effectively kill NPM1c expressing cells *in vitro* and *in vivo.* Without being limited by theory, it is believed that the engineered CAR expressing ML NK cells of the disclosure provide therapeutic benefits over CAR expressing T cells at least by avoiding, reducing or eliminating cytokine release syndrome, neurotoxicity and/or graft versus host disease (GVHD). The engineered CAR expressing ML NK cells of the disclosure have been shown to have enhanced interferon-$\gamma$ (INF-$\gamma$ ) production and cytotoxicity against tumor cells relative to conventional NK cells. As demonstrated herein, incorporating an anti-NPM1c CAR into such ML NK cells resulted in cells that induced apoptosis of target cells expressing NPM1c and reduced overall survival of such target cells. Further, when NPM1c expressing AML cells were injected into mice, the engineered CAR expressing ML NK cells of the disclosure reduced overall tumor burden *in vivo.* As further described herein, the efficacy of such engineered CAR expressing ML NK cells is increased when such cells express human IL-15, in secreted or membrane bound form. In particular, engineered CAR expressing ML NK cells of the disclosure expressing a membrane bound form of human IL-15 showed increased persistence and survival, and enhanced efficacy.

Accordingly, the ML NK cells expressing CAR polypeptides described herein are useful for targeted immunotherapy to treat cancers that carry an NPM1c mutation. For example, the ML NK cells expressing CAR polypeptides disclosed herein are useful for targeted immunotherapy to treat AML. In one aspect, provided herein are ML NK cells expressing a CAR that specifically binds to an antigen comprising an NPM1c neoepitope when such epitope is in complex with (or presented by) MHC class I protein (e.g., HLA-A2). In one aspect, provided herein are ML NK cells expressing a CAR that specifically binds to one or more of the neoepitopes having the following amino acid sequences: AIQDLCLAV (SEQ ID NO:1), AIQDLCVAV (SEQ ID NO: 71), CLAVEEVSL (SEQ ID NO: 72), VEEVSLRK (SEQ ID NO: 73), AVEEVSLR (SEQ ID NO: 74), AVEEVSLRK (SEQ ID NO: 75), CLAVEEVSLRK (SEQ ID NO: 76), when such epitope is in complex with a MHC class I protein (e.g., HLA-A2). In one aspect, provided herein are ML NK cells expressing a CAR that does not bind to, or substantially does not bind to, an MHC class I protein alone. In one aspect, provided herein are ML NK cells expressing a CAR that does not bind to, or substantially does not bind to, a control peptide in complex with an MHC class I protein (e.g., wherein the control peptide is an NY-ESO-1 epitope (e.g., a peptide comprising SEQ ID NO: 62) or influenza virus M1 epitope (e.g., a peptide comprising SEQ ID NO: 63). In one aspect, provided herein are ML NK cells expressing a CAR that does not bind to, or substantially does not bind to, an NPM1c neoepitope alone (without an MHC class I protein). In some aspects, the NPM1c neoepitope comprises the amino acid sequence AIQDLCLAV (SEQ ID NO: 1), and the MHC class I protein is an HLA-A2 protein (e.g., a protein encoded by the HLA-A*02:01 allele). In some aspects, the antigen is on the surface of a cancer cell (e.g., where the cancer is NPM1c+, e.g., where the cancer is AML). In one aspect, provided herein are ML NK cells expressing a CAR that specifically binds to an amino acid sequence comprising AIQDLCLAV (SEQ ID NO: 1) in complex with an HLA-A2 protein (e.g., a protein encoded by the HLA-A*02:01 allele).

In one aspect, provided herein are pharmaceutical compositions comprising the CAR expressing memory-like NK cells described herein (and, optionally, a pharmaceutically acceptable carrier).

In one aspect, a CAR polypeptide expressed by the cytokine-induced memory-like NK cells described herein comprises an intracellular domain, a transmembrane domain and an extracellular domain, wherein the extracellular domain specifically binds to an amino acid sequence comprising AIQDLCLAV (SEQ ID NO:1) in complex with an HLA-A2 protein (e.g., a protein encoded by the HLA-A*02:01 allele).

In one aspect, expression of the CAR polypeptide targets the ML NK cell to a cancer cell (e.g., wherein the cancer is AML) displaying on its surface an NPM1c neoepitope in complex with a class I major histocompatibility complex (MHC class I) protein (e.g., HLA-A2). In one aspect, expression of the CAR polypeptide targets the ML NK cells to a cancer cell (e.g., wherein the cancer is AML) displaying on its surface the amino acid sequence AIQDLCLAV (SEQ ID NO:1) in complex with an HLA-A2 protein (e.g., a protein encoded by the HLA-A*02:01 allele).

In one aspect, provided herein are methods of treating cancer in a subject (e.g., a human), wherein the cell surface of cells comprising the cancer displays an NPM1c neoepitope in complex with a MHC class I protein (e.g., HLA-A2), the method comprising administering to the subject an anti-NPM1c CAR expressing memory-like NK cell or population of said cells as described herein. In one aspect, the cell surface of cells comprising the cancer displays an NPM1c neoepitope in complex with a MHC class I protein (e.g., HLA-A2). In one aspect, the cell surface of cells comprising the cancer displays an amino acid sequence comprising AIQDLCLAV (SEQ ID NO: 1) in complex with an HLA-A2 protein (e.g., a protein encoded by the HLA-A*02:01 allele).

In some aspects, provided herein are methods of treating NPM1c-positive cancer in a subject (e.g., a human), the method comprising administering to the subject an anti-NPM1c CAR expressing memory-like NK cell or population of said cells as described herein.

In one aspect, provided herein are methods of treating AML in a subject (e.g., a human), the method comprising administering to the subject an anti-NPM1c CAR expressing memory-like NK cell or population of said cells as described herein .

## Anti-NPM1c Chimeric Antigen Receptor Expressing Cytokine-Induced Memory-Like NK Cells

In some aspects, the disclosure provides a cytokine-induced memory-like (ML) NK cell, or population of said cells, engineered to express a chimeric antigen receptor (CAR) polypeptide comprising an extracellular domain that specifically binds to an antigen comprising an NPM1c neoepitope in complex with a class I major histocompatibility complex (MHC class I) protein.

## Chimeric Antigen Receptor Polypeptide

In some aspects, a cytokine-induced memory-like NK cell is engineered to express a CAR polypeptide. In some aspects, the CAR polypeptide comprises an extracellular domain comprising an antibody, or antigen binding fragment thereof, or a bispecific molecule described herein.

CARs are genetically-engineered, artificial membrane-bound proteins that, when expressed in an immune effector cell (e.g., cytokine-induced memory-like NK cell), direct such immune effector cell to an antigen, and generally stimulate the immune effector cell to kill the cell displaying the antigen. Thus, the CARs can be used to impart a desired antigenic specificity to immune effector cells, such as an anti-tumor specificity (in particular, the antigenic specificity of is imparted by the extracellular domain of the CAR).

CARs generally comprise an extracellular domain that binds one or more antigens displayed on a cell, a transmembrane domain, and an intracellular domain that transmits an activation signal to the immune effector cell upon binding of the extracellular domain to the one or more antigens. In certain aspects, CARs contain three domains: 1) an extracellular domain typically comprising a signal peptide, a ligand or antigen recognition region (e.g. scFv), and a flexible spacer; 2) a transmembrane (TM) domain; 3) an intracellular domain (also known as a cytoplasmic domain) typically comprising one or more signaling domains. The extracellular domain of the CAR resides outside of the cell and exposed to the extracellular space, whereby it is accessible for interaction with its ligand/antigen. The TM domain allows the CAR to be anchored into the cell membrane of the effector cell. The intracellular domain of a CAR may comprise one or more cytoplasmic domains derived from signal transducing proteins different from the protein from which the extracellular domain is derived. The intracellular domain aids in effector cell activation upon binding of the CAR to its ligand/antigen. In some aspects, effector cell activation comprises induction of cytokine and chemokine production, as well as activation of the cytolytic activity of the effector cell. In some aspects, the CARs redirect cytotoxicity toward tumor cells.

Engagement of the antigen binding domain of the CAR with its target antigen on the surface of a target cell results in clustering of the CAR and delivers an activation stimulus to the CAR-containing cell. In some aspects, the main characteristic of CARs are their ability to redirect immune effector cell specificity, thereby triggering proliferation, cytokine production, phagocytosis or production of molecules that can mediate cell death of the target antigen expressing cell in a major histocompatibility (MHC) independent manner, exploiting the cell specific targeting abilities of monoclonal antibodies, soluble ligands or cell specific co-receptors CAR polypeptides provided herein comprise an extracellular domain that binds a neoantigen (e.g., a cancer or tumor neoantigen). A cancer neoantigen is an antigen that is present solely in cancer cells due to mutations that occur in such cancer cells. The cancer antigen may be expressed intracellularly and presented by an MHC class I protein on the surface of the cancer cell. For example, the cancer neoantigen targeted by a CAR polypeptide contemplated herein may be NPM1c:HLA-A2. In some aspects, the antibodies or antigen binding fragments (e.g., scFv) described herein are used to make CAR polypeptides. In some aspects, an antibody or antigen binding fragment thereof (e.g., an scFv) that binds NPM1c:HLA-A2 is used to generate a CAR polypeptide. In some aspects, the extracellular binding domain of the CAR polypeptide binds to a mutant nucleophosmin protein neoepitope (such as NPM1c neoepitope) in complex with (or presented by) a class I major histocompatibility complex (MHC class I) protein (e.g., HLA-2).

In some aspects, provided herein are CAR polypeptides that include an extracellular (antigen-binding) domain, a transmembrane domain, and an intracellular (cytoplasmic) domain that includes a cytoplasmic sequence of CD3ζ sequence sufficient to stimulate a NK cell when the antigen-binding domain binds to the antigen, and optionally, a cytoplasmic sequence of one or more (e.g., two, three, or four) co-stimulatory proteins (e.g., a cytoplasmic sequence of one or more of B7-H3, BTLA, CD2, CD7, CD27, CD28, CD30, CD40, CD40L, CD80, CD160, CD244, ICOS, LAG3, LFA-1, LIGHT, NKG2C, 4-1BB, OX40, PD-1, PD-L1, TIM3, 2B4, DAP10, CD137, DAP12, and a ligand that specifically binds to CD83) that provides for co-stimulation of the NK cell when the antigen-binding domain binds to the antigen.

In some aspects, a CAR can further include a linker. Additional aspects of CARs and CAR-expressing immune effector cells, including exemplary extracellular (antigen-binding) domains, linkers, transmembrane domains, and intracellular (cytoplasmic) domains, are described in, e.g., Kakarla et al., Cancer J. 20:151-155, 2014; Srivastava et al., Trends Immunol. 36:494-502, 2015; Nishio et al., Oncoimmunology 4(2): e988098, 2015; Ghorashian et al., Br. J. Haematol. 169:463-478, 2015; Levine, Cancer Gene Ther. 22:79-84, 2015; Jensen et al., Curr. Opin. Immunol. 33:9-15, 2015; Singh et al., Cancer Gene Ther. 22:95-100, 2015; Li et al., Zhongguo Shi Yan Xue Ye Xue Za Zhi 22:1753-1756, 2014; Gill et al., Immunol. Rev. 263:68-89, 2015; Magee et al., Discov. Med. 18:265-271, 2014; Gargett et al., Front. Pharmacol. 5:235, 2014; Yuan et al., Zhongguo Shi Yan Xue Ye Xue Za Zhi 22:1137-1141, 2014; Pedgram et al., Cancer J. 20:127-133, 2014; Eshhar et al., Cancer J. 20:123-126, 2014; Ramos et al., Cancer J. 20:112-118, 2014; Maus et al., Blood 123:2625-2635, 2014; Jena et al., Curr. Hematol. Malig. Rep. 9:50-56, 2014; Maher et al., Curr. Gene Ther. 14:35-43, 2014; Riches et al., Discov. Med. 16:295-302, 2013; Cheadle et al., Immunol. Rev. 257:83-90, 2014; Davila et al., Int. J. Hematol. 99:361-371, 2014; Xu et al., Cancer Lett. 343:172-178, 2014; Kochenderfer et al., Nat. Rev. Clin. Oncol. 10:267-276, 2013; Hosing et al., Curr. Hematol. Malig. Rep. 8:60-70, 2013; Hombach et al., Curr. Mol. Med. 13:1079-1088, 2013; Xu et al., Leuk. Lymphoma 54:255-260, 2013; Gilham et al., Trends Mol. Med. 18:377-384, 2012; Lipowska-Bhalla et al., Cancer Immunol. Immunother. 61:953-962, 2012; Chmielewski et al., Cancer Immunol. Immunother. 61:1269-1277, 2013; Jena et al., Blood 116:1035-1044, 2010; Dotti et al, Immunology Reviews 257(1): 107-126, 2013; Dai et al., Journal of the National Cancer Institute 108(7): djv439, 2016; Wang and Riviere, Molecular Therapy-Oncolytics 3: 16015, 2016; U.S. Patent Application Publication Nos. 2018/0057609; 2018/0037625; 2017/0362295; 2017/0137783; 2016/0152723, 2016/0206656, 2016/0199412, 2016/0208018, 2015/0232880, 2015/0225480; 2015/0224143; 2015/0224142; 2015/0190428; 2015/0196599; 2015/0152181; 2015/0140023; 2015/0118202; 2015/0110760; 2015/0099299; 2015/0093822; 2015/0093401; 2015/0051266; 2015/0050729; 2015/0024482; 2015/0023937; 2015/0017141; 2015/0017136; 2015/0017120; 2014/0370045; 2014/0370017; 2014/0369977; 2014/0349402; 2014/0328812; 2014/0322275; 2014/0322216; 2014/0322212; 2014/0322183; 2014/0314795; 2014/0308259; 2014/0301993; 2014/0296492; 2014/0294784; 2014/0286973; 2014/0274909; 2014/0274801; 2014/0271635; 2014/0271582; 2014/0271581; 2014/0271579; 2014/0255363; 2014/0242701; 2014/0242049; 2014/0227272; 2014/0219975; 2014/0170114; 2014/0134720; 2014/0134142; 2014/0120622; 2014/0120136; 2014/0106449; 2014/0106449; 2014/0099340; 2014/0086828; 2014/0065629; 2014/0050708; 2014/0024809; 2013/0344039; 2013/0323214; 2013/0315884; 2013/0309258; 2013/0288368; 2013/0287752; 2013/0287748; 2013/0280221; 2013/0280220; 2013/0266551; 2013/0216528; 2013/0202622; 2013/0071414; 2012/0321667; 2012/0302466; 2012/0301448; 2012/0301447; 2012/0060230; 2011/0213288; 2011/0158957; 2011/0104128; 2011/0038836; 2007/0036773; and 2004/0043401.

Additional aspects of CARs and CAR-expressing immune effector cells, including exemplary extracellular (antigen-binding) domains, linkers, transmembrane domains, and intracellular (cytoplasmic) domains, are described in WO 2016/168595; WO 12/079000; 2015/0141347; 2015/0031624; 2015/0030597; 2014/0378389; 2014/0219978; 2014/0206620; 2014/0037628; 2013/0274203; 2013/0225668; 2013/0116167; 2012/0230962; 2012/0213783; 2012/0093842; 2012/0071420; 2012/0015888; 2011/0268754; 2010/0297093; 2010/0158881; 2010/0034834; 2010/0015113; 2009/0304657; 2004/0043401; 2014/0322253; 2015/0118208; 2015/0038684; 2014/0024601; 2012/0148552; 2011/0223129; 2009/0257994; 2008/0160607; 2008/0003683; 2013/0121960; 2011/0052554; and 2010/0178276.

In some aspects, provided herein are CARs that comprise an intracellular domain, a transmembrane domain and an extracellular domain, wherein the extracellular domain comprises any antibody, or antigen binding fragment thereof, or a bispecific molecule described herein. In some aspects, provided herein are chimeric antigen receptors (CARs) comprising an intracellular domain, a transmembrane domain and an extracellular binding domain, wherein the extracellular binding domain comprises any antibody, or antigen binding fragment thereof, or a bispecific molecule described herein, wherein such antibody, antigen binding fragment thereof, or bispecific molecule binds to an antigen comprising an NPM1c neoepitope in complex with (or presented by) a class I major histocompatibility complex (MHC class I) protein.

In some aspects, provided herein are chimeric antigen receptors (CARs) having the intracellular, transmembrane and/or extracellular domains of NPM1c CAR described in the Examples section (see, e.g., Example 1).

In some aspects, provided herein are CARs comprising an intracellular domain comprising one or more costimulatory domains of one or more costimulatory molecules selected from the group consisting of: CD27, CD28, 4-1BB, OX40, CD30, CD40, PD-1, ICOS, 2B4, DAP10, CD137 and DAP12. In specific aspects, provided herein are CARs comprising an intracellular domain comprising a CD3-zeta signaling domain and, optionally, a 4-1BB costimulatory domain. In some aspects, provided herein are CARs comprising a transmembrane domain of CD3-zeta, CD8, CD28, NKG2D, CD16, NKp44 or NKp46. In specific aspects, provided herein are CARs comprising a transmembrane domain comprising a CD8 transmembrane domain. In some aspects, provided herein are CARs comprising an extracellular domain comprising any antibody or antigen binding fragment thereof described herein (e.g., scFv). In specific aspects, provided herein are CARs comprising an extracellular domain comprising any antibody or antigen binding fragment thereof described herein (e.g., scFv) that specifically binds to an antigen comprising a mutant nucleophosmin protein epitope (e.g., an NPM1c neoepitope) in complex with (or presented by) a class I major histocompatibility complex (MHC class I) protein (e.g., HLA-A2). In specific aspects, provided herein are CARs comprising an extracellular domain comprising any antibody or antigen binding fragment thereof described herein (e.g., scFv) that specifically binds to an antigen comprising AIQDLCLAV (SEQ ID NO: 1) neoepitope in complex with (or presented by) a class I major histocompatibility complex (MHC class I) protein (e.g., HLA-A2). In specific aspects, provided herein are CAR polypeptides comprising an extracellular domain comprising any antibody or antigen binding fragment thereof described herein (e.g., scFv) comprising a VH and a VL, wherein the VH comprises the amino acid sequence of SEQ ID NO: 5 or an amino acid sequence that is at least 75%, 80%, 85%, 90%, 95%, 98% or 99% identical to SEQ ID NO: 5, and wherein the VL comprises the amino acid sequence of SEQ ID NO: 3 or an amino acid sequence that is at least 75%, 80%, 85%, 90%, 95%, 98% or 99% identical to SEQ ID NO: 3. In specific aspects, provided herein are CAR polypeptides comprising an extracellular domain comprising any antibody or antigen binding fragment thereof described herein (e.g., scFv) comprising a VH comprising VH CDR1 having amino acid sequence of SEQ ID NO: 9, VH CDR2 having amino acid sequence of SEQ ID NO: 10, CDR3 having amino acid sequence of SEQ ID NO: 11, and/or comprising a VL comprising VL CDR1 having amino acid sequence of SEQ ID NO: 6, VL CDR2 having amino acid sequence of SEQ ID NO: 7, and VL CDR3 having amino acid sequence of SEQ ID NO: 8. In specific aspects, provided herein are CAR polypeptides comprising an extracellular domain comprising any antibody or antigen binding fragment thereof described herein (e.g., scFv) comprising a VH comprising VH CDR1, VH CDR2 and VH CDR3 being the CDRs of a VH that has an amino acid sequence of SEQ ID NO: 5, and/or comprising a VL comprising VL CDR1, VL CDR2 and VL CDR3 being the CDRs of a VL that has an amino acid sequence of SEQ ID NO: 3. In specific aspects, provided herein are CAR polypeptides comprising an extracellular domain comprising an scFv that has the amino acid sequence of SEQ ID NO: 2, or an scFv that has amino acid sequence that is at least 75%, 80%, 85%, 90%, 95%, 98% or 99% identical to SEQ ID NO: 2.

### Extracellular (Antigen Binding) Domains of CARs

In some aspects, a CAR polypeptide expressed in a cytokine-induced memory-like NK cell comprises an extracellular domain. In some aspects, the extracellular domain comprises an antigen binding domain.

Non-limiting examples of antigen binding domains include: a monoclonal antibody (e.g., IgG1, IgG2, IgG3, IgG4, IgM, IgE, and IgD) (e.g., a fully human or a chimeric (e.g., a humanized) antibody), an antigen binding fragment of an antibody (e.g., Fab, Fab', or F(ab')$_2$ fragments) (e.g., a fragment of a fully human or a chimeric (e.g.. humanized) antibody), a diabody, a triabody, a tetrabody, a minibody, a scFv, scFv-Fc, (scFv)$_2$, scFab, bis-scFv, hc-IgG, a BiTE, a single domain antibody (e.g., a V-NAR domain or a VhH domain), IgNAR, and a multispecific (e.g., bispecific antibody) antibody. In some aspects, the antigen binding domain comprises an scFv. Methods of making these antigen-binding domains are known in the art.

In some aspects, an antigen binding domain includes at least one (e.g., one, two, three, four, five, or six) CDR (e.g., any of the three CDRs from an immunoglobulin light chain variable domain and/or any of the three CDRs from an immunoglobulin heavy chain variable domain) of an antibody that is capable of specifically binding to the target antigen, such as immunoglobulin molecules (e.g., light or heavy chain immunoglobulin molecules) and immunologically-active (antigen-binding) fragments of immunoglobulin molecules.

In some aspects, an antigen binding domain is a single-chain antibody (e.g., a V-NAR domain or a $V_HH$ domain, or any of the single-chain antibodies as described herein). In some aspects, an antigen binding domain is a whole antibody molecule (e.g., a human, humanized, or chimeric antibody) or a multimeric antibody (e.g., a bi-specific antibody).

In some aspects, antigen-binding domains include antibody fragments and multispecific (e.g., bi-specific) antibodies or antibody fragments. Examples of antibodies and antigen-binding fragments thereof include but are not limited to: single-chain Fvs (scFvs), Fab fragments, Fab' fragments, F(ab')$_2$, disulfide-linked Fvs (sdFvs), Fvs, and fragments containing either a VL or a VH domain.

Additional antigen binding domains provided herein are polyclonal, monoclonal, multispecific (multimeric, e.g., bi-specific), human antibodies, chimeric antibodies (e.g., human-mouse chimera), single-chain antibodies, intracellularly-made antibodies (i.e., intrabodies), and antigen-binding fragments thereof. The antibodies or antigen-binding fragments thereof can be of any type (e.g., IgG, IgE, IgM, IgD, IgA, and IgY), class (e.g., $IgG_1$, $IgG_2$, $IgG_3$, $IgG_4$, $IgA_1$, and $IgA_2$), or subclass.

Additional examples of antigen binding domains are antigen-binding fragments of an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4), an antigen-binding fragment of an IgA (e.g., an antigen-binding fragment of IgA1 or IgA2) (e.g., an antigen-binding fragment of a human or humanized IgA, e.g., a human or humanized IgA1 or IgA2), an antigen-binding fragment of an IgD (e.g., an antigen-binding fragment of a human or humanized IgD), an antigen-binding fragment of an IgE (e.g., an antigen-binding fragment of a human or humanized IgE), or an antigen-binding fragment of an IgM (e.g., an antigen-binding fragment of a human or humanized IgM).

In some aspects, an antigen binding domain can bind to a particular antigen (e.g., a tumor-associated antigen) with an affinity ($K_D$) about or higher than $1 \times 10^{-7}$ M (e.g., about or higher than $1 \times 10^{-8}$ M, about or higher than $1 \times 10^{-9}$ M, about or higher than 500 nM, about or higher than 100 nM, about or higher than 25 nM, about or higher than 15 nM, about or higher than 7 nM, about or higher than 5 nM, or about or higher than 1 nM), e.g., in saline or in phosphate buffered saline.

As can be appreciated by those in the art, the choice of the antigen binding domain to include in the CAR depends upon the type and number of ligands that define the surface of a cell (e.g., cancer cell or tumor) to be targeted in a subject in need thereof. For example, the antigen binding domain may be chosen to recognize a tumor specific antigen (TSA), such as a cancer neoantigen. For example, the tumor specific antigen may be an NMPlc neoantigen in complex with (or presented by) a MHC Class I protein (e.g., HLA-A2), such as NPM1c:HLA-A2. In some aspects, the NMPlc neoantigen comprises the amino acid sequence AIQDLCLAV (SEQ ID NO: 1)

The CAR polypeptide comprises an antigen binding domain that recognizes an acute myeloid leukemia neoantigen that is an NPM1c neoantigen. In one aspect, the NMP1c neoantigen is in complex with (or presented by) a MHC Class I protein (e.g., HLA-A2), such as NPM1c:HLA-A2.

Tumor antigens, (e.g. tumor-associated antigens (TAAs) and tumor-specific antigens (TSAs)) that may be targeted by CAR effector cells (e.g., CAR ML NK cells), include, but are not limited to NPM1c:HLA-A2. In one aspect, the tumor specific antigen is NPM1c:HLA-A2.

In some aspects, the CAR comprises an extracellular domain that specifically binds to an antigen comprising a neoepitope (e.g., a cancer neoepitope) in complex with (or presented by) an MHC protein (e.g., MHC class I protein). In some aspects, the extracellular domain does not bind to, or substantially does not bind to, the MHC protein alone. In some aspects the extracellular domain does not bind to, or substantially does not bind to, a control peptide in complex with the MHC protein. In some aspects, the extracellular domain does not bind to, or substantially does not bind to, the neoepitope alone (without an MHC protein).

Functional MHC Class I molecules comprise a heavy $\alpha$ chain and a $\beta$2-microglobulin chain. Peptide binding by MHC Class I molecules is accomplished by interaction of the peptide amino acid side chains with discrete pockets within the peptide-binding groove of the MHC molecule that is formed by the $\alpha$1 and $\alpha$2 domains of the heavy chain. Typically, for the human leukocyte antigen (HLA), the main binding energy is derived from the interaction of residues in position 2 and the C-terminus of the peptide and the B and F binding pockets of the MHC molecule, respectively, though side chains throughout the peptide can promote or diminish MHC binding capacity (see, e.g., Guo, et al (1992) Nature 360:364; Silver et al (1992) Nature 360:367; Gorga et al (1992) Proteins 12;87; Madden (1995) Annu Rev Immunol 13:587; Madden et al (1993) Cell 75;693; Madden et al (1992) Cell 70:1035; Bjorkman, et al (1987) Nature 329:512; Saper et al (1991) J Mol Biol 219:277). For a peptide with 9 amino acid residues, the C-terminal residue (position 9) interacts with the F binding pocket of the MHC molecule.

MHC molecules are extremely polymorphic, and thousands of allelic variants have been identified at the class I A and B loci. Most of the polymorphism occurs at the peptide binding pocket, such that MHC molecules have a range of peptide binding specificities. Despite the polymorphism, it is known in the art that HLA class I molecules can be clustered into groups (i.e., supertypes) based upon shared peptide binding specificity. Each group (supertype) is defined by a peptide consensus sequence that reflects the positions of the peptide that are "anchor residues" or residues that are important for MHC binding. For example, HLA Class I molecules of the A2-supertype (i.e., HLA-A2 or a protein encoded by the HLA-A*02 allele group) share specific binding for peptides with small and aliphatic residues (e.g., alanine, tyrosine, serine, valine, leucine, isoleucine, methionine, glutamine) at position 2 and aliphatic (e.g., leucine, isoleucine, valine, methionine) or small hydrophobic residues (e.g., alanine, valine) at the C-terminus of the peptide (see, e.g., Sidney, et al (2008) BMC Immunology 9:1).

In some aspects, that the extracellular domain binds (e.g., specifically binds) to an antigen comprising acute myeloid leukemia (AML)-associated mutant nucleophosmin protein neoepitope in complex with (or presented by) an MHC class I protein such as HLA-A2 (such as NPM1c:HLA-A2).

Genomic analysis of AML has shown a lower mutational load than most other adult cancers, with an average of 13 coding mutations per AML patient (see Ley et al., N Engl J Med 368: 2059 (2013); Alexandrov et al.. NATURE 500: 415 (2013); Kandoth et al., NATURE 502 333 (2013)). However, somatic mutations in AML often occur in the same genes (see Ley et al., N Engl J Med 368: 2059 (2013); Papaemmanuil et al., N Engl J Med 374: 2209 (2016)) and neoantigens derived from these hotspot mutations therefore become attractive targets for tumor-specific immunotherapy (see van der Lee et al., J CLIN INVEST 129: 774 (2019)). Among the most commonly occurring mutations is a 4-nucleotide duplication in a critical driver gene encoding nucleophosmin (NPM1; encoded by *NPM1*), which occurs in 30-35% of all adult AML patients (see Ley et al., N Engl J Med 368: 2059 (2013); Papaemmanuil et al., N Engl J Med 374: 2209 (2016); Falini et al., N Engl J Med 352: 254 (2005)). Such mutations in NPM1 result in its aberrant cytoplasmic localization, and the mutant protein is referred to as NPM1c. The AML-associated NPM1c mutant protein generates a leukemic neoantigen that is HLA class I restricted and presented on leukemic blasts of patients with the HLA-A*02:01 allele and some other alleles. For example, NPM1c produces a leukemia-specific neoantigen epitope (AIQDLCLAV (SEQ ID NO: 1), abbreviated as AIQ) that is presented by the most common HLA-A*0201 allele (~50% of human population) (see Greiner et al., BLOOD 120: 1282 (2012)).

The extracellular domain binds to an antigen comprising a NPM1c neoepitope in complex with (or presented by) an MHC Class I protein such as HLA-A2. The length of the NPM1c neoepitope is any length that is reasonable for a peptide that binds an MHC Class I molecule. In some aspects, the length of the NPM1c neoepitope is 5-20 amino acids, 6-19 amino acids, 7-18 amino acids, 8-17 amino acids. 8-16 amino acids, 8-15 amino acids, 8-15 amino acids, 8-14 amino acids, 8-13 amino acids, 8-12 amino acids, 9-12 amino acids, or 9-11 amino acids. In some aspects, the length of the NPM1c neoepitope is 20, 19, 18, 17, 16, 15, 14, 13, 12, 11, 10, 9, 8, 7, 6, or 5 amino acids. In some aspects, the length of the NPM1c neoepitope is 12 amino acids. In some aspects, the length of the NPM1c neoepitope is 11 amino acids. In some aspects, the length of the NPM1c neoepitope is 10 amino acids. In some aspects, the length of the NPM1c neoepitope is 9 amino acids. In some aspects, the length of the NPM1c neoepitope is 8 amino acids. In some aspects, the NPM1c neoepitope is a peptide of 20, 19, 18, 17, 16, 15, 14, 13, 12, 11, 10, 9, 8, 7, 6, or 5 consecutive amino acids within a polypeptide of 10, 15, 20, 30, 40, 50, or 100 amino acid residues in length.

In some aspects, the NPM1c neoepitope binds to a MHC Class I protein that is HLA-A2. In some aspects, the NPM1c neoepitope that binds HLA-A2 comprises an amino acid sequence wherein position 2 of the amino acid sequence is a small and aliphatic residue (e.g., alanine, tyrosine, serine, valine, leucine, isoleucine, methionine, glutamine), and wherein the C-terminal residue of the amino acid sequence is an aliphatic residue (e.g., leucine, isoleucine, valine, methionine) or a small hydrophobic residue (e.g., alanine, valine). In some aspects, the NPM1c neoepitope that binds HLA-A2 comprises an amino acid sequence wherein position 2 of the amino acid sequence is valine, isoleucine or leucine and the C-terminal residue of the amino acid sequence is valine, leucine, or isoleucine. In some aspects, wherein the NPM1c neoepitope is 8 amino acid residues in length, the C-terminal amino acid is position 8. In some aspects, wherein the NPM1c neoepitope is 9 amino acid residues in length, the C-terminal amino acid is position 9. In some aspects, wherein the NPM1c neoepitope is 10 amino acid residues in length, the C-terminal amino acid is position 10. In some aspects, wherein the NPM1c neoepitope is 11 amino acid residues in length, the C-terminal amino acid is position 11. In some aspects, wherein the NPM1c neoepitope is 12 amino acid residues in length, the C-terminal amino acid is position 12.

Neoepitopes derived from NPM1c that bind to HLA-A2 are known in the art. For example, Greiner (2012) Blood 120:1282 identified amino acid sequences for 9-mer NPM1c neoepitopes that bind HLA-A2, including: AIQDLCLAV (SEQ ID NO: 1) and AIQDLCVAV (SEQ ID NO: 71). As a further example, van der Lee (2019) J Clin Invest 129:774 identified amino acid sequences of NPM1c neoepitopes that bind HLA-A2 Class I molecules, including CLAVEEVSL (SEQ ID NO: 72), as well as amino acid sequences of NPM1c neoepitopes that bind to MHC Class I molecules encoded by other HLA haplotypes, including VEEVSLRK (SEQ ID NO:73), AVEEVSLR (SEQ ID NO:74), AVEEVSLRK (SEQ ID NO: 75), and CLAVEEVSLRK (SEQ ID NO: 76).

In some aspects, the extracellular domain binds (e.g., specifically binds) to an antigen comprising a neoepitope of a mutant nucleophosmin protein in complex with (or presented by) an MHC class I protein (such as NPM1c:HLA-A2), wherein the mutation in the nucleophosmin protein is due to a four-nucleotide duplication in the gene encoding nucleophosmin. In some aspects, the extracellular domain binds (e.g., specifically binds) to an antigen comprising a cytoplasmic (located in the cytoplasm) mutant nucleophosmin protein neoepitope in complex with (or presented by) an MHC class I protein (such as NPM1c:HLA-A2). In some aspects, the neoepitope is an 8, 9, 10, 11, or 12 amino acid peptide derived from mutant nucleophosmin protein. In some aspects, the neoepitope is an 8, 9, 10, 11, or 12 amino acid peptide derived from 10, 15, 20, 25, 30, 35, 40, 45, 50, 55, 60, 65, 70, 75, 80, 85, 90, 95, or 100 amino acid residues of mutant nucleophosmin protein. In some aspects, the neoepitope is an 8, 9, 10, 11, or 12, amino acid peptide derived from 10, 15, 20, 25, 30, 35, 40, 45, 50, 55, 60, 65, 70, 75, 80, 85, 90, 95, or 100 amino acid residues at the C-terminus of mutant nucleophosmin protein. In some aspects, the mutant nucleophosmin protein comprises an amino acid sequence as set forth by SEQ ID NO: 56. In some aspects, the mutant nucleophosmin protein neoepitope is an 8, 9, 10, 11, or 12 amino acid peptide derived from a protein comprising the amino acid sequence of SEQ ID NO: 56. In some aspects the mutant nucleophosmin protein neoepitope is an 8, 9, 10, 11, or 12 amino acid peptide derived from 10, 15, 20, 25, 30, 35, 40, 45, 50, 55, 60, 65, 70, 75, 80, 85, 90, 95, or 100 amino acid residues of the amino acid sequence set forth by SEQ ID NO: 56. In some aspects, the mutant nucleophosmin protein neoepitope is an 8, 9, 10, 11, or 12 amino acid peptide derived from the 10, 15, 20, 25, 30, 35, 40, 45, 50, 55, 60, 65, 70, 75, 80, 85, 90, 95, or 100 amino acid residues at the C-terminus of a protein with the amino acid sequence set forth by SEQ ID NO:56. In some aspects, the extracellular domain specifically binds to an antigen comprising a neoepitope of a protein comprising the amino acid sequence of SEQ ID NO: 56 in complex with (or presented by) an MHC class I protein (e.g., HLA-A2 protein).

In some aspects, the mutant nucleophosmin protein comprises the C-terminal amino acid sequence MTDQEAIQDLCLAVEEVSLRK (SEQ ID NO: 57). In some aspects, the extracellular domain specifically binds to an antigen comprising a neoepitope of a NPM1c protein comprising the C-terminal amino acid sequence MTDQEAIQDLCLAVEEVSLRK (SEQ ID NO: 57) in complex with (or presented by) an MHC class I protein (e.g., HLA-A2 protein). In some aspects, the neoepitope is an 8, 9, 10, 11, or 12 amino acid peptide derived from the C-terminal amino acid sequence MTDQEAIQDLCLAVEEVSLRK (SEQ ID NO:57) of the NPM1c protein. In some aspects, the extracellular domain specifically binds to an antigen comprising an NPM1c neoepitope in complex with (or presented by) an HLA-A2 protein or a protein encoded by the HLA-A*02 allele group (i.e., NPM1c:HLA-A2). In some aspects, NPM1c is a human NPM1c.

In some aspects, the extracellular domain binds to (e.g., specifically binds to) an antigen comprising a cytoplasmic mutant nucleophosmin protein neoepitope in complex with (or presented by) an MHC class I protein (e.g., an HLA-A2 protein or a protein encoded by the HLA-A*02 allele group), wherein the amino acid sequence of the neoepitope comprises: AIQDLCLAV (SEQ ID NO: 1), AIQDLCVAV (SEQ ID NO: 71), CLAVEEVSL (SEQ ID NO:72), VEEVSLRK (SEQ ID NO: 73), AVEEVSLR (SEQ ID NO: 74), AVEEVSLRK (SEQ ID NO: 75) or CLAVEEVSLRK (SEQ ID NO: 76). In some aspects, the extracellular domain binds to an antigen comprising an amino acid sequence selected from AIQDLCLAV (SEQ ID NO: 1), AIQDLCVAV (SEQ ID NO: 71), CLAVEEVSL (SEQ ID NO: 72), VEEVSLRK (SEQ ID NO: 73), AVEEVSLR (SEQ ID NO: 74), AVEEVSLRK (SEQ ID NO: 75) and CLAVEEVSLRK (SEQ ID NO: 76) presented by HLA-A2. In some aspects, the extracellular domain binds to an antigen comprising an amino acid sequence AIQDLCLAV (SEQ ID NO: 1) presented by HLA-A2.

In some aspects, the extracellular domain does not bind to, or substantially does not bind to, an MHC class I protein alone and/or a control peptide in complex with an MHC class I protein (e.g., wherein the control peptide has the same number of amino acids as the neoepitope but is derived from a protein different from the protein from which the neoepitope is derived).

In some aspects, the extracellular domain does not bind to, or substantially does not bind to, a cytoplasmic mutant nucleophosmin protein neoepitope alone (without an MHC class I protein such as HLA-A2).

In some aspects, the NPM1c neoepitope comprises the amino acid sequence AIQDLCLAV (SEQ ID NO:1), and the MHC class I protein is an HLA-A2 protein (e.g., a protein encoded by the HLA-A*02:01 allele). In some aspects, the NPM1c neoepitope comprises an amino acid sequence selected from AIQDLCVAV (SEQ ID NO:71), CLAVEEVSL (SEQ ID NO:72), VEEVSLRK (SEQ ID NO:73), AVEEVSLR (SEQ ID NO:74), AVEEVSLRK (SEQ ID NO:75) and CLAVEEVSLRK (SEQ ID NO: 76), and the MHC class I protein is an HLA-A2 protein (e.g., a protein encoded by the HLA-A*02:01 allele).

In some aspects, the extracellular domain specifically binds to an antigen comprising a neoepitope comprising the amino acid sequence AIQDLCLAYV (SEQ ID NO:1) in complex with a class I major histocompatibility complex (MHC class I) protein, wherein any one, two, three, four, five or six amino acids of the amino acid sequence AIQDLCLAV (SEQ ID NO:1) are substituted. In some aspects, the extracellular domain specifically binds to an antigen comprising a neoepitope comprising the amino acid sequence AIQDLCLAV in complex with a class I major histocompatibility complex (MHC class I) protein, wherein any one, two, three or four amino acids of the amino acid sequence AIQDLCLAV are substituted. In some aspects, the amino acid substitution is a conservative amino acid substitution. In some aspects, the amino acid substitution is a substitution with an amino acid residue of a similar size as the size of the existing residue in the AIQDLCLAV sequence (SEQ ID NO:1). In some aspects, the amino acid substitution does not affect (or does not substantially affect) the binding of the extracellular domain to the antigen.

In some aspects, the extracellular domain specifically binds to an antigen comprising a neoepitope comprising the amino acid sequence AIQDLCLAV in complex with a class I major histocompatibility complex (MHC class I) protein, wherein one, two, or more anchor residues of the amino acid sequence AIQDLCLAV (SEQ ID NO:1) are substituted (e.g., position 2 and/or position 9 of SEQ ID NO:1, e.g., underlined residues of AIQDLCLAV (SEQ ID NO:1)). In some aspects, the amino acid substitution does not affect (or does not substantially affect) the binding of the extracellular domain to the antigen or the binding of the neoepitope to the class I major histocompatibility complex (MHC class I) protein (e.g., HLA-A2). In some aspects, the amino acid residue I in the second position of AIQDLCLAV (SEQ ID NO:1) is substituted with the amino acid residue L (leucine). In some aspects, the amino acid residue I in the second position of AIQDLCLAV (SEQ ID NO:1) is substituted with the amino acid residue V (valine), M (methionine), tyrosine (T), serine (S), glutamine (Q) or A (alanine). In some aspects, the amino acid residue V in the ninth position of AIQDLCLAV (SEQ ID NO:1) is substituted with the amino acid residue I (isoleucine), L (leucine), M (methionine), or A (alanine).

In some aspects, the extracellular domain specifically binds to an antigen comprising a neoepitope comprising the amino acid sequence AIQDLCLAV in complex with a class I major histocompatibility complex (MHC class I) protein, wherein any one, two, three, four, five or six amino acids of the amino acid sequence AIQDLCLAV (SEQ ID NO:1) are substituted, and wherein the substitution is a conservative amino acid substitution. In some aspects, the extracellular domain specifically binds to an antigen comprising a neoepitope comprising an amino acid sequence identified in **Table 1** in complex with a MHC class I protein.

**Table 1: Conservative substitution of AIQ neoepitope**

| Sequence name | Sequence | | | | | | | | | SEQ ID NO |
|---|---|---|---|---|---|---|---|---|---|---|
| AIQ residues | $X_1$ | $X_2$ | $X_3$ | $X_4$ | $X_5$ | $X_6$ | $X_7$ | $X_8$ | $X_9$ | |

(continued)

| Sequence name | Sequence | | | | | | | | | SEQ ID NO |
|---|---|---|---|---|---|---|---|---|---|---|
| AIQ neoepitope | A | I | Q | D | L | C | L | A | V | 1 |
| Conservative substitution of residue $X_1$ | **V** | I | Q | D | L | C | L | A | V | 77 |
| | **L** | I | Q | D | L | C | L | A | V | 78 |
| | **I** | I | Q | D | L | C | L | A | V | 79 |
| Conservative substitution of residue $X_3$ | A | I | **N** | D | L | C | L | A | V | 80 |
| Conservative substitution of residue $X_4$ | A | I | Q | **E** | L | C | L | A | V | 81 |
| Conservative substitution of residue $X_5$ | A | I | Q | D | **I** | C | L | A | V | 82 |
| | A | I | Q | D | **V** | C | L | A | V | 83 |
| | A | I | Q | D | **M** | C | L | A | V | 84 |
| | A | I | Q | D | **A** | C | L | A | V | 85 |
| | A | I | Q | D | **F** | C | L | A | V | 86 |
| Conservative substitution of residue $X_6$ | A | I | Q | D | L | **S** | L | A | V | 87 |
| | A | I | Q | D | L | **A** | L | A | V | 88 |
| Conservative substitution of residue $X_7$ | A | I | Q | D | L | C | **I** | A | V | 89 |
| | A | I | Q | D | L | C | **V** | A | V | 90 |
| | A | I | Q | D | L | C | **M** | A | V | 91 |
| | A | I | Q | D | L | C | **A** | A | V | 92 |
| | A | I | Q | D | L | C | **F** | A | V | 93 |
| Conservative substitution of residue $X_8$ | A | I | Q | D | L | C | L | **V** | V | 94 |
| | A | I | Q | D | L | C | L | **L** | V | 95 |
| | A | I | Q | D | L | C | L | **I** | V | 96 |

In some aspects, the extracellular domain specifically binds to the amino acid sequence AIQDLCLAV (SEQ ID NO:1) in complex with a class I major histocompatibility complex (MHC class I) protein, wherein any one, two, three, four, five or six amino acids of the amino acid sequence AIQDLCLAV (SEQ ID NO:1) are substituted, wherein the extracellular domain had the same or substantially the same binding affinity to the amino acid sequence AIQDLCLAYV (SEQ ID NO:1) in complex with the MHC class I protein. In some aspects, the extracellular domain specifically binds to the amino acid sequence AIQDLCLAV (SEQ ID NO:1) in complex with a class I major histocompatibility complex (MHC class I) protein, wherein any one, two, three, four, five or six amino acids of the amino acid sequence AIQDLCLAV (SEQ ID NO:1) are substituted, wherein the extracellular domain specifically binds with the same or better affinity than to the amino acid sequence AIQDLCLAYV (SEQ ID NO:1) in complex with the MHC class I protein. In some aspects, the extracellular domain binds to the amino acid sequence AIQDLCLAV (SEQ ID NO:1) in complex with a class I major histocompatibility complex (MHC class I) protein, wherein any one, two, three, four, five or six amino acids of the amino acid sequence AIQDLCLAV (SEQ ID NO:1) are substituted, wherein the antibody or antigen binding fragment has a KD of 0.1 to 100 nM (e.g., 0.1 to 50 nM, 0.1 to 25 nM, 1 0.1 to 15 nM). In some aspects, the extracellular domain binds to the amino acid sequence AIQDLCLAV (SEQ ID NO:1) in complex with a class I major histocompatibility complex (MHC class I) protein, wherein any one, two, three, four, five or six amino acids of the amino acid sequence AIQDLCLAV (SEQ ID NO: 1) are substituted, wherein the extracellular domain binds with a $K_D$ of less than 100 nM (e.g., less than 50 nM, less than 25 nM, less than 15 nM, less than 7 nM, less than 6 nM, less than 5 nM, less than 4 nM, less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, less than 0.2 nM, or less than 0.1 nM).

In some aspects, the extracellular domain binds to an NPM1c epitope presented by an MHC class I protein such as HLA-A2 (NPM1c:HLA-A2) and have an anti-cancer or anti-tumor effect (e.g., an anti-cancer effect in vivo, optionally, wherein the cancer is AML).

In some aspects, the extracellular domain specifically binds to an antigen comprising an NPM1c neoepitope in complex with a class I major histocompatibility complex (MHC class I) protein, wherein the extracellular domain comprises a heavy chain variable region (VH) and a light chain variable region (VL). In some aspects, the neoepitope comprises an amino acid sequence comprising AIQDLCLAV (SEQ ID NO:1). In some aspects, the MHC class I protein is encoded by an HLA-A allele comprising the HLA-A*02 allele group. In some aspects, the HLA-A allele is HLA-A*02:01.

In some aspects, the extracellular domain comprises an anti-NPM1c:HLA-A2 antibody or antigen binding fragment thereof having heavy chain variable regions and/or light chain variable regions described herein. In some aspects, the extracellular domain comprises an anti-NPM1c:HLA-A2 antibody or antigen binding fragment thereof having one or more complementarity determining regions (CDRs) described herein (e.g., having CDRs of NPM1c scFv, see, e.g., Sequences section and the Examples). In some aspects, the extracellular domain that binds to NPM1c:HLA-A2 comprises an scFv. An exemplary amino acid sequence for an scFv that specifically binds to NPM1c:HLA-A2 is set forth in SEQ ID NO: 2. In some aspects, the scFv comprises an amino acid sequence having at least 75%, at least 80%, at least 85%, at least 90%, at least 95%, at least 98% or at least 99% identity to the amino acid sequence set forth in SEQ ID NO: 2. In some aspects, the scFv comprises an amino acid sequence having at least 75%, 80%, at least 85%, at least 90%, at least 95%, at least 98% or at least 99% identity to the amino acid sequence set forth in SEQ ID NO: 2, wherein at least 95% of the differences in identity with the amino acid sequence set forth in SEQ ID NO: 2 are in the framework regions (or not in the complementarity determining regions (CDRs)) of the scFv.

In some aspects, the extracellular domain comprises an anti-NPM1c:HLA-A2 antibody or antigen binding fragment thereof comprising a heavy chain variable region (VH) having the amino acid sequence SEQ ID NO: 5. In some aspects, the extracellular domain comprises an anti-NPM1c:HLA-A2 antibody or antigen binding fragment thereof comprising a heavy chain variable region (VH) having at least 75%, at least 80%, at least 85%, at least 90%, at least 95%, at least 98% or at least 99% amino acid sequence identity to the amino acid sequence set forth in SEQ ID NO:5. In some aspects, the VH comprises an amino acid sequence having at least 75%, 80%, at least 85%, at least 90%, at least 95%, at least 98% or at least 99% identity to the amino acid sequence set forth in SEQ ID NO: 5, wherein at least 95% of the differences in identity with the amino acid sequence set forth in SEQ ID NO: 5 are in the framework regions (or not in the complementarity determining regions (CDRs)) of the VH.

In some aspects, the extracellular domain comprises an anti-NPM1c:HLA-A2 antibody or antigen binding fragment thereof comprising a light chain variable region (VL) having the amino acid sequence SEQ ID NO:3 (the amino acid sequence of the VL of NPM1c scFv). In some aspects, the extracellular domain comprises an anti-NPM1c:HLA-A2 antibody or antigen binding fragment thereof comprising a light chain variable region (VL) comprising an amino acid sequence having at least 75%, at least 80%, at least 85%, at least 90%, at least 95%, at least 98% or at least 99% identity to the amino acid sequence set forth in SEQ ID NO:3 . In some aspects, the VL comprises an amino acid sequence having at least 75%, 80%, at least 85%, at least 90%, at least 95%, at least 98% or at least 99% identity to the amino acid sequence set forth in SEQ ID NO: 3, wherein at least 95% or all of the differences in identity with the amino acid sequence set forth in SEQ ID NO: 3 are in the framework regions (or not in the complementarity determining regions (CDRs)) of the VL.

In some aspects, the extracellular domain comprises an anti-NPM1c:HLA-A2 antibody or antigen binding fragment thereof comprising a heavy chain variable region (VH) having the amino acid sequence SEQ ID NO: 5, and a light chain variable region (VL) having the amino acid sequence SEQ ID NO: 3. In some aspects, the extracellular domain comprises an anti-NPM1c:HLA-A2 antibody or antigen binding fragment thereof comprising a heavy chain variable region (VH) comprising an amino acid sequence having at least 75%, at least 80%, at least 85%, at least 90%, at least 95%, at least 98% or at least 99% identity to the amino acid sequence set forth in SEQ ID NO:5, and a light chain variable region (VL) comprising an amino acid sequence having at least 75%, at least 80%, at least 85%, at least 90%, at least 95%, at least 98% or at least 99% identity to the amino acid sequence set forth in SEQ ID NO: 3. In some aspects, the VH and VL each comprise amino acid sequences having at least 75%, 80%, at least 85%, at least 90%, at least 95%, at least 98% or at least 99% identity to the amino acid sequence set forth in SEQ ID NO: 5 and SEQ ID NO: 3, respectively, wherein at least 95% or all of difference in identity with the amino acid sequence set forth in SEQ ID NO: 5 and SEQ ID NO: 3 are in the framework regions (or not in the complementarity determining regions (CDRs)) of the VH and the VL.

CDRs of the antigen binding fragments of the disclosure are defined in various ways in the art, including the Kabat, Chothia, AbM, Contact, and IMGT.

In some aspects, the CDRs are defined according to the Kabat system, which is based on sequence variability (see, e.g., Kabat EA & Wu TT (1971) Ann NY Acad Sci 190: 382-391; Kabat EA et al, (1991) Sequences of Proteins of Immunological Interest, Fifth Edition, U.S. Department of Health and Human Services, NIH Publication No. 91-3242. The Kabat CDR positions are determined according to methods known in the art. In one aspect, the CDRs of the antibodies and fragments thereof described herein are determined using the Kabat system. In some aspects, the extracellular domain comprises an anti-NPM1c:HLA-A2 antibody or antigen binding fragment thereof having one or more complementarity determining regions (CDRs) of NPM1c scFv as determined using the Kabat system.

In some aspects, the CDRs are defined according to the Chothia system, which is based on the location of immunoglobulin structural loop regions (see, e.g., Chothia C & Lesk AM, (1987), J Mol Biol 196: 901-917; Al-Lazikani B et al., (1997) J Mol Biol 273 : 927-948; Chothia C et al, (1992) J Mol Biol 227: 799-817; Tramontano A et al, (1990) J Mol Biol 215(1): 175-82; and U.S. Patent No. 7,709,226). The term "Chothia CDRs," and like terms are recognized in the art and refer to antibody CDR sequences as determined according to the method of Chothia and Lesk, 1987, J. Mol. Biol., 196:901-917, which is referred to herein as the "Chothia CDRs" (see also, e.g., U.S. Patent No. 7,709,226 and Martin, A., "Protein Sequence and Structure Analysis of Antibody Variable Domains," in Antibody Engineering, Kontermann and Diibel, eds., Chapter 31, pp. 422-439, Springer- Verlag, Berlin (2001)). The Chothia CDR positions are determined according to methods known in the art. In some aspects, the CDRs are determined using the Chothia system. In some aspects, the extracellular domain comprise an anti-NPM1c:HLA-A2 antibody or antigen binding fragment thereof having one or more complementarity determining regions (CDRs) of NPM1c scFv as determined using the Chothia system.

In some aspects, the CDRs are defined according to the AbM system, which is based on AbM hypervariable regions that represent a compromise between the Kabat CDRs and Chothia structural loops, and where CDRs are determined using OxfordMolecular's AbM antibody modeling software (Oxford Molecular Group, Inc.). The AbM CDR positions is determined according to methods known in the art. In one aspect, the CDRs are determined using the AbM system. In some aspects, the extracellular domain comprises an anti-NPM1c:HLA-A2 antibody or antigen binding fragment thereof having one or more complementarity determining regions (CDRs) of NPM1c scFv as determined using the AbM system.

In some aspects, the CDRs are defined according to the IMGT system (see "IMGT®, the international ImMunoGeneTics information system® website imgt.org, founder and director: Marie-Paule Lefranc, Montpellier, France; see, e.g., Lefranc, M.-P., 1999, The Immunologist, 7: 132-136 and Lefranc, M.-P. et al., 1999, Nucleic Acids Res., 27:209-212, both of which are incorporated herein by reference in their entirety). The IMGT CDR positions are determined according to methods known in the art. In one aspect, the CDRs are determined using the IMGT system. In some aspects, the extracellular domain comprises an anti-NPM1c:HLA-A2 antigen binding fragment having one or more complementarity determining regions (CDRs) of NPM1c scFv as determined using the IMGT system.

In some aspects, the CDRs are defined according to the Contact system. The Contact definition is based on an analysis of the available complex crystal structures (bioinf.org.uk/abs) (see MacCallum RM et al., (1996) J Mol Biol 5: 732-745; see also, e.g., Martin A. "Protein Sequence and Structure Analysis of Antibody Variable Domains," in Antibody Engineering, Kontermann and Diibel, eds., Chapter 31, pp. 422-439, Springer- Verlag, Berlin (2001)). The Contact CDR positions are determined according to methods known in the art. In one aspect, the CDRs are determined using the Contact system. In some aspects, the extracellular domain comprises an anti-NPM1c:HLA-A2 antigen binding fragment having one or more complementarity determining regions (CDRs) of NPM1c scFv as determined using the Contact system.

In some aspects , the extracellular domain comprises an antigen binding fragment that specifically binds to an NPM1c epitope presented by HLA-A2 and comprises one, two, or three VH CDRs and/or one, two, or three VL CDRs of NPM1c scFv as defined according to any of the above-described systems. For example, in some aspects, the extracellular domain comprises an antigen binding fragment that specifically binds to an NPM1c epitope presented by HLA-A2 and comprise one, two, or all three VH CDRs and/or one, two, or all three VL CDRs of NPM1c scFv as defined by IMGT.

As is known in the art, VHs and VLs contain CDRs surrounded by framework regions (the CDRs and FR sequences appear in the following sequence in the VH and VL: FR1-CDR1-FR2-CDR2-FR3-CDR3-FR4). Optionally, the framework regions are human framework regions.

In some aspects, the extracellular domain comprises an anti-NPM1c:HLA-A2 antigen binding fragment comprising a heavy chain variable region (VH) having one, two or all three VH CDRs of a VH having the amino acid sequence SEQ ID NO:5 . In some aspects, the extracellular domain comprises an anti-NPM1c:HLA-A2 antigen binding fragment comprising a heavy chain variable region (VH) having one, two, or all three VH CDRs of a VH having the amino acid sequence SEQ ID NO: 5 as defined by IMGT.

In some aspects, the extracellular domain comprises an anti-NPM1c:HLA-A2 antigen binding fragment comprising a light chain variable region (VL) having one, two or all three VL CDRs of a VL having the amino acid sequence SEQ ID NO: 3. In some aspects, the extracellular domain comprises an anti-NPM1c:HLA-A2 antigen binding fragment comprising a light chain variable region (VL) having one, two or all three VL CDRs of a VL having the amino acid sequence SEQ ID NO: 3 as defined by IMGT.

In some aspects, the extracellular domain comprises an anti-NPM1c:HLA-A2 antigen binding fragment comprising a heavy chain variable region (VH) having one, two or all three VH CDRs of a VH having the amino acid sequence SEQ ID NO: 5 , and a light chain variable region (VL) having one, two or all three VL CDRs of a VL having the amino acid sequence SEQ ID NO: 3.

In some aspects, the extracellular domain comprises an anti-NPM1c:HLA-A2 antigen binding fragment comprising a heavy chain variable region (VH) having VH CDR1 of amino acid sequence SEQ ID NO: 9, VH CDR2 of amino acid sequence SEQ ID NO: 10, and/or VH CDR3 of amino acid sequence SEQ ID NO: 11. In some aspects, the extracellular domain comprises an anti-NPM1c:HLA-A2 antigen binding fragment comprising a heavy chain variable region (VH) having VH CDR1 of amino acid sequence SEQ ID NO: 9, VH CDR2 of amino acid sequence SEQ ID NO: 10, and VH CDR3 of amino acid sequence SEQ ID NO: 11, wherein one, two, three, four or five amino acids of SEQ ID NO: 9, SEQ ID NO: 10, or SEQ ID NO: 11 have been substituted. In some aspects, the amino acid substation is a conservative substitution. In some aspects, the amino acid substitution is a substitution with an amino acid residue of a similar size. In certain aspects, the amino acid substitution does not affect (or does not substantially affect) or improves the binding to the antigen.

In some aspects, the extracellular domain comprises an anti-NPM1c:HLA-A2 antigen binding fragment comprising a light chain variable region (VL) having VL CDR1 of amino acid sequence SEQ ID NO: 6, VL CDR2 of amino acid sequence SEQ ID NO: 7, and/or VL CDR3 of amino acid sequence SEQ ID NO: 8. In some aspects, the extracellular domain comprises an anti-NPM1c:HLA-A2 antigen binding fragment comprising a light chain variable region (VL) having VL CDR1 of amino acid sequence SEQ ID NO:6, VL CDR2 of amino acid sequence SEQ ID NO:7, and/or VL CDR3 of amino acid sequence SEQ ID NO:8, wherein one, two, three, four or five amino acids of SEQ ID NO:6, SEQ ID NO:7, or SEQ ID NO:8 have been substituted. In some aspects, the amino acid substation is a conservative substitution. In some aspects, the amino acid substitution is a substitution with an amino acid residue of a similar size. In certain aspects, the amino acid substitution does not affect (or does not substantially affect) or improves the binding to the antigen.

In some aspects, the extracellular domain comprises an anti-NPM1c:HLA-A2 antigen binding fragment comprising a heavy chain variable region (VH) having VH CDR1 of amino acid sequence SEQ ID NO:9, VH CDR2 of amino acid sequence SEQ ID NO:10, and VH CDR3 of amino acid sequence SEQ ID NO:11, and/or a light chain variable region (VL) having VL CDR1 of amino acid sequence SEQ ID NO:6, VL CDR2 of amino acid sequence SEQ ID NO:7, and VL CDR3 of amino acid sequence SEQ ID NO:8. In certain aspects, the extracellular domain comprises an anti-NPM1c:HLA-A2 antigen binding fragment comprising a heavy chain variable region (VH) having VH CDR1 of amino acid sequence SEQ ID NO:9, VH CDR2 of amino acid sequence SEQ ID NO:10, and VH CDR3 of amino acid sequence SEQ ID NO:11, and a light chain variable region (VL) having VL CDR1 of amino acid sequence SEQ ID NO:6, VL CDR2 of amino acid sequence SEQ ID NO:7, and VL CDR3 of amino acid sequence SEQ ID NO:8. In some aspects, the extracellular domain comprises an anti-NPM1c:HLA-A2 antigen binding fragment comprising a VH and a VL described herein, wherein one, two, three, four or five amino acids of the VH and/or a VL CDRs have been substituted.

In some aspects, one or more CDRs in the VH and/or VL region described herein may vary by one, two, three, four or five amino acids as long as specific binding to NPM1c:HLA-A2 is maintained.

In some aspects, an antibody fragment provided herein has been affinity matured, i.e., has one or more alterations in one or more complementarity determining regions compared to the described antibody or fragment, wherein such one or more alterations result in an improvement in the affinity of the antibody or fragment to the antigen relative to the described antibody or fragment. In some aspects, the antibodies or fragments provided herein have a Kd to the antigen (e.g., NPM1c:HLA-A2) of less than 100 nM (e.g., less than 50 nM, less than 25 nM, less than 15 nM, less than 7 nM, less than 6 nM, less than 5 nM, less than 4 nM, less than 3 nM, less than 2 nM, less than 1 nM, less than 0.9 nM, less than 0.8 nM, less than 0.7 nM, less than 0.6 nM, less than 0.5 nM, less than 0.4 nM, less than 0.3 nM, less than 0.2 nM, or less than 0.1 nM). In some aspects, the antibodies or fragments provided herein have a Kd to the antigen (e.g., NPM1c:HLA-A2) of less than 15 nM, less than 10 nM, less than 7 nM, less than 5 nM or less than 1 nM (e.g., 0.01 to 15 nM, 0.01 to 10 nM, 0.01 to 7 nM, 0.01 to 5 nM, 0.01 to 1 nM, 0.1 to 15 nM, 0.1 to 10 nM, 0.1 to 7 nM, 0.1 to 5 nM, 0.1 to 1 nM, 1 to 15 nM, 1 to 10 nM, 1 to 7 nM, 1 to 5 nM, 5 to 15 nM, 5 to 10 nM, or 5 to 7 nM).

In some aspects, the extracellular domain comprises an antigen binding fragment comprising three light chain variable region complementarity determining regions (VL CDRs 1-3) and three heavy chain variable region complementarity determining regions (VH CDRs 1-3). In some aspects, the VH CDR1 comprises amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequence set forth in SEQ ID NO: 9. In some aspects, the VH CDR2 comprises an amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequence set forth in SEQ ID NO: 10. In some aspects, the VH CDR3 comprises an amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequence set forth in SEQ ID NO: 11.

In some aspects, the VL CDR1 comprises an amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequences set forth in SEQ ID NO: 6. In some aspects, the VL CDR2 comprises an amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequence set forth in SEQ ID NO: 7. In some aspects, the VL CDR3 comprises an amino acid sequence having at least 80% sequence identity, or at least 81% sequence identity, or at least 82% sequence identity, or at least 83% sequence identity, or at least 84% sequence identity, or at least 85% sequence identity, or at least 86% sequence identity, or at least 87% sequence identity, or at least 88% sequence identity, or at least 89% sequence identity, or at least 90% sequence identity, or at least 91% sequence identity, or at least 92% sequence identity, or at least 93% sequence identity, or at least 94% sequence identity, or at least 95% sequence identity, or at least 96% sequence identity, or at least 97% sequence identity, or at least 98% sequence identity, or at least 99% sequence identity to the amino acid sequence set forth in SEQ ID NO: 8.

In some aspects, the extracellular domain comprises an antigen binding fragment having a binding affinity (Kd) to the antigen (e.g., NPM1c:MHC class I) of at least $10^{-7}$M. In certain aspects, the antigen binding fragment has a binding affinity (Kd) to the NPM1c:MHC class I antigen (e.g., NPM1c:HLA-A2) of at least $10^{-7}$ M or higher, at least $10^{-8}$ M or higher, at least $10^{-9}$ M or higher, at least 500 nM or higher, at least 250 nM or higher, at least 100 nM or higher, at least 50 nM or higher, at least 25 nM or higher, at least 20 nM or higher, at least 15 nM or higher, or at least 10 nM or higher. In some aspects, the antibody or antigen binding fragment thereof has a binding affinity (Kd) to the NPM1c:MHC class I antigen (e.g., NPM1c:HLA-A2) of at least about 25 nM or higher, at least about 15 nM or higher, or at least about 10 nM or higher. In some aspects, the antigen binding fragment has a binding affinity (Kd) to the NPM1c:HLA-A2 antigen has a binding affinity (Kd) to the NPM1c:MHC class I antigen (e.g., NPM1c:HLA-A2) between (or from and to) 0.1 nM and 500 nM, 0.1 nM and 100 nM, 0.5 nM and 100 nM, 0.1 nM and 50 nM, 0.5 nM and 50 nM, 0.1 nM and 25 nM, 0.5 nM and 25 nM. 0.1 nM and 15 nM, 0.5 nM and 15 nM, 0.1 nM and 10 nM, or 0.5 nM and 10 nM, or 1 nM to 100 nM (or any value in between). In some aspects, the antigen binding fragment has a binding affinity (Kd) to the NPM1c:MHC class I antigen (e.g., NPM1c:HLA-A2) between (or from and to) about 0.1 nM and about 100 nM or about 0.5 nM to about 100 nM. In some aspects, the antigen binding fragment has a binding affinity (Kd) to the NPM1c:MHC class I antigen (e.g., NPM1c:HLA-A2) between (or from and to) about 0.1 nM and about 50 nM or about 0.5 nM to about 50 nM.

In some aspects, the extracellular domain comprises an antigen binding fragment having a Kon for the NPM1c:MHC class I antigen (e.g., NPM1c:HLA-A2) of at least $0.5 \pm 0.02 \times 10^4$ Ms$^{-1}$ or higher. In some aspects, the antigen binding fragment described herein has a Kon for the NPM1c:MHC class I antigen (e.g., NPM1c:HLA-A2) of at least $1 \pm 0.02 \times 10^4$ Ms$^{-1}$ or higher. In some aspects, the antigen binding fragment described herein has a Kon for the NPM1c:MHC class I antigen (e.g., NPM1c:HLA-A2) of at least $2.5 \pm 0.02 \times 10^4$ Ms$^{-1}$ or higher. In some aspects, the antigen binding fragment described herein has a Kon for the NPM1c:MHC class I antigen (e.g., NPM1c:HLA-A2) of at least $5 \pm 0.02 \times 10^4$ Ms$^{-1}$ or higher. In some aspects, the antigen binding fragment described herein has a Kon for the NPM1c:MHC class I antigen (e.g., NPM1c:HLA-A2) between (or from and to) $0.5 \pm 0.02 \times 10^4$ Ms$^{-1}$ and $50 \pm 0.02 \times 10^4$ Ms$^{-1}$. In some aspects, the antigen binding fragment described herein has a Kon for the NPM1c:MHC class I antigen (e.g., NPM1c:HLA-A2) between (or from and to) $1 \pm 0.02 \times 10^4$ Ms$^{-1}$ and $10 \pm 0.02 \times 10^4$ Ms$^{-1}$.

In some aspects, the extracellular domain comprises an antigen binding fragment having a Koff for the NPM1c:MHC class I antigen (e.g., NPM1c:HLA-A2) of less than $50 \pm 0.02 \times 10^{-4}$s$^{-1}$. In some aspects, the antigen binding fragment described herein has a Koff for the NPM1c:MHC class I antigen (e.g., NPM1c:HLA-A2) of less than $10 \pm 0.02 \times 10^{-4}$s$^{-1}$. In some aspects, the antigen binding fragment described herein has a Koff for the NPM1c:MHC class I antigen (e.g., NPM1c:HLA-A2) of less than $5 \pm 0.02 \times 10^{-4}$s$^{-1}$. In some aspects, the antigen binding fragment described herein has a Koff for the NPM1c:MHC class I antigen (e.g., NPM1c:HLA-A2) between (or from and to) $0.5 \pm 0.02 \times 10^{-4}$s$^{-1}$ and $50 \pm 0.02 \times 10^{-4}$s$^{-1}$. In some aspects, the antigen binding fragment described herein has a Koff for the NPM1c:MHC class I antigen (e.g., NPM1c:HLA-A2) between (or from and to) $1 \pm 0.02 \times 10^{-4}$s$^{-1}$ and $15 \pm 0.02 \times 10^{-4}$s$^{-1}$.

Antigen binding molecules provided herein specifically bind to the antigen (such as NPM1c:HLA-A2). In some aspects, the antigen bound by the antigen binding molecule, is presented by an MHC class I molecule (e.g., HLA-A2) on the surface of a cancer cell. In some aspects, the cancer cell is an AML cell.

In some aspects, the extracellular domain comprises a single chain antibody, e.g., a single chain Fv (scFv). In some aspects, the scFv is a human or humanized scFv. In some aspects, the scFv comprises a linker. In some aspects, the linker is a peptide linker. In some aspects, the peptide linker is a Gly-Ser linker. In some aspects, the Gly-Ser linker is selected from the group consisting of (Gly4Ser)1 (SEQ ID NO:58), (Gly4Ser)2 (SEQ ID NO: 59), (Gly4Ser)3 (SEQ ID NO: 60), and (Gly4Ser)4 (SEQ ID NO: 61). In some aspects, the Gly-Ser linker comprises the amino acid sequence SGSSGGSSSG (SEQ ID NO:4). In some aspects, the extracellular domain comprises an antigen binding fragment of an antibody, where the fragment can be, without limitation an Fv fragment, a Fab fragment, a F(ab') fragment, a F(ab')$_2$ fragment, or a disulfide-linked Fv (sdFv). In one aspect, the extracellular domain comprises an Fv fragment. In oneaspect, aspects, the extracellular domain comprises a Fab fragment. In one aspects, the extracellular domain comprises a F(ab') fragment. In one aspect, the extracellular domain comprises a F(ab')$_2$ fragment.

### Transmembrane Domains

In some aspects, the CAR polypeptides provided herein comprise a transmembrane domain. In some aspects, the transmembrane domain is derived from a natural source. In some aspects, embediments- the transmembrane domain is derived from any membrane-bound or transmembrane protein. Non-limiting examples of transmembrane domains that may be used in CARs described herein may be derived from (e.g., comprise at least the transmembrane sequence or a part of the transmembrane sequence of) the alpha, beta, or zeta chain of the T-cell receptor, CD28, CD3 epsilon, CD33, CD37, CD64, CD80, CD45, CD4, CD5, CDS, CD9, CD16, CD22, CD86, CD 134, CD137, or CD154. In one aspect, the transmembrane domain is from a CD4 molecule. In one aspect, the transmembrane domain is from a CD8 molecule.

In some aspects, the transmembrane domain is synthetic. For example, in some aspects where the transmembrane domain is from a synthetic source, the transmembrane domain may include (e.g., predominantly include) hydrophobic residues (e.g., leucine and valine). In some aspects, the synthetic transmembrane domain includes at least one (e.g., at least two, at least three, at least four, at least five, or at least six) triplet of phenylalanine, tryptophan, and valine at the end of a synthetic transmembrane domain. In some aspects, the transmembrane domain of a CAR includes a CD8 hinge domain.

In some aspects the transmembrane domain is naturally associated with a sequence in the cytoplasmic domain. In some aspects, the transmembrane domain is modified by one or more (e.g., two, three, four, five, six, seven, eight, nine, or ten) amino acid substitutions to avoid the binding of the domain to other transmembrane domains (e.g., the transmembrane domains of the same or different surface membrane proteins) to minimize interactions with other members of the receptor complex.

In some aspects, the transmembrane domain of a CAR polypeptide described herein comprises the CD8 hinge and transmembrane regions. In some aspects, the transmembrane domain comprises the amino acid sequence set forth in SEQ ID NO: 25. In some aspects, the transmembrane domain comprises an amino acid sequence having at least 90%, 95%, 96%, 97%, 98%, or 99% identity to the amino acid sequence set forth in SEQ ID NO: 25. In some aspects, the transmembrane domain is encoded by the nucleotide sequence set forth in SEQ ID NO: 33. In some aspects, the transmembrane domain is encoded by a nucleotide sequence having at least 80%, 85%, 90%, 95%, 96%, 97%. 98%, or 99% identity to the nucleotide sequence set forth in SEQ ID NO: 33.

In some aspects, the transmembrane domain of CARs provided herein comprises the transmembrane domain of CD3-zeta, CD8, CD28, DAP12, 2B4, NKG2D, CD16, NKp44, FcYRIIIa, NKp30, actKIR, NKG2C, IL15Rb, or NKp46. In some aspects, the transmembrane domain of CARs provided herein comprises the transmembrane domain of CD3-zeta, CD8 or CD28. In some of these aspects, the intracellular domain of the CAR comprises a costimulatory domain of a costimulatory molecule selected from the group consisting of: CD27, CD28, 4-1BB, OX40, CD30, CD40, PD-1, ICOS, and any combination thereof.

### Intracellular (Cytoplasmic) Domains

In some aspects, the CAR polypeptides described herein comprise an intracellular domain. The intracellular domain can be any polypeptide domain known to function to transmit a signal causing, for example, activation of an immune effector cell such as a NK cell or a ML NK cell. Such a domain or motif may transmit a primary antigen-binding signal that is necessary for the activation of a T lymphocyte in response to the binding of the extracellular domain of the CAR to the target antigen. Examples of intracellular domains include, without limitation, ILR chain, CD28, 4-1BB and CD3ζ.

Typically, the intracellular domain comprises an ITAM (immunoreceptor tyrosine-based activation motif).

In one aspect the intracellular domain is or comprises a CD3 ζ signaling sequence (e.g., an ITAM-containing portion thereof). In one aspect, the intracellular domain comprises a lymphocyte receptor chain. In one aspect, the intracellular domain comprises a TCR/CDR3 complex protein. In one aspect, the intracellular domain comprises an Fc receptor subunit. In one aspect, the intracellular domain comprises an IL-2 receptor subunit.

The intracellular domain of CARs provided herein can include two distinct classes of cytoplasmic signaling sequences: signaling sequences that initiate antigen-dependent activation through the TCR (primary cytoplasmic signaling sequences) (e.g., a CD3ζ cytoplasmic signaling sequence) and a cytoplasmic sequence of one or more of co-stimulatory proteins that act in an antigen-independent manner to provide a secondary or co-stimulatory signal (secondary cytoplasmic signaling sequences).

In certain aspects, provided herein are CARs that comprise an intracellular signaling domain that includes a cytoplasmic sequence of CD3ζ sufficient to stimulate a T cell when the antigen binding domain binds to the antigen, and optionally, a cytoplasmic sequence of one or more of co-stimulatory proteins (e.g., a cytoplasmic sequence of one or more of CD27, CD28, 4-1BB, OX40, CD30, CD40L, CD40, PD-1, PD-L1, ICOS, LFA-1, CD2, CD7, CD160, LIGHT, BTLA, TIM3, CD244, CD80, LAG3, NKG2C, B7-H3, 2B4, DAP10, CD137, DAP12, DNAM-1, NKp80, NTBA, CRACC, CD2, CD3ζ one or more integrins, IL-15R, IL-18R, IL-12R, IL-21 R, IRE1a, a ligand that specifically binds to CD83, and any of the ITAM sequences described herein or known in the art) that provides for co-stimulation of the NK cell. In some aspects, the entire intracellular signaling domain of a co-stimulatory protein is included in the intracellular domain of a CAR. In some aspects, the intracellular domain includes a truncated portion of an intracellular signaling domain of a co-stimulatory protein (e.g., a truncated portion of the intracellular signaling domain that transduces an effector function signal in the CAR-expressing immune effector cell). In some aspects, the intracellular domain of a CAR can be designed to include the CD3ζ signaling domain by itself or combined with any other desired cytoplasmic signaling sequence(s) useful in the context of a CAR. In some aspects, the cytoplasmic domain of a CAR can include a CD3ζ chain portion and a costimulatory cytoplasmic signaling sequence. The costimulatory cytoplasmic signaling sequence refers to a portion of a CAR including a cytoplasmic signaling sequence of a costimulatory protein (e.g., CD27, CD28, 4-IBB (CD 137), OX40, CD30, CD40, PD-1, ICOS, lymphocyte function-associated antigen- 1 (LFA-1), CD2, CD7, LIGHT, NKG2C, B7-H3, and a ligand that specifically binds with CD83).

In some aspects, the cytoplasmic signaling sequences within the intracellular domain of a CAR are positioned in a random order. In some aspects, the cytoplasmic signaling sequences within the intracellular domain of a CAR are linked to each other in a specific order. In some aspects, a linker (e.g., any of the linkers described herein) can be used to form a linkage between different cytoplasmic signaling sequences.

In some aspects, the intracellular domain is designed to include the cytoplasmic signaling sequence of CD3ζ and the cytoplasmic signaling sequence of the costimulatory protein CD28. In some aspects, the intracellular domain is designed to include the cytoplasmic signaling sequence of CD3ζ and the cytoplasmic signaling sequence of costimulatory protein 4-IBB. In some aspects, the intracellular domain is designed to include the cytoplasmic signaling sequence of CD3ζ and the cytoplasmic signaling sequences of costimulatory proteins CD28 and 4-1BB. In some aspects, the intracellular domain does not include the cytoplasmic signaling sequences of 4-1BB.

In some aspects, the CAR polypeptide comprises the amino acid sequence set forth in SEQ ID NO: 26. In some aspects, the CAR polypeptide comprises an amino acid sequence having at least 90%, 95%, 96%, 97%, 98%, or 99% identity to the amino acid sequence set forth in SEQ ID NO: 26. In some aspects, the CAR polypeptide comprises the amino acid sequence set forth in SEQ ID NO: 27. In some aspects, the CAR polypeptide comprises an amino acid sequence having at least 90%, 95%, 96%, 97%, 98%, or 99% identity to the amino acid sequence set forth in SEQ ID NO: 27.

In some aspects, the CAR polypeptide comprises the amino acid sequences set forth in SEQ ID NOs: 26 and 27. In some aspects, the CAR polypeptide comprises amino acid sequences having at least 90%, 95%, 96%, 97%, 98%, or 99% identity to the amino acid sequences set forth in SEQ ID NOs: 26 and 27.

In some aspects, a nucleotide sequence encoding the CAR polypeptide comprises the nucleic acid sequence set forth in SEQ ID NO: 34. In some aspects, a nucleotide sequence encoding the CAR polypeptide comprises a nucleic acid sequence having at least 80%, 85%, 90%, 95%, 96%, 97%, 98%, or 99% identity to the nucleic acid sequence set forth in SEQ ID NO: 34. In some aspects, a nucleotide sequence encoding the CAR polypeptide comprises the nucleic acid sequence set forth in SEQ ID NO: 35. In some aspects, a nucleotide sequence encoding the CAR polypeptide comprises a nucleic acid sequence having at least 80%, 85%, 90%, 95%, 96%, 97%, 98%, or 99% identity to the nucleic acid sequence set forth in SEQ ID NO: 35.

In some aspects, a nucleotide sequence encoding the CAR polypeptide comprises the nucleic acid sequences set forth in SEQ ID NOs: 34 and 35. In some aspects, a nucleotide sequence encoding the CAR polypeptide comprises nucleic acid sequences having at least 90%, 95%, 96%, 97%, 98%, or 99% identity to the nucleic acid sequences set forth in SEQ ID NOs: 34 and 35.

In some aspects, the CAR comprises one or more co-stimulatory domains derived from a protein such as CD28, CD137 (also known as 4-IBB), CD134 (also known as OX40) and CD278 (also known as ICOS). In some aspects, the CAR does not comprise a co-stimulatory domain derived from CD137.

In certain aspects, the intracellular domain further comprises a cytokine. In some aspects, the intracellular domain further comprises a self-cleaving domain (e.g., the P2A self-cleaving peptide) and a cytokine, wherein the cleavage of the self-cleaving domain releases the cytokine. In some aspects, the self-cleaving domain (e.g., the P2A self-cleaving peptide) and the cytokine are positioned at the C-terminal end of the CAR protein and its intracellular domain. In some aspects, the cytokine is one or more of the following: IL-12, IL-7, IL-13, IL-15, IL-4, IL-10, TNF-$\alpha$, IFN-$\gamma$, TGF-$\beta$ and CCL19. In one aspect, the cytokine is IL-12. In one aspect, the cytokine is IL-7. In one aspect, the cytokine is IL-13. In one aspect, the cytokine is IL-15. In one aspect, the cytokine is IL-4. In one aspect, the cytokine is IL-10. In one aspect, the cytokine is TNF-$\alpha$. In one aspect, the cytokine is IFN-$\gamma$. In one aspect, the cytokine is TGF-$\beta$. In one aspect, the cytokine is CCL19. Immune effector cells modified to express a cytokine are known in the art (see, e.g., Adachi et al, 2018, Nature Biotechnology, doi:10.1038/nbt.4086; Liu et al., 2019, J. Immunol., doi:10.4049/jimmunol.1800033; Krenciute et al., 2017, Cancer Immunol. Res. 597):571-581, doi:10.1158/2326-6066,CIR-16-0376; Liu et al., 2018, Leukemia 32:520-531). In certain aspects, the modification of immune effector cells described herein to express a cytokine is the same as that described in Adachi et al, 2018, Nature Biotechnology, doi:10.1038/nbt.4086; Liu et al., 2019, J. Immunol., doi:10.4049/jimmunol.1800033; Krenciute et al., 2017, Cancer Immunol. Res. 597):571-581, doi:10.1158/2326-6066,CIR-16-0376; or Liu et al., 2018, Leukemia 32:520-531, or in accordance with the methods described therein.

### Linkers Between CAR Domains

In some aspects, the CAR polypeptides described herein comprise a linker between at least one domain in the CAR. In some aspects, a CAR described herein includes a linker:

(1) between the extracellular (antigen binding) domain and the transmembrane domain, and/or (2) between the transmembrane domain and the intracellular (cytoplasmic) domain. In some aspects, the linker can be a polypeptide linker. For example, the linker can have a length of between about 1 amino acid and about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, about 12 amino acids, about 10 amino acids, about 8 amino acids, about 6 amino acids, about 4 amino acids, or about 2 amino acids; about 2 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, about 12 amino acids, about 10 amino acids, about 8 amino acids, about 6 amino acids, or about 4 amino acids; about 4 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, about 12 amino acids, about 10 amino acids, about 8 amino acids, or about 6 amino acids; about 6 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, about 12 amino acids, about 10 amino acids, or about 8 amino acids; about 8 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, about 12 amino acids, or about 10 amino acids; about 10 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, about 14 amino acids, or about 12 amino acids; about 12 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, about 16 amino acids, or about 14 amino acids; about 14 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, about 18 amino acids, or about 16 amino acids; about 16 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, about 20 amino acids, or about 18 amino acids; about 18 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, about 25 amino acids, or about 20 amino acids; about 20 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, about 30 amino acids, or about 25 amino acids; about 25 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, about 35 amino acids, or about 30 amino acids; about 30 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, about 40 amino acids, or about 35 amino acids; about 35 amino acids to about 500 amino acids, about 400 amino acids, about 300 amino acids, about 200 amino acids, about 100 amino acids, about 90 amino acids, about 80 amino acids, about 70 amino acids, about 60 amino acids, about 50 amino acids, or about 40 amino acids; about 40 amino acids to about 500 amino acids, about 400 amino acids, about 300 amino acids, about 200 amino acids, about 100 amino acids, about 90 amino acids, about 80 amino acids, about 70 amino acids, about 60 amino acids, or about 50 amino acids; about 50 amino acids to about 500 amino acids, about 400 amino acids, about 300 amino acids, about 200 amino acids, about 100 amino acids, about 90 amino acids, about 80 amino acids, about 70 amino acids, or about 60 amino acids; about 60 amino acids to about 500 amino acids, about 400 amino acids, about 300 amino acids, about 200 amino acids, about 150 amino acids, about 100 amino acids, about 90 amino acids, about 80 amino acids, or about 70 amino acids; about 70 amino acids to about 500 amino acids, about 400 amino acids, about 300 amino acids, about 200 amino acids, about 100 amino acids, about 90 amino acids, or about 80 amino acids; about 80 amino acids to about 500 amino acids, about 400 amino acids, about 300 amino acids, about 200 amino acids, about 100 amino acids, or about 90 amino acids; about 90 amino acids to about 500 amino acids, about 400 amino acids, about 300 amino acids, about 200 amino acids, or about 100 amino acids; about 100 amino acids to about 500 amino acids, about 400 amino acids, about 300 amino acids, or about 200 amino acids; about 200 amino acids to about 500 amino acids, about 400 amino acids, or about 300 amino acids; about 300 amino acids to about 500 amino acids or about 400 amino acids; or about 400 amino acids to about 500 amino acids.

## Methods of Making CARs

Methods for making CAR polypeptides and nucleic acid molecules encoding such polypeptides are known to those of skill in the art. Exemplary methods are described herein.

The antibodies and fragments suitable for use in the CARs described herein can be produced by any method known in the art.

Methods of making antibody fragments are well known in the art. For example, Fab and F(ab')2 fragments can be produced by proteolytic cleavage of immunoglobulin molecules using enzymes such as pepsin (to produce F(ab')2 fragments) or papain (to produce Fab fragments). Methods of making scFv fragments are also known in the art (see, e.g., Ahmad et al., 2012, Clinical and Developmental Immunology, doi: 10.1155/2012/980250; Wang et al., 2006, Anal. Chem. 78, 997-1004; Pansri et al., 2009, BMC Biotechnology 9:6; Chao et al., 2006, Nature Protocols 1(2):755-768). scFv having desired antigen-binding properties can be selected by phage display technology or yeast surface display technology. scFv can be constructed by fusing variable domains of heavy and light chains of immunoglobulins via short polypeptide linkers (using recombinant expression techniques). Methods of making single domain antibodies (e.g., antibodies lacking the light chains) are well known in the art (see, e.g., Riechmann & Muyldermans, 1999, J Immunol 231:25- 38; Nuttall et al, 2000, Curr Pharm Biotechnol 1(3):253-263; Muyldermans, 2001, J Biotechnol 74(4): 277-302).

Methods for producing bispecific antibodies are well known in the art (see, e.g., Konterman, 2012, MAbs 4: 182-197; Gramer et al., 2013, MAbs 5:962-973).

In aspects, where the selected CDR amino acid sequences are short sequences (e.g., fewer than 10-15 amino acids in length), nucleic acids encoding the CDRs can be chemically synthesized as described in, e.g., Shiraishi et al. (2007) Nucleic Acids Symposium Series 51(1):129-130 and U.S. Patent No. 6,995,259. For a given nucleic acid sequence encoding an acceptor antibody, the region of the nucleic acid sequence encoding the CDRs can be replaced with the chemically synthesized nucleic acids using standard molecular biology techniques. The 5' and 3' ends of the chemically synthesized nucleic acids can be synthesized to comprise sticky end restriction enzyme sites for use in cloning the nucleic acids into the nucleic acid encoding the variable region of the donor antibody.

Any of the antibodies described herein can be screened and/or tested for their ability to modulate any of the activities or functions ascribed to the antigen, e.g., NPM1c:HLA-A2, either *in vitro* or *in vivo,* using any immunological or biochemical-based methods known in the art.

The polypeptides described herein can be produced using a variety of techniques known in the art of molecular biology and protein chemistry. For example, a nucleic acid encoding one or both of the heavy and light chain polypeptides of an antibody can be inserted into an expression vector that contains transcriptional and translational regulatory sequences, which include, e.g., promoter sequences, ribosomal binding sites, transcriptional start and stop sequences, translational start and stop sequences, transcription terminator signals, polyadenylation signals, and enhancer or activator sequences. The regulatory sequences include a promoter and transcriptional start and stop sequences. In addition, the expression vector can include more than one replication system such that it can be maintained in two different organisms, for example in mammalian or insect cells for expression and in a prokaryotic host for cloning and amplification.

Several possible vector systems are available for the expression of any polypeptide described herein (e.g., cloned heavy chain and light chain polypeptides) from nucleic acids in mammalian cells. One class of vectors relies upon the integration of the desired gene sequences into the host cell genome. Cells which have stably integrated DNA can be selected by simultaneously introducing drug resistance genes such as *E. coli* gpt (Mulligan and Berg (1981) Proc Natl Acad Sci USA 78:2072) or *Tn5* neo (Southern and Berg (1982) Mol Appl Genet 1:327). The selectable marker gene can be either linked to the DNA gene sequences to be expressed, or introduced into the same cell by co-transfection (Wigler et al. (1979) Cell 16:77). A second class of vectors utilizes DNA elements which confer autonomously replicating capabilities to an extrachromosomal plasmid. These vectors can be derived from animal viruses, such as bovine papillomavirus (Sarver et al. (1982) Proc Natl Acad Sci USA, 79:7147), cytomegalovirus, polyoma virus (Deans et al. (1984) Proc Natl Acad Sci USA 81:1292), or SV40 virus (Lusky and Botchan (1981) Nature 293:79).

The expression vectors can be introduced into cells in a manner suitable for subsequent expression of the nucleic acid. The method of introduction is largely dictated by the targeted cell type, discussed below. Exemplary methods include CaPO4 precipitation, liposome fusion, cationic liposomes, electroporation, viral infection, dextran-mediated transfection, polybrene-mediated transfection, protoplast fusion, and direct microinjection.

Appropriate host cells for the expression of polypeptides include yeast, bacteria, insect, plant, and mammalian cells. Of particular interest are bacteria such as *E. coli,* fungi such as *Saccharomyces cerevisiae* and *Pichia pastoris,* insect cells such as SF9, mammalian cell lines (e.g., human cell lines), as well as primary cell lines.

The polypeptides can be produced from the cells by culturing a host cell transformed with the expression vector containing nucleic acid encoding the antibodies or fragments, under conditions, and for an amount of time, sufficient to allow expression of the proteins. Such conditions for protein expression will vary with the choice of the expression vector and the host cell, and will be easily ascertained by one skilled in the art through routine experimentation. For example, antibodies expressed in *E. coli* can be refolded from inclusion bodies (see, e.g., Hou et al. (1998) Cytokine 10:319-30). Bacterial expression systems and methods for their use are well known in the art (see Current Protocols in Molecular Biology, Wiley & Sons, and Molecular Cloning--A Laboratory Manual --3rd Ed., Cold Spring Harbor Laboratory Press, New York (2001)). The choice of codons, suitable expression vectors and suitable host cells will vary depending on a number of factors, and may be easily optimized as needed. An antibody (or fragment thereof) described herein can be expressed in mammalian cells or in other expression systems including but not limited to yeast, baculovirus, and *in vitro* expression systems (see, e.g., Kaszubska et al. (2000) Protein Expression and Purification 18:213-220).

Following expression, the polypeptides can be isolated. Standard purification methods include electrophoretic, molecular, immunological, and chromatographic techniques, including ion exchange, hydrophobic, affinity, and reverse-phase HPLC chromatography. For example, an antibody can be purified using a standard anti-antibody column (e.g., a protein-A or protein-G column). Ultrafiltration and diafiltration techniques, in conjunction with protein concentration, are also useful. See, e.g., Scopes (1994) "Protein Purification, 3rd edition," Springer-Verlag, New York City, New York. The degree of purification necessary will vary depending on the desired use. In some instances, no purification of the expressed antibody or fragments thereof will be necessary.

Methods for determining the yield or purity of a polypeptide are known in the art and include, e.g., Bradford assay, UV spectroscopy, Biuret protein assay, Lowry protein assay, amido black protein assay, high pressure liquid chromatography (HPLC), mass spectrometry (MS), and gel electrophoretic methods (e.g., using a protein stain such as Coomassie Blue or colloidal silver stain).

The polypeptides can be modified following their expression and purification. The modifications can be covalent or noncovalent modifications. Such modifications can be introduced into the polypeptides by, e.g., reacting targeted amino acid residues of the polypeptide with an organic derivatizing agent that is capable of reacting with selected side chains or terminal residues. Suitable sites for modification can be chosen using any of a variety of criteria including, e.g., structural analysis or amino acid sequence analysis of the antibodies or fragments.

In some aspects, the polypeptides can be conjugated to a heterologous moiety. The heterologous moiety can be, e.g., a heterologous polypeptide, a therapeutic or a cytotoxic agent (e.g., a toxin or a drug), or a detectable label such as, but not limited to, a radioactive label, an enzymatic label, a fluorescent label, a heavy metal label, a luminescent label, or an affinity tag such as biotin or streptavidin. Suitable heterologous polypeptides include, e.g., an antigenic tag (e.g., FLAG (DYKDDDDK (SEQ ID NO: 44)), polyhistidine (6-His; HHHHHH (SEQ ID NO: 45), hemagglutinin (HA; YPYDVPDYA (SEQ ID NO: 46)), glutathione-S-transferase (GST), or maltose-binding protein (MBP)) for use in purifying the antibodies or fragments. Heterologous polypeptides also include polypeptides (e.g., enzymes) that are useful as diagnostic or detectable markers, for example, luciferase, a fluorescent protein (e.g., green fluorescent protein (GFP)), or chloramphenicol acetyl transferase (CAT). Suitable radioactive labels include, e.g., 32P, 33P, 14C, 125I, 131I, 35S, and 3H. Suitable fluorescent labels include, without limitation, fluorescein, fluorescein isothiocyanate (FITC), green fluorescent protein (GFP), DyLight™ 488, phycoerythrin (PE), propidium iodide (PI), PerCP, PE-Alexa Fluor® 700, Cy5, allophycocyanin, and Cy7. Luminescent labels include, e.g., any of a variety of luminescent lanthanide (e.g., europium or terbium) chelates. For example, suitable europium chelates include the europium chelate of diethylene triamine pentaacetic acid (DTPA) or tetraazacyclododecane-1,4,7,10-tetraacetic acid (DOTA). Enzymatic labels include, e.g., alkaline phosphatase, CAT, luciferase, and horseradish peroxidase.

Two proteins (e.g., an antibody and a heterologous moiety) can be crosslinked using any of a number of known chemical cross linkers. Examples of such cross linkers are those which link two amino acid residues via a linkage that includes a "hindered" disulfide bond. In these linkages, a disulfide bond within the cross-linking unit is protected (by hindering groups on either side of the disulfide bond) from reduction by the action, for example, of reduced glutathione or the enzyme disulfide reductase. One suitable reagent, 4- succinimidyloxycarbonyl-α-methyl-α(2-pyridyldithio) toluene (SMPT), forms such a linkage between two proteins utilizing a terminal lysine on one of the proteins and a terminal cysteine on the other. Heterobifunctional reagents that cross-link by a different coupling moiety on each protein can also be used. Other useful crosslinkers include, without limitation, reagents which link two amino groups (e.g., N-5-azido-2-nitrobenzoyloxysuccinimide), two sulfhydryl groups (e.g., 1,4-bis-maleimidobutane), an amino group and a sulfhydryl group (e.g., mmaleimidobenzoyl-N-hydroxysuccinimide ester), an amino group and a carboxyl group (e.g., 4-[p-azidosalicylamido] butylamine), and an amino group and a guanidinium group that is present in the side chain of arginine (e.g., p-azidophenyl glyoxal monohydrate).

In some aspects, a radioactive label can be directly conjugated to the amino acid backbone of a polypeptide. Alternatively, the radioactive label can be included as part of a larger molecule (e.g., 125I in meta-[125I]iodophenyl-N-hydroxysuccinimide ([125I]mIPNHS) which binds to free amino groups to form meta-iodophenyl (mIP) derivatives of relevant proteins (see, e.g., Rogers et al. (1997) J Nucl Med 38:1221-1229) or chelate (e.g., to DOTA or DTPA) which is in turn bound to the protein backbone. Methods of conjugating the radioactive labels or larger molecules/chelates containing them to the polypeptides described herein are known in the art. Such methods involve incubating the proteins with the radioactive label under conditions (e.g., pH, salt concentration, and/or temperature) that facilitate binding of the radioactive label or chelate to the protein (see, e.g., U.S. Patent No. 6,001,329).

Methods for conjugating a fluorescent label (sometimes referred to as a "fluorophore") to a protein (e.g., an antibody) are known in the art of protein chemistry. For example, fluorophores can be conjugated to free amino groups (e.g., of lysines) or sulfhydryl groups (e.g., cysteines) of proteins using succinimidyl (NHS) ester or tetrafluorophenyl (TFP) ester moieties attached to the fluorophores. In some aspects, the fluorophores can be conjugated to a heterobifunctional cross-linker moiety such as sulfo-SMCC. Suitable conjugation methods involve incubating an antibody protein, or fragment thereof, with the fluorophore under conditions that facilitate binding of the fluorophore to the protein. See, e.g., Welch and Redvanly (2003) "Handbook of Radiopharmaceuticals: Radiochemistry and Applications," John Wiley and Sons (ISBN 0471495603).

In some aspects, the polypeptides described herein can be glycosylated. In some aspects, a polypeptide described herein can be subjected to enzymatic or chemical treatment, or produced from a cell, such that the polypeptide has reduced or absent glycosylation. Methods for producing antibodies with reduced glycosylation are known in the art and described in, e.g., U.S. patent no. 6,933,368; Wright et al. (1991) EMBO J 10(10):2717-2723; and Co et al. (1993) Mol Immunol 30:1361.

## Exemplary CAR Polypeptides

In some aspects, the CAR polypeptide comprises an extracellular domain that binds to a NPM1c antigen, a DAP12 transmembrane and intracellular domain. In some aspects, the CAR polypeptide comprises an extracellular domain that binds to a NPM1c antigen, a CD8 transmembrane domain, a 4-1BB intracellular domain, and a CD3ζ signaling domain. In some aspects, the CAR polypeptide comprises an extracellular domain that binds to a NPM1c antigen, a CD28 transmembrane domain, a CD28 intracellular domain, and a CD3ζ signaling domain. In some aspects, the CAR polypeptide comprises an extracellular domain that binds to a NPM1c antigen, a CD8 transmembrane domain, a 2B4 intracellular domain, and a CD3ζ signaling domain. In some aspects, the CAR polypeptide comprises an extracellular domain that binds to a NPM1c antigen, a 2B4 transmembrane and intracellular domain and a CD3ζ signaling domain. In some aspects, the CAR polypeptide comprises an extracellular domain that binds to a NPM1c antigen, a CD28 transmembrane domain and intracellular domain, a 4-1BB intracellular domain, and a CD3ζ signaling domain. In some aspects, the CAR polypeptide comprises an extracellular domain that binds to a NPM1c antigen, a NKp46 transmembrane domain, a 2B4 intracellular domain, and a CD3ζ signaling domain. In some aspects, the CAR polypeptide comprises an extracellular domain that binds to a NPM1c antigen, a CD16 transmembrane domain, a 2B4 intracellular domain, and a CD3ζ signaling domain. In some aspects, the CAR polypeptide comprises an extracellular domain that binds to a NPM1c antigen, a NKp44 transmembrane domain, a DAP10 intracellular domain, and a CD3ζ signaling domain. In some aspects, the CAR polypeptide comprises an extracellular domain that binds to a NPM1c antigen, a NKG2D transmembrane domain, a 4-1BB intracellular domain, and a CD3ζ signaling domain. In some aspects, the CAR polypeptide comprises an extracellular domain that binds to a NPM1c antigen, a NKG2D transmembrane domain, a 4-1BB intracellular domain, and a CD3ζ signaling domain. In some aspects, the CAR polypeptide comprises an extracellular domain that binds to a NPM1c antigen, an NKG2D transmembrane domain and a CD3ζ signaling domain. In some aspects, the CAR polypeptide comprises an extracellular domain that binds to a NPM1c antigen, an NKG2D transmembrane domain, a DAP12 intracellular domain, a 2B4 intracellular domain, and a CD3ζ signaling domain. In some aspects, the CAR polypeptide comprises an extracellular domain that binds to a NPM1c antigen, an NKG2D transmembrane domain, a DAP10 intracellular domain, a 2B4 intracellular domain, and a CD3ζ signaling domain. In some aspects, the polypeptide comprises an extracellular domain that binds to a NPM1c antigen, an NKG2D transmembrane domain, a 4-1BB intracellular domain, a 2B4 intracellular domain, and a CD3ζ signaling domain.

In some aspects, the CAR polypeptide is operably linked to a cytokine such that the cytokine is also expressed in the cell. In some aspects, the CAR polypeptide is linked to a cytokine via a cleavable linker such that both the CAR polypeptide and cytokine are separately expressed after cleavage. In some aspects, the CAR polypeptide is operably linked to an IL-15 polypeptide described herein. In some aspects, the CAR polypeptide is linked to an IL-15 polypeptide described herein via a cleavable linker.

In some aspects, the CAR polypeptide comprises a CD8 hinge and transmembrane domain, a 4-1BB signaling domain, and a CD3 ζ signaling domain. In some aspects, the CAR polypeptide comprises an NPM1c binding scFv, a transmembrane domain, a 4-1BB intracellular domain, and a CD3 ζ signaling domain. In some aspects, the CAR polypeptide comprises a CD8 hinge and transmembrane region of SEQ ID NO: 33, the 4-1BB signaling domain of SEQ ID NO: 34, and the CD3 ζ signaling domain of SEQ ID NO: 35. In some aspects, the CAR polypeptide is encoded by the nucleic acid sequence of SEQ ID NO: 30.

In some aspects, the CAR polypeptide comprises an amino acid sequence comprising: (i) an anti-NPM1c scFv; (ii) a CD8 hinge domain; (iii) a CD8 transmembrane domain; (iv) a 4-1BB intracellular domain; and (v) a CD3 ζ signaling domain.

In some aspects, the CAR polypeptide comprises an amino acid sequence comprising: (i) an anti-NPM1c scFv; (ii) a CD8 transmembrane domain; (iii) a 4-1BB intracellular domain; and (iv) a CD3 ζ signaling domain.

In some aspects, the CAR polypeptide comprises an amino acid sequence comprising: (i) an anti-NPM1c scFv; (ii) a CD28 transmembrane domain; (iii) a CD28 intracellular domain; and (iv) a CD3 ζ signaling domain.

In some aspects, the CAR polypeptide comprises an amino acid sequence comprising: (i) an anti-NPM1c scFv; (ii) a DAP12 transmembrane domain; and (iii) a DAP12 intracellular domain.

In some aspects, the CAR polypeptide comprises an amino acid sequence comprising: (i) an anti-NPM1c scFv; (ii) a CD28 transmembrane domain; (iii) a 2B4 intracellular domain; and (iv) a CD3 ζ signaling domain.

In some aspects, the CAR polypeptide comprises an amino acid sequence comprising: (i) an anti-NPM1c scFv; (ii) a 2B4 transmembrane domain; (iii) a 2B4 intracellular domain; and (iv) a CD3 ζ signaling domain.

In some aspects, the CAR polypeptide comprises an amino acid sequence comprising: (i) an anti-NPM1c scFv; (ii) a CD28 transmembrane domain; (iii) a CD28 intracellular domain; (iv) a 4-1BB intracellular domain; and (v) a CD3 ζ signaling domain.

In some aspects, the CAR polypeptide comprises an amino acid sequence comprising: (i) an anti-NPM1c scFv; (ii) a CD16 transmembrane domain; (iii) a 2B4 intracellular domain; and (iv) a CD3 ζ signaling domain.

In some aspects, the CAR polypeptide comprises an amino acid sequence comprising: (i) an anti-NPM1c scFv; (ii) a NKp44 transmembrane domain; (iii) a DAP10 intracellular domain; and (iv) a CD3 ζ signaling domain.

In some aspects, the CAR polypeptide comprises an amino acid sequence comprising: (i) an anti-NPM1c scFv; (ii) a NKp46 transmembrane domain; (iii) a 2B4 intracellular domain; and (iv) a CD3 ζ signaling domain.

In some aspects, the CAR polypeptide comprises an amino acid sequence comprising: (i) an anti-NPM1c scFv; (ii) a NKG2D transmembrane domain; (iii) a 2B4 intracellular domain; and (iv) a CD3 ζ signaling domain.

In some aspects, the CAR polypeptide comprises an amino acid sequence comprising: (i) an anti-NPM1c scFv; (ii) a NKG2d transmembrane domain; (iii) a 4-1BB intracellular domain; and (iv) a CD3 ζ signaling domain.

In some aspects, the CAR polypeptide comprises an amino acid sequence comprising: (i) an anti-NPM1c scFv; (ii) a NKG2D transmembrane domain; (iii) a 2B4 intracellular domain; (iv) a DAP12 intracellular domain; and (v) a CD3 ζ signaling domain.

In some aspects, the CAR polypeptide comprises an amino acid sequence comprising: (i) an anti-NPM1c scFv; (ii) a NKG2D transmembrane domain; (iii) a 2B4 intracellular domain; (iv) a DAP10 intracellular domain; and (v) a CD3 ζ signaling domain.

In some aspects, the CAR polypeptide comprises an amino acid sequence comprising: (i) an anti-NPM1c scFv; (ii) a NKG2D transmembrane domain; (iii) a 4-1BB intracellular domain; (iv) a 2B4 intracellular domain; and (v) a CD3 ζ signaling domain.

In some aspects, the CAR polypeptide comprises an amino acid sequence comprising: (i) an anti-NPM1c scFv; (ii) a NKG2D transmembrane domain; and (iii) a CD3 ζ signaling domain.

In some aspects, the CAR polypeptide comprises an amino acid sequence comprising: (i) an anti-NPM1c scFv comprising the amino acid sequence set forth in SEQ ID NO: 24; (ii) a CD8 hinge and transmembrane domain comprising the amino acid sequence set forth in SEQ ID NO: 25; (iii) a 4-1BB intracellular domain comprising the amino acid sequence set forth in SEQ ID NO: 26; and (iv) a CD3 $\zeta$ signaling domain comprising the amino acid sequence set forth in SEQ ID NO: 27. In some aspects, the CAR polypeptide further comprises an IL-15 polypeptide comprising the amino acid sequence set forth in SEQ ID NO: 97. In some aspects, the IL-15 polypeptide further comprises a heterologous transmembrane domain.

In some aspects, the CAR polypeptide is encoded by a nucleotide sequence comprising: (i) an anti-NPM1c scFv encoded by the nucleic acid sequence set forth in SEQ ID NO: 32; (ii) a CD8 hinge and transmembrane domain encoded by the nucleic acid sequence set forth in SEQ ID NO: 33; (iii) a 4-1BB intracellular domain encoded by the nucleic acid sequence set forth in SEQ ID NO: 34; and (iv) a CD3 $\zeta$ signaling domain encoded by the nucleic acid sequence set forth in SEQ ID NO: 35. In some aspects, the nucleotide sequence encoding the CAR further comprises the nucleic acid sequence set forth in SEQ ID NO: 98, encoding an IL-15 polypeptide. In some aspects, the nucleic acid sequence encoding an IL-15 polypeptide is operably linked to a nucleic acid sequence encoding a heterologous transmembrane domain.

In some aspects, the CAR polypeptide is encoded by a nucleotide sequence comprising: (i) an anti-NPM1c scFv encoded by a nucleic acid sequence having at least 80%, 85%, 90%, 95%, 96%, 97%, 98%, or 99% identity to the nucleic acid sequence set forth in SEQ ID NO: 32; (ii) a CD8 hinge and transmembrane domain encoded by a nucleic acid sequence having at least 80%, 85%, 90%, 95%, 96%, 97%, 98%, or 99% identity to the nucleic acid sequence set forth in SEQ ID NO: 33; (iii) a 4-1BB intracellular domain encoded by a nucleic acid sequence having at least 80%, 85%, 90%, 95%, 96%, 97%, 98%, or 99% identity to the nucleic acid sequence set forth in SEQ ID NO: 34; and (iv) a CD3 $\zeta$ signaling domain encoded by a nucleic acid sequence having at least 80%, 85%, 90%, 95%, 96%, 97%, 98%, or 99% identity to the nucleic acid sequence set forth in SEQ ID NO: 35. In some aspects, the nucleotide sequence encoding the CAR further comprises a nucleic acid sequence at least 80%, 85%, 90%, 95%, 96%, 97%, 98%, or 99% identity to the nucleic acid sequence set forth in SEQ ID NO: 98, encoding an IL-15 polypeptide. In some aspects, the nucleic acid sequence encoding an IL-15 polypeptide is operably linked to a nucleic acid sequence encoding a heterologous **t**ransmembrane domain.

**Vectors Encoding the CAR Polypeptide**

In some aspects, the CAR polypeptides described herein are encoded by a vector.

Once coding sequences for a CAR polypeptide described herein has been prepared or isolated, such sequences can be cloned into any suitable vector or replicon. A "coding sequence" or a sequence which "encodes" a selected polypeptide (e.g., CAR polypeptide), is a nucleic acid molecule which is transcribed (in the case of DNA) and translated (in the case of mRNA) into a polypeptide in vivo when placed under the control of appropriate regulatory sequences (or "control elements"). The boundaries of the coding sequence are determined by a start codon at the 5' (amino) terminus and a translation stop codon at the 3' (carboxy) terminus. A coding sequence can include, but is not limited to, cDNA from viral, procaryotic or eucaryotic mRNA, genomic DNA sequences from viral or procaryotic DNA, and even synthetic DNA sequences. A transcription termination sequence may be located 3' to the coding sequence. Transcription and translation of coding sequences are typically regulated by "control elements," including, but not limited to, transcription promoters, transcription enhancer elements, transcription termination signals, polyadenylation sequences (located 3' to the translation stop codon), sequences for optimization of initiation of translation (located 5' to the coding sequence), and translation termination sequences.

Numerous cloning vectors are known to those of skill in the art, and the selection of an appropriate cloning vector is a matter of choice. Ligations to other sequences are performed using standard procedures, known in the art.

As used herein, the term "vector" has the same meaning as commonly understood by one of ordinary skill in the art, and refers to a nucleic acid molecule capable of transporting another nucleic acid molecule to which it has been linked. One type of vector is a "plasmid," which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors used in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

Moreover, the term "vector" can refer to a nucleic acid molecule that can transfer or transport another nucleic acid molecule. The transferred nucleic acid can be linked to, e.g., inserted into, the vector nucleic acid molecule. A vector can include sequences that direct autonomous replication in a cell, or may include sequences sufficient to allow integration into host cell DNA. Useful vectors include, for example, plasmids (e.g., DNA plasmids or RNA plasmids), transposons, cosmids, bacterial artificial chromosomes, and viral vectors. Useful viral vectors include, e.g., replication defective retroviruses and lentiviruses.

As used herein, the term "transduction" or "cell transduction" refers to the process of transferring nucleic acid(s) into a cell using a DNA or RNA virus.

In some aspects, a nucleic acid molecule described herein is provided in an expression vector. In some aspects, the vector comprises the nucleic acid molecule that codes for the peptides operatively linked to appropriate expression control sequences. Methods of affecting this operative linking, either before or after the nucleic acid molecule is inserted into the vector, are well known. Expression control sequences include promoters, activators, enhancers, operators, ribosomal nuclease domains, start signals, stop signals, cap signals, polyadenylation signals, and other signals involved with the control of transcription or translation.

A "promoter" is a nucleotide sequence which initiates transcription of a polypeptide-encoding polynucleotide. Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is repressed by an analyte, cofactor, regulatory protein, etc.), and constitutive promoters. In addition, such promoters can also have tissue specificity, for example, the CD80 promoter is only inducible in certain immune cells, and the myoD promoter is only inducible in muscle cells. It is intended that the term "promoter" or "control element" includes full-length promoter regions and functional (e.g.. controls transcription or translation) segments of these regions. A promoter is "derived from" a gene encoding a co-stimulatory molecule if it has the same or substantially the same basepair sequence as a region of the promoter region of the co-stimulatory molecule, complements thereof, or if it displays sequence identity as described below.

In some aspects, the nucleic acid molecules described herein are provided in a viral vector. As used herein, the term "viral vector" can refer to, for example, a nucleic acid molecule (e.g., a transfer plasmid) that includes virus-derived nucleic acid elements that facilitate transfer of the nucleic acid molecule or integration into the genome of a cell, or to a viral particle that mediates nucleic acid transfer. Viral particles can include various viral components and sometimes also host cell components in addition to nucleic acid(s).

Viral vectors that are suitable for use include, for example, retroviral, adenoviral, and adeno-associated vectors, herpes virus, simian virus 40 (SV40), and bovine papilloma virus vectors (see, for example, Gluzman (Ed.), Eukaryotic Viral Vectors, CSH Laboratory Press, Cold Spring Harbor, N.Y.). As used herein, the term "retroviral vector" can refer to a retrovirus that has been modified to express the gene of interest (i.e., gene encoding a candidate polypeptide or a heterologous polypeptide). Retroviral vectors can be used to efficiently transfer genes (i.e., gene(s) encoding a candidate polypeptide or a heterologous polypeptide) to host cells by utilizing viral infection processes. Foreign or heterologous target genes cloned into the retroviral genome (i.e., inserted using molecular biology techniques) can be efficiently delivered to host cells susceptible to infection by retroviruses. Known genetic manipulations can disrupt the replication capacity of the retroviral genome. The resulting replication-deficient vector can be used to introduce new genetic material into the cell but they cannot be replicated. Helper virus or packaging cell lines may be used to allow vector particle assembly and release from cells. Such retroviral vectors may comprise nucleic acid sequences encoding one or more genes of interest (i.e., polycistronic nucleic acid sequences may encode several genes of interest), 5 'retroviral long chain terminal repeats (5' LTR), and 3 '. Replication-defective retrovirus genomes containing retrovirus long chain terminal repeats (3 ' LTR).

In some aspects, the viral vector is a lentiviral vector. As used herein, the term "lentiviral vector" refers to lentivirus families (e.g., HIV, MIV, equine infectious anemia virus, caprin arthritis-encephalitis virus) that can be incorporated into non-dividing cells. (See, eg, US Pat. Nos. 5,994,136 and 6,013,516).

In some aspects, the viral vector is a pseudotyped lentiviral vector. As used herein, the term "pseudo lentiviral vector" or "pseudotyped lentiviral vector" refers to a lentiviral vector containing heterologous membrane proteins, such as heterologous viral envelopes to alter their tropism. See, e.g., Cronin et al (2005) CURR. GENE THER. 5:387-398. For example, in some aspects, the envelope glycoprotein is from vesicular stomatitis virus (VSVG). Pseudotyping lentiviral vectors with a diverse set of naturally occurring or engineered viral envelopes allows targeted transduction of specific cell types. For example, lentiviral vectors pseudotyped with a baboon retroviral envelope glycoprotein (BaEV-LVs) have been developed. Lentiviral vectors pseudotyped with a modified BaEVg can transduce NK cells 20-fold or higher in comparison to VSVg pseudotyped lentiviral vector, in large part because activated NK cells resulted in the upregulationg of ASCT-2, making them highly susceptible to transduction with BaEV pseudotyped lentiviral vectors.

In some aspects, the viral vector is capable of expressing the CAR polypeptide. In some aspects, the viral vector comprises a nucleic acid sequence containing an origin of replication. In some aspects, aspects, the viral vector is a plasmid. In some aspects, the viral vector is an expression construct, which is generally a plasmid that is used to introduce a specific gene into a target cell. Once the expression vector is inside the cell, the protein that is encoded by the gene is produced by the cellular transcription and translation machinery ribosomal complexes. In some aspects, the plasmid is engineered to contain regulatory sequences that act as enhancer and promoter regions and lead to efficient transcription of the gene carried on the expression vector. The viral vectors of the present disclosure express large amounts of stable messenger RNA, and therefore proteins.

In some aspects, the viral vectors have expression signals such as a strong promoter, a strong termination codon, adjustment of the distance between the promoter and the cloned gene, and the insertion of a transcription termination sequence and a PTIS (portable translation initiation sequence).

In some aspects, the viral vector is a circular plasmid or a linear nucleic acid. The circular plasmid and linear nucleic acid are capable of directing expression of a particular nucleotide sequence in an appropriate subject cell. In some aspects, the viral vector comprises a promoter operably linked to the nucleotide sequence encoding the CAR polypeptide, which may be operably linked to termination signals. In some aspects, the viral vector comprising the nucleotide sequence encoding the CAR polypeptide, meaning that at least one of its components is heterologous with respect to at least one of its other components. In some aspects, the expression of the nucleotide sequence in the expression cassette is under the control of a constitutive promoter or of an inducible promoter, which initiates transcription only when the host cell is exposed to some particular external stimulus.

**Cytokine Induced Memory-Like Natural Killer Cells**

In some aspects, the disclosure provides an engineered cytokine-induced memory-like (ML) natural killer (NK) cell, or population of said cells, comprising a CAR polypeptide described herein. In some aspects, the ML NK cell or population of cells is differentiated from or derived from an autologous or allogeneic NK cell, such as a human donor NK cell. In some aspects, the ML NK cell or population of cells is differentiated from or derived from NK cell(s) from cord blood (e.g., human cord blood). In some aspects, the ML NK cell or population of cells is differentiated from or derived from human PBMCs. In some aspects, the ML NK cell or population of cells is differentiated from or derived from an iPSC-derived NK cell (e.g., human iPSCs).

In some aspects, the ML NK cell or population of cells is differentiated from CD56+CD3- human NK cells. In some aspects, CD56+CD3- human NK cells are purified from human PBMCs (e.g., donor human PBMCs, e.g., autologous or allogeneic). In some aspects, >95% CD56+CD3- human NK cells are purified from PBMCs. In some aspects, CD56+CD3- human NK cells are isolated using immunodensity cell separation, e.g., RosetteSep. In some aspects, a method for purifying CD56+CD3- human NK cells from PBMCs comprises (i) crosslinking non-NK cells with red blood cells using specific antibodies to form immunorosettes; (ii) subjecting the cells to density gradient centrifugation to pellet the immunorosettes; (iii) isolating the NK cells.

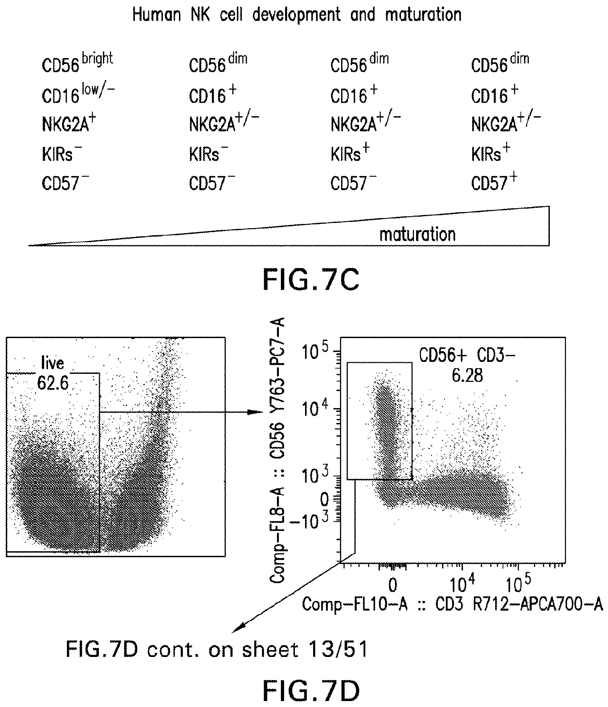

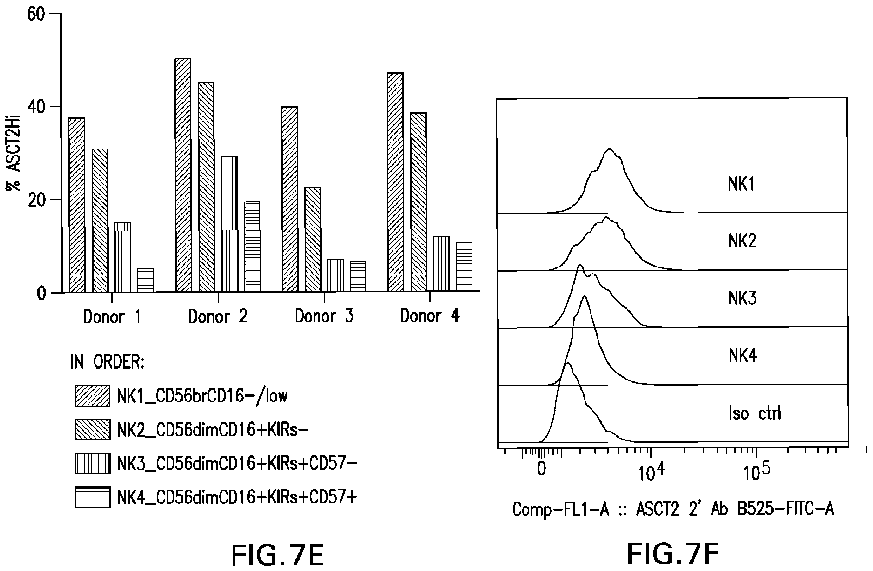

In some aspects, the population of human NK (hNK) cells comprises subsets at different stages of maturation and/or development. In some aspects, the stage of maturation and/or development is determined by expression of phenotypic markers. In some aspects, the stage of maturation and/or development is determined based on the phenotypic markers shown in FIG. 7C. In some aspects, a subset of hNK cells that is less mature, less developed, more stem-like, and/or highly proliferative expresses the following phenotypic markers: CD56$^{bright}$ CD16$^{low/-}$NKG2A$^+$KIRs$^-$CD57$^-$. In some aspects, a subset of hNK cells at an intermediate maturation and/or development stage expresses the following phenotypic markers: CD56$^{dim}$, CD16$^+$NKG2A$^{+/-}$KIRs$^-$CD57$^-$ or CD56$^{dim}$CD16$^+$NKG2A$^{+/-}$KIRs$^+$CD57$^-$. In some aspects, a subset of hNK cells that is matured and/or developed expresses the following phenotypic markers: CD56$^{dim}$CD16$^+$NKG2A$^{+/-}$KIRs$^+$CD57$^+$. In some aspects, a subset of hNK cells that is less mature and/or less developed has average expression of ASCT2 that is increased (e.g., by about 1.1-fold, 1.2-fold, 1.3-fold, 1.4-fold, 1.5-fold, 1.6-fold, 1.7-fold, 1.8-fold, 1.9-fold, 2-fold, 2.5-fold, 3-fold) relative to the average expression of ASCT of a subset of hNK cells that is more mature and/or more developed, e.g., as measured by flow cytometry.

In some aspects, a subset of hNK cells that is CD56$^{bright}$CD16$^{low/-}$NKG2A$^+$KIRs$^-$CD57$^-$ has average expression of ASCT2 that is increased (e.g., by about 1.1-fold, 1.2-fold, 1.3-fold, 1.4-fold, 1.5-fold, 1.6-fold, 1.7-fold, 1.8-fold, 1.9-fold, 2-fold, 2.5-fold, 3-fold) relative to the average expression of ASCT of a subset of hNK cells that is CD56$^{dim}$CD16$^+$NKG2A$^{+/-}$KIRs$^-$CD57$^-$ e.g., as measured by flow cytometry. In some aspects, a subset of hNK cells that is CD56$^{bright}$CD16$^{low/-}$NKG2A$^+$KIRs$^-$CD57$^-$ has average expression of ASCT2 that is increased (e.g., by about 1.1-fold, 1.2-fold, 1.3-fold, 1.4-fold, 1.5-fold, 1.6-fold, 1.7-fold, 1.8-fold, 1.9-fold, 2-fold, 2.5-fold, 3-fold) relative to the average expression of ASCT of a subset of hNK cells that is CD56$^{dim}$CD16$^+$NKG2A$^{+/-}$KIRs$^+$CD57$^-$, e.g., as measured by flow cytometry. In some aspects, a subset of hNK cells that is CD56$^{bright}$CD16$^{low/-}$NKG2A$^+$KIRs$^-$CD57$^-$ has average expression of ASCT2 that is increased (e.g., by about 1.1-fold, 1.2-fold, 1.3-fold, 1.4-fold, 1.5-fold, 1.6-fold, 1.7-fold, 1.8-fold, 1.9-fold, 2-fold, 2.5-fold, 3-fold) relative to the average expression of ASCT of a subset of hNK cells that is CD56$^{dim}$CD16$^+$NKG2A$^{+/-}$KIRs$^+$CD57$^+$, e.g., as measured by flow cytometry.

Phenotype

In some aspects, cytokine-induced memory-like (ML) NK cells express a unique set of markers compared to a control NK cell or population of cells. In some aspects, the control NK cell is a human NK cell activated in the presence of IL-15 alone (e.g., human IL-15) or a human NK cell line activated in the presence of IL-15 alone. In some aspects, the control NK cell has the same phenotype as the NK cell from which the ML NK cell is derived.

In some aspects, expression of at least one cell surface marker expressed on a control NK cell is decreased. In some aspects, at least one cell surface marker expressed on a control NK cell is not expressed on ML NK cells. In some aspects, expression of at least one cell surface marker not expressed on a control NK cell is expressed on ML NK cells. In some aspects, expression of at least one cell surface marker having low levels of expression on a control NK cell is increased on ML NK cells.

In some aspects, a ML NK cell or population of said cells is characterized as described in the following: Berrien-Elliott, M. M. et al. Cancer Discovery, DOI: 10.115812159-8290.CD-20-0312, December 2020; Romee. R. et al. Sci Transl Med Vol. 8(357), 2016 Sep 21.

In some aspects, expression of one or more of the following polypeptides is increased in an ML NK cell or population of said cells relative to a control NK cell: CD94/NKG2A, NKp30, NKp44, NKGD2 and CD25. In some aspects, expression of one or more of the following polypeptides is increased in an ML NK cell or population of said cells relative to a control NK cell: CD94/NKG2A, NKp30, NKp44, NKp46, NKG2D, CD62L and CD25. In some aspects, one or more of the following polypeptides is increased in an ML NK cell or population of said cells relative to a control NK cell: TRAIL, CD69, CD62L, NKG2A, and NKp30. In some aspects, expression of the polypeptide is increased by at least 1.5, 2, 3, 4, 5, 6, 7, 8, 9 or 10 fold.

In some aspects, a population of ML NK cells comprises an increased frequency of TRAIL+CD69+CD62L+NKG2A+NKp30+ NK cells. In some aspects, the frequency of the cell population is increased by at least 1.5, 2, 3, 4, 5, 6, 7, 8, 9 or 10 fold. In some aspects, a population of ML NK cells comprises a decreased frequency of CD27+CD127+ NK cells. In some aspects, the frequency of the cell population is decreased by at least 1.5, 2, 3, 4, 5, 6, 7, 8, 9 or 10 fold.

In some aspects, expression of CD16 and/or CD11b is decreased in an ML NK cell or population of said cells relative to a control NK cell. In some aspects, expression of the polypeptide is decreased by at least 1.5, 2, 3, 4, 5, 6, 7, 8, 9 or 10 fold.

In some aspects, expression of one or more of the following polypeptides is relatively unchanged in the ML NK cell or said population of cells relative to a primary/conventional NK cell: KIR, CD57, NKG2C, DNAM-1, CD137, and CD11b.

In some aspects, a ML NK cell or said population of cells have the following phenotype: CD11b$^{high}$CD27$^{low}$KLRG1 $^{high}$ CD43$^{high}$.

In some aspects, a ML NK cell or said population of cells is CD25+NKG2A+NKp30+NKp44+.

In some aspects, the ML NK cells have increased CD56 expression compared to control NK cells. In some aspects, ML NK cells have increased CD69 expression compared to control NK cells. In some aspects, the ML NK cells have increased NKG2A expression compared to control NK cells. In some aspects, ML NK cells have increased expression of NKG2C compared to control NK cells. In some aspects, the ML NK cells have increased expression of CD94 compared to control NK cells. In some aspects, the ML NK cells have increased expression of NKp46 compared to control NK cells.

In some aspects, expression of the cell surface markers described herein is induced within 2-24 or 14-16 hours of exposure to at least one cytokine. In some aspects, the expression profile described herein is induced within 2-24 or 14-16 hours of exposure to at least one cytokine.

## Functional Characteristics

In some aspects, cytokine-induced memory-like (ML) NK cells have enhanced functional characteristics compared to a control NK cell. In some aspects, the control NK cell is a human NK cell activated in the presence of IL-15 alone or a human NK cell line activated in the presence of IL-15 alone. In some aspects, the control NK cell has the same phenotype as the NK cell from which the ML NK cell is derived.

In some aspects, the memory-like (ML) NK cells described herein have increased proliferative capacity. In some aspects, the ML NK cells have increased proliferation compared to a control NK cell. Methods for measuring cell proliferation are known to those of skill in the art and include, but are not limited to, measuring the number of cells and/or measuring proliferation markers. In some aspects, proliferation is measured using cytoplasmic proliferation dyes, in which a cell permeable fluorescent chemical binds to cytosolic components and is diluted in half every cell division. Such dyes can be used in vitro and in vivo. Examples include measuring carbozyfluorescein diacetate (CFSE). In some aspects, proliferation is measured by quantifying the level of a cell-cycle associated protein. Multiple techniques are applicable to measure cell proliferation. Examples of techniques include flow cytometry, western blot analysis, and tissue microscopy. Manual methods for determining cell proliferation may be used including counting total cell number.

In some aspects, the memory-like (ML) NK cells produce IFNγ. In some aspects, IFN γ production is increased in ML NK cells relative to a control NK cell (e.g., a human NK cell or activated human NK cell). In some aspects, ML NK cells have increased IFN γ production upon stimulation of the NK cells. In some aspects, the NK cells are stimulated by an activating receptor or tumor target. In some aspects, the ML NK cells have increased IFN γ production compared to a control NK cell upon exposure to cancer cells. In some aspects, a ML NK cell has increased IFN γ production compared to a control NK cell upon exposure to cancer cells. In some aspects, IFN γ production increases by at least 10%, at least 20%, at least 30%, at least 40%, at least 50%, at least 60%, at least 70%, at least 80%, at least 90%, or at least 99% compared to conventional NK cells. In some aspects, IFN γ production is increased upon stimulation 1-7 days, 7-14 days, 14-21 days, and up to 30 days after activation. In some aspects, ML NK cells maintain increased IFN γ 1-7 days after implantation into a subject. In some aspects, ML NK cells maintain increased IFN γ for at least 1 month, at least two months, or at least three months after implantation into a subject. In some aspects, the ML NK cells maintain increased IFN γ for at least one week, at least two weeks, at least three weeks, at least one month, at least two months, or at least three months after implantation. In some aspects, the ML NK cells pass on enhanced IFNγ to progeny cells.

In some aspects, ML NK cells have enhanced antibody-dependent cellular cytotoxicity (ADCC) relative to a control NK cell. ADCC is a process that can kill sensitive targets, including tumor cells and virally infected cells, in which NK cells are the effectors. ADCC is triggered when receptors on the NK cell surface recognize IgGI or IgG3 antibodies bound to the surface of a cell. This triggers release of cytoplasmic granules containing perforin and granzymes, leading to target cell death. Methods for measuring ADCC of NK cells are known to those of skill in the art.

In some aspects, ML NK cells have enhanced anti-tumor efficacy relative to a control NK cell. Methods for measuring anti-tumor efficacy of NK cells are known to those of skill in the art and described herein.

In some aspects, ML NK cells (i) produce increased IFNγ in the presence of one or more cytokines and/or tumor targets; (ii) have enhanced ADCC; (iii) have enhanced anti-tumor efficacy; or (iv) any combination of (i)-(iii).

## Methods of Making Memory-Like NK Cells

In some aspects, NK cells are differentiated into memory-like (ML) NK cells. In some aspects, NK cells are activated and then differentiate into ML NK cells over a period of time (e.g., hours, days). In some aspects, activation of NK cells results in differentiation into ML NK cells. In some aspects, NK cells are activated using cytokines, such as IL-2, IL-7, IL-12, IL-15, IL-18 and IL-21 and any combination thereof.

In some aspects, NK cells are activated by exposure for a period of time to IL-12 and IL-15; IL-12 and IL-18; IL-15 and IL-18; or IL-12, IL-15 and IL-18.

In some aspects, the NK cells are activated by exposure for a period of time to IL-12 in a concentration range from 1-20 ng/mL. In some aspects, NK cells are activated with at least 1 ng/mL, at least 2 ng/mL, at least 3 ng/mL, at least 4 ng/mL, at least 5 ng/mL, at least 6 ng/mL, at least 7 ng/mL, at least 8 ng/mL, at least 9 ng/mL, at least 10 ng/mL, at least 15 ng/mL, or at least 20 ng/mL of IL-12. In some aspects, NK cells are activated with 10ng/mL of IL-12.

In some aspects, the NK cells are activated by exposure for a period of time to IL-15 in a concentration range from 1-50 ng/mL. In some aspects, the NK cells are activated by exposure for a period of time to IL-15 in a concentration range from 1-100 ng/mL. In some aspects, NK cells are activated with at least 50 ng/mL, at least 60 ng/mL, at least 70 ng/mL, at least 80 ng/mL, at least 90 ng/mL, at least 95 ng/mL, at least 100 ng/mL, at least 110 ng/mL, at least 120 ng/mL, at least 130 ng/mL, at least 140 ng/mL, or at least 150 ng/mL of IL-15. In some aspects, NK cells are activated with 1 ng/mL of IL-15. In some aspects, NK cells are activated with 50 ng/mL of IL-15.

In some aspects, NK cells are activated by exposure for a period of time to IL-18 in a concentration range from 10-100 ng/mL. In some aspects, NK cells are activated with 50ng/mL of IL-18. In some aspects, NK cells are activated with at least 20 ng/mL, at least 30 ng/mL, at least 40 ng/mL, at least 50 ng/mL, at least 60 ng/mL, at least 70 ng/mL, at least 80 ng/mL, at least 90 ng/mL, at least 95 ng/mL, or at least 100 ng/mL of IL-18. In some aspects, NK cells are activated with 50 ng/mL IL-18.

In some aspects, NK cells are activated by exposure for a period of time to 1-20 ng/mL IL-12, 1-50 ng/mL IL-15 and 10-100 ng/mL IL-18. In some aspects, NK cells are activated by exposure for a period of time to 10 ng/mL IL-12, 1 ng/mL IL-15 and 50 ng/mL IL-18. In some aspects, NK cells are activated by exposure for a period of time to 10 ng/mL IL-12, 50 ng/mL IL-15 and 50 ng/mL IL-18.

In some aspects, NK cells are incubated in the presence of the cytokines for an amount of time sufficient to form cytokine-activated memory-like (ML) NK cells. In some aspects, the amount of time sufficient to form cytokine-activated memory-like (ML) NK cells is between about 8 and about 24 hours, about 12 hours, or about 16 hours. In some aspects, the amount of time sufficient to form cytokine-activated memory-like (ML) NK cells is between about 16 hours to about 20 hours. In some aspects, the amount of time sufficient to form cytokine-activated memory-like (ML) NK cells is at least about 1 hour; about 2 hours; about 3 hours; about 4 hours; about 5 hours; about 6 hours; about 7 hours; about 8 hours; about 9 hours; about 10 hours; about 1 1 hours; about 12 hours; about 13 hours; about 14 hours; about 15 hours; about 16 hours; about 17 hours; about 18 hours; about 19 hours; about 20 hours; about 21 hours; about 22 hours; about 23 hours; about 24 hours; about 25 hours; about 26 hours; about 27 hours; about 28 hours; about 29 hours; about 30 hours; about 31 hours; about 32 hours; about 33 hours; about 34 hours; about 35 hours; about 36 hours; about 37 hours; about 38 hours; about 39 hours; about 40 hours; about 41 hours; about 42 hours; about 43 hours; about 44 hours; about 45 hours; about 46 hours;

about 47 hours; or about 48 hours.

In some aspects, ML NK cells are activated by exposure to at least one cytokine for 12-24 hours, 12-48 hours, or 14-16 hours. In some aspects, ML NK cells are activated by exposure to at least one cytokine for 16-20 hours. In some aspects, ML NK cells are activated by exposure to at least one cytokine for 16 hours.

In some aspects, ML NK cells are activated by exposure to 1-20 ng/mL IL-12, 1-50 ng/mL IL-15 and 10-100 ng/mL IL-18 for 12-24 hours or 14-16 hours. In some aspects, ML NK cells are activated by exposure to 10 ng/mL IL-12, 1 ng/mL IL-15 and 50 ng/mL IL-18 for 12-24 hours or 14-16 hours. In some aspects, ML NK cells are activated by exposure to 10 ng/mL IL-12, 50 ng/mL IL-15 and 50 ng/mL IL-18 for 12-24 hours, 16-20 hours, or 14-16 hours.

In some aspects, the ML NK cells are maintained *in vitro* with IL-15. In some aspects, the ML NK cells are maintained *in vitro* with 1ng/mL of IL-15. In some aspects, the ML NK cells are contacted with IL-15 every day, every two days, every three days, every four days, or every five days. In some aspects, the ML NK cells are contacted with IL-15 every two days. In some aspects, the ML NK cells are contacted with IL-15 every three days.

In some aspects, the NK cells are activated by co-culturing the NK cells with cells expressing at least one cytokine. In some aspects, the ML NK cells are generated by co-culture of NK cells with one or more of dendritic cells and macrophages. In some aspects, the ML NK cells are generated by co-culture of NK cells with dendritic cells. In some aspects, the dendritic cells secrete enough of any one or more of IL-12, IL-15, and IL-18 to produce ML NK cells when co-cultured with NK cells. In some aspects, the ML NK cells are generated by co-culture of NK cells with macrophages. In some aspects, the macrophages secrete enough of any one or more of IL-12, IL-15, and IL-18 to produce ML NK cells when co-cultured with NK cells.

**Exemplary Memory-Like NK Cells**

In some aspects, an ML NK cell or population of said cells comprise a phenotype and at least one functional characteristic as described herein.

In some aspects, an ML NK cell or population of said cells is CD25+NKG2A+NKp30+NKp44+ and produces IFNγ in the presence of one or more cytokines and/or tumor targets relative to a control NK cell.

In some aspects, an ML NK cell or population of said cells is CD25+NKG2A+NKp30+NKp44+ and (i) produces IFNγ in the presence of one or more cytokines and/or tumor targets relative to a control NK cell and (ii) has enhanced ADCC activity relative to a control NK cell.

In some aspects, an ML NK cell or population of said cells is CD25+NKG2A+NKp30+NKp44+ and (i) produces IFNγ in the presence of one or more cytokines and/or tumor targets relative to a control NK cell and (ii) has enhanced anti-tumor efficacy relative to a control NK cell.

In some aspects, an ML NK cell or population of said cells is CD25+NKG2A+NKp30+NKp44+ and (i) has enhanced anti-tumor efficacy relative to a control NK cell and (ii) has enhanced ADCC activity relative to a control NK cell.

In some aspects, an ML NK cell or population of said cells is CD25+NKG2A+NKp30+NKp44+ and (i) produces IFNγ in the presence of one or more cytokines and/or tumor targets relative to a control NK cell; (ii) has enhanced ADCC activity relative to a control NK cell; and (iii) has enhanced anti-tumor efficacy relative to a control NK cell.

In some aspects, an ML NK cell or population of said cells has increased expression of CD94/NKG2A, NKp30, NKp44, NKG2D, CD25 or any combination thereof, and produces IFNγ in the presence of one or more cytokines and/or tumor targets, relative to a control NK cell.

In some aspects, an ML NK cell or population of said cells (i) has increased expression of CD94/NKG2A, NKp30, NKp44, NKG2D, CD25 or any combination thereof; (ii) produces IFNγ in the presence of one or more cytokines and/or tumor targets; and (iii) has enhanced ADCC activity, wherein (i)-(iii) are relative to a control NK cell.

In some aspects, an ML NK cell or population of said cells (i) has increased expression of CD94/NKG2A, NKp30, NKp44, NKG2D, CD25 or any combination thereof; (ii) produces IFNγ in the presence of one or more cytokines and/or tumor targets; and (iii) has enhanced anti-tumor efficacy, wherein (i)-(iii) are relative to a control NK cell.

In some aspects, an ML NK cell or population of said cells (i) has increased expression of CD94/NKG2A, NKp30, NKp44, NKG2D, CD25 or any combination thereof; (ii) has enhanced ADCC activity; and (iii) has enhanced anti-tumor efficacy, wherein (i)-(iii) are relative to a control NK cell.

In some aspects, an ML NK cell or population of said (i) has increased expression of CD94/NKG2A, NKp30, NKp44, NKG2D, CD25 or any combination thereof; (ii) produces IFNγ in the presence of one or more cytokines and/or tumor targets; (iii) has enhanced ADCC activity; and (iv) has enhanced anti-tumor efficacy, wherein (i)-(iv) are relative to a control NK cell.

In some aspects, an ML NK cell or population of said cells (i) has increased expression of CD94/NKG2A, NKp30, NKp44, NKG2D, CD25 or any combination thereof; (ii) has decreased expression of CD16 and/or CD11lb; and (iii) produces IFNγ in the presence of one or more cytokines and/or tumor targets, wherein (i)-(iii) are relative to a control NK cell.

In some aspects, an ML NK cell or population of said cells (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; and (iv) has enhanced ADCC activity, wherein (i)-(iv) are relative to a control NK cell.

In some aspects, an ML NK cell or population of said cells (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; and (iv) has enhanced anti-tumor efficacy, wherein (i)-(iv) are relative to a control NK cell.

In some aspects, an ML NK cell or population of said cells (i) has increased expression of CD94/NKG2A, NKp30, NKp44, NKG2D, CD25 or any combination thereof; (ii) has decreased expression of CD16 and/or CD11b; (iii) has enhanced ADCC activity; and (iv) has enhanced anti-tumor efficacy, wherein (i)-(iv) are relative to a control NK cell.

In some aspects, an ML NK cell or population of said (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; (iv) has enhanced ADCC activity; and (v) has enhanced anti-tumor efficacy, wherein (i)-(v) are relative to a control NK cell.

In some aspects, an ML NK cell or population of said (i) is $CD56^{bright}$, $CD16^{low/-}$, $NKG2A^+$, $KIRs^-$, $CD57$; (ii) is highly proliferative; (iii) is less mature; (iv) has increased expression of ASCT2, wherein (i)-(iv) are relative to a control NK cell that is $CD56^{dim}$, $CD16^+$, $NKG2A^{+/-}$, $KIRs^-$, $CD57^-$; $CD56^{dim}$, $CD16^+$, $NKG2A^{+/-}$, $KIRs^+$, $CD57^-$; or $CD56^{dim}$, $CD16^+$, $NKG2A^{+/-}$, $KIRs^+$, $CD57^+$.

Methods for Increasing the Transduction Efficiency of a Cell

Aspects of the disclosure are directed towards methods for increasing the transduction efficiency of a cell. In some aspects, the method comprises contacting at least one $ASCT2^+$ cell with a cell culture medium comprising a vector encoding a candidate polypeptide. In some aspects, the $ASCT2^+$ cell has been activated with a cytokine. In some aspects, the disclosure is directed to methods for increasing the transduction efficiency of a cell comprising contacting at least one $ASCT2^+$ cell with a cell culture medium comprising a baboon envelope (BaEV) lentiviral vector encoding a candidate polypeptide

Alanine/serine/cysteine transporters, including ASCT1 (encoded by *SLC1A4)* (ASCT1 (human, Gene Name SLC1A4, Gene ID: 6509) and ASCT2 (encoded by *SLC1A5)* (ASCT2 (human), Gene Name SLC1A5, Gene ID: 6510), mediate sodium-dependent exchange of small neutral amino acids such as Ala, Ser, Cys and Thr. Their structure is predicted to be similar to that of the glutamate transporter. Alanine/Serine/Cysteine-preferring Transporter 2 (ASCT2), for example, is a ubiquitously expressed, broad-specificity, sodium-dependent neutral amino acid exchanger that appears to play a role in the regulation of extracellular and intracellular amino acid pools. Under physiological conditions, ASCT2 is distributed ubiquitously in the body. ASCT2 has been implicated in cancer. For example, overexpression of ASCT2 is driven by oncogenes like MYC and has been observed in cancers of the prostate, lung, breast, kidney, the gastrointestinal tract and liver, the female reproductive tract, and the nervous system.

As described herein, expression levels of ASCT2 are abundant in primary conventional NK cells and are further enhanced in cytokine-induced memory-like (CIML) NK cells. ASCT2 is a receptor of baboon envelope glycoprotein BaEV-gp, indicating that inefficient transduction of NK cells, such as primary NK cells and CIML NK cells, can be overcome utilizing an unconvention BaEV pseudotyped lentivirus. Thus, the presence or level of ASCT2 (i.e., an $ASCT2^+$ cell) results in the cell being more receptive to transduction by the BaEV-lentiviral vector.

In some aspects, an $ASCT2^+$ cell can be characterized by the presence or absence of ASCT2 or expression thereof. For example, the presence or absence of ASCT2 or expression thereof can be determined by techniques known in the art. For example, procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Theory of Enzyme Immunoassays", P. Tijssen, Elsevier Science Publishers, Amsterdam, 1985.

In some aspects, the $ASCT2^+$ cell can be characterized by increased expression of the gene encoding the ASCT2 protein, or increased levels of ASCT2 protein relatively to a control cell. For example, an $ASCT2^+$ cell may have increased expression or protein levels of ASCT2 relative to a control cell. As used herein, a "control" or "control cell" can refer to a cell that provides a reference point for measuring changes in genotype or phenotype of a cell, such as ASCT2 expression or level. For example, a control cell can comprise a wild-type cell. In other aspects, a control cell can comprise a genetically-modified cell, such as an $ASCT2^{-/-}$ cell. In still other aspects, a control cell can comprise an immature cell or a mature cell. In some aspects, the control cell can be an inactivated NK cell.

"Changed as compared to a control" sample or cell, such as increased ASCT2 level relative to a control cell, can refer to having a level of the analyte or diagnostic or therapeutic indicator (e.g., marker, such as ASCT2) to be detected at a level that is statistically different than a sample from a normal, untreated, or abnormal state control sample. Determination of statistical significance is within the ability of those skilled in the art, e.g., the number of standard deviations from the mean that constitute a positive or negative result.

In some aspects, the ASCT2$^+$ cell has expression or protein levels that are about 10%, about 20%, about 30%, about 40%, about 50%, about 60%, about 70%, about 80%, about 90% or about 100% greater than that of a control cell.

In some aspects, the ASCT2$^+$ cell can be characterized by the expression of a gene encoding ASCT2 or a level of ASCT2 above a threshold. The term "threshold", for example an ASCT2 threshold, can refer to a value derived from a plurality of biological samples, such as donor cells, for a biomarker, such as a polypeptide corresponding to ASCT2, above which threshold is associated with an effect, such as increased transfection efficiency.

In some aspects, the ASCT2$^+$ cell is an NK cell. An "NK cell" can refer to a subpopulation of lymphocytes that is associated with innate or non-conventional immunity. NK cells can be characterized by several features and biological properties, for example expression of specific surface antigens comprising CD56 and/or CD16 on human NK cells, absence of alpha/beta or gamma/delta TCR complexes on the cell surface, the ability to bind to cells that do not express "self" MHC / HLA antigens by activation of the cell, thereby killing the cells, the ability to kill tumor or other diseased cells expressing a ligand for the NK activating receptor, ("NK cell activity") of releasing a protein molecule called cytokine that stimulates or represses. Any subpopulation of NK cells is also included in the term NK cell.

Key functions of NK cells include killing virus-infected cells, contribution to human reproduction (dominant lymphocyte in pregnant decidua), and exhibiting anti-tumor responses. For example, longitudinal studies correlated low NK cell activity with increased risk of cancer. Also, cancer patients often have defective NK cell number and/or function.

NK cells can kill cancer target cells (e.g., leukemic blasts) without prior sensitization. Key mediators include, for example, ADCC (Rituximab, Cetuximab etc.), via Fc receptor (CD16a). In aspects, the cell can be a primary cell, such as a primary NK cell. For example, the primary NK cell can be isolated from PBMCs, such as by methods known in the art, including magnetic labeling, or depleting non-NK cells (i.e., T cells, B cells, stem cells, dendritic cells, monocytes, granulocutes, and erythroid cells).

In some aspects, the cell is a mouse primary NK cell. Such cells have a transduction rate of about 20%, and thus can be resistant to transduction even more so than human cells. Therefore, aspects described herein provide opportunities for otherwise difficult studies without creating a transgenic mouse model.

In some aspects, the cell can be a cytokine induced memory-like (CIML) NK cell. A CIML NK cell can refer to an NK cell that has been pre-activated with cytokines which results in extensive proliferation, and differentiation into cytokine-induced memory-like (CIML) NK cells. See, for example, Romee, Rizwan, et al. "Cytokine activation induces human memory-like NK cells." Blood 120.24 (2012): 4751-4760. For example, the resting NK cell is activated with cytokines such as IL-7, IL-12, IL-15, IL-18, IL-21, or any combination thereof. Referring to FIG. 1, for example, naive NK cells are pre-activated with IL-12, IL-18 and, optionally, IL-15, to produce cytokine-induced memory-like NK cells. In aspects, CIML NK cells can be identified by enhanced IFN-$\gamma$ production relative to a control cell, such as a cell that has not been activated. Other suitable biomarkers of memory-like NK cells include, but are not limited to, CD94, NKG2A, NKG2C, CD69, and NKp46.

In one aspect, an NK cell (for example, a cytokine induced memory-like (CIML) NK cell, a resting NK cell, a naive NK cell) can be pre-activated with a cytokine of the interleukin-12 family. Members of the interleukin-12 family include, for example, the heterodimeric cytokines IL-12 (comprising subunits IL-12A (p35) and IL-12B (p40)), IL-23 (comprising subunits IL-12B (p40) and IL-23 p19), IL-27 (comprising subunits EBI3 and IL-27 p28), and IL-35 (comprising subunits EBI3 and IL-12A (p35)). EBI3 is a homologue to IL-12 p40. Without wishing to be bound by theory, an NK cell (for example, a cytokine induced memory-like (CIML) NK cell, a resting NK cell, a naive NK cell) can be pre-activated with a IL-12B (p40) subunit or a homologue thereof (such as EBI3). *See also,* Sun et al., Cytokine. 2015 Oct; 75(2): 249-255.

In some aspects, the cell is a mammalian cell, such as a mammalian NK cell. The term "mammalian cell" can refer to a cell from a mammal. In aspects, the cell is a human cell, such as a human NK cell, while in other aspects, the cells are obtained from domestic animals, laboratory animals, livestock, or companion animals (e.g., rodents, cattle, pigs, sheep, goats, dogs, cats, horses, rabbits, etc.). It is not intended that the invention be limited to cells from any particular species, as the invention finds use with any type of mammalian cell. The invention also finds use with normal cells, cancerous cells, pre-cancerous cells, healthy cells, diseased cells, virus-infected cells, cells from different tissues, cells at different developmental stages such as adult and fetal cells, etc., obtained from any type of animal. In further aspects, the invention finds use with mutant cells, including naturally occurring mutant cells, mutant cells which are genetically engineered using knockout technology, insertion, deletion, or replacement, chemically-induced mutant cells, radiation-induced mutant cells, etc., obtained from any type of animal. The invention further finds use with primary cultured cells, cell line cells, and cells infected with a pathogen such as a virus, bacteria, protozoa, fungus, etc., from any type of animal.

As described herein, aspects of the disclosure are directed towards methods for increasing the transduction efficiency of a cell. For example, the method comprises contacting at least one ASCT2$^+$ cell with a cell culture medium comprising a vector encoding a target gene. In aspects, the method can comprise a step of obtaining, isolating, or identifying an ASCT2$^+$ cell. In some aspects, an ASCT2$^+$ cell can be provided by culturing (or activating) an ASCT2$^-$ cell with one or more cytokines, such as IL-12, IL-18, and/or IL-15. "Culturing" a cell can refer to contacting a cell with a cell culture medium under conditions suitable to the survival and/or growth and/or proliferation of the cell. Referring to FIG. 1, for example, the naïve NK cell is an ASCT⁻ cell which, after culturing with and pre-activation by one or more cytokines, differentiates into cytokine-induced memory-like NK cells, which are ASCT2⁺ cells. In some aspects, the cell can be cultured for a period of time. For example, the cells can be cultured prior to, during, or after pre-activation with one or more cytokines. For example, after pre-activation with IL-12, IL-18, and/or IL-15, cells can be cultured for a period of time with IL-15. As another example, the cells can be cultured prior to, during, or after transduction.

Thus, some aspects can comprise the step of obtaining, isolating, or identifying an ASCT2⁺ cell, such as a cytokine-induced memory-like (CIML) NK cell. As described herein, expression levels of ASCT2 are enhanced in cytokine-induced memory-like (CIML) NK cells, and thus expression levels or protein levels of ASCT2 can be used to identify an ASCT2⁺ cell. ASCT2 is a receptor of baboon envelope glycoprotein BaEV-gp, indicating that inefficient transduction of NK cells, such as CIML NK cells, can be overcome utilizing an unconventional BaEV pseudotyped lentivirus. Thus, the presence or level of ASCT2 (i.e., an ASCT2⁺ cell) results in the cell being more receptive to transduction by the BaEV-lentiviral vector.

## Candidate polypeptides

Tumor-specific antigens that control cell growth, proliferation, and death can be intracellular. Recognition of intracellular proteins requires ability to recognize antigen peptides presented by HLA molecules. Normally NK cells have no way of recognizing epitopes including neoepitopes presented by HLA molecules. Thus, genetically-engineered NK cells, as described herein, can target intracellular antigens. For example, a specific group of antibodies called T cell receptor (TCR)-like/mimic antibodies target these intracellular antigens. The intracellular tumor-specific antigens can go through the major histocompatibility complex (MHC) class I signaling pathway and present as tumor-specific peptide/MHC complexes on the tumor cell surfaces. TCR-like antibodies recognize the peptide/MHC complexes on the tumor cell surfaces in the same manner as authentic TCRs. The recognition of the peptide/MHC complex by TCRs expressed on the surface of T cells can trigger various effects, such as T cell proliferation and differentiation and cytokine or chemokine secretion. The recognition of the peptide/MHC complex by TCR-like antibodies, however, can trigger much broader pharmacological pathways than that of the TCRs in T cells. TCR-like antibodies can trigger ADCC, CDC, antibody-dependent cellular phagocytosis (ADCP), or the direct induction of apoptosis. Such antibody targets include, but are not limited to, MAGE1, GP100, hTERT, MUC1, NY-ESO-1, FLT3, TP53, spliceosome factors, MAGE3, hCGβ, Her2/Neu, Melan-A/MART-1, TARP, p53, Tyrosinase, p68, MIF, Proteinase 3, WT1, HA-1H, and PRAME. See, for example, He, Qinghua, et al. "TCR-like antibodies in cancer immunotherapy." Journal of hematology & oncology 12.1 (2019): 99.

Nucleophosmin 1 (NPM1) is the phosphoprotein involved in ribosome assembly/transport, cytoplasmic/nuclear trafficking, regulation of DNA polymerase alpha activity, centrosome duplication, and regulation of p53. NPM1 is encoded by the *NPM1* gene in humans and is an intracellular target for acute myelogenous leukemia (AML). Mutations in NPM1 resulting in localization to the cytoplasm can be referred to as the mutant protein cytoplasmic Nucleophosmin 1 (NPM1c). NPM1c contains a nuclear export signal (NES) at its C-terminus.

The candidate polypeptide comprises a chimeric antigen receptor (or CAR). "Chimeric antigen receptor" can refer to an artificial immune cell receptor that is engineered to recognize and bind to an antigen expressed by a tumor cell. A CAR can be designed for an immune effector cell, such as a T cell or NK cell, and can be a chimera of the signaling domain, such as of the T cell receptor (TcR) complex, and an antigen recognition domain (eg a single chain fragment (scFv) of an antibody) (Enblad Et al., Human Gene Therapy., 2015, 26 (8): 498-505).

Thus, CARs can be used to graft the specificity of an antibody or fragment thereof onto an NK cell or T cell, wherein the transfer of its coding sequence is facilitated by a retroviral vector. These receptors are called chimeras because they are composed of portions of different origin. Upon encountering a target cell, e.g., a cancer cell, the CAR NK cell destroys the cancer cell by, for example, the following mechanisms: the general stimulation of cell proliferation, increasing the degree to which cells are toxic to other living cells (i.e., cytotoxicity), and increasing the production of factors secreted by cells in the immune system, which have an effect on other cells within the organism.

As used herein, the vectors as described herein, such as the BaEV-LVs, express a gene of interest (i.e., a gene encoding a candidate polypeptide, a gene encoding a heterologous polypeptide, a gene encoding a CAR described herein). As used herein, a candidate polypeptide can refer to any polypeptide or fragment thereof in which expression on or in the ASCT2+ cell is desired. For example, the candidate polypeptide can be an antibody or fragment thereof, a toxin, a hormone, a growth factor, a receptor, or a signaling molecule. In some aspects the candidate polypeptide or the heterologous polypeptide is a CAR described herein. Suitable candidate polypeptides comprise antibodies and fragments thereof, non-limiting examples of which comprise immune checkpoint antibodies (i.e., immune checkpoint inhibitors). Such antibody targets include, but are not limited to, 4-1BB Ligand, B7-H3, B7-H4, BTLA, CD27, CD28, CD30, CD40, IDO, LIGHT, Nectin 2, OX40, CD70, CD80, CD86, CD96, CD137, CD153, CD154, CD155, CD223, OX40L, PD-1, PD-L1, TIGIT, CD226, CD273, CD357, CTLA-4, DR3, Galectin 9, GITRL, ICOS, ICOSLG, TIM3, TL1A, TNFRSF14, and VISTA.

As used herein, the term "transduction" or "cell transduction" can refer to the process of transferring nucleic acid(s) into a cell using a DNA or RNA virus.

Aspects as described herein can improve or increase transduction efficiency relative to conventional lentiviral transduction approaches. "Increasing transduction efficiency" or "improving transduction efficiency" can refer to the ability of the pseudotyped lentiviral vector to improve the percentage or proportion of a population of target cells (for example, ASCT2+ CIML NK cells) into which a gene encoding a polypeptide is delivered intracellularly across the plasma membrane. Immunofluorescence microscopy, flow cytometry, and other suitable methods can be used to assess transduction efficiency. In some aspects, methods described herein can enable a transduction efficiency of at least 25%, 30%, 35%, 40%, 45%, 50%, 55%, 60%, 65%, 70%, 75%, 80%, or 85%, for example as measure by immunofluorescence microscopy, flow cytometry, FACS, and other suitable methods. For example, methods describe herein can result in transduction of at least 40% of the cells after about 3 days.

## Exemplary Methods for Increasing Transduction Efficiency in a Cell

In aspects, the invention provides a method for increasing the transduction efficiency of a cell. For example, the method comprises contacting at least one ASCT2$^+$ cell, for example an NK cell, with a cell culture medium comprising a baboon envelope (BaEV) lentiviral vector encoding a candidate polypeptide. In some aspects, the ASCT2$^+$ cell has been activated with an internleukin-12 family member and IL-18. In aspects, the ASCT2$^+$ cell has been activated with IL-12, IL-18, and IL-15. In aspects, the method comprises the step of obtaining, isolating, or identifying an NK cell and activating the NK cell, such as an ASCT2$^-$ cell, with an interleukin-12 family member and IL-18 and, optionally, IL-15. In further aspects, the interleukin-12 family member comprises IL-12, IL-23, IL-27, or IL-35. In further aspects, activating the NK cell produces an ASCT2$^+$ cell. In aspects, the ASCT2$^+$ cell is a cytokine-induced memory-like (CIML) NK cell. In further aspects, the method comprises the step of obtaining, isolating, or identifying a cytokine-induced memory-like (CIML) NK cells. In certain aspects, the expression or level of ASCT2$^+$ is increased relative to a control cell. In further aspects, the control cells is an inactivated NK cell. In aspects, the control cell is a mature NK cell. In aspects, ASCT2 expression is increased about 20% to about 30% in the ASCT2$^+$ cell relative to the control cell. In aspects, the presence or level of ASCT2 results in the cell being more receptive to transduction by the BaEV lentiviral vector. In aspects, the ASCT2$^+$ cell has been activated with one or more of IL-7, IL-12, IL-15, IL-18, IL-21, IL-23 or any combination thereof. In aspects, the ASCT2$^+$ cell is a mammalian cell. In further aspects, the mammalian cell is a human cell. In further aspects, the human cell is/was isolated from peripheral blood mononuclear cells (PBMCs). In aspects, the lentiviral vector is pseudotyped with a baboon envelope glycoprotein (BaEV-gp). In aspects, at least 40% of the at least one ASCT2$^+$ cells are transduced after about 3 days. In aspects, the transduction efficiency is improved relative to conventional lentiviral transduction approach. In aspects, the candidate polypeptide comprises an antibody or fragment thereof, a toxin, a hormone, a growth factor, a receptor, or a signaling molecule, or a chimeric antigen receptor thereof. In further aspects, the antibody or fragment is specific for a checkpoint inhibitor. In further aspects, the antibody is an anti-T-cell receptor antibody or a T-cell receptor-like antibody. In further aspects, the antibody or fragment thereof is specific for NPM1, NPM1c, MAGE1, GP100, hTERT, MUC1, NY-ESO-1, FLT1, TP53, spliceosome factors, MAGE3, hCHβ, Her2/Neu, Melan-A/MART-1, TARP, p53, Tyrosinase, p68, MIF, Proteinase 3, WT1, HA-1H, or PRAME. In aspects, the antibody targets a tumor-specific intracellular protein. In further aspects, the intracellular protein comprises NPM1c. In some aspects, the culture medium comprises IL-12/15/21 and IL-7. Aspects of the disclosure are also drawn towards cells produced by any of the methods described herein.

Further, aspects of the disclosure are drawn towards methods for treating cancer. For example, such methods comprise administering to a subject in need thereof a cell produced by any one of the methods described herein.

Sill further, aspects of the disclosure are drawn towards methods for making genetically engineered cells. For example, such methods comprise contacting at least one ASCT2$^+$ cell with a cell culture medium comprising a baboon envelope (BaEV) lentiviral vector encoding a polypeptide. In aspects, the ASCT2$^+$ cell has been activated with an interleukin-12 family member and IL-18. In aspects, the interleukin-12 family member comprises IL-12, IL-23, IL-27, or IL-35.

Aspects of the disclosure are also drawn towards genetically engineered cells produced by contacting at least one ASCT2$^+$ cell with a cell culture medium comprsing a baboon envelope (BaEV) lentiviral vector encoding a polypeptide. In aspects, the ASCT2$^+$ cell has been activated with an interleukin-12 family member and IL-18.

Further, aspects of the disclosure are drawn towards an immunotherapy comprising a cell produced by any method described herein or genetically engineered cell produced by contacting at least one ASCT2$^+$ cell with a cell culture medium comprsing a baboon envelope (BaEV) lentiviral vector encoding a polypeptide. In aspects, in the ASCT2$^+$ cell has been activated with an interleukin-12 family member and IL-18.

Also, aspects of the disclosure are drawn towards methods for treating cancer. For example, such methods comprise administering to a subject in need thereof the immunotherapy comprising a cell produced by any method described herein or genetically engineered cell produced by contacting at least one ASCT2$^+$ cell with a cell culture medium comprsing a baboon envelope (BaEV) lentiviral vector encoding a polypeptide. In aspects, the ASCT2$^+$ cell has been activated with an interleukin-12 family member and IL-18.

Other objects and advantages of this invention will become readily apparent from the ensuing description.

Methods of Transducing Cytokine-Induced Memory-Like NK Cells

Provided herein are methods for producing a population of cytokine-induced memory-like NK cells expressing a heterologous polypeptide. In some aspects, the heterologous polypeptide comprises any CAR polypeptide described herein.

In some aspects, the method comprises transducing the population of cytokine-induced memory-like NK cells with a pseudotyped lentiviral vector. As described herein, a pseudotyped lentiviral vector comprises a heterologous glycoprotein, e.g., a glycoprotein from a different enveloped virus. In some aspects, the pseudotyped lentiviral vector is capable of recognizing and transducing a particular host cell based on whether the receptor recognized by the glycoprotein is present or expressed by the host cell. For example, canonical pseudotyped lentiviral vectors comprise the glycoprotein VSV-G which binds to the LDL receptor, wherein the pseudotyped lentiviral vector recognizes and transduced host cells expressing the LDL receptor. Without being bound by theory, LDL receptor is poorly expressed on NK cells (e.g., conventional NK cells, cytokine-induced memory-like NK cells), resulting in ineffective transduction efficiency in these cells using a pseudotyped lentiviral vector comprising the VSV-G glycoprotein.

Accordingly, the method of the disclosure comprise transducing the population of cytokine-induced memory-like NK cells with a pseudotyped lentiviral vector comprising a heterologous glycoprotein that binds to a receptor present on the cells. In some aspects, the cytokine-induced memory-like NK cells express ASCT-2, and the pseudotyped lentiviral vector comprises a glycoprotein that binds ASCT-2. Without being bound by theory, the abundant expression of ASCT-2 on cytokine-induced memory-like NK cells results in effective transduction in these cells using a pseudotyped lentiviral vector comprising a glycoprotein that binds ASCT-2.

In some aspects, the glycoprotein that binds ASCT-2 is the BaEV glycoprotein or a variant thereof that maintains binding to ASCT-2. In some aspects, the glycoprotein that binds ASCT-2 comprises an amino acid sequence having at least 70%, 75%, 80%, 85%, 86%, 87%, 88%, 89%, 90%, 91%, 92%, 93%, 94%, 95%, 96%, 97%, 98%, or 99% sequence identity to SEQ ID NO: 107. In some aspects, the glycoprotein that binds ASCT-2 comprises SEQ ID NO: 107. In some aspects, the glycoprotein that binds ASCT-2 consists of SEQ ID NO: 107.

In some aspects, the glycoprotein that binds ASCT-2 comprises a polypeptide encoded by a nucleotide sequence having at least 70%, 75%, 80%, 85%, 86%, 87%, 88%, 89%, 90%, 91%, 92%, 93%, 94%, 95%, 96%, 97%, 98%, or 99% sequence identity to SEQ ID NO: 108. In some aspects, the glycoprotein that binds ASCT-2 comprises a polypeptide encoded by a nucleotide sequence set forth by SEQ ID NO: 108. In some aspects, the glycoprotein that binds ASCT-2 consists of a polypeptide encoded by nucleotide sequence set forth by SEQ ID NO: 108.

In some aspects, the population comprises cytokine-induced memory-like NK cells expressing ASCT-2. In some aspects, the method for producing a population of cytokine-induced memory-like NK cells expressing a heterologous polypeptide comprises contacting the population of cytokine-induced memory-like NK cells with a pseudotyped lentiviral vector encoding the heterologous polypeptide (e.g., under conditions to transduce the population of cytokine-induced memory-like NK cells), wherein the pseudotyped lentiviral vector comprises a glycoprotein that binds to ASCT-2 (e.g., BaEV).

In some aspects, the method comprises providing a population of cytokine-induced memory-like NK cells according to a method described herein. In some aspects, the population of cytokine-induced memory-like NK cells comprises one or more phenotypic markers and/or functional characteristics described herein relative to population of control NK cells. In some aspects, the population of cytokine-induced memory-like NK cells is obtained from a population of primary human NK cells. In some aspects, the population of control NK cells is obtained from the same or a different population of primary human NK cells. In some aspects, the population of primary human NK cells is derived from (e.g., autologous or allogeneic) iPSCs, cord blood, or PBMCs according to a method described herein. In some aspects, the population of cytokine-induced memory-like NK cells and the population of control NK cells is obtained from the same population of primary human NK cells, wherein the population of primary human NK cells is derived from (e.g., autologous or allogeneic) iPSCs, cord blood, or PBMCs according to a method described herein.

In some aspects, the population of cytokine-induced memory-like NK cells is obtained by pre-activating a population of primary human NK cells. In some the population of cytokine-induced memory-like NK cells is obtained according to a method described in Romee R, et al. Blood. 2012;120(24) and/or Romee R, Sci. Transl. Med. 2016; 21:357ra123. In some aspects , the population of primary human NK cells is pre-activated by exposure for a period of time to one or more cytokines. In some aspects, the one or more cytokines are selected from: IL-12, IL15, IL18, and a combination thereof. In some aspects, the population of primary human NK cells is pre-activated by exposure for a period of time to IL-12 and IL-15; IL-12 and IL-18; IL-18 and IL-15; or IL-12, IL-15, and IL-18.

In some aspects, the population of cytokine-induced memory-like NK cells is obtained by pre-activating a population of primary human NK cells, wherein the population of primary human NK cells is exposed for a period of time to IL-12 in a concentration range between about 1 ng/mL and about 20 ng/mL; IL-15 in a concentration range between about 1 ng/mL and about 50 ng/mL; and IL-18 in a concentration range between about 10 ng/mL and about 100 ng/mL. In some aspects, the population of primary human NK cells is exposed for a period of time to IL-12 in a concentration range between about 5 ng/mL and about 15 ng/mL; IL-15 in a concentration range between about 25 ng/mL and about 75 ng/mL; and IL-18 in a concentration range between about 25 ng/mL and about 75 ng/mL. In some aspects, the population of primary human NK cells is exposed for a period of time to IL-12 in a concentration range between about 5 ng/mL and about 15 ng/mL; and IL-18 in a concentration range between about 25 ng/mL and about 75 ng/mL. In some aspects, the population of primary human NK cells is exposed for a period of time to IL-12 at a concentration of about 5, 6, 7, 8, 9, 10, 11, 12, 13, 14, or 15 ng/mL; IL-15 at a concentration of about 40, 45, 50, 55, or 60 ng/mL; and IL-18 at a concentration of about 40, 45, 50, 55, or 60 ng/mL. In some aspects, the population of primary human NK cells is exposed for a period of time to IL-12 at a concentration of about 5, 6, 7, 8, 9, 10, 11, 12, 13, 14, or 15 ng/mL; and IL-18 at a concentration of about 40, 45, 50, 55, or 60 ng/mL

In some aspects, the population of primary human NK cells is pre-activated by exposure to the one or more cytokines for a period of time between about 8 and about 24 hours. In some aspects, the population of primary human NK cells is pre-activated by exposure to the one or more cytokines for a period of time between about 12 and about 20 hours. In some aspects, the population of primary human NK cells is pre-activated by exposure to the one or more cytokines for a period of time between about 12 and about 16 hours. In some aspects, the population of primary human NK cells is pre-activated by exposure to the one or more cytokines for a period of time between about 16 and about 20 hours. In some aspects, the population of primary human NK cells is pre-activated by exposure to the one or more cytokines for a period of time between about 14 and about 16 hours. In some aspects, the population of primary human NK cells is pre-activated by exposure to the one or more cytokines for a period of time of about 12, 13, 14, 15, 16, 17, 18, 19, or 20 hours.

In some aspects, the population of cytokine-induced memory-like NK cells is obtained by pre-activating a population of primary human NK cells, wherein the population of primary human NK cells is exposed to IL-12, IL-15, and IL-18 for a period of time between about 8 hours and about 24 hours. In some aspects, the population of primary human NK cells is exposed to IL-12, IL-15, and IL-18 for a period of time between about 12 hours and about 20 hours. In some aspects, the population of primary human NK cells is exposed to IL-12, IL-15, and IL-18 for a period of time between about 12 and about 16 hours. In some aspects, the population of primary human NK cells is exposed to IL-12, IL-15, and IL-18 for a period of time between about 16 hours and about 20 hours. In some aspects, the population of primary human NK cells is exposed to IL-12, IL-15, and IL-18 for a period of time between about 14 hours and about 16 hours. In some aspects, the population of primary human NK cells is exposed to IL-12, IL-15, and IL-18 for a period of time between about 12, 13, 14, 15, 16, 17, 18, 19, or 20 hours.

In some aspects, the population of cytokine-induced memory-like NK cells is obtained by pre-activating a population of primary human NK cells, wherein the population of primary human NK cells is exposed to IL-12 and IL-18 for a period of time between about 8 hours and about 24 hours. In some aspects, the population of primary human NK cells is exposed to IL-12 and IL-18 for a period of time between about 12 hours and about 20 hours. In some aspects, the population of primary human NK cells is exposed to IL-12 and IL-18 for a period of time between about 12 and about 16 hours. In some aspects, the population of primary human NK cells is exposed to IL-12 and IL-18 for a period of time between about 16 hours and about 20 hours. In some aspects, the population of primary human NK cells is exposed to IL-12 and IL-18 for a period of time between about 14 hours and about 16 hours. In some aspects, the population of primary human NK cells is exposed to IL-12 and IL-18 for a period of time between about 12, 13, 14, 15, 16, 17, 18, 19, or 20 hours.

In some aspects, the population of cytokine-induced memory-like NK cells is obtained by pre-activating a population of primary human NK cells, wherein the population of primary human NK cells is exposed to IL-12 in a concentration range between about 1 ng/mL and about 20 ng/mL; IL-15 in a concentration range between about 1 ng/mL and about 50 ng/mL; and IL-18 in a concentration range between about 10 ng/mL and about 100 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours. In some aspects, the population of primary human NK cells is exposed to IL-12 in a concentration range between about 5 ng/mL and about 15 ng/mL; IL-15 in a concentration range between about 25 ng/mL and about 75 ng/mL; and IL-18 in a concentration range between about 25 ng/mL and about 75 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours. In some aspects, the population of primary human NK cells is exposed for to IL-12 at a concentration of about 5, 6, 7, 8, 9, 10, 11, 12, 13, 14, or 15 ng/mL; IL-15 at a concentration of about 40, 45, 50, 55, or 60 ng/mL; and IL-18 at a concentration of about 40, 45, 50, 55, or 60 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours.

In some aspects, the population of cytokine-induced memory-like NK cells is obtained by pre-activating a population of primary human NK cells, wherein the population of primary human NK cells is exposed to IL-12 in a concentration range between about 1 ng/mL and about 20 ng/mL; and IL-18 in a concentration range between about 10 ng/mL and about 100 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours. In some aspects, the population of primary human NK cells is exposed to IL-12 in a concentration range between about 5 ng/mL and about 15 ng/mL; and IL-18 in a concentration range between about 25 ng/mL and about 75 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours. In some aspects, the population of primary human NK cells is exposed for to IL-12 at a concentration of about 5, 6, 7, 8, 9, 10, 11, 12, 13, 14, or 15 ng/mL; and IL-18 at a concentration of about 40, 45, 50, 55, or 60 ng/mL for a period of time between about 8 hours and about 24 hours; between about 12 hours and about 16 hours; between about 16 hours and about 20 hours; between about 14 and about 16 hours; or about 12, 13, 14, 15, 16, 17, 18, 19, or 20 hours.

In some aspects, the population of cytokine-induced memory-like NK cells is transduced following pre-activation. In some aspects, the population of cytokine-induced memory-like NK cells is rested for a period of time prior to transducing according to a method described herein. In some aspects, the population of cytokine-induced memory-like NK cells is exposed to IL-15 during the resting. In some aspects, the resting comprises exposure to a concentration of IL-15 between about 0.5 ng/mL and 10 ng/mL. In some aspects, the resting comprises exposure to a concentration of IL-15 between about 0.5 ng/mL and 5 ng/mL. In some aspects, the resting comprises exposure to a concentration of IL-15 between about 0.5 ng/mL and 2 ng/mL. In some aspects, the resting comprises exposure to a concentration of IL-15 of about 0.5, 0.6, 0.7, 0,8, 0.9, 1.0, 1.5, or 2 ng/mL. In some aspects, the resting is performed for a period of time between about 12 and about 96 hours, between about 12 and about 72 hours, between about 12 hours and about 48 hours, between about 12 hours and about 24 hours, between about 24 hours and about 72 hours, or between about 24 hours and about 48 hours prior to the transducing.

In some aspects, the population of control NK cells is obtained by exposure of the primary human NK cells for a period of time to IL-15 only. In some aspects, the population of control NK cells is obtained by exposure of the primary human NK cells to IL-15 at a concentration of about 40, 45, 50, 55, or 60 ng/mL, optionally for a period of time between about 8 and about 24 hours, between about 16 and about 20 hours, between about 12 and between about 16 hours, between about 14 and about 16 hours, or between about 16 hours. In some aspects, the population of control NK cells is maintained by exposure to IL-15. In some aspects, the population of control NK cells is maintained by exposure to a concentration of IL-15 of about 0.5, 0.6, 0.7, 0,8, 0.9, 1.0, 1.5, or 2 ng/mL for a period of time between about 12 and about 96 hours, between about 12 and about 72 hours, between about 12 hours and about 48 hours, between about 12 hours and about 24 hours, between about 24 hours and about 72 hours, or between about 24 hours and about 48 hours.

In some aspects, the population of cytokine-induced memory-like NK cells expresses one or more unique markers relative to the control population of NK cells (e.g., control population of NK cells activated in the presence of IL-15 alone), as further described herein. In some aspects, the population of cytokine-induced memory-like NK cells has one or more unique functional characteristics relative to the control population of NK cells (e.g., control population of NK cells activated in the presence of IL-15 alone), as further described herein.

In some aspects, the population of cytokine-induced memory-like NK cells has increased ASCT-2 expression relative to the control population of NK cells (e.g., control population of NK cells activated in the presence of IL-15 alone). In some aspects, the population of cytokine-induced memory-like NK cells has increased surface expression of ASCT-2 relative to the control population of NK cells. Methods of measuring expression of surface markers are known in the art, and include flow cytometry, imaging mass spectrometry (e.g., CyTOF), histology, and microscopy. In some aspects, the population of cytokine-induced memory-like NK cells has increased surface expression of ASCT-2 relative to the control population of NK cells, wherein the increase is at least 1.1-fold, about 1.2-fold, 1.3-fold, 1.4-fold, 1.5-fold, 1.6-fold, 1.7-fold, 1.8-fold, 2-fold, 2.5-fold, 3-fold, 3.5-fold, or 4-fold, e.g., as measured by flow cytometry. In some aspects, the increase is about 1.5-3 fold.

In some aspects, the population of cytokine-induced memory-like NK cells has increased expression of ASCT-2 RNA transcript relative to the control population of NK cells. Methods for quantifying RNA expression are known in the art, and include quantitative PCR and RNA sequencing. In some aspects, expression of ASCT-2 RNA transcript in the population of cytokine-induced memory-like NK cells is increased by at least about 1.1-fold, about 1.2-fold, 1.3-fold, 1.4-fold, 1.5-fold, 1.6-fold, 1.7-fold, 1.8-fold, 2-fold, 2.5-fold, 3-fold, 3.5-fold, 4-fold, 4.5-fold, or 5-fold relative to the control population of memory-like NK cells. In some aspects, the expression of ASCT-2 RNA transcript is increased by about 2-fold to about 4-fold.

In some aspects, the population of engineered cytokine-induced memory-like NK cell expressing a heterologous polypeptide (e.g., a CAR described herein) is obtained by contacting the population of cytokine-induced memory-like NK cells expressing ASCT-2 with a pseudotyped lentiviral vector encoding the heterologous polypeptide under conditions to transduce the population of cytokine-induced ML NK cells, wherein the pseudotyped lentiviral vector comprises a glycoprotein that binds ASCT-2 (e.g., BaEV).

Methods for obtaining a pseudotyped lentiviral vector are known in the art. In some aspects, the pseudotyped lentiviral vector is produced by (i) transfecting one or more lentiviral packaging plasmids, a transfer plasmid, and/or an envelope plasmid into target cells (e.g., A293T cells); (ii) growing the target cells to near or complete confluence; (iii) harvesting the cell culture medium; and (iv) collecting the pseudotyped lentiviral vector. In some aspects, the titer of the pseudotyped lentiviral vector is determined using a method described in the art, such as flow cytometry.

In some aspects, the population of engineered cytokine-induced memory-like NK is contacted with a titer of the pseudotyped lentiviral vector (e.g., BaEV lentiviral vector) sufficient to transduce the population. In some aspects, the population of engineered cytokine-induced memory-like NK is contacted with a multiplicity of infection (MOI) of the pseudotyped lentiviral vector (e.g., BaEV lentiviral vector) that is about $5 \times 10^3$, about $6 \times 10^3$, about $7 \times 10^3$, about $8 \times 10^3$, about $9 \times 10^3$, about $10 \times 10^3$, about $11 \times 10^3$, about $12 \times 10^3$, about $13 \times 10^3$, about $14 \times 10^3$, or about $15 \times 10^3$. In some aspects, the population of engineered cytokine-induced memory-like NK is contacted with the pseudotyped lentiviral vector (e.g., BaEV lentiviral vector) for a period of time that is between about 1 hour and about 5 hours, optionally about 1 hour to about 1.5 hours.

In some aspects, contacting the population of cytokine-induced memory-like NK with the pseudotyped lentiviral vector (e.g., BaEV lentiviral vector) encoding a heterologous polypeptide (e.g., CAR polypeptide described herein) results in transduction of at least about 10%, 15%, 20%, 25%, 30%, 35%, 40%, 45%, 50%, 55%, 60%, 65%, 70%, 75%, 80%, 85%, or 90% of the population. In some aspects, the transduction is measured by quantifying expression of mRNA encoding the heterologous polypeptide and/or the heterologous polypeptide using methods known in the art. In some aspects, the transduction is measured by quantifying surface expression of the heterologous polypeptide using methods known in the art, e.g., by flow cytometry or CyTOF.

In some aspects, the method further comprises isolating the population of cytokine-induced memory like NK cells. In some aspects, the method further comprises expanding the population of cells.

**Cytokine Expressing NK Cells**

In some aspects, the ML NK cells are engineered to express a cytokine. In some aspects, the ML NK cells are engineered to express an IL-15 polypeptide. In some aspects, the ML NK cells are engineered to express a membrane-bound IL-15 polypeptide. In some aspects, a membrane-bound IL-15 polypeptide comprises a heterologous transmembrane domain. In some aspects, the ML NK cells are engineered to express a secreted IL-15 polypeptide.

In some aspects, the ML NK cells are engineered to express an IL-15 polypeptide comprising the amino acid sequence set forth in SEQ ID NO: 96. In some aspects, the ML NK cells are engineered to express an IL-15 polypeptide comprising an amino acid sequence having at least 90%, 95%, 96%, 97%, 98%, or 99% identity to the amino acid sequence set forth in SEQ ID NO: 96.

In some aspects, the ML NK cells are engineered to express an IL-15 polypeptide comprising the amino acid sequence set forth in SEQ ID NO: 96 and operably linked to a heterologous transmembrane domain. In some aspects, the ML NK cells are engineered to express an IL-15 polypeptide comprising an amino acid sequence having at least 90%, 95%, 96%, 97%, 98%, or 99% identity to the amino acid sequence set forth in SEQ ID NO: 96 and operably linked to a heterologous transmembrane domain.

In some aspects, the ML NK cells are engineered to express IL15Ra. In some aspects the ML NK cells are engineered to express at least one of: (i) co-expression of IL15 and IL15Ra by using a self-cleaving peptide; (ii) a fusion protein of IL15 and IL5Ra; (iii) an IL15/IL15Ra fusion protein with intracellular domain of IL15Ra truncated; (iv) a fusion protein of IL15 and membrane bound Sushi domain of IL15Ra; (v) a fusion protein of IL15 and IL15R; (vi) a homodimer of ILI5R; and (v) an IL-15 polypeptide fused to a transmembrane domain.

In some aspects, the ML NK cells are engineered to express at least one of: (i) co-expression of IL15 and IL15Ra by using a self-cleaving peptide; (ii) a fusion protein of IL15 and IL5Ra; (iii) an IL15/IL15Ra fusion protein with intracellular domain of IL15Ra truncated; (iv) a fusion protein of IL15 and membrane bound Sushi domain of IL15Ra; (v) a fusion protein of IL15 and IL15R; (vi) a homodimer of ILI5R; (v) an IL-15 polypeptide fused to a transmembrane domain, wherein any one of (i)-(v) can be co-expressed with a CAR in separate constructs or in a bi-cistronic construct. In some aspects, the partial or full peptide of a cell surface exogenous cytokine or a receptor is transiently expressed in the cell provided herein.

In some aspects, the ML NK cell is engineered to express any one or more of IL-15, membrane-bound IL-15, secreted IL-15, and IL-15Ra. In some aspects, the engineered ML NK cell has increased cell proliferation and survival when engineered to express any IL-15 polypeptide described herein. In some aspects, the memory-like NK cell engineered to express an IL-15 polypeptide has increased cell proliferation compared to a memory-like NK cell not expressing the IL-15 polypeptide.

In some aspects, the memory-like NK cell engineered to express an IL-15 polypeptide has increased cell proliferation compared to a control NK cell. In some aspects the engineered memory-like NK cell expressing an IL-15 polypeptide proliferates at least 10%, at least 20%, at least 30%, at least 40%, at least 50%, at least 60%, at least 70%, at least 80%, or at least 90% faster than conventional NK cells or ML NK cells not expressing the IL-15 polypeptide. In some aspects, the memory-like NK cell has increased cell survival compared to conventional NK cells when expressing any IL15 polypeptide described herein. In some aspects, engineered ML NK cells survive at least 10%, at least 20%, at least 30%, at least 40%, at least 50%, at least 60%, at least 70%, at least 80%, or at least 90% longer than conventional NK cells.

**Exemplary Anti-NPM1c CAR-Expressing Cytokine-Induced Memory-Like NK Cells**

In some aspects, provided herein are cytokine-induced memory-like human NK cells expressing any chimeric antigen receptor (CAR) polypeptide described herein. In some aspects, a cytokine-induced memory-like NK cell or said population of cells is transformed with a nucleic acid encoding any chimeric antigen receptor (CAR) polypeptide described herein.

In some aspects, provided herein are cytokine-induced memory-like human NK cells engineered to express (i) a CAR polypeptide comprising an scFv comprising the amino acid sequence set forth in SEQ ID NO: 24, and (ii) an IL-15 polypeptide comprising the amino acid sequence set forth in SEQ ID NO: 96.

In some aspects, provided herein are cytokine-induced memory-like human NK cells engineered to express (i) a CAR polypeptide comprising an scFv comprising the amino acid sequence set forth in SEQ ID NO: 24, and (ii) a membrane-bound IL-15 polypeptide comprising the amino acid sequence set forth in SEQ ID NO: 96.

In some aspects, provided herein are cytokine-induced memory-like human NK cells engineered to express (i) a CAR polypeptide comprising an scFv comprising the amino acid sequence set forth in SEQ ID NO: 24, and (ii) an IL-15 polypeptide comprising the amino acid sequence set forth in SEQ ID NO: 96 and fused to a heterologous transmembrane domain.

In some aspects, an anti-NPM1c CAR ML NK cell or population of said cells expresses a CAR polypeptide comprising an scFv comprising the amino acid sequence set forth in SEQ ID NO: 24, and is CD25+NKG2A+NKp30+NKp44+ and produces IFN$\gamma$ in the presence of one or more cytokines and/or tumor targets relative to a control NK cell.

In some aspects, an ML NK cell or population of said cells expresses a CAR polypeptide comprising an scFv comprising the amino acid sequence set forth in SEQ ID NO: 24, is CD25+NKG2A+NKp30+NKp44+ and (i) produces IFN$\gamma$ in the presence of one or more cytokines and/or tumor targets relative to a control NK cell and (ii) has enhanced ADCC activity relative to a control NK cell.

In some aspects, an ML NK cell or population of said cells expresses a CAR polypeptide comprising an scFv comprising the amino acid sequence set forth in SEQ ID NO: 24, is CD25+NKG2A+NKp30+NKp44+ and (i) produces IFN$\gamma$ in the presence of one or more cytokines and/or tumor targets relative to a control NK cell and (ii) has enhanced anti-tumor efficacy relative to a control NK cell.

In some aspects, an ML NK cell or population of said cells expresses a CAR polypeptide comprising an scFv comprising the amino acid sequence set forth in SEQ ID NO: 24, is CD25+NKG2A+NKp30+NKp44+ and (i) has enhanced anti-tumor efficacy relative to a control NK cell and (ii) has enhanced ADCC activity relative to a control NK cell.

In some aspects, an ML NK cell or population of said cells expresses a CAR polypeptide comprising an scFv comprising the amino acid sequence set forth in SEQ ID NO: 24, is CD25+NKG2A+NKp30+NKp44+ and (i) produces IFN$\gamma$ in the presence of one or more cytokines and/or tumor targets relative to a control NK cell; (ii) has enhanced ADCC activity relative to a control NK cell; and (iii) has enhanced anti-tumor efficacy relative to a control NK cell.

In some aspects, an ML NK cell or population of said cells expresses a CAR polypeptide comprising an scFv comprising the amino acid sequence set forth in SEQ ID NO: 24, has increased expression of CD94/NKG2A, NKp30, NKp44, NKG2D, CD25 or any combination thereof, and produces IFN$\gamma$ in the presence of one or more cytokines and/or tumor targets, relative to a control NK cell.

In some aspects, an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) produces IFN$\gamma$ in the presence of one or more cytokines and/or tumor targets; and (iii) has enhanced ADCC activity, wherein (i)-(iii) are relative to a control NK cell.

In some aspects, an ML NK cell or population of said cells expresses a CAR polypeptide comprising an scFv comprising the amino acid sequence set forth in SEQ ID NO: 24 and (i) has increased expression of CD94/NKG2A, NKp30, NKp44, NKG2D, CD25 or any combination thereof; (ii) produces IFN$\gamma$ in the presence of one or more cytokines and/or tumor targets; and (iii) has enhanced anti-tumor efficacy, wherein (i)-(iii) are relative to a control NK cell.

In some aspects, an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) has enhanced ADCC activity; and (iii) has enhanced anti-tumor efficacy, wherein (i)-(iii) are relative to a control NK cell.

In some aspects, an ML NK cell or population of said cells expresses a CAR polypeptide comprising an scFv comprising the amino acid sequence set forth in SEQ ID NO: 24, (i) has increased expression of CD94/NKG2A, NKp30, NKp44, NKG2D, CD25 or any combination thereof; (ii) produces IFN$\gamma$ in the presence of one or more cytokines and/or tumor, targets; (iii) has enhanced ADCC activity; and (iv) has enhanced anti-tumor efficacy, wherein (i)-(iv) are relative to a control NK cell.

In some aspects, an ML NK cell or population of said cells expresses a CAR polypeptide comprising an scFv comprising the amino acid sequence set forth in SEQ ID NO: 24, and(i) has increased expression of CD94/NKG2A, NKp30, NKp44, NKG2D, CD25 or any combination thereof; (ii) has decreased expression of CD16 and/or CD11b; and (iii) produces IFNγ in the presence of one or more cytokines and/or tumor targets, wherein (i)-(iii) are relative to a control NK cell.

In some aspects an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; and (iv) has enhanced ADCC activity, wherein (i)-(iv) are relative to a control NK cell.

In some aspects, an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; and (iv) has enhanced anti-tumor efficacy, wherein (i)-(iv) are relative to a control NK cell.

In some aspects, an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) has decreased expression of CD16 and/or CD11b; (iii) has enhanced ADCC activity; and (iv) has enhanced anti-tumor efficacy, wherein (i)-(iv) are relative to a control NK cell.

In some aspects, an ML NK cell or population of said cells expresses a CAR polypeptide comprising an scFv comprising the amino acid sequence set forth in SEQ ID NO: 24, and (i) has increased expression of CD94/NKG2A, NKp30, NKp44, NKG2D, CD25 or any combination thereof; (ii) has decreased expression of CD16 and/or CD11b; (iii) produces IFNγ in the presence of one or more cytokines and/or tumor targets; (iv) has enhanced ADCC activity; and (v) has enhanced anti-tumor efficacy, wherein (i)-(v) are relative to a control NK cell.

In some aspects, the ML NK cells provided herein are derived from NK cells isolated from, or expanded from, peripheral blood, cord blood, or lymph. In some aspects, the NK cells are allogeneic. In some aspects , the NK cells are autologous.

In some aspects, the ML NK cells provided herein are autologous to a subject to whom they are to be administered (after their modification to express a CAR described herein). In some aspects, the ML NK cells provided herein are allogeneic to a subject to whom they are to be administered (after their modification to express a CAR described herein). Where allogeneic ML NK cells are used to prepare CAR-expressing immune effector cells, the immune effector cells can be co-administered with one or more immunosuppressive agents.

In some aspects, ML NK cells are derived from a patient with a disease or condition (such as cancer, e.g., AML) and genetically modified *in vitro* to express at least one CAR with specificity for any antigen described herein (e.g., neoantigen). For example, the antigen can be a cancer neoantigen presented by an MHC class I protein (such as an antigen comprising a mutant nucleophosmin protein neoepitope in complex with an MHC class I protein, e.g., NPM1c:HLA-A2). In some aspects, the ML NK cells genetically modified to express a CAR with specificity for be a cancer neoantigen presented by an MHC class I protein (e.g., NPM1c:HLA-A2) is then administered to treat cancer in the patient (e.g., NPM1c -positive cancer, e.g., AML). In some aspects, the ML NK cells perform at least one effector function (e.g. induction of cytokines) that is stimulated or induced by the specific binding of the ligand or antigen to the CAR and that is useful for treatment of the same patient's disease or condition.

The stimulation of a ML NK cell comprising a CAR (e.g., by binding of the extracellular domain of the CAR to a cancer neoantigen) can result in the activation of one or more anti-cancer activities of the CAR cell. For example, in some aspects, stimulation of a CAR ML NK cell can result in an increase in the cytolytic activity or helper activity of the CAR cell, including the secretion of cytokines.

In some aspects, CAR effector cells (e.g., CAR-ML NK cells) comprise a CAR molecule that binds to any antigen described herein (e.g., NPM1c:HLA-A2). In some aspects, the ML NK cell comprising a CAR molecule useful in the methods disclosed herein expresses a CAR comprising an extracellular domain that binds an NPM1c neoepitope in complex with (or presented by) an MHC class I protein (e.g., HLA-A2), such as NPM1c:HLA-A2. In some aspects, the immune effector cell comprising a CAR molecule (e.g., CAR-ML NK cell) useful in the methods disclosed herein expresses a CAR comprising an NPM1c:HLA-A2 binding domain.

In some aspects, the CAR construct is capable of expressing or functioning in a memory-like natural killer (ML NK) cell.

In some aspects, the CAR expressing ML NK cells have increased cytotoxicity toward target cells compared to NK cells not expressing the CAR. Methods for measuring cytotoxicity are known to those of skill in the art and include, but are not limited to, measuring cell number of the target cell of interest, measuring markers for cell death/viability, and luminesence. For example, in some aspects, flow cytometry analysis is use to quantify total cell number. In some aspects, cells are stained using Live/Dead Fixable stain kits and quantified using flow cytometry. In some aspects, cytotoxicity is measured using luciferase-expressing target cells. When cultured with effector cells, luminescence of target cell lysates is used to calculate cell cytotoxicity.

In some aspects, the CAR expressing ML NK cells (e.g., CAR comprising an NPM1c:HLA-A2 binding domain) have increased expression of one or more phenotypic and/or functional markers when contacted with target cells expressing the antigen (e.g., NPM1c:HLA-A2) targeted by the CAR antigen recognition domain. In some aspects, the one or more phenotypic and/or functional markers with increased expression is selected from: (i) IFNgamma; (ii) granzyme B; (iii) one or more activation markers selected from: CD25, CD69, ICOS, CD226, CD107a, and CD62L; (iv) one or more activating receptors selected from: NKp30, NKG2D, NKp44; (vi) one or more maturation markers selected from: CD56 and NKG2A; and/or (viii) TIGIT, wherein (i)-(viii) are relative to control NK cell (e.g., non-CAR expressing ML NK cells). In some aspects, the CAR expressing ML NK cells (e.g., CAR comprising an NPM1c:HLA-A2 binding domain) have increased expression of IFN-gamma when contacted with target cells expressing the antigen (e.g., NPM1c:HLA-A2) targeted by the CAR antigen recognition domain relative to control NK cell (e.g., non-CAR expressing ML NK cells). In some aspects, the CAR expressing ML NK cells (e.g., CAR comprising an NPM1c:HLA-A2 binding domain) have increased expression of granzyme B when contacted with target cells expressing the antigen (e.g., NPM1c:HLA-A2) targeted by the CAR antigen recognition domain relative to control NK cell (e.g., non-CAR expressing ML NK cells). In some aspects, the CAR expressing ML NK cells (e.g., CAR comprising an NPM1c:HLA-A2 binding domain) have increased expression of CD107a when contacted with target cells expressing the antigen (e.g., NPM1c:HLA-A2) targeted by the CAR antigen recognition domain relative to control NK cell (e.g., non-CAR expressing ML NK cells). In some aspects, the CAR expressing ML NK cells (e.g., CAR comprising an NPM1c:HLA-A2 binding domain) have increased expression of TIGIT when contacted with target cells expressing the antigen (e.g., NPM1c:HLA-A2) targeted by the CAR antigen recognition domain relative to control NK cell (e.g., non-CAR expressing ML NK cells). In some aspects, the CAR expressing ML NK cells (e.g., CAR comprising an NPM1c:HLA-A2 binding domain) have increased expression of an activation markers when contacted with target cells expressing the antigen (e.g., NPM1c:HLA-A2) targeted by the CAR antigen recognition domain relative to control NK cell (e.g., non-CAR expressing ML NK cells). In some aspects, the activation marker is CD62L. In some aspects the activation marker is CD25. In some aspects, the activation marker is CD69. In some aspects, the activation marker is ICOS. In some aspects, aspects, the activation marker is CD226. In some aspects, the CAR expressing ML NK cells (e.g., CAR comprising an NPM1c:HLA-A2 binding domain) have increased expression of an activating receptors when contacted with target cells expressing the antigen (e.g., NPM1c:HLA-A2) targeted by the CAR antigen recognition domain. In some aspects, the activating receptor is NKG2D. In some aspects, the activating receptor is NKp30. In some aspects, the activating receptor is NKp44.

In some aspects, the CAR expressing ML NK cells (e.g., CAR comprising an NPM1c:HLA-A2 binding domain) have decreased expression of one or more phenotypic and/or functional markers when contacted with target cells expressing the antigen (e.g., NPM1c:HLA-A2) targeted by the CAR antigen recognition domain. In some aspects, the one or more phenotypic and/or functional markers with decreased expression is selected from (i) CD57; and/or (ii) TRAIL wherein (i)-(ii) are relative to control NK cell (e.g., non-CAR expressing ML NK cells). In some aspects, the CAR expressing ML NK cells (e.g., CAR comprising an NPM1c:HLA-A2 binding domain) have decreased expression of TRAIL when contacted with target cells expressing the antigen (e.g., NPM1c:HLA-A2) targeted by the CAR antigen recognition domain relative to control NK cell (e.g., non-CAR expressing ML NK cells). In some aspects, the CAR expressing ML NK cells (e.g., CAR comprising an NPM1c:HLA-A2 binding domain) have decreased expression of CD57 when contacted with target cells expressing the antigen (e.g., NPM1c:HLA-A2) targeted by the CAR antigen recognition domain relative to control NK cell (e.g., non-CAR expressing ML NK cells).

In some aspects, the CAR expressing ML NK cells (e.g., CAR comprising an NPM1c:HLA-A2 binding domain) when contacted with target cells expressing the antigen (e.g., NPM1c:HLA-A2) targeted by the CAR antigen recognition domain have: (i) increased expression of IFNgamma; (ii) increased expression of granzyme B; (iii) increased expression of one or more activation markers selected from: CD25, CD107a, CD69, ICOS, CD226, and CD62L; (iv) increased expression of one or more activating receptors selected from: NKp30, NKG2D, NKp44; (v) increased expression of one or more maturation markers selected from: CD56 and NKG2A; (vi) decreased expression of CD57; (vii) increased expression of TIGIT; and/or (viii) has decreased expression of TRAIL; wherein (i)-(vii) are relative to control NK cell (e.g., non-CAR expressing ML NK cells). In some aspects, the CAR expressing ML NK cells (e.g., CAR comprising an NPM 1c:HLA-A2 binding domain) when contacted with target cells expressing the antigen (e.g., NPM1c:HLA-A2) targeted by the CAR antigen recognition domain have increased expression of IFNgamma and increased expression of CD107a relative to control NK cell (e.g., non-CAR expressing ML NK cells). In some aspects, the CAR expressing ML NK cells (e.g., CAR comprising an NPM1c:HLA-A2 binding domain) when contacted with target cells expressing the antigen (e.g., NPM1c:HLA-A2) targeted by the CAR antigen recognition domain have increased expression of IFNgamma, CD107a, and granzyme B relative to control NK cell (e.g., non-CAR expressing ML NK cells). In some aspects, the CAR expressing ML NK cells (e.g., CAR comprising an NPM1c:HLA-A2 binding domain) when contacted with target cells expressing the antigen (e.g., NPM1c:HLA-A2) targeted by the CAR antigen recognition domain have increased expression of IFNgamma, CD107a, granzyme B relative to control NK cell (e.g., non-CAR expressing ML NK cells); and decreased expression of TRAIL relative to control NK cell (e.g., non-CAR expressing ML NK cells).

**Methods of Making CAR-Expressing Cytokine-Induced Memory-Like NK Cells**

Disclosed herein are methods that can be used to generate any of the cytokine-induced memory-like NK cells described herein comprising any CAR polypeptide described herein.

In some aspects, a CAR polypeptide or nucleic acid molecule encoding the CAR polypeptide is introduced into a ML NK cell or said population of cells using any method known to those of skill in the art. For example, in some aspects, a nucleic acid molecule encoding a CAR polypeptide described herein is introduced into the cell by electroporation, transfection, or transduction.

In some aspects, a nucleic acid molecule encoding a CAR polypeptide is introduced into a ML NK cell via transduction. In some aspects, a lentivirus comprising a nucleic acid molecule encoding a CAR polypeptide is introduced into a ML NK cell via transduction.

A variety of different methods known in the art can be used to introduce any of the nucleic acids encoding a CAR polypeptide described herein or expression vectors comprising a nucleic acid encoding a CAR polypeptide described herein into a ML NK cell.

In some aspects, a method for producing a CAR expressing ML NK cell described herein comprises: (i) obtaining cells from peripheral blood, cord blood or lymph (e.g., from peripheral blood mononuclear cells (PMBC)), (ii) optionally, purifying the obtained cells, (iii) optionally, expanding the cells, (iv) activating the cells (e.g., with cytokines such as, but not limited to IL-15, IL12, and IL-18) to form cytokine-induced memory-like NK cells, (v) optionally, expanding the activated cells, (vi) transducing the cells with an expression vector comprising a CAR polypeptide described herein, (vii) isolating the cells expressing the CAR, and (viii) optionally, expanding the isolated cells.

In some aspects, a method for producing a CAR expressing ML NK cell described herein comprises: (i) obtaining a pluripotent stem cell (iPSC) (ii) inducing iPSC to differentiate into a NK cell, (ii) pre-activating the NK cell into a cytokine-induced memory-like NK cell, (iii) optionally, expanding the activated cells, (iv) transducing the activated cells with an expression vector comprising a CAR polypeptide described herein, (v) isolating the ML NK cells expressing the CAR, and (vi) optionally, expanding the isolated cells.

In some aspects, any of the genetic modifications described herein are transduced into a cell using lentivirus. In some aspects, any CAR polypeptide described herein is transduced into ML NK cells using baboon retroviral envelope glycoprotein variant (BaEV-LV). NK cells express the BaEV receptor, alanine, serine, cysteine transporter 2 (ASCT2). In some aspects, the BaEV-LV transduction is performed as described in Bari, R., et al. 2019 Frontiers in Immuno. Vol. 10, 2001. In some aspects, any CAR polypeptide described herein is transduced into ML NK cells using vesicular stomatitis virus GP (VSV-G)-LV. NK cells express the VSV-G receptor low-density lipoprotein (LDL-R). In some aspects, the VSV-G-LV transduction is performed as described in Tomás, H., et al. 2019 Mol Ther Methods Clin Dev. 15: 1-8. In some aspects, the glycoprotein of BaEV replaces the VSV glycoprotein. In some aspects, the VSV genome expresses a BaEV glycoprotein.

In some aspects, a CAR polypeptide is transduced via a viral vector (e.g., lentivirus) into the cytokine-induced ML NK cells in the presence of IL-15 for an amount of time sufficient to virally transduce CAR into the cells, resulting in CAR-expressing ML NK cells. In some aspects, the amount of time sufficient to form CAR-expressing ML NK cells is between about 12 hours and about 24 hours. In some aspects, the amount of time sufficient to virally transduce CAR into the ML NK cells (forming CAR-expressing ML NK cells) can be at least about 1 hour; about 2 hours; about 3 hours; about 4 hours; about 5 hours; about 6 hours; about 7 hours; about 8 hours; about 9 hours; about 10 hours; about 11 hours; about 12 hours; about 13 hours; about 14 hours; about 15 hours; about 16 hours; about 17 hours; about 18 hours; about 19 hours; about 20 hours; about 21 hours; about 22 hours; about 23 hours; about 24 hours; about 25 hours; about 26 hours; about 27 hours; about 28 hours; about 29 hours; about 30 hours; about 31 hours; about 32 hours; about 33 hours; about 34 hours; about 35 hours; about 36 hours; about 37 hours; about 38 hours; about 39 hours; about 40 hours; about 41 hours; about 42 hours; about 43 hours; about 44 hours; about 45 hours; about 46 hours; about 47 hours; or about 48 hours.

In some aspects, ML NK cells transduced with a vector comprising a nucleic acid encoding a CAR polypeptide is incubated in the presence of IL-15 for an amount of time sufficient to express the vector and to form CAR-expressing ML NK cells. In some aspects, the amount of time sufficient to form CAR-expressing ML NK cells is between about 3 days and about 8 days. In some aspects, the amount of time sufficient to form ML NK cells can be at least about 1 day; about 2 days; about 3 days; about 4 days; about 5 days; about 6 days; about 7 days; about 8 days; about 9 days; about 10 days; about 11 days; about 12 days; about 13 days; or about 14 days.

**Compositions**

In some aspects, provided herein are compositions (e.g., pharmaceutical compositions) comprising any anti-NPM1c CAR expressing cytokine-induced memory-like NK cell, or population of said cells, disclosed herein.

Pharmaceutical compositions may comprise a pharmaceutically acceptable carrier. Appropriate pharmaceutically acceptable carriers, including but not limited to excipients and stabilizers, are known in the art (see, e.g., Remington's Pharmaceutical Sciences (1990) Mack Publishing Co., Easton, PA).

Pharmaceutical compositions can be sterile compositions that comprise cells, tethering means (e.g., lipid nanoparticles) and/or proteins or peptides, preferably in a pharmaceutically-acceptable carrier (e.g., one or more compatible solid or liquid filler, diluents or encapsulating substances that are suitable for administration to a human or other subject contemplated herein). The carrier can be an organic or inorganic ingredient, natural or synthetic, with which the cells, tethering means (e.g., lipid nanoparticles) and/or proteins or peptides are combined to facilitate administration. The components of the pharmaceutical compositions are commingled in a manner that precludes interaction that would substantially impair their desired pharmaceutical efficiency.

Pharmaceutically acceptable carriers may include, without limitation, a buffer, an emulsifying agent, a suspending agent, a dispersing agent, an isotonic agent, a wetting agent, a chelating agent, a sequestering agent, a pH buffering agent, a solubility enhance, an antimicrobial agent, an anesthetic, and/or an antioxidant.

Various excipients for formulating pharmaceutical compositions and techniques for preparing the composition are known in the art (see Remington: The Science and Practice of Pharmacy, 21st Edition, A. R. Gennaro, Lippincott, Williams & Wilkins, Baltimore, MD, 2006; incorporated herein by reference in its entirety). The use of a conventional excipient medium may be contemplated within the scope of the present disclosure, except insofar as any conventional excipient medium may be incompatible with a substance or its derivatives, such as by producing any undesirable biological effect or otherwise interacting in a deleterious manner with any other component(s) of the pharmaceutical composition. Excipients may include, for example: antiadherents, antioxidants, binders, coatings, compression aids, disintegrants, dyes (colors), emollients, emulsifiers, fillers (diluents), film formers or coatings, glidants (flow enhancers), lubricants, preservatives, printing inks, sorbents, suspending or dispersing agents, sweeteners, and waters of hydration. Exemplary excipients include, but are not limited to: saline, butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, sucrose, dextrose, magnesium stearate, malt, maltitol, mannitol, methionine, methylcellulose, methyl paraben, microcrystalline cellulose, polyethylene glycol, glycerol, ethanol, polyvinyl pyrrolidone, povidone, starch (e.g., pregelatinized starch), propylene, propyl paraben, retinyl palmitate, shellac, silica gel, silicon dioxide, sodium carboxymethyl cellulose, sodium citrate, sodium stearate, sodium starch glycolate, sorbitol, starch (corn), stearic acid, talc, base cream, titanium dioxide, vitamin A, vitamin E, vitamin C, and xylitol.

In some aspects, the pharmaceutical compositions disclosed herein may include at least one pharmaceutically acceptable salt. Examples of pharmaceutically acceptable salts that may be included in a composition of the disclosure include, but are not limited to, acid addition salts, alkali or alkaline earth metal salts, mineral or organic acid salts of basic residues such as amines; alkali or organic salts of acidic residues such as carboxylic acids; and the like. Representative acid addition salts include acetate, acetic acid, adipate, alginate, ascorbate, aspartate, benzenesulfonate, benzene sulfonic acid, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like

The pharmaceutical compositions can be formulated such that they are suitable for administration to a subject (e.g., a human). A pharmaceutical composition may be formulated for any route of administration.

The pharmaceutical compositions, when it is desirable to deliver them systemically, may be formulated for parenteral administration by injection, e.g., by bolus injection or continuous infusion. Such formulations can be prepared as liquid solutions, suspensions, emulsions or solid forms suitable making into a solution or suspension prior to injection. Formulations for injection may be presented in unit dosage form, *e.g.,* in ampoules or in multi-dose containers. Pharmaceutical parenteral formulations include aqueous solutions of the ingredients. Aqueous injection suspensions may contain substances which increase the viscosity of the suspension, such as sodium carboxymethyl cellulose, sorbitol, or dextran. Alternatively, suspensions of ingredients may be prepared as oil-based suspensions. Suitable lipophilic solvents or vehicles include fatty oils such as sesame oil, or synthetic fatty acid esters, such as ethyl oleate or triglycerides, or liposomes. If administered parenterally, suitable pharmaceutically acceptable carriers may include, without limitation, physiological saline or phosphate buffered saline (PBS), or solutions containing, e.g., polyethylene glycol, polypropylene glycol or glucose.

The CAR-expressing cell or population of said cells described herein can be used or present in a therapeutically effective amount in the pharmaceutical composition disclosed herein. The therapeutically effective amount can be determined by standard clinical techniques.

The pharmaceutically acceptable compositions contemplated herein may include, in addition to the CAR-expressing cell or population of said cells described herein, an additional anti-cancer agent (e.g., any one, two, three or more anti-cancer agents described herein).

**Therapeutic Methods and Uses**

Approximately 20,830 new cases of AML were diagnosed, and 10,460 deaths were attributed to this disease in 2015 in the United States alone. As older age is a risk factor for AML, disease incidence can increase with an aging population. Patients who are able to tolerate conventional chemotherapy and allogeneic stem cell transplantation can achieve a complete response, however, a majority experience relapse and die from the disease. Therefore, there is a need to develop less toxic and more effective targeted therapies that take advantage of our evolving understanding of cancer immunotherapy.

The disclosure provides methods for treating cancer (e.g., inhibiting cancer proliferation, inhibiting cancer progression) in a subject in need thereof comprising administering to the subject any immune effector cell (e.g., a ML NK cell) expressing a CAR polypeptide described herein, or any pharmaceutical composition described herein. In certain aspects, the disclosure provides methods for treating an NPM1c-positive cancer. As used herein a "NPM1c-positive cancer" refers to a cancer comprising tumor cells with a mutation in the *NPM1* gene (e.g., a 4 nt duplicative mutation in *NPM1*), wherein the mutation in *NPM1* results in increased cytoplasmic localization of NPM1 protein when compared to cells expressing wild-type *NPM1*. Methods of measuring gene expression in a cancer to determine the presence of a particular genetic mutation (e.g., 4 nt duplicative mutation in *NPM1*) are known in the art, and comprise analysis of a malignant tumor sample collected from a subject (e.g., blood, bone marrow, tumor, and/or tissue sample). In some aspects, methods to detect small duplications, insertions, or deletions in a gene are performed using real time quantitative polymerase chain reaction (RT-PCR), droplet digital PCR, Sanger sequencing, and next-generation sequencing (e.g., whole-genome sequencing, e.g., whole-exome sequencing). In some aspects, a NPM1c-positive cancer is detected to have a mutation in the *NPM1* gene (e.g., a 4 base pair frameshift insertion in exon 12 of the gene, a mutation encoding C-terminal 11 amino acids in an alternative reading frame, or an *NPM1* mutation resulting in expression of a protein comprising the following C-terminal amino acid sequence: MTDQEAIQDLCLAVEEVSLRK (SEQ ID NO:57)). In some aspects, a NPM1c-positive cancer comprises tumor cells with increased cytoplasmic localization of NPM1 protein. Methods of assessing NPM1 cellular localization are known in the art, for example, using a labeled anti-NPM1 antibody and assessing localization by microscopy or flow cytometry. In some aspects, tumor cells isolated from a NPM1c-positive cancer have increased cytoplasmic localization of NPM1 protein when compared to cells isolated from a healthy non-cancerous tissue sample.

In some aspects, the disclosure provides methods for treating NPM1c-positive cancer (e.g., inhibiting proliferation or progression of the cancer) in a subject in need thereof comprising administering to the subject a ML NK cell expressing a CAR polypeptide described herein, a population of said cells, or a pharmaceutical composition described herein.

In some aspects, the disclosure provides for treating AML (e.g., inhibiting proliferation or progression of AML) in a subject in need thereof comprising administering to the subject any a ML NK cell expressing a CAR polypeptide described herein, a population of said cells, or any pharmaceutical composition described herein. In certain aspects, the disclosure provides for treating an NPM1c-positive AML.

In some aspects, a ML NK cell expressing a CAR polypeptide, a population of said cells, or pharmaceutical compositions of the disclosure can be used in the development of targeted immunotherapy for treating cancer.

In some aspects, a ML NK cell expressing a CAR polypeptide a population of said cells, or pharmaceutical compositions of the disclosure can be used for the treatment of AML.

In some aspects, a ML NK cell expressing a CAR polypeptide, a population of said cells, or pharmaceutical compositions of the disclosure can be used as cytotoxic agents to kill AML cells.

In some aspects, the disclosure provides methods for treating a NPM1c-positive cancer (e.g., AML) in a subject carrying an allele encoding HLA-A2 (i.e., HLA-A*02:01 allele). In some aspects, the NPM1c-positive cancer (e.g., AML) comprises tumor cells with expression of HLA-A2. Methods of determining HLA expression are known in the art, and includes flow cytometry, immunohistochemistry, and western blot using labeled antibodies that recognize HLA-A2. HLA expression may also be determined by RT-PCR and RNA sequencing.

In some aspects, the disclosure provides methods for treating cancer (e.g., NPM1c-positive cancer, e.g., AML) in a subject in need thereof, wherein the cell surface of cells comprising the cancer displays an NPM1c neoepitope in complex with an MHC class I protein (e.g., HLA-A2), the treating comprising administering to the subject a ML NK cell expressing a CAR polypeptide described herein, a population of said cells, or any pharmaceutical composition described herein.

In some aspects, the disclosure provides for treating cancer (e.g., NPM1c-positive cancer, e.g., AML) in a subject in need thereof, wherein the cell surface of cells comprising the cancer displays AIQDLCLAV (SEQ ID NO: 1) neoepitope in complex with an MHC class I protein (e.g., HLA-A2 or a protein encoded by the HLA-A*02 allele group, such a protein encoded by the HLA-A*02:01 allele), the treating comprising administering to the subject any ML NK cells expressing a CAR polypeptide described herein, or any pharmaceutical composition described herein.

In some aspects, the disclosure provides for reducing cancer burden or increasing survival in a subject with cancer (e.g., wherein the cancer is NPM1c-positive, e.g., wherein the cancer is AML) comprising administering to the subject any ML NK cells expressing a CAR polypeptide described herein, or any pharmaceutical composition described herein. In certain aspects, the cell surface of cells comprising the cancer displays an NPM1c neoepitope (e.g., SEQ ID NO:1) in complex with an MHC class I protein (e.g., HLA-A2).

In some aspects, the disclosure provides for preventing cancer in a subject in remission from cancer comprising administering to the subject any ML NK cells expressing a CAR polypeptide described herein, or any pharmaceutical composition described herein.

In some aspects, the cancer is a relapsed cancer. In some aspects, the cancer is a refractory cancer. In one aspect, the cancer is an advanced stage cancer. In some aspects, the cancer is resistant to one or more other therapies (e.g., chemotherapy, radiotherapy, stem cell transplantation, or another immunotherapy).

In some aspects, the disclosure provides for preventing AML in a subject in need thereof comprising administering to the subject any ML NK cells expressing a CAR polypeptide described herein, or any pharmaceutical composition described herein. In one aspect, the disclosure provides for preventing AML in a subject in remission from AML.

In some aspects, the cancer to be treated is AML. In some aspects, the cancer is relapsed AML. In some aspects the cancer is refractory AML. In some aspects, the cancer is advanced AML. In some aspects, the cancer is AML resistant to one or more other therapies (e.g., chemotherapy, radiotherapy, stem cell transplantation, or another immunotherapy).

The effectiveness of any therapy described herein can be assessed by evaluating a parameter (e.g., tumor burden) before and after administration of the therapy (e.g., to the subject being treated or an animal model of the cancer being treated). Any assay known in the art can be used to evaluate the therapeutic effectiveness of the therapies described herein.

## Methods of Administration

The therapies described herein can be administered to a subject by any suitable means which include, but are not limited to, parenteral route of administration. In some aspects, the composition is administered to the patient parenterally. Non-limiting examples of suitable routes of parenteral administration include intravenous, intramuscular, intraarterial, subcutaneous, intratumoral, intrathecal and intraperitoneal administration. In some aspects, the therapies described herein are administered intravenously. In some aspects, the therapies described herein are administered intraperitoneally. In some aspects, the therapies described herein are administered intramuscularly. In some aspects, the therapies described herein are administered subcutaneously. In certain aspects, the administration is intravenous, intrathecal, intraosseous or into the spinal cord. In one aspect, the therapies described herein are administered into the spinal cord or the spinal canal. In one aspect, the therapies described herein are administered intrathecally. In one aspect, the therapies described herein are administered intraosseously. In one aspect, the therapies described herein are administered into the bone marrow.

The appropriate dosage will vary with the particular cancer being treated, the age, weight and physical condition of the subject being treated, the severity of the cancer, the route of administration, the duration of the treatment, the responsiveness of the subject being treated, the nature of the concurrent or combination therapy (if any), the specific route of administration and like factors within the knowledge and expertise of the health practitioner. In certain aspects, a maximum tolerable dose is to be used, that is, the highest safe dose according to sound medical judgment. In preferred aspects, the therapies are to be administered in effective amounts. An effective amount is a dosage of the composition sufficient to provide a medically desirable result.

For example, if the subject has a tumor, an effective amount may be that amount that reduces the tumor volume or load (as for example determined by imaging the tumor). Effective amounts may also be assessed by the presence and/or frequency of cancer cells in the blood or other body fluid or tissue (e.g., a biopsy). If the tumor is impacting the normal functioning of a tissue or organ, then the effective amount may be assessed by measuring the normal functioning of the tissue or organ.

In certain aspects, the engineered ML NK immune effector cells are administered in an amount of about or at least $1x10^4$, $5x10^4$, $1x10^5$, $5x10^5$, $1x10^6$, $5x10^6$, $1x10^7$, $5x10^7$, $1x10^8$, $5x10^8$, $1x10^9$, $5x10^9$, $1x100^{10}$, $5x100^{10}$, $1x10^{11}$, or $5x10^{11}$, $1x10^{112}$, or $5x10^{12}$ cells (or any value or range in between).

In some aspects, patients treated with the any CAR ML NK cell described herein experience complete remission. In some aspects, patients are dosed at $0.5x10^6$ cells/kg with any engineered ML NK cell. In some aspects, patients are dosed at $1.0x10^6$ cells/kg with a CAR-CIML NK cell. In some aspects, patients are dosed at $10x10^6$ cells/kg with a CAR-CIML NK cell.

Various dosing schedules of the therapies described herein are contemplated including single administration or multiple administrations over a period of time. The methods of administration include, without limitation, bolus administration and infusions (e.g., continues or pulse infusions).

The therapeutic regimen for use in the methods described herein may include administration of a therapy twice a week, once every week, once every two weeks, once every three weeks, once every month or 4 weeks, once every six weeks, once every two months or eight weeks, or once every three months or twelve weeks. In certain aspects, the subject receives a single dose of any therapy described herein. In certain aspects, the subject receives from at least two, at least three, at least four, at least five, at least six, at least eight, or at least ten doses of any therapy described herein. In certain aspects, a therapy described herein is administered daily, every other day, or two times a week. In certain aspects, a therapy described herein is administered for a period of time, such as one week, two weeks, three weeks, four weeks, six weeks, two months, three months, four months, five months, six months, or one year.

In some aspects, the initial treatment period (where the therapy is administered, e.g., a single time, twice a week, once a week, twice in two weeks, or once a month) is followed by a withdrawal period in which the antibody is not administered (for, e.g., a week, two weeks, three weeks, 1 month or four weeks, six weeks, two months or 8 weeks, three months, four months, five months, six months, or 1 year), and then followed by a second treatment period (where the therapy is administered, e.g., a single time, twice a week, once a week, twice in two weeks, or once a month). Such initial treatment and such second treatment periods can last, for example, two weeks, three weeks, four weeks, six weeks, two months, or three months (where the initial treatment period can be the same or different from the second treatment period).

## Patient Populations

The subject being treated in accordance with the methods described herein include, but are not limited to, humans and non-human vertebrates. In certain aspects, the subject being treated in accordance with the methods described herein is a mammal, such as a household pet (e.g., a dog, a cat, a rabbit, a ferret, etc.), a livestock or farm animal (e.g., a cow, a pig, a sheep, a goat, a pig, a chicken or another poultry), a horse (e.g., a thoroughbred horse), a monkey, a laboratory animal (e.g., a mouse, a rat, a rabbit, etc.), and the like. Subjects also include fish and other aquatic species. In a preferred aspect, the subject being treated in accordance with the methods described herein is a human. In one aspect, the disclosure can be practiced in any subject that is likely to benefit from targeted immunotherapy for the treatment of acute myeloid leukemia (AML). In some aspects, the disclosure is for use in a subject that has a NPM1c-positive cancer (e.g., AML).

In some aspects, the therapeutic methods and uses of the disclosure can be practiced in any subject that has (e.g., has been diagnosed with) a cancer that may (or is likely to) benefit from any immunotherapy described herein. A subject having a cancer (e.g., NPM1c-positive cancer, e.g., AML) is a subject that has detectable cancer cells. The disclosure contemplates administration of a ML NK cells expressing a CAR polypeptide described herein to subjects having a cancer (e.g., NPM1c-positive cancer, e.g., AML).

In some aspects, the therapeutic methods and uses of the disclosure can be practiced in any subject that has cancer characterized by (e.g., known to have, expected to have, or detected to have) a mutation in the *NPM1* gene (e.g., a 4 base pair frameshift insertion in exon 12 of the gene, a mutation encoding C-terminal 11 amino acids in an alternative reading frame, or an *NPM1* mutation resulting in expression of a protein comprising the following C-terminal amino acid sequence: MTDQEAIQDLCLAVEEVSLRK (SEQ ID NO:57). In certain aspects, embediments- the therapeutic methods and uses of the disclosure can be practiced in any subject that has cancer characterized by expression (e.g., known to express, expected to express, or detected to express) of a mutant NPM1 protein (e.g., an NPM1c mutant protein having cytoplasmic localization, a protein having a mutation in the C-terminal domain, a mutant protein lacking a folded C-terminal domain, a protein comprising the following C-terminal amino acid sequence: MTDQEAIQDLCLAVEEVSLRK (SEQ ID NO:57), a protein set forth by SEQ ID NO:56, or NPM1c). The mutated C-terminal sequence of NPM1c is known in the art (see, e.g., van der Lee et al., 2019, J. Clin. Invest. 129(2):774-785, see e.g., Figure 1). In some aspects, the therapeutic methods and uses of the disclosure can be practiced in any subject that has cancer, wherein the cell surface of cells comprising the cancer displays (e.g., known to display, expected to display, or detected to display) a mutant nucleophosmin neoepitope (such as NPM1c neoepitope, e.g., AIQDLCLAV (SEQ ID NO:1)) in complex with a class I major histocompatibility complex (MHC class I) protein (e.g., HLA-A2). In some aspects, the therapeutic methods and uses of the disclosure are practiced in any subject that has cancer, wherein a class I major histocompatibility complex (MHC class I) protein (e.g., HLA-A2) displays or presents an NPM1c neoepitope (e.g., AIQDLCLAV (SEQ ID NO:1)) on the cell surface of cells comprising the cancer.

Optionally, the cancer cells of the prospective patient to be treated in accordance with the methods described herein are tested for a mutation in the *NPM1* gene or NPM1 protein, or are tested to determine whether the cell surface of cells comprising the cancer display an antigen comprising an NPM1c neoepitope (e.g., AIQDLCLAV (SEQ ID NO:1)) in complex with a class I major histocompatibility complex (MHC class I) protein (e.g., HLA-A2). In some aspects, the patient is treated in accordance with the methods described herein if such a test is positive for a mutation in the NPM1 gene or a mutation in NPM1 protein, or is determined to display an antigen comprising an NPM1c neoepitope (e.g., AIQDLCLAV (SEQ ID NO:1)) in complex with a class I major histocompatibility complex (MHC class I) protein (e.g., HLA-A2) on the cell surface of cancer cells.

In some aspects, the therapeutic methods and uses of the disclosure can be practiced in a subject that has Acute Myeloid Leukemia (AML). In a specific aspect, the therapeutic methods and uses of the disclosure are practiced in a subject that has been diagnosed with AML.

Tests for diagnosing the cancers to be treated by the methods described herein are known in the art and will be familiar to the ordinary medical practitioner. These laboratory tests include, without limitation, microscopic analyses, cultivation dependent tests (such as cultures), and nucleic acid detection tests. These include wet mounts, stain-enhanced microscopy, immune microscopy (e.g., FISH), hybridization microscopy, particle agglutination, enzyme-linked immunosorbent assays, urine screening tests, DNA probe hybridization, serologic tests, etc. The medical practitioner generally takes a full history and conducts a complete physical examination in addition to running the laboratory tests listed above.

Methods for the detection of AML include, but are not limited to, flow cytometry of PBMC for leukemic cells, followed by PCR and sequencing for NPM1c mutation. Clinical methods for AML diagnosis are known in the art. Risk factors for the development of AML include smoking, chemotherapy, radiation therapy, certain blood disorder, and age.

In one aspect, the subject being treated has been diagnosed with an early stage cancer (e.g., AML). In one aspect, the subject being treated has been diagnosed with an advanced stage cancer (e.g., AML).

In certain aspects, the subject being treated has any stage of AML progression.

In some aspects, the subject being treated has previously undergone one or more other cancer therapies (e.g., chemotherapy, radiotherapy, or stem cell transplantation). In certain aspects, the subject being treated has previously undergone one or more other cancer therapies (e.g., chemotherapy, radiotherapy, or stem cell transplantation), and the subject's cancer has relapsed. In certain aspects, the subject being treated has previously undergone one or more other cancer therapies (e.g., chemotherapy, radiotherapy, or stem cell transplantation), and the subject has developed resistance to the one or more other cancer therapies. In certain aspects, the subject being treated is in remission (e.g., in partial remission or in complete remission of cancer). In certain aspects, the subject being treated is refractory to one or more other cancer therapies (e.g., chemotherapy, radiotherapy, or stem cell transplantation).

In other aspects, contemplated herein is treating a subject that is at risk of developing cancer that may (or is likely to) benefit from any immunotherapy described herein in accordance with the therapeutic methods and uses of the disclosure. A subject at risk of developing a cancer (e.g., AML) is a subject that has a higher than normal probability of developing cancer. These subjects include, for instance, subjects having a genetic abnormality that has been demonstrated to be associated with a higher likelihood of developing a cancer, subjects having a familial disposition to cancer, subjects exposed to cancer causing agents (i.e., carcinogens) such as tobacco, asbestos, or other chemical toxins, and subjects previously treated for cancer and in apparent remission. The disclosure contemplates administration of ML NK cells expressing a CAR polypeptide described herein to subjects at risk of developing a cancer (e.g., AML).

In some aspects, aspect, the subject being treated is an adult. In one aspect, the subject is a human subject over 18 years of age. In one aspect, the subject is a human subject over 21 years of age. In one aspect, the subject is a human subject over 45 years of age. In one aspect, the subject is a human subject over 65 years of age. In one aspect, the subject is a human subject under 18 years of age. In one aspect, the subject is a human subject under 45 years of age (or between 18 and 45 years of age, or between 21 and 45 years of age). In one aspect, the subject is a human subject under 65 years of age (or between 18 and 65 years of age, between 21 and 65 years of age, or between 45 and 65 years of age).

**Combination Therapies**

In some aspects, the therapeutic methods and uses described herein further include treatment of the subject with additional agents that enhance therapeutic responses, such as enhance an anti-tumor response in the subject and/or that are cytotoxic to the tumor (e.g., chemotherapeutic agents).

In some aspects, a therapy described herein is administered to a subject in combination with one or more anti-cancer therapy, e.g., a chemotherapy, a radiation therapy, stem cell transplantation, a small molecule with an anti-cancer activity, another anti-cancer immunotherapy (e.g., another anti-cancer antibody or fragment thereof, or another T cell therapy), or any other anti-cancer therapy known in the art.

In some aspects, CAR-expressing ML NK cells described herein are administered to a subject in combination with one or more anti-cancer therapy, e.g., a chemotherapy, a radiation therapy, stem cell transplantation, a small molecule with an anti-cancer activity, another anti-cancer immunotherapy (e.g., another anti-cancer antibody or fragment thereof, or another T cell therapy), or any other anti-cancer therapy known in the art.

In some aspects, one or more of the CAR-expressing ML NK cells, or compositions comprising the same, described herein are administered to a subject in combination with stem cell transplantation or another anti-cancer immunotherapy (e.g., another anti-cancer antibody or fragment thereof, or another T cell therapy). For administration with the ML NK cells comprising CARs, any combination therapies that would not negatively affect the viability of the cells are contemplated herein.

Suitable therapeutic agents for use in combination therapy include small molecule chemotherapeutic agents, including protein tyrosine kinase inhibitors, as well as biological anti-cancer agents, such as anti-cancer antibodies, including but not limited to those discussed further below.

In some aspects, combination therapy includes administering to the subject an immune checkpoint inhibitor to enhance anti-tumor immunity, such as a PD-1 inhibitor, a PD-L1 inhibitor, a PD-L2 inhibitor, or a CTLA-4 inhibitor. Other modulators of immune checkpoints may target TIM-3, OX-40, OX-40L or ICOS. In one aspect, an agent that modulates an immune checkpoint is an antibody (e.g., an antagonistic antibody to PD-1, PD-L1, PD-L2, CTLA-4, TIM-3, or OX-40). In another aspect, an agent that modulates an immune checkpoint is a protein or small molecule modulator. In another aspect, the agent (such as an mRNA) encodes an antibody modulator of an immune checkpoint. In one aspect, any therapy described herein is administered in combination with a TIM-3 inhibitor. In one aspect, any therapy described herein is administered in combination with a PD-1 inhibitor. In one aspect, any therapy described herein is administered in combination with a PD-L1 inhibitor. In one aspect, any therapy described herein is administered in combination with a CTLA-4 inhibitor. Non-limiting examples of immune checkpoint inhibitors that can be used in combination therapy include pembrolizumab, alemtuzumab, nivolumab, pidilizumab, ofatumumab, rituximab, MEDI0680, PDR001, AMP-224, PF-06801591, BGB-A317, REGN2810, SHR-1210, TSR-042, affimer, avelumab (MSB0010718C), atezolizumab (MPDL3280A), durvalumab (MEDI4736), BMS936559, ipilimumab, tremelimumab, AGEN1884, MEDI6469 and MOXR0916.

In a specific aspect, a therapy described herein is administered to a subject in combination with chemotherapy. Examples of types of chemotherapeutic agents that can be used in the combination therapy described herein include, without limitation, an alkylating agent, a nitrosourea agent, an antimetabolite, a platinum complex derivative, a topoisomerase inhibitor, an aromatase inhibitor, an alkaloid derived from a plant, a hormone antagonist, an antitumor antibiotic, and a P-glycoprotein inhibitor. Specific examples of chemotherapeutic drugs that can be used in the combination therapy described herein include, without limitation, taxol, paclitaxel, nab-paclitaxel, 5-fluorouracil (5-FU), gemcitabine, doxorubicin, daunorubicin, colchicin, mitoxantrone, tamoxifen, cyclophosphamide, mechlorethamine, melphalan, chlorambucil, busulfan, uramustine, mustargen, ifosamide, bendamustine, carmustine, lomustine, semustine, fotemustine, streptozocin, thiotepa, mitomycin, diaziquone, tetrazine, altretamine, dacarbazine, mitozolomide, temozolomide, procarbazine, hexamethylmelamine, altretamine, hexalen, trofosfamide, estramustine, treosulfan, mannosulfan, triaziquone, carboquone, nimustine, ranimustine, azathioprine, sulfanilamide, fluoropyrimidine, thiopurine, thioguanine, mercaptopurine, cladribine, capecitabine, pemetrexed, fludarabine, methotrexate, hydroxyurea, nelarabine or clofarabine, cytarabine, decitabine, pralatrexate, floxuridine, thioquanine, azacitidine, cladribine, pentostatin, mercaptopurine, imatinib, dactinomycin, cerubidine, bleomycin, actinomycin, luteomycin, epirubicin, idarubicin, plicamycin, vincristin, vinblastine, vinorelbine, vindesine, vinflunine, paclitaxel, docetaxel, etoposide, teniposide, periwinkle, vinca, taxane, irinotecan, topotecan, camptothecin, teniposide, pirarubicin, novobiocin, merbarone, aclarubicin, amsacrine, antiandrogen, anti-estrogen, bicalutamide, medroxyprogesterone, fluoxymesterone, diethylstilbestrol, estrace, octreotide, megestrol, raloxifene, toremifene, fulvestrant, prednisone, flutamide, leuprolide, goserelin, aminoglutethimide, testolactone, anastrozole, letrozole, exemestane, vorozole, formestane, fadrozole, androstene, resveratrol, myosmine, catechin, apigenin eriodictyol isoliquiritigenin, mangostin, amiodarone, azithromycin, captopril, clarithromycin, cyclosporine, piperine, quercetine, quinidine, quinine, reserpine, ritonavir, tariquidar, verapamil, cisplatin, carboplatin, oxaliplatin, transplatin, nedaplatin, satraplatin, triplatin and carboplatin.

In a specific aspect, a therapy described herein is administered to a subject in combination with one or more chemotherapies for treatment of AML. In some aspects, the one or more chemotherapies is selected from: cytarabine, daunorubicin, idarubicin, cladribine, fludarabine, mitoxantrone, etoposide, 6-thioguanine, hydroxyurea, prednisone, dexamethasone, methotrexate, 6-mercaptopurine, azacytidine, and decitabine.

In a specific aspect, a therapy described herein is administered to a subject in combination with radiation therapy.

In a specific aspect, any therapy described herein is administered to a subject in combination with stem cell transplantation.

In certain aspects, any therapy described herein can be administered before, during (i.e., concurrently) or after one or more additional anti-cancer therapy. In one aspect, the subject being treated in accordance with the methods described herein has not previously received an anti-cancer therapy. In one aspect, the subject being treated in accordance with the methods described herein has previously received an anti-cancer therapy (e.g., a chemotherapy, a radiation therapy, or a stem cell transplant).

**Kits**

Aspects of the invention are directed towards kits.

The term "kit" can refer to a set of articles that facilitates the process, method, assay, anaylsis, or manipulation of a sample. The kit can include instructions for using the kit (eg, instructions for the method of the invention), materials, solutions, components, reagents, chemicals, or enzymes required for the method, and other optional components.

For example, cells, vectors, and culture medium as described herein can be provided in a kit. In one aspect, the kit includes (a) a container that contains a composition that includes vectors or components thereof, and optionally (b) informational material. The informational material can be descriptive, instructional, marketing or other material that encompasses the methods described herein and/or the use of the agents for therapeutic benefit. In an aspect, the kit also includes a second agent, such as cells. For example, the kit includes a first container that contains the vector or composition comprising the same, and a second container that includes the second agent, such as cells.

The informational material of the kits is not limited in its form. In one aspect, the informational material can include information about production of the compound, molecular weight of the compound, concentration, date of expiration, batch or production site information, and so forth. In one aspect, the informational material encompasses methods of transducing the cells of the kit or methods of administering genetically-modified cells to a subject, e.g., in a suitable dose, dosage form, or mode of administration (e.g., a dose, dosage form, or mode of administration described herein), to treat a subject). The information can be provided in a variety of formats, include printed text, computer readable material, video recording, or audio recording, or information that provides a link or address to substantive material.

In addition to the vector and/or cells, the composition in the kit can include other ingredients, such as a solvent or buffer, culture media, a stabilizer, or a preservative. The compositions of the kit thereof can be provided in any form, e.g., liquid, dried or lyophilized form, and can be substantially pure and/or sterile. When the compositions are provided in a liquid solution, the liquid solution can be an aqueous solution or an alcohol solution. When the compositions or components thereof are provided as a dried form, reconstitution, for example, is by the addition of a suitable solvent. The solvent, e.g., sterile water or buffer, can optionally be provided in the kit.

The kit can include one or more containers for the composition or compositions containing the agents. In some aspects, the kit contains separate containers, dividers or compartments for the composition and informational material. For example, the composition can be contained in a bottle, vial, or syringe, and the informational material can be contained in a plastic sleeve or packet. In other aspects, the separate elements of the kit are contained within a single, undivided container. For example, the composition is contained in a bottle, vial or syringe that has attached thereto the informational material in the form of a label. In some aspects, the kit includes a plurality (e.g., a pack) of individual containers, each containing one or more unit dosage forms (e.g., a dosage form described herein) of the agents. The containers can include a combination unit dosage, e.g., a unit that includes the vector and/or cells and the second agent, e.g., in a desired ratio. For example, the kit includes a plurality of syringes, ampules, foil packets, blister packs, or medical devices, e.g., each containing a single combination unit dose. The containers of the kits can be air tight, waterproof (e.g., impermeable to changes in moisture or evaporation), and/or light-tight. The kit optionally includes a device suitable for administration of the composition, e.g., a syringe or other suitable delivery device. The device can be provided preloaded with one or both of the agents or can be empty, but suitable for loading.

In one aspect, provided herein are kits comprising one or more containers comprising: (i) a ML NK cell expressing a CAR polypeptide described herein, population of said cells, or a pharmaceutical composition described herein; (ii) optionally, one or more additional anti-cancer agents (e.g., a chemotherapeutic agent), and (iii) instructions for use in treating cancer in a subject.

In one aspect, the kits may comprise, in the same or separate suitable containers, a ML NK cell expressing a CAR polypeptide which binds to an antigen comprising an NPM1c neoepitope in complex with an MHC class I protein (e.g., NPM1c:HLA-A2), and a pharmaceutically acceptable carrier (e.g., a buffer).

The suitable containers may include, without limitation, a vial, well, test tube, flask, bottle, syringe, infusion bag, or other container means, into which the ML NK cell expressing a CAR polypeptide described herein, may be placed (and in some instances, suitably aliquoted). Where an additional component is provided, the kit can contain additional containers into which this component may be placed. The containers may further include injection or blow-molded plastic containers in which the desired vials are retained. Containers and/or kits can include labeling with instructions for use and/or warnings.

## Definitions

As used herein, the term "NPM1c" refers to a mutant nucleophosmin protein (NPM1), resulting from a 4-nucleotide duplication in the *NPM1* gene, which has cytoplasmic localization. Human nucleophosmin encoded by the wild-type *NPM1* gene has an amino acid sequence as set forth by SEQ ID NO:54 (accession number NM_002520). An exemplary NPM1c protein that is encoded by the *NPM1* gene with a 4-nucleotide duplication has an amino acid sequence as set forth by SEQ ID NO:56. Furthermore, the C-terminal amino acid sequence of said exemplary NPM1c protein comprises MTDQEAIQDL**CLAVEEVSLRK** (SEQ ID NO:57), with the portion of the sequence that is mutated relative to wild-type nucleophosmin protein highlighted in bold (see, e.g., van der Lee et al., 2019, JCI 129(2):774-785; Verhaak, R. et al (2005) Blood 106:3747). In some aspects, a NPM1c neoepitope of the disclosure comprises a neoepitope derived from a NPM1c protein comprising the amino acid sequence MTDQEAIQDLCLAVEEVSLRK (SEQ ID NO:57). In some aspects, a NPM1c neoepitope of the disclosure comprises a neoepitope derived from the portion of NPM1c having an amino acid sequence comprising MTDQEAIQDLCLAVEEVSLRK (SEQ ID NO:57).

As used herein, the term "NPM1c:HLA-A2" refers to a neoepitope of NPM1c in complex with an HLA-A2 protein. In some aspects, the neoepitope of NPM1c comprises an amino acid sequence AIQDLCLAV (SEQ ID NO:1).

As used herein, the term "about," when used to modify a numerical value, indicates that deviations of up to 10% above and below the numerical value remain within the intended meaning of the recited value.

As used herein, the terms "VH" or "V$_H$" refer to the heavy chain variable region of an antibody.

As used herein, the terms "VL" or "V$_L$" refer to the light chain variable region of an antibody.

As used herein, the term "percent (%) amino acid sequence identity" or "percent sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity. Alignment for purposes of determining percent amino acid sequence identity can be achieved in various ways that are known in the art, for instance, using publicly available computer software such as BLASTp, BLAST-2, ALIGN (e.g., ALIGN-2) or Megalign (DNASTAR) software. To obtain gapped alignments for comparison purposes, Gapped BLAST can be utilized as described in Altschul et al., (1997) Nucleic Acids Res. 25(17):3389-3402. In addition, the percent identity between two amino acid sequences can be determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "antibody" generally refers to an antibody comprising two light chain polypeptides and two heavy chain polypeptides (unless the context in which this term is used suggests otherwise). Antibodies include different antibody isotypes including IgM, IgG, IgA, IgD, and IgE antibodies. The term "antibody" includes, without limitation, a polyclonal antibody, a monoclonal antibody, a chimerized or chimeric antibody, a humanized antibody, a primatized antibody, a deimmunized antibody, and a fully human antibody. The antibody can be made in or derived from any of a variety of species, e.g., mammals such as humans, non-human primates (e.g., orangutan, baboons, or chimpanzees), horses, cattle, pigs, sheep, goats, llama, dogs, cats, rabbits, guinea pigs, gerbils, hamsters, rats, and mice. The antibody can be a purified or a recombinant antibody

As used herein, the term "antibody fragment," "antigen binding fragment," or similar terms refer to a fragment of an antibody that retains the ability to bind to a target antigen. Such fragments include, without limitation, a single chain antibody, a single chain Fv fragment (scFv), a Fab fragment, a Fab' fragment, and a F(ab')$_2$ fragment. This term also includes, e.g., single domain antibodies such as camelid single domain antibodies. See, e.g., Muyldermans et al. (2001) Trends Biochem Sci 26:230-235; Nuttall et al. (2000) Curr Pharm Biotech 1:253-263; Reichmann et al. (1999) J Immunol Meth 231:25-38; PCT application publication nos. WO 94/04678 and WO 94/25591; and U.S. patent no. 6,005,079. In some aspects, the disclosure provides single domain antibodies comprising two VH domains with modifications such that single domain antibodies are formed. In addition, intrabodies, minibodies, triabodies, and diabodies are also included in the definition of antibody fragments and are compatible for use in the methods described herein. See, e.g., Todorovska et al. (2001) J Immunol Methods 248(1):47-66; Hudson and Kortt (1999) J Immunol Methods 231(1):177-189; Poljak (1994) Structure 2(12):1121-1123; Rondon and Marasco (1997) Annual Review of Microbiology 51:257-283.

As used herein, the term an "amino acid substitution" or "substituted" (when such term is referred to a substituted amino acid) refers to the replacement of at least one existing amino acid residue in a predetermined amino acid sequence with a different amino acid residue. The term "amino acid insertion" refers to the incorporation of at least one additional amino acid into a predetermined amino acid sequence. While the insertion will usually consist of the insertion of one or two amino acid residues, larger "peptide insertions," can also be made. The replaced or inserted amino acid residue(s) may be naturally occurring or non-naturally occurring (modified). The term "amino acid deletion" refers to the removal of at least one amino acid residue from a predetermined amino acid sequence.

As used herein, a "conservative amino acid substitution" is one in which the amino acid residue is replaced with an amino acid residue having a similar side chain. Families of amino acid residues having similar side chains have been defined in the art, including:

(1) hydrophobic side chains: norleucine, Met, Ala, Val, Leu;
(2) neutral hydrophilic side chains: Cys, Ser, Thr, Asn, Gln;
(3) acidic side chains: Asp, Glu;
(4) basic side chains: His, Lys, Arg;
(5) side chains that influence chain orientation: Gly, Pro; and
(6) aromatic side chains: Trp, Tyr, Phe.

For example, a non-conservative amino acid substitution is a substitution of an amino acid residue with an amino acid residue with a substantially different side chain (i.e., an amino acid residue that is a member of a different family).

In some aspects, a conservative amino acid substitution is made by considering the hydropathic index of the amino acid residue. Each amino acid is assigned a hydropathic index on the basic of its hydrophobicity and charge characteristics. They are: Ile (+4.5); Val (+4.2); Leu (+3.8); Phe (+2.8); Cys (+2.5); Met (+1.9); Ala (+1.8); Gly (-0.4); Thr (-0.7); Ser (-0.8); Trp (-0.9); Tyr (-1.3); Pro (-1.6); His (-3.2); Glu (-3.5); Gln (-3.5); Asp (-3.5); Asn (-3.5); Lys (-3.9); and Arg (-4.5). The importance of the hydropathic amino acid index in conferring interactive function on a polypeptide is understood in the art (see, e.g., Kyte et al (1982) J Mol Biol 157:105-131). In some aspects, a conservative amino acid substitution is made by replacing one amino acid residue with another amino acid residue having a the same or similar (e.g., within about +2, +1.5, +1, +0.5, -0.5, -1, -1.5, or -2) hydropathic index.

In some aspects, a conservative amino acid substitution is made by considering the hydrophilicity of the amino acid residue. The following hydrophilicity values have been assigned: Arg (+3.0); Lys (+3.0±1); Asp (+3.0±1); Glut (+0.2); Gly (0); Thr (-0.4); Pro (-0.5±1); Ala (-0.5); His (-0.5); Cys (-1.0); Met (-1.3); Val (-1.5); Leu (-1.8); Ile (-1.8); Tyr (-2.3); Phe (-2.5); and Trp (-3.4). In some aspects, a conservative amino acid substitution is made by replacing one amino acid residue with another amino acid residue having a the same or similar (e.g., within about +2, +1.5, +1, +0.5, -0.5, -1, -1.5, or -2) hydrophilicity. Exemplary amino acid substitutions are set forth in **Table 2.**

**Table 2: Conservative amino acid substitutions**

| Original Residue | Exemplary Substitution | Preferred Substitution |
|---|---|---|
| Ala | Val, Leu, Ile | Val |
| Arg | Lys, Gln, Asn | Lys |
| Asn | Gln | Gln |
| Asp | Gln | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Nle | Leu |
| Leu | Nle, Ile, Val, Met, Ala, Phe | Ile |
| Lys | Arg, Dbu, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Nle | Leu |
| Nle = norleucine<br>Dbu = 2,4-diaminobutyric acid | | |

As used herein, the term "cytokine-induced memory-like NK cell" or "ML NK cell" refers to a NK cell derived from an NK cell which has been activated *ex vivo* with at least one cytokine and maintains an enhanced memory-like function after challenge in the absence of the same cytokines.

As used herein, the term "CIML NK cell" refers to a NK cell derived from an NK cell which has been activated with at least one cytokine and exhibits enhanced activation and interferon-gamma responses.

As used herein, the term "isolated nucleic acid molecule" refers to a nucleic acid molecule that has been separated from a component of its natural environment. An isolated nucleic acid molecule is present at a location that is different from its natural chromosomal location.

As used herein, the term "neoepitope" refers to a disease-specific antigen comprising a peptide that arises from disease-specific mutations, which are recognized as different from self and presented on the surface of cells affected by the disease but not normal cells. A "tumor neoepitope" or "cancer neoepitope" refers to a tumor- or cancer-specific antigen comprising a peptide that arises from tumor- or cancer-specific mutations, which are recognized as different from self and presented on the surface of tumor/cancer cells but not normal cells. Presentation of a tumor- or cancer-specific neoepitope occurs following intracellular processing and cleavage of a tumor- or cancer-specific antigen within a tumor cell, thereby producing one or more distinct peptides of 8-15 amino acids comprising the tumor- or cancer-specific mutations. The subset of these peptides that bind MHC class I or II molecules for presentation to CD8+ or CD4+ T cells, respectively, constitute tumor neoepitopes.

As used herein the term "$K_D$" or "Kd" or "$K_d$" has the same meaning as commonly understood by one of ordinary skill in the art, and refers to the equilibrium dissociation constant of a binding reaction between an antibody (or antigen binding fragment thereof) and an antigen. The value of $K_D$ is a numeric representation of the ratio of the antibody off-rate constant (koff) to the antibody on-rate constant (kon). The value of $K_D$ is inversely related to the binding affinity of an antibody to an antigen. The smaller the $K_D$ value the greater the affinity of the antibody for its antigen. Affinity can be measured by any method known in the art.

As used herein, the term "koff" or "$k_{off}$" has the same meaning as commonly understood by one of ordinary skill in the art, and refers to the off-rate constant for the dissociation of an antibody from an antibody/antigen complex. The value of koff is a numeric representation of the fraction of complexes that decay or dissociate per second, and is expressed in units $sec^{-1}$.

As used herein, the term "kon" or "$k_{on}$" has the same meaning as commonly understood by one of ordinary skill in the art, and refers to the on-rate constant for the association of an antibody with an antigen. The value of kon is a numeric representation of the number of antibody/antigen complexes formed per second in a 1 molar (1M) solution of antibody and antigen, and is expressed in units $M^{-1}sec^{-1}$.

As used herein, the terms "specific binding," "specifically binds," "selective binding," and "selectively binds," are intended to mean that an antibody or antigen-binding fragment thereof, or extracellular domain, that exhibits appreciable affinity for a particular antigen or epitope (e.g., NPM1c:HLA-A2) and, generally, does not bind to, or substantially does not bind to, other antigens and epitopes (e.g., HLA-A2 alone, e.g., NPM1c neoepitope alone, e.g., non-NPM1c neoepitope in complex with HLA-A2). "Appreciable" or preferred binding includes binding with a $K_D$ of $10^{-7}$, $10^{-8}$, $10^{-9}$, or $10^{-10}$ M or better. The $K_D$ of an antibody antigen interaction (the affinity constant) indicates the concentration of antibody at which 50% of antibody and antigen molecules are bound together. Thus, at a suitable fixed antigen concentration, 50% of a higher (i.e., stronger) affinity antibody will bind antigen molecules at a lower antibody concentration than would be required to achieve the same percent binding with a lower affinity antibody. Thus a lower $K_D$ value indicates a higher (stronger) affinity. As used herein, "better" affinities are stronger affinities, and are of lower numeric value than their comparators, with a $K_D$ of $10^{-7}$ M being of lower numeric value and therefore representing a better affinity than a $K_D$ of $10^{-6}$ M. Affinities better (*i.e.,* with a lower $K_D$ value and therefore stronger) than $10^{-7}$ M, preferably better than $10^{-8}$ M, are generally preferred. Values intermediate to those set forth herein are also contemplated, and a preferred binding affinity can be indicated as a range of affinities, for example preferred binding affinities for antibodies that bind NPM1c:HLA-A2 disclosed herein are, $10^{-7}$ to $10^{-12}$ M, more preferably $10^{-8}$ to $10^{-12}$ M.

An antibody, antigen binding fragment or extracellular domain that "does not bind to" or "substantially does not bind to" an antigen is one that will not appreciably bind to an off-target antigen (e.g., MHC class I protein alone, e.g., neoepitope alone e.g., a control peptide in complex with the MHC class I protein). For example, in one aspect, binds to NPM1c:HLA-A2 will exhibit at least a two, and preferably three, or four or more orders of magnitude better binding affinity (*i.e.,* binding exhibiting a two, three, or four or more orders of magnitude lower $K_D$ value) for NPM1c:HLA-A2 than, e.g., HLA-A2 alone, NPM1c neoepitope alone, and/or a non-NPM1c neoepitope in complex with HLA-A2. Specific or selective binding can be determined according to any art-recognized means for determining such binding, including, for example, according to Scatchard analysis, Biacore analysis, bio-layer interferometry, and/or competitive (competition) binding assays.

As used herein, the term "HLA-A" has the same meaning as commonly understood by one of ordinary skill in the art and refers to a group of human leukocyte antigens (HLA) that are encoded by the HLA-A locus in humans. HLA is a major histocompatibility complex (MHC) antigen specific to humans. HLA-A is one of three major types of human MHC class I cell surface receptors. The others are HLA-B and HLA-C. The HLA-A protein is a heterodimer, and is composed of a heavy α chain and smaller β chain. The α chain is encoded by a variant HLA-A gene, and the β chain (β2-microglobulin) is an invariant β2 microglobulin molecule. The β2 microglobulin protein is coded for by a separate region of the human genome. HLA-A*02 (A*02) is a human leukocyte antigen serotype within the HLA-A serotype group. The serotype is determined by the antibody recognition of the α2 domain of the HLA-A α-chain. For A*02, the α chain is encoded by the HLA-A*02 gene and the β chain is encoded by the B2M locus.

As used herein, the term "effective dose" or "effective amount" refers to an amount sufficient to achieve or at least partially achieve the desired therapeutic effect.

The following examples are offered by way of illustration and not by way of limitation. Various other aspects of the invention may be practiced, given the general description provided herein.

## EXAMPLES

Cellular therapies that harness the ability of immune cells to recognize, respond and kill neoplastic cells represent a promising approach for treating advanced malignancies. Efficient cellular transduction has been achieved for T cells, but not for NK cells-a major technical drawback in improving NK cell-based cancer immunotherapy. NK cells are innate lymphoid cells that can eliminate virus-infected and malignantly transformed cells. The conventional lentiviral transduction approach uses a canonical lentivirus pseudotyped by vesicular stomatitis virus G (VSVG); however, NK cells are known to be very resistant to VSVG-peudotyped lentiviral transduction with a gene editing rate 1-3%. As described herein, we have identified compositions and methods to increase the transduction efficiency of NK cells. By studying the unique receptor expression pattern of NK cells, our data showed that in sharp contrast to LDL-R, expression levels of ASCT2 are abundant in primary conventional NK cells and are further enhanced in cytokine-induced memory-like (CIML) NK cells. ASCT2 is an amino acid transporter and corresponding receptor of baboon envelope glycoprotein (BaEV-gp1). By utilizing an unconventional BaEV pseudotyped lentivirus, we overcome the transduction block in primary human NK cells and in CIML NK cells. Our data with BaEV lentivirus confirms that primary NK cells are readily accessible to this unconventional gene-editing approach while CIML NK cells are more amenable to lentiviral transduction than their naive counterparts, which is well correlated to their ASCT2 expression levels. Our transduction efficiency with CIML NK cells is now close to 40-70%

### Abbreviations

AIQ, AIQDLCLAV (SEQ ID NO: 1); AML, acute myeloid leukemia; alloSCT, allogeneic hematopoietic stem cell transplantation; BLI, Bioluminescence imaging; CAR, chimeric antigen receptor; CAR-NK, chimeric antigen receptor NK cell; FACS, fluorescence-activated cell sorting; FBS, fetal bovine serum; GIL, GILGFVFTL (SEQ ID NO: 63); HSCs, hematopoietic stem cells; MACS, magnetic-activated cell sorting; NPM1, nucleophosmin; NPM1c, mutant nucleophosmin; scFv, single-chain variable fragment; SLL, SLLMWITQC (SEQ ID NO: 62); TAAs, tumor-associated antigens; HSPCs, hematopoietic stem/progenitor cells.

### The following materials and methods were used for the following Examples

#### Cell line culture

OCI-AML3 cells were purchased from ATCC. OCI-AML2 cells were purchased from DSMZ. OCI-AML3 cells and OCI-AML2 were cultured in RPMI 1640 medium (Gibco) supplemented with 10% FBS (Life Tech and VWR) and 2 mM L-Glutamine (Thermo Fisher Scientific).

#### CAR vector design

The sequence of CAR, consisting of the anti-NPM1c scFv (SEQ ID NO: 2), the CD8α leader sequence, extracellular hinge domain and transmembrane domain, the 4-1BB co-stimulatory domain, and the CD3zeta activation domain, was custom-synthesized by Integrated DNA Technologies (IDT). In some constructs, the CAR was linked to secreted or membrane bound IL-15 nucleotide sequence via a cleavable linker. The pHIV vector (plasmid #21373) was doubly digested with the enzymes XbaI and ClaI. After gel purification of the vector backbone, the pHIV backbone, CAR fragment and P2A-GFP fragment were assembled basing on their overlap region at 5' and 3' terminals using HiFi DNA Assembly Master Mix (New England BioLabs) according to the manufacturer's protocol. The resulting plasmids were sequenced using following sequencing primers: 5'-GTTAGGCCAGCTTGGCACTTGATGT-3' (SEQ ID NO: 69) (forward) and 5'-AGGCACAATCAGCATTGGTAGCTG-3' (SEQ ID NO: 70) (reverse). The plasmid with the correct sequence was named pHIV-CAR-GFP.

#### Generation of CAR-expressing primary human NK cells

Lentivirus was generated by transfecting 293T cells with pHIV-CAR-GFP, BaEV-gp (or pCMV-VSVG), pCMV-Δ8.9, and pAdv plasmids. Culture supernatants were collected at 48 and 72 hrs and lentivirus particles were pelleted by ultracentrifugation at 25,000 rpm, 4 °C for 2 hrs. Lentivirus particles were suspended in 100 μL if serum-free DMEM media and frozen at -80 °C. Human NK cells were isolated from donor peripheral blood mononuclear cells (PBMCs) using ficoll centrifugation and were purified using Rosette Sep (StemCell Technologies, $\geq$ 95% CD56+CD3-). The NK cells were pre-activated by plating 3-5x10$^6$ cells and activating for 16 hours using rhIL-12 (10 ng/mL) + rhIL-18 (50 ng/mL) + rhIL-15 (50 ng/mL) or control conditions (rhIL-15, 50ng/mL), washed 3 times with PBS to remove cytokines, and cultured in complete RPMI 1640 medium containing 10% human AB serum (Sigma-Aldrich) supplemented with rhIL-15 (1 ng/mL) to support survival, with 50% of the medium being replaced every 2-3 days with fresh cytokines. NK cells were transduced with lentivirus (MOI=10) 1 day after pre-activation. CAR-NK cells were expanded in media supplemented with 1ng/mL or rhIL-15. Cells were rested after pre-activation and transduction for 10 days.

### *Cytotoxicity assay by measuring luciferase activity*

Cytotoxicity assays of CAR-NK cells were performed using luciferase-expressing target cell lines. NK cells were incubated with target cells at indicated effector:target (E:T) ratios for 24 hr. Cells were then rinsed once in PBS, lysed in luciferase cell culture lysis reagent (Promega), and subsequently mixed with luciferase assay reagent (Promega). Luminescence of the lysates was analyzed using a plate spectrophotometer (Infinite M200PRO, TECAN). The luminescence of target cells alone was used as a baseline control. Specific lysis of each sample was calculated using the following formula: specific lysis (%) = $100 \times$ {1 - [(luminescence in CAR-T group /luminescence in target cells alone)/(luminescence in untransduced **T** group /luminescence in target cells alone)] }.

### *CAR-NK cells killing of target cells in vivo*

NOD-scid IL2rg$^{null}$ (NSG) mice were purchased from the Jackson Laboratories and housed in the specific pathogen-free (SPF) vivarium at the Dana-Farber Cancer Institute. All experiments with mice were approved by the Institutional Animal Care and Use Committee. Briefly, luciferase-expressing OCI-AML3 cells ($1 \times 10^6$), were injected in 200uL of PBS into irradiated NSG mice by tail vein injection. After 4 days, 500k CAR-NK cells were injected with a total of 1x10$^6$ NK cells into the tumor-bearing mice. Bioluminescence imaging (BLI) was performed every three days using a Xenogen IVIS-200 Spectrum camera.

### *Exemplary Preparation of Lentivirus:*

*(1) Expansion of Plasmids:*

All 3 lentiviral packaging plasmids are grown in Stbl3 chemically competent *E. coli* **(Thermo Fisher C737303).** To grow the validated plasmids, use 100ml of LB broth with 100 $\mu$g/mL ampicillin for Midi Prep **(Qiagen # 2945),** or 1000ml of LB broth with 100 $\mu$g/mL ampicillin for Endo-free Maxi Prep **(Qiagen #12362).**

*(2) Growing* 293T cells:

293T cells cultured in 20 ml 10% FBS DMEM, and reach 100% confluence. The cells are not passaged past 15 splits. The cells are not passaged to grow to near 100% confluency. 150mm x 25mm tissue culture dishes **(Thermo Fisher Scientific #0877224).** Complete DMEM media (with 10% FBS + P/S+2 mM L-glut)

*(3) Transfection of 293T cells:*

Two to three days before transfection, plate 5E6 cells/15cm plate. Allow to grow to almost 100% confluence. Allow all transfection reagents to warm to room-temperature before use. Transfect as follows:

| Reagent | Amount | | |
|---|---|---|---|
| Transfer Plasmid | 20 μg | A:<br><br>Mix and stay for 5 mins. | Mix A solution and B solution, stay for 30 mins at RT |
| BaEV | 10 μg | | |
| Delta 8.9 | 10 μg | | |
| pAdv | 5 μg | | |
| Opti-MEM **(Life Technologies #31985088)** | To 2.5 mL | | |
| | | | |
| TransIT-LT1 **(Mirus #2306)** | 135 μL | B:<br><br>Mix and stay for 5 mins. | |
| Opti-MEM **(Life Technologies #31985088)** | 2.365 mL | | |

Transfer dropwise to 293T in a spiral fashion to evenly culture dish.

(Optional): at 8 hours-post transfection, check for syncytia formation and (if applicable) fluorescence.

Day +2 (48 hours after transfection): Harvest and replace with 15 mL pre-warmed complete DMEM. Store the supernatant in 4°C for one day before moving forward to concentration together with the secondary harvest.

Day +3 (72 hours after transfection): Harvest. Bleach and toss plate.

(4) *Concentration of lentivirus*

Collect the virus supernatant.

Spin supernatant in centrifuge for 5 minutes 350xg to pellet cellular debris. Filter supernatant (0.45 um syringe filter) into ultracentrifuge tube. Add medium up to a total volume of ~35mL, balancing with holder.

Spin 2h, 25,000 rpm, 4°C.

Pour off supernatant; completely remove remaining medium and add 100 ul serum-free DMEM media to dissolve pellet overnight at 4 °C. Do not pipette up and down. 5. Next day, gently pipette up and down to mix (careful not to form bubbles), and transfer over into a 1.5 mL Eppendorf tube.

Add 100 uL serum-free DMEM media into the ultracentrifuge tube to wash. Gently pipette up and down and transfer into the same 1.5 mL Eppendorf tube.

Freeze 20-50 ul aliquots -80°C.

(5) *Titering of lentivirus*

Titer the virus by FACS using Jurkat cell line.

Grow Jurkat in complete RPMI (containing 10% FBS, P/S, and 2mM L-glut).

Plate out 1 million/1 mL 12-well x 2 well (duplicates) per virus, supplemented with 1 uL polybrene (f.c. 10ug/mL), with two additional wells for untransduced controls.Add 1 μL of concentrated BaEV lentivirus per well.

Spinfect in a centrifuge at 1000xg for 1 hour at 32°C, brake off or brake-low (to prevent spillage).

At 72 hours, flow for GFP+ (or ScFv+).

Gate on FSC/SSC, Live/Dead dye negative, GFP+ (or ScFv+).

Assuming MOI=1 for Jurkat, calculate the viral titer as follows:

Subtract background GFP+ (or ScFv+) from untransduced control,

$$\text{virus concentration (infection unit } /\mu L) = (1E6) * \%\text{positive}$$

(6) *Spinfection of human primary memory-like NK cells*

Coat a plate using 20ug/ml RetroNectin (PBS) with a volume corresponding to 4 ug/cm$^2$ plate area. Allow the plate to stand for 2h at room temperature (or alternatively overnight at 4°C).

*Dispense 0.5ml into each well of a 24-well plate or 2 ml into each well of a 6-well plate. Non-treated, cell culture-grade tissue culture plates or dishes can be used in this step.

Remove the RetroNectin solution and then block with an appropriate volume of sterile 2% BSA in PBS. Allow the plate to stand at room temperature for 30 minutes.

Use 0.5ml for each well of a 24-well plate or 2ml for each well of a 6-well plate.

Remove the BSA solution, and wash the plate once with an appropriate volume of PBS. After removing the wash solution, the plate is ready for use.

NK cells were isolated from fresh human PBMC (collar) the day before, and preactivated with rhIL-2 (500 IU/ml) + rhIL-12 (10ng/ml) +rhIL-18 (50ng/ml) at $10^6$ cells/mL in Miltenyi NK expansion medium with 5% human AB serum.

After 16-20 hrs of cytokine activation, the cells were washed twice and then suspended at $5 \times 10^6$ cells/mL in serum-free NK expansion medium with 500 U/mL IL-2. Mixture (300ul) of Vectofusin-1 and lentivirus (final MOI=10) was added into 200 uL cell suspension for transduction in one well of 24-well plate. Final concentration of Vectofusin-1 was 10$\mu$g/mL.

After spinoculation at 1000xg, 32°C for 1 h (very simple and one-time spinfection), the cells were cultured with the lentivirus for 48 h. The cell culture medium was then exchanged with fresh complete cell culture medium containing 5% human AB serum and 500 U/mL IL-2.

Measure CAR expression at day 3-4 post-transduction via FACS and and assess in vitro NK cell anti-tumor function at day 5-6 post-transduction via FACS and luciferase assays. For in vivo efficacy and safety test, transfer CAR-NK cells at day 6-10 post-transduction into tumor bearing NSG mice.

**Statistical analysis**

Data are expressed as mean $\pm$ s.e., from at least three independent experiments, unless otherwise stated. A two-tailed t-test was used to compare two independent groups. Tumor growth in vivo was compared using two-way repeated-measures ANOVA. The Kaplan-Meier method was used to analyze survival patterns in tumor-bearing mice and statistical differences were evaluated according to the Mantel-Cox log-rank test. A *p* value < 0.05 was considered statistically significant. All of statistical analysis was performed using SPSS Statistics 22 software.

**Example 1: Engineering Memory-Like NK Cells with a CAR construct**

*Acute myeloid leukemia (AML) Continues to be a Major Therapeutic Challenge.*

Approximately 20,830 new cases of AML were diagnosed, and 10,460 deaths were attributed to this disease in 2015 in the United States alone. As older age is a risk factor for AML, disease incidence can increase with an aging population. Patients who are able to tolerate conventional chemotherapy and allogeneic stem cell transplantation can achieve a complete response, however, a majority experience relapse and die from the disease (Falini B, et al. Discov. Med. 2010;10(53):281-92). Therefore, there is a need to develop less toxic and more effective targeted therapies that take advantage of our evolving understanding of cancer immunotherapy. Although allogeneic hematopoietic stem cell transplantation (allo-HSCT) is an effective treatment, many patients are not candidates due to progressive disease, age, co-morbidities, or lack of an HLA-matched donor, and long-term clinical success is limited by graft versus host disease (GVHD), infection, and organ toxicity. Graft versus leukemia (GVL), the underlying immune therapeutic basis for allo-HSCT, has traditionally been ascribed to T cells, however more recent studies have identified Natural Killer (NK) cells to play an important role for additional GVL (Venstrom JM, et al.N. Engl. J. Med. 2012;367(9):805-816; Cooley S, Blood. 2010;116(14):2411-9). Here, we describe a new approach to arm NK cells for adoptive immunotherapy based on innate NK cell memory.

*Cytokine Induced Memory-Like (CIML) NK Cells and Cancer Immunotherapy.*

Cellular therapies that harness the ability of immune cells to recognize, respond and kill neoplastic cells represent a promising approach for treating advanced malignancies. In this context, dramatic clinical responses observed with chimeric antigen receptor (CAR) T cells demonstrate the great potential of this approach. However, the absence of tumor specific antigens in a great number of cancers, loss of tumor target antigen, poor in vivo persistence and severe toxicity including cytokine release syndrome (CRS) and neurotoxicity remain major challenges with CAR T cell therapies. Furthermore, patients who relapse after CAR T cell infusion have very limited treatment options. NK cells are innate lymphoid cells that intrinsically can eliminate virus-infected and malignantly transformed cells. Conventional NK cells have shown some promise in early phase clinical trials, however these infusions resulted in relatively short remissions in a minority of patients. Recently, paradigm-shifting studies have shown that NK cells exhibit innate immune memory. We discovered that brief *in vitro* pre-activation with IL-12, IL-15, and IL-18 results in the activation and differentiation of resting NK cells to generate cytokine-induced memory-like (CIML) NK cells with potent anti-leukemia activity **(FIG. 1)** (Romee R, et al. Blood. 2012;120(24)). In our first-in-human phase 1 trial, CIML NKs induced clinical responses in >50% of patients with relapsed refractory AML, with no apparent toxicity (Romee R, Sci. Transl. Med. 2016; 21:357ra123). Furthermore the cells proliferated, expanded and maintained enhanced anti-leukemia activity after adoptive transfer into the patients. Id. These data demonstrate that CIML NK cell therapy is effective,safe, and offers anti-leukemic clinical activity. CIML NK cells are currently being evaluated in several studies including our ongoing trial in patients with myeloid malignancies relapsed after allo-HSCT.

### CIML NK Cells as a CAR Platform.

Recent studies using allogeneic cord blood derived NK cells as a platform for generating CD19-CARs have shown promising clinical responses in patients with relapsed B cell malignancies (Rezvani et al, ASH 2018). Notably clinical responses were achieved with minimal toxicity and no cases of severe cytokine release syndrome (CRS) or neurotoxicity. These clinical observations indicate that NK cells represent a new cellular platform for genetically modified adoptive cell therapy. We will build on these observations along with our prior clinical experience with CIML NK cell therapy to develop methods for generating CIML NK CARs and to evaluate the efficacy of these genetically modified NK cells in preclinical models. CIML NK cells provide a unique platform for development of NK cell CARs based on the favorable safety profile, increased proliferation, prolonged persistence and enhanced anti-leukemia function seen *in vivo* in pre-clinical animal models and in patients treated with genetically un-modified CIML NK cells. Furthermore, their intrinsic propensity to target myeloid blasts makes them attractive for AML where CAR T cells have shown only modest to none benefit primarily due to lack for good surface target antigens.

### CAR Targets in AML.

AML is a molecularly diverse malignancy. Mutations in NPM1c result in aberrant cytoplasmic localization of proteins and/or presentation by MHC. We have successfully generated and validated a new CAR construct which is able to target a neoantigen generated from NPM1c on the cell surface of AML blasts.

### Generating NPM1c targeted CAR using memory-like NK cell platform.

Aspects described herein comprise an NK cell-based CAR against AML with mutated NPM1c. Compared to TCR and conventional CAR approaches, our approach has an advantage to arm NK cells. NK cells can recognize and kill target cells that have no or low HLA expression. This can be important because leukemia cells that lack HLA expression or have low levels of HLA molecules can be killed by NK cells, and those leukemia cells that express high level of HLA can be efficiently killed by our CAR-NK cells. As antigen loss is a mechanism of tumor resistance following CAR-T therapy, our CAR-NK cells can prove effective in reducing resistance to therapy and disease relapse. Without wishing to be bound by theory, our mem-NK CAR approach will significantly enhance targeting of leukemic blasts without affecting the normal hematopoietic stem cells and progenitors, which have been one of the challenges with T cell, based CAR approaches in AML.

### Optimization of the methods for <u>in vitro</u> generation of CIML CAR-NK cells.

We are optimizing transduction of CIML NK cells using our CAR constructs. CIML NK are being generated from conventional peripheral blood NK cells (from normal healthy volunteer donors) using our previously described methods (Romee R, et al. Blood. 2012;120(24) and Romee R, Sci. Transl. Med. 2016; 21:357ra123). Conventional and CIML NK cells (from the same donors) will be transduced with our CAR constructs and compared for their transduction efficiency. Data shows cytokine activated NK cells are more amenable to lentiviral transduction as well as nucleofection. The transduced NK cells are assessed for *in vitro* activity against OCI-AML3 (HLA-A2+ antigen +), GMB (HLA-A2- antigen -) and K562 (HLA negative but susceptible to NK cells). We will also test their activity against HLA-A2+ antigen+ primary AML blasts from patients. We have also developed an NK cell specific CyTOF panel, which allows us to assess up to 38 markers in a single cell fashion enabling a detailed immune subset analysis.
We are in the process of expanding aspects herein to effectively target other mutated proteins in AML and other myeloid malignancies (e.g., MDS, MPN).

### Compare activity of CIML NK and T cell CARs (with the same CAR constructs) <u>in vitro</u> and <u>in vivo</u> using xenograft and PDX mouse models.

We will assess CIML CAR-NK cells and the T cell CARs (bearing the same constructs) for their *in vitro* and *in vivo* activation, proliferation, persistence and efficacy in xenograft and PDX mouse model. The animals will be sacrificed at 7, 14, 28 and 60 days after adoptive transfer to assess *in vivo* persistence, expansion and activity (based on BLI tumor imaging and survival) and exhaustion of the cells. We will also assess trafficking/homing of these cells into the key organs including bone marrow and spleen in these mice.

***Optimize expansion methods to develop an off-shelf product for an early phase clinical trial.***

Various ex vivo expansion methods using feeder cell lines (IL21/41BB transduced K562 cells) and feeder free methods (Sutlu et al, Cytotherapy 2010) will be compared for optimal short-term expansion and fresh infusion without cryopreservation (Sutlu T, et al. Cytotherapy. 2010;12(8):1044-55; Denman CJ, et al, PLoS One. 2012;7(1):e30264). These expansion methods will also be tested on the CliniMACS Prodigy system, which allows manufacturing process in an automated and closed system and thus ideal for our future trials. We will develop an automated cell manufacturing approach with short turn-around time that can be easily exported to other GMP facilities. After optimizing the process as outlined herein, we will transfer this process to the CMCF (Cell Manipulation Core Facility) at Dana Farber. We already have 2 active IND's for manufacturing of CIML NK cells and we have already validated the manufacturing process of these cells. We intend to submit our IND for using CIML NK cell CAR.

**Example 2: Optimization of transduction efficiency of the memory-like NK cells based on the ASCT2 expression**

The technical aspects of producing genetically manipulated, potent CAR-NK cells are critical. Efficient transduction has been achieved for T cells, but not for NK cells-a major technical drawback in improving NK cell-based cancer immunotherapy. The conventional lentiviral transduction approach uses a canonical lentivirus pseudotyped by vesicular stomatitis virus G (VSVG); however, NK cells are known to be very resistant to VSVG-peudotyped lentiviral transduction with a gene editing rate 1-3%.

It was evaluated if CIML NK cells have a distinct receptor expression relative to conventional NK cells that can be exploited to mediate transduction by pseudotyped lentivirus. CIML NK cells were prepared from conventional peripheral blood NK cells isolated from normal healthy volunteer donors as described in Romee, R. et al. Blood 120, 4751-4760 (2012) and Romee, R. et al. Sci Transl Med 8, 357ra123 (2016) and shown in **FIG. 1.** Briefly, hNK cells were isolated from donor peripheral blood mononuclear cells (PBMCs) using ficoll centrifugation and purification by Rosette Sep (StemCell Technologies, $\geq$ 95% CD56+CD3-). The hNK cells were pre-activated by plating 3-5x10$^6$ cells and activating for 16 hours using rhIL-12 (10 ng/mL) + rhIL-18 (50 ng/mL) + rhIL-15 (50 ng/mL) or control conditions (rhIL-15, 50 ng/mL), washed 3 times with PBS to remove cytokines, and cultured in complete RPMI 1640 medium containing 10% human AB serum (Sigma-Aldrich) supplemented with rhIL-15 (1 ng/mL) to support survival, with 50% of the medium being replaced every 2-3 days with fresh cytokines.

Expression of LDL-R, the receptor ligated by VSVG protein, was evaluated by flow cytometry. Both conventional NK cells and CIML NK cells were found to have low expression of LDL-R **(FIG. 2A)**. Accordingly, the transduction block in NK cells (i.e., using VSVG-LV) can be attribute to their minimal expression of low-density lipoprotein receptor (LDL-R), the corresponding receptor for VSVG protein **(FIG. 2C)**. Ligation of VSVG protein with LDL-R is critical for lentivirus adhesion following invasion of target cells and thus is a limiting factor of transduction efficiency **(FIG. 2C).** By studying the unique receptor expression pattern of NK cells, our data showed that in sharp contrast to LDL-R, expression levels of ASCT2 are abundant in primary conventional NK cells and are further enhanced in cytokine-induced memory-like (CIML) NK cells (Romee, R. et al. Blood 120, 4751-4760 (2012); Romee, R., et al. Scientifica (Cairo) 2014, 205796 (2014); Romee, R. et al. Sci Transl Med 8, 357ra123 (2016)) **(FIG. 2B; FIG. 2C).** ASCT2 is an amino acid transporter and corresponding receptor of baboon envelope glycoprotein (BaEV-gp1). Thus, without wishing to be bound by theory, utilizing an unconventional BaEV pseudotyped lentivirus, we will overcome the transduction block in primary human NK cells and especially in CIML NK cells **(FIG. 2C)**

**Example 3: Generation of anti-NPM1c CAR memory-like NK cells.**

Chimeric antigen receptor (CAR) T-cell therapy is a known method in targeting cancer antigens. However, CAR-T cells are limited due to their persistence and negative side-effects. Generation of CAR-NK cells which remain active and demonstrates potency toward cancer cells are needed.

***CIML NK cells as a CAR Platform.***

Studies using allogeneic cord blood-derived NK cells as a platform for generating CD19-CARs have shown clinical responses in patients with relapsed B cell malignancies (Liu et al. NEJM. (2020) 382:545-53). Clinical responses were achieved with minimal toxicity and no cases of severe cytokine release syndrome (CRS) or neurotoxicity, indicating that NK cells represent a new cellular platform for genetically modified adoptive cell therapy. We will build on these observations along with our prior clinical experience with cytokine-induced memory-like (CIML) NK cell therapy (Romee R, et al Blood (2012) 120(24):4751-4760; Romee R, et al. Sci Transl Med (2016) 8(357):357ra123) to develop methods for generating CIML NK CARs and to evaluate the efficacy of these genetically modified NK cells in preclinical models. CIML NK cells **(FIG. 1)** provide a unique platform for development of NK cell CARs based on the favorable safety profile, increased proliferation, prolonged persistence and enhanced anti-leukemia function seen in pre-clinical models and in patients treated with un-modified CIML NK cells. Id.

### *Mutated NPM1c as a CAR Target in AML.*

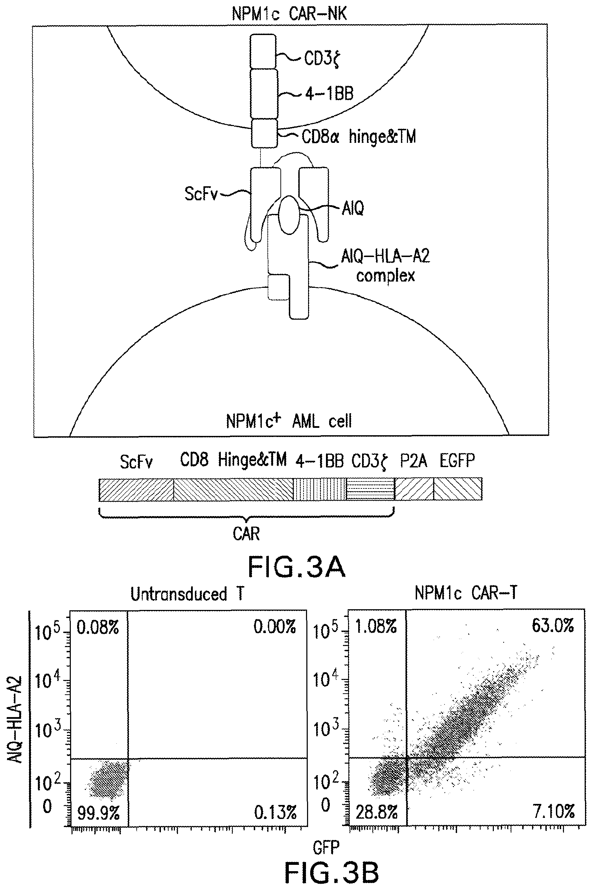

Most CAR-T cell therapies target tumor-associated antigens (TAAs), which can lead to on-target/off-tumor toxicity due to low level expression in normal tissues and tumor resistance due to loss of antigen expression by tumor cells. One way to overcome this drawback is to target tumor-specific oncogenic driver gene mutations. The four-nucleotide duplication in nucleophosmin, referred to as NPM1c, is a driver oncogene mutation in ~30% of AML. NPM1c generates a leukemia-specific neo-antigen (AIQDLCLAV; SEQ ID NO: 1) that is presented by the HLA-A*0201 allele. Using yeast surface display, we have isolated a human single-chain variable fragment (scFv) that specifically binds to the NPM1c epitope-HLA-A2 complex. The previously identified scFv targeting this antigen is described in US Provisional Application No. 62/987,612 (herein incorporated by reference). We have created a CAR construct by cloning the scFv in-frame into a CAR backbone containing a CD8α hinge and transmembrane (TM) domain, a 4-1BB co-stimulatory domain and a CD3z activation domain, followed by self-cleavage P2A and EGFP **(FIG. 3A** and **Table 3).**

**Table 3 Components of anti-NPM1c CAR construct**

| Component (5' to 3') | SEQ ID NO (aa) | SEQ ID NO (DNA) |
|---|---|---|
| Leader sequence | 23 | 31 |
| anti-NPM1c scFv | 24 | 32 |
| CD8 hinge & transmembrane | 25 | 33 |
| 4-1BB co-stimulatory domain | 26 | 34 |
| CD3z activation domain | 27 | 35 |
| P2A | 28 | 36 |
| EGFP | 29 | 37 |
| Full-length construct | 22 | 30 |

As described previously, anti-NPM1c CAR-T cells specifically recognize the AIQ-HLA-A2 complex. Briefly, GFP+ anti-NPM1c CAR-T cells (with the anti-NPM1c CAR of SEQ ID NO: 30) incubated with biotinylated AIQ-HLA-A2 followed by streptavidin-APC staining were shown to bind to AIQ-HLA-A2 complex **(FIG. 3B),** but not to HLA-A2 presenting control peptide epitope (e.g., SLL; SLLMWITQC (SEQ ID NO:62)) or HLA-A2 alone (data not shown). Untransduced T cells did not show binding to any of the three complexes. These results confirm the specificity of NPM1c-CAR-T cells (with the anti-NPM1c CAR of SEQ ID NO: 30) to AIQ-HLA-A2 complex. Engineered CAR-T cells with the isolated scFv exhibited potent cytotoxicity both in vitro and in vivo against NPM1c+ HLA-A2+ leukemia cells, but not NPM1c- HLA-A2+ or HLA-A2- tumor cells (data not shown).

Here we will develop a NK cell-based CAR against AML by using CIML NK cells transduced with the CAR construct able to recognize the NPM1c neo-antigen in complex with HLA-A*0201. NK cells can recognize and kill target cells that have no or low HLA expression. This can be important because leukemia cells that have low levels of HLA can be killed by NK cells via CAR-independent mechanisms, while those leukemia cells that express high levels of HLA and thus more NPM1c neoantigen-HLA-A2 targets can be targeted and killed by NPM1c CAR-NK cells. As antigen loss is an important mechanism of tumor resistance following CAR-T therapy, NPM1c CAR-NK cells can prove effective in reducing resistance to therapy and disease relapse.

### *Potent anti-AML function of CAR CIML NK cells.*

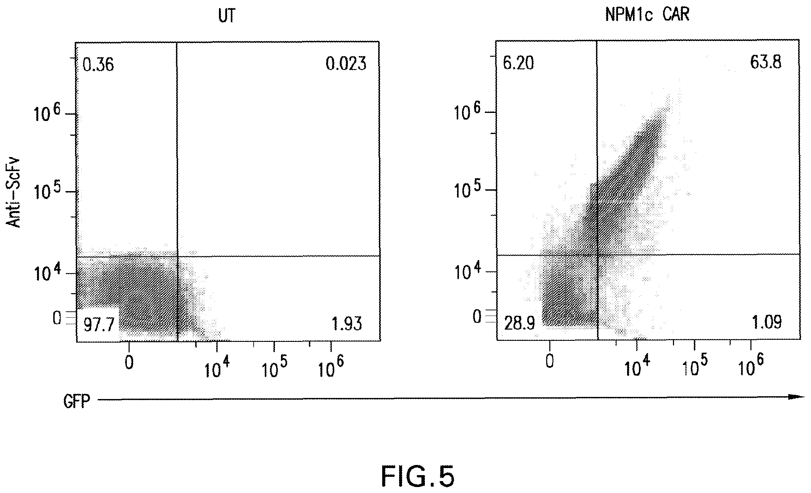

As described further below, we have introduced anti-NPM1c CAR into CIML NK cells via an unconventional lentiviral transduction approach and achieved above 50% CAR-editing efficiency **(FIG. 5).** Compared with their untraduced counterparts, CIML NK cells carrying anti-NPM1c CAR (according to SEQ ID NO: 30) acquired enhanced anti-AML function, indicated by the elevated levels of IFN-gamma production as well as increased expression of the degranulation marker CD107a when pulsed with NPM1c+ target AML cells (OCI-AML3). In summary, we have built a CAR-NK platform to specifically and efficiently target the tumor-specific intracellular proteins like NPM1c.

### Memory-like NK cells

Cytokine-induced memory-like (CIML) NK cells are NK cells with long-term enhanced functionality caused by pre-activation with cytokines including IL-12, IL-18, and IL15. The CIML NK cells mount a recall response during future infections or challenge even in the absence of the initial cytokine stimulus. To generate CIML NK cells, the methods of Example 2 were used. Briefly, peripheral blood mononuclear cells were isolated from humans and stimulated for 16 hours with rhIL-12 (10 ng/mL) + rhIL-18 (50 ng/mL) + rhIL-15 (50 ng/mL) or control conditions (rhIL-15, 50 ng/mL), washed 3 times to remove cytokines, and cultured in complete RPMI 1640 medium containing 10% human AB serum (Sigma-Aldrich) supplemented with rhIL-15 (1 ng/mL) to support survival.

### Transduction

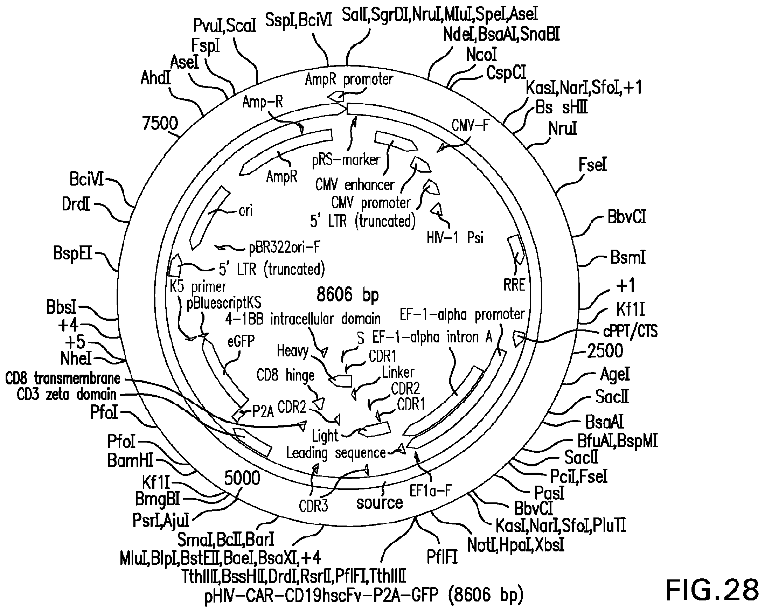

Transduction of primary human and mouse NK cells poses various challenges, and minimal success is observed with different gene transfer protocols. To mediate this process, a pseudotyped lentivirus-based method was utilized. Both VSVG- (vesicular-stomatitis-virus-G protein) and a BaEV-LV (Baboon envelope glycoprotein) are known to successfully transfer genetic material to NK cells. NK cells express the VSVG receptor Low-density lipoprotein (LDL-R) and the BaEV receptor alanine, serine, cysteine transporter 2 (ASCT2). Surface expression of both of these receptors was measured on conventional (not cytokine induced) and ML NK cells using flow cytometry. As described in Example 2, LDL-R levels **(FIG. 2A)** were found to be expressed less than ASCT2 **(FIG. 2B).** Therefore, to generate a more potent lentiviral approach, the glycoprotein from BaEV (amino acid sequence set forth by SEQ ID NO: 107; nucleotide sequence set forth by SEQ ID NO: 108) was inserted into the coding sequence to generate a pseudotyped lentivirus with BaEV glycoprotein that will recognize ASCT2. A map depicting a lentiviral construct pseudotyped with BaEV glycoprotein and encoding a anti-CD19 CAR is depicted in **FIG. 28.** The lentiviral expression construct was prepared to express the anti-NPM1c CAR identified in **FIG. 3A** and **Table 3** and pseudotyped with BaEV glycoprotein (referred to as "BaEV-LV"). The construct was cloned into a lentiviral packaging plasmid under control of an EF-1-alpha promoter and lentivirus was generated by transient transfection in HEK 293T cells. Titers of LV containing supernatants were quantified by FACS.

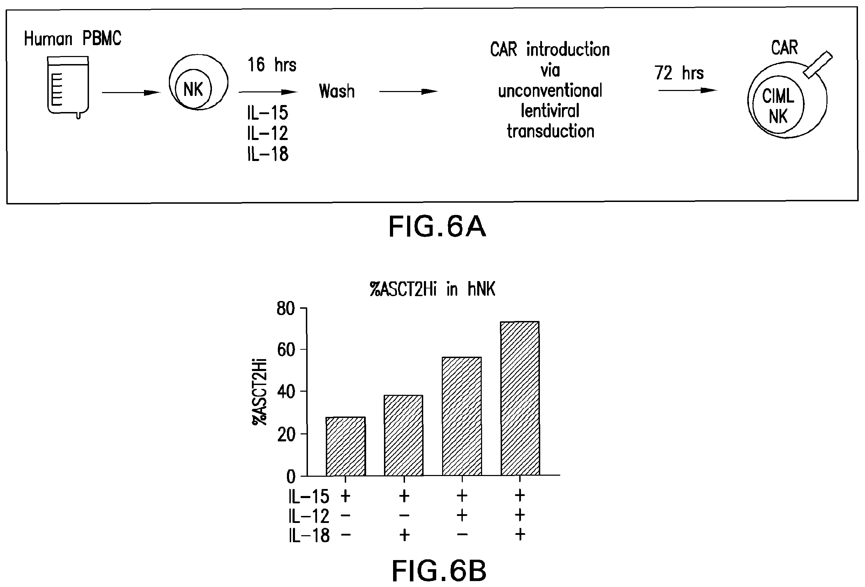

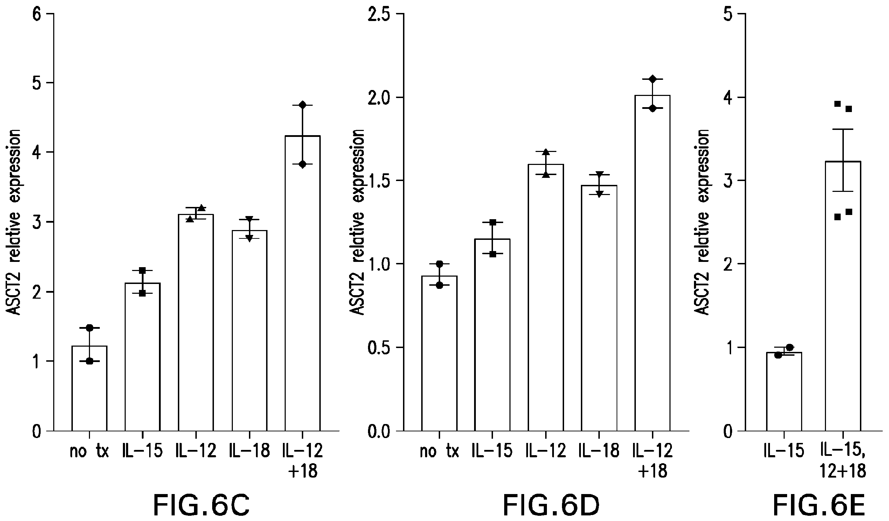

To understand if induction of ML NK cells with different cytokines prior to CAR transduction impacted expression of ASCT2, cells were induced and assayed for expression. Specifically, human peripheral blood mononuclear cells (PBMCs) were collected, and human NK (hNK) cells were purified from the sample. hNK cells were then stimulated for 16 hours with different combinations of IL-15, IL-12, and/or IL-18 using the doses described above (i.e., rhIL-12 at 10 ng/mL; rhIL-18 at 50 ng/mL; rhIL-15 at 50 ng/mL). Cells were then washed and transduced with the anti-NPM1c CAR (nucleotide sequence set forth in SEQ ID NO: 30) using the BaEV-LV lentiviral transduction described above, and 72 hours later, functional anti-NPM1c-CAR-ML hNK cells were generated **(FIG. 6A).**

When hNK cells were stimulated with one or more of IL-15, IL-12, and IL-18, the surface expression of ASCT2 increased with stimulation with more than one of IL-15, IL-12, and IL-18 as measured by flow cytometry **(FIG. 6B).** Specifically, simultaneous treatment with IL-15, IL-12, and IL-18 increased expression more than IL-15 alone.

It was further confirmed if ASCT2 expression was altered at the mRNA level in CIML NK cells relative to conventional NK cells using quantitative PCR (qPCR). Briefly, primary human NK cells were isolated from PBMCs obtained from three different healthy human donors using the method described above. The hNK cells were plated at $3-5\times10^6$ cells per well and activating for 16 hours by exposure to (i) rhIL-12 (10 ng/mL) + rhIL-18 (50 ng/mL); or (ii) rhIL-12 (10 ng/mL) + rhIL-18 (50 ng/mL) + rhIL15 (50-ng/mL);. Control conditions were exposure to rhIL-12 (10 ng/mL) alone, rhIL-18 (50 ng/mL) alone, or rhIL-15 (50 ng/mL) alone. Following activation, the cells were washed 3 times with PBS to remove cytokines, and RNA was extracted for qPCR assay. Quantification of ASCT2 RNA transcripts was performed using the $\Delta\Delta Ct$ method. **FIGS. 6C-6E** provide quantification for activated hNK cells from three different human donors. As shown in **FIGS. 6C-6D,** NK cells activated with IL-12 and IL-18 had increased expression of ASCT2 RNA transcripts relative to control hNK cells, particularly compared to control hNK cells activated with IL-15 only. As shown in **FIG. 6E,** NK cells activated with IL-15, IL-12, and IL-18 had >3-fold increase in ASCT2 RNA transcript relative to control hNK cells activated with IL-15 only.

Together, these findings indicate that activation with IL-12 and IL18 or all three cytokines (IL-12, IL-18, IL-15) improves expression of ASCT2 RNA transcript and surface expression in the hNK cells and may increase transduction efficiency with BaEV-LV.

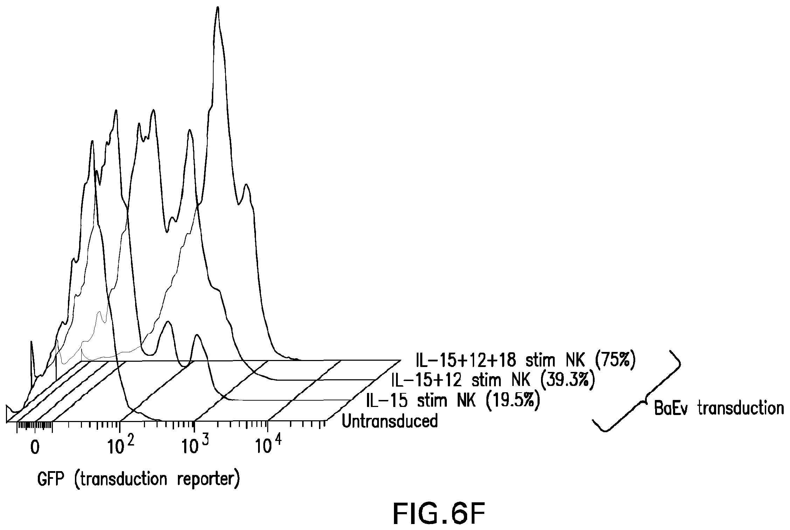

The ML-NK cells were then transduced with the anti-NPM1c CAR and transduction efficiency was measured. Specifically, the CAR construct described above contains a GFP reporter. The GFP expression levels were measured in transduced cells. **FIG. 6F** shows pre-activation with all three cytokines improved transduction efficiency of BaEV-LV.

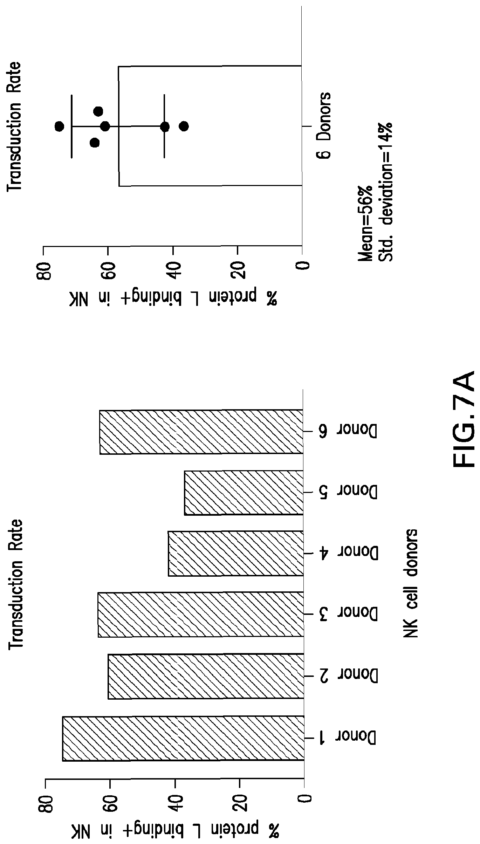

Transduction with the BaEV-LV lentiviral transduction was evaluated in CIML-NK cells obtained from different human donors. CAR expression was measured by flow cytometry using Protein L (see, e.g., Zheng, et al (2012) J. Transl Med. 10:29). As shown in **FIG. 7A,** the overall transduction rate, determined as the expression of the anti-NPM1c CAR by protein L binding after transduction, was $56\pm14\%$ for six different donors .

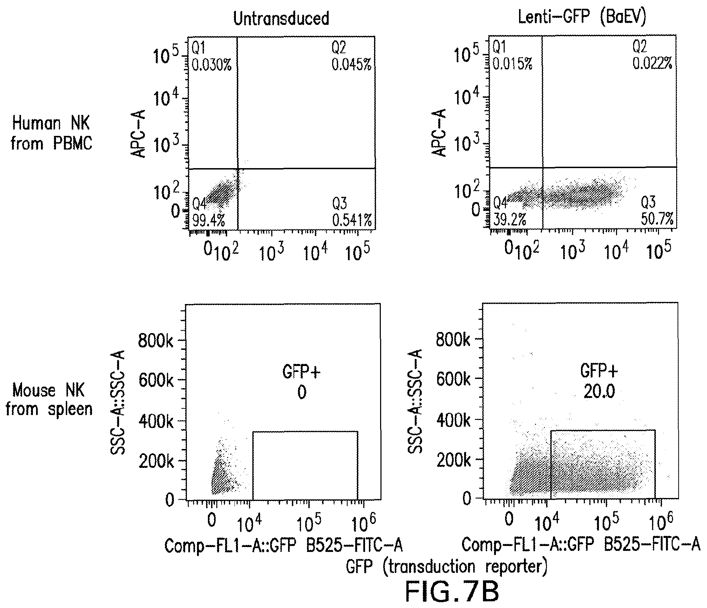

Additionally, transduction with the BaEV-LV lentiviral transduction was evaluated in CIML-NK cells obtained from human and mouse donors. Human CIML-NK cells were obtained by pre-activation with rhIL-12, rhIL-15, and rhIL-18 of primary hNK cells obtained from PBMCs as described above. Mouse CIML-NK cells were obtained by harvesting mouse splenocytes, isolating mouse NK cells, and pre-activating with recombinant mouse IL-12, IL-15, and IL-18. The lentivirus pseudotyped with BaEV encoded GFP (Lenti-GFP (BaEV)). As shown in **FIG. 7B,** the rate of transduction as measured by GFP expression was high in both human and mouse CIML-NK cells.

**Example 4: Expression of ASCT2 Among ML NK Cell Subsets**

It was further evaluated whether ASCT2 expression varied among subsets of NK cells. As shown in **FIG. 7C,** hNK cells express different phenotypic markers based upon their stage of development and maturation. The characteristic phenotypic markers from less mature (e.g., more "stem-like") to fully matured are indicated by **Table 4** below.

**Table 4: Phenotypic Markers of hNK Cell Subsets Based on Stage of Maturation**

| Stage of maturity | CD56 | CD16 | NKG2A | KIRs (Killer-cell immunoglobulin-like receptors) | CD57 |
|---|---|---|---|---|---|
| Less mature | Bright | Low/- | + | - | - |
| Intermediate | Dim | + | +/- | - | - |
| | Dim | + | +/- | + | - |
| Matured | Dim | + | +/- | + | + |

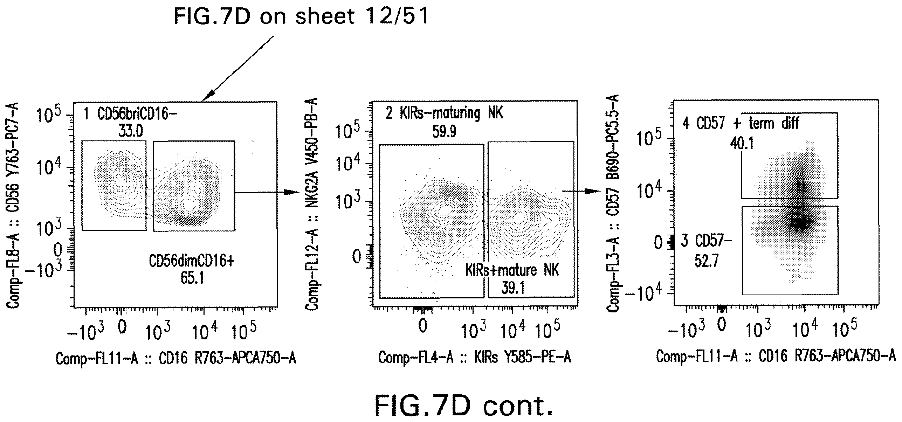

Flow cytometry analysis was used to analyze human NK cells subsets based on the phenotypic markers shown above, and ASCT2 expression was determined for each subset. Briefly, hNK cells were isolated from fresh or frozen PBMCs obtained from four different human donors as described in Example 3. The hNK cells were then rested overnight. The cells were stained with labeled antibodies targeting CD56, CD3, CD16, KIRs, CD57, and ASCT2, washed, then subjected to flow cytometry analysis. The gating strategy used is shown in **FIG. 7D,** which allowed identification of the following hNK subsets (in order from less mature to more mature):

(i) NK1: CD56bright; CD16-/low;
(ii) NK2: CD56dim; CD16+; KIRs-;
(iii) NK3: CD56dim; CD16+; KIRs-; CD57-;
(iv) NK4: CD56dim; CD16+; KIRs+; CD57+.

As shown in **FIGS. 7E-7F,** the proportion of the population expressing high surface levels of ASCT2 correlated with the stage of maturation of the hNK cell subsets. Specifically, the ASCT2 surface expression from high to low was NK1>NK2>NK3>NK4 for hNK cells. The data was consistent across hNK cell subsets obtained from different human donors.

**Example 5: Anti-NPM1c CAR-ML NK cells are potent against Acute Myeloid Leukemia**

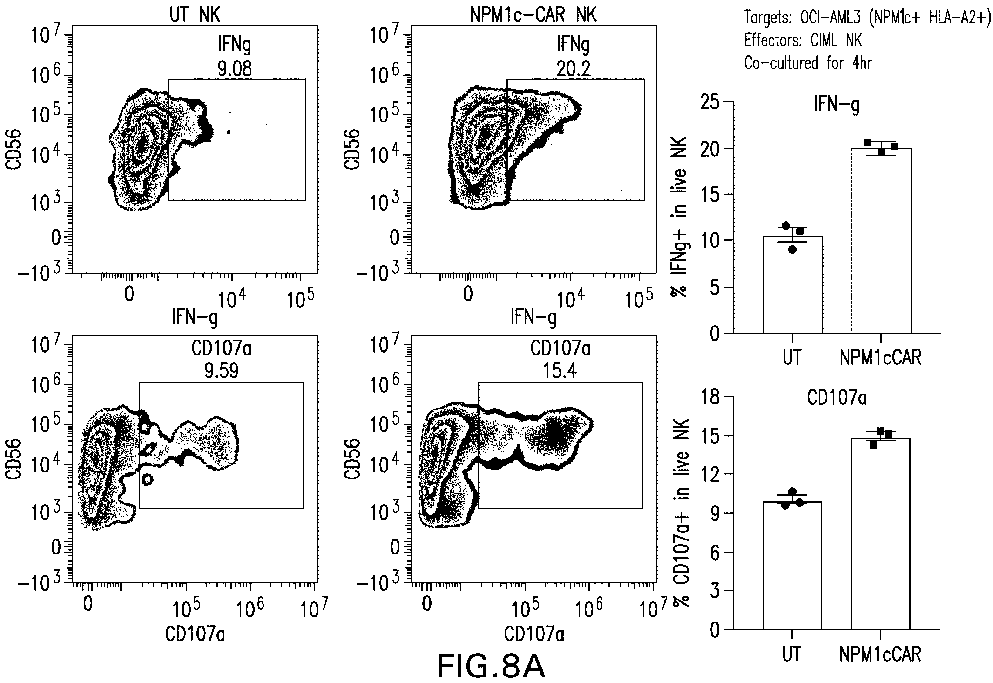

Acute Myeloid Leukemia (AML) is known to express NPM1c. To investigate if anti-NPM1c CAR-ML NK cells are more potent than untransduced NK cells against AML, each cell population was cultured with an AML cell line and expression of IFN-γ and CD107a, both of which are markers for NK cell activation, were measured. Specifically, OCI-AML3 cells which are NPM1c+/HLA-A2+ were co-cultured with anti-NPM1c CAR-ML NK cells for 4 hours. Cells were collected and flow cytometry analysis was performed. Cells were stained for CD56, CD107a, and IFNγ. Anti-NPM1c-CAR-ML NK cells demonstrated increased expression of IFN-γ and CD107a compared to untransduced cells (ML NK cells stimulated with IL15, IL12, and IL18) **(FIG. 8A).** This increased expression suggests higher potency and NK cell activation compared to untransduced cells in the present of target AML cells.

Mass cytometry allows for high-throughput analysis of a large number of parameters on single cells, and is used to deeply immunophenotype of human NK cells (Strauss-Albee (2015) Sci Transl Med 7:297ra115; Amir, et al (2013) Nat Biotech 31:545). Accordingly, an extensive analysis of expression profile of anti-NPM1c CAR ML-NK cells following co-culture with NPM1c+ target cells was performed using an NK-specific CyTOF panel (see, e.g., Romee et al (2016) Sci Transl Med 8:357ra123). Briefly, anti-NPM1c CAR ML-NK cells or untransduced ML-NK cells were co-cultured with OCI-AML3 cells for 6 hours. The cells were then analyzed using mass cytometry panels. Diversity was evaluated for three human donors. Quantification was performed as previously described (Diggins, et al (2015) Methods 82:55). As shown in **FIG. 8B,** CD107a, IFNγ, and granzyme B were enhanced in CAR ML-NK cells relative to untransduced NK cells. These data suggest CAR-NK cells have enhanced functionality for killing leukemia cells. As shown in **FIG. 8C,** multiple activation markers (CD25, CD69, ICOS, and CD226) were increased in CAR ML-NK cells. The activation marker CD62L was also increased, (particularly in CAR ML-NK cells from one donor). These data show CAR-NK cells were more active than their untransduced counterparts. As shown in **FIG. 8D,** multiple activating receptors (NKp30, NKG2D, and NKp44) were increased in CAR-NK cells. Overall, these data indicate CAR-NK cells express higher levels of activating receptors compared to the untransduced NK cells. As shown in **FIG. 8E,** certain maturation markers (CD56, NKG2A) were enhanced in CAR-NK cells, while the marker CD57 was decreased. These data indicate CAR-NK cells are less mature compared to the untransduced NK cells. As shown in **FIG. 8F,** the exhaustion marker TIGIT was enhanced in CAR-NK cells. As shown in **FIG. 8G,** the apoptosis marker TRAIL was decreased in CAR-NK cells, indicating the CAR-NK cells have improved longevity relative to untransduced NK cells.

**Example 6: Co-transduction with IL-15 improves potency of anti-NPM1c CAR-ML NK cells**

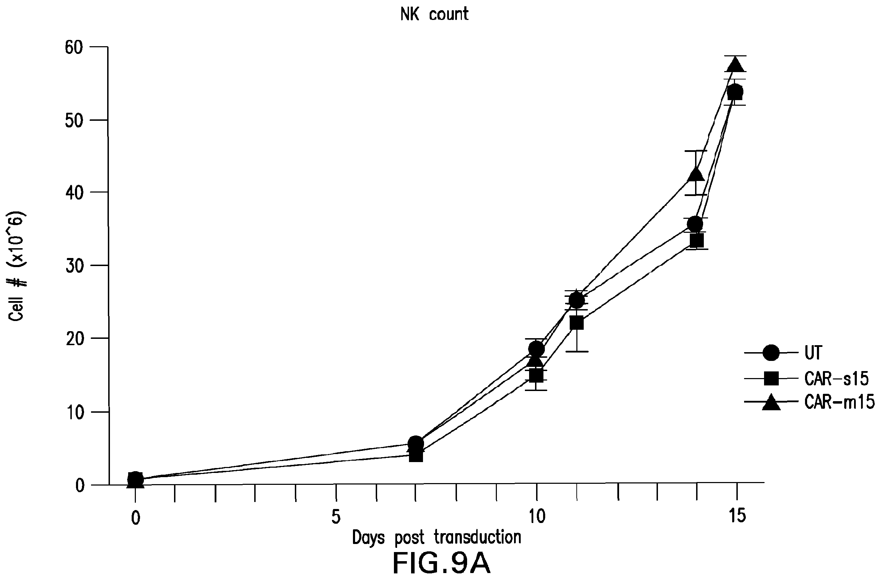

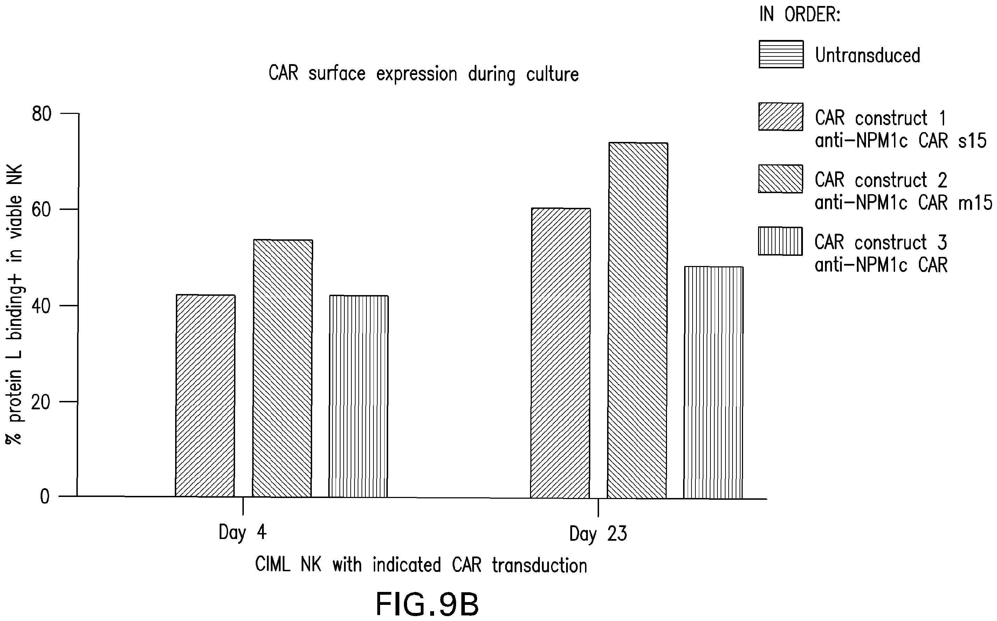

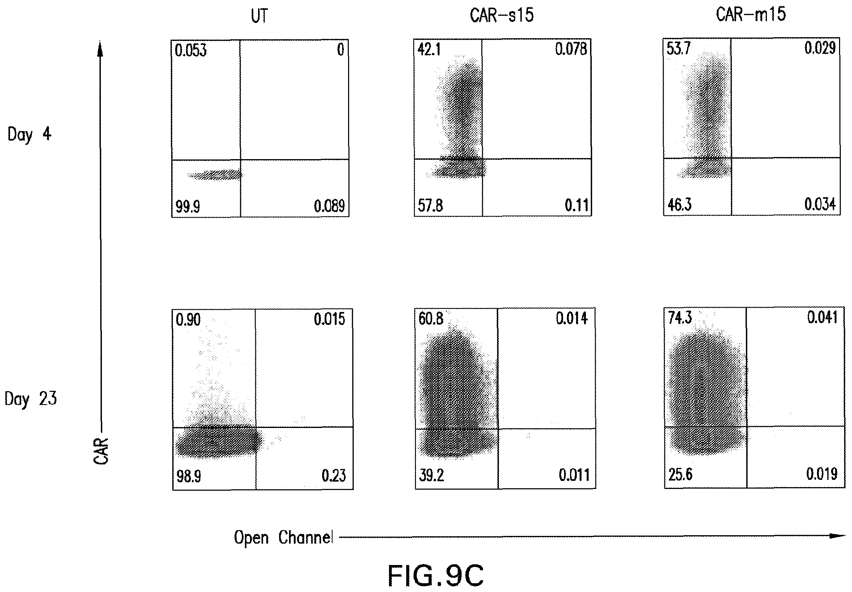

It was evaluated if co-transduction with IL-15 would further enhance the potency of the anti-NPM1c CAR-ML NK cells. The lentivirus construct was prepared to encode an anti-NPM1c CAR linked via a P2A self-cleavable peptide to a membrane bound IL-15 (mIL-15) or a soluble version of IL-15 that could undergo secretion (sIL-15). The mIL-15 included a C-terminal CD8 hinge and transmembrane domain. The full-length amino acid sequence and nucleotide sequence of mIL-15 and sIL-15 are set forth in **Table 5.** A lentivirus expression construct pseudotyped with BaEV glycoprotein was prepared to encode the anti-NPM1c-mIL-15 or anti-NPM1c-sIL15 (referred to as "CAR-m15" and "CAR-s15" respectively). When CIML NK cells were transduced with CAR-m15 or CAR-s15, the transduced cells maintained expansion *in vitro,* similar to untransduced (UT) cells **(FIG. 9A).** Comparison was made to CIMNL NK cell transduced with anti-NPM1c CAR only as described in Example 3. Even throughout expansion, surface expression of the CAR (as measured by percent Protein L binding) was maintained when measured on day 4 and day 23 post transduction by flow cytometry **(FIGS. 9B-9C).**

**Table 5: Sequence information for constructs having an anti-NPM1c CAR fused to membrane-bound IL-15 (mIL-15) or secreted IL-15 (sIL-15)**

| Construct arranged 5' to 3' for DNA N' to C' for amino acid | anti-NPM1c-mIL-15 | | anti-NPM1c-sIL-15 | |
|---|---|---|---|---|
| | SEQ ID NO (aa) | SEQ ID NO (DNA) | SEQ ID NO (aa) | SEQ ID NO (DNA) |
| CD8 signal peptide | 23 | 31 | 23 | 31 |
| Anti-NPM1c scFv | 24 | 32 | 24 | 32 |
| CD8 hinge | 104 | 103 | 104 | 103 |
| CD8 transmembrane domain | 106 | 105 | 106 | 105 |
| 4-1BB co-stimulatory domain | 26 | 34 | 26 | 34 |
| CD3z | 27 | 35 | 27 | 35 |
| P2A | 28 | 36 | 28 | 36 |
| CD8 signal peptide | 23 | 31 | 23 | 31 |
| Mature IL-15 | 97 | 98 | 97 | 98 |
| CD8 hinge | 104 | 103 | - | - |
| CD8 transmembrane domain | 106 | 105 | - | - |
| Full length | 100 | 99 | 102 | 101 |

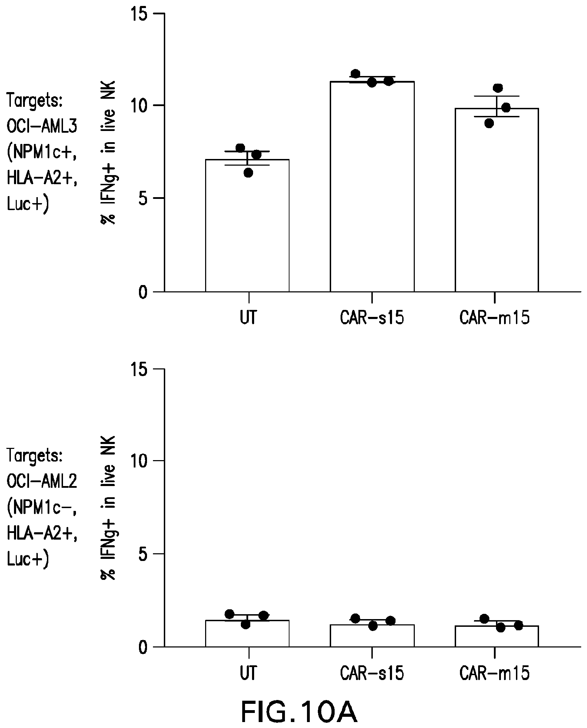

Next, specific killing by anti-NPM1c CAR-ML NK cells of AML target cells expressing NPM1c was compared to AML cells lacking expression of NPM1c. To do so, it was investigated whether the anti-NPM1c CAR-ML-NK cells were potent against NPM1c- AML cells in addition to their potency toward NPM1c+ cells. The CIML NK cells were transduced with CAR-s15 LV or CAR-m15 LV. Target cell killing of two luciferase expressing AML cell lines were assayed; OCI-AML3 (NPM1c+, HLA-A2+, Luc+) and OCI-AML2 (NPM1c-, HLA-A2+, Luc+). First, cells were co-cultured at a 1:1 ratio of anti-NPM1c CAR-CIML NK to AML cells, and collected 5 hours post co-culture. IFNγ expression, measured by flow cytometry analysis, was increased in anti-NPM1c CAR ML-NK cells cocultured with NPM1c+ (OCI-AML3) cells **(FIG. 10A).**

Additionally, the apoptotic rate of target cells (AML) was measured by co-culturing at different effector:target (E:T) ratios for four hours. Apoptosis was increased in effector cells expressing NPM1c compared to NPM1c negative cells **(FIG.**

**10B).** Both CAR constructs were successful at inducing apoptosis in NPM1c positive cells, which showed early apoptosis when measured by Annexin V flow-cytometry.

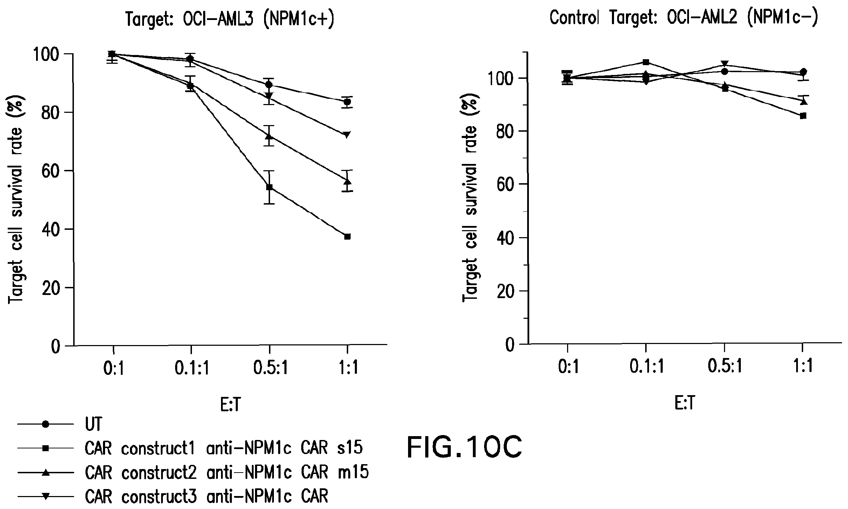

Effective cell killing was further measured by co-culturing the NK cells with each AML cell line for 24 hours followed by measurement of luciferase expression. Comparison was made to ML-NK cells transduced with anti-NPM1c CAR only. When cultured at different E:T ratios for 24 hours, survival rate of target cells was reduced in OCI-AML3 cells (left panel of **FIG. 10C**) whereas survival was not affected in the NPM1c- OCI-AML2 cells (right panel of **FIG. 10C**). Moreover, ML-NK cells co-transduced with IL15 demonstrated increased killing of NPM1c-expressing target cells relative to ML-NK cells transduced with anti-NPM1c CAR only. Together, these results demonstrate successful potency of anti-NPM1c CAR-CIML NK cells, particularly when co-transduced with IL-15, against NPM1c expressing AML cells and no cytotoxic effects against AML cells not expressing NPM1c.

Further comparison of CIML NK cells transduced with CAR alone or CAR and mIL15 were performed. As explained above, transduction with CAR was found to be efficient in primary human CIML NK cells, as measured at 72 hours following transduction, using BaEV-LV encoding anti-NPM1c CAR **(FIG. 10D)**. However, by using BaEV-LV to co-transduce the CAR and mIL-15, greater expansion was observed relative to cells transduced with BaEV-LV encoding CAR only **(FIG. 10E)**. Expression of IFN-gamma and CD107a in CIML NK cells transduced with BaEV encoding CAR and mIL-15 was similar to CIML NK cells transduced with BaEV encoding CAR only following a 4 hour stimulation with OCI-AML target cells **(FIG. 10F)**. However, CIML NK cells co-transduced with CAR and mIL-15 were more effective at killing target OCI-AML3 cells when measured four hours following co-culture **(FIG. 10G)**. Cell death was measured by flow cytometric analysis using 7-AAD.

### Example 7: Anti-NPM1c CAR-ML NK cells demonstrate efficacy against AML *in vivo*

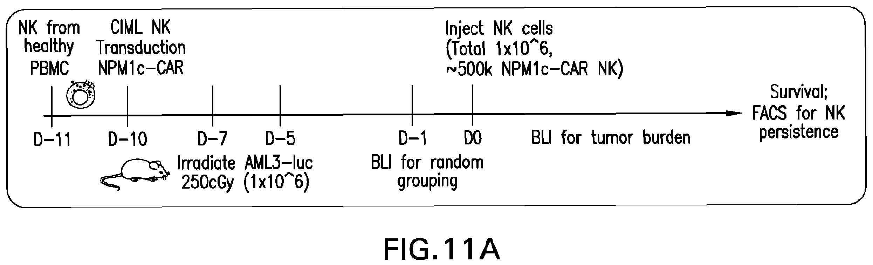

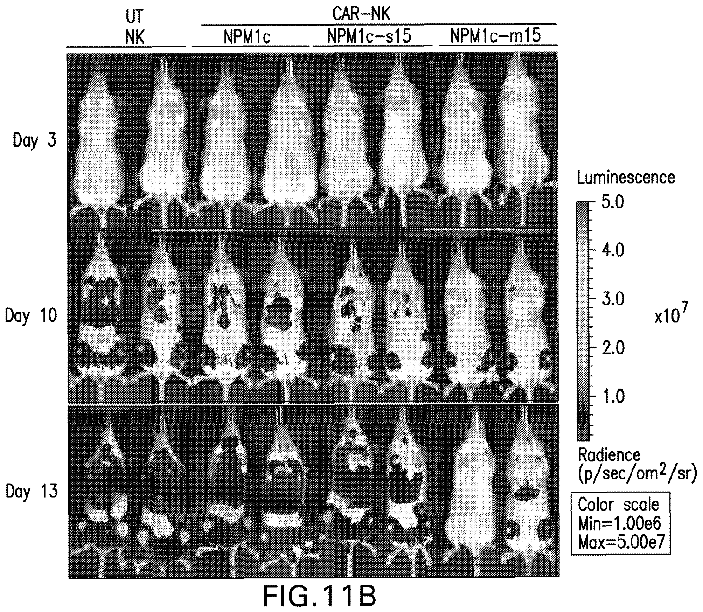

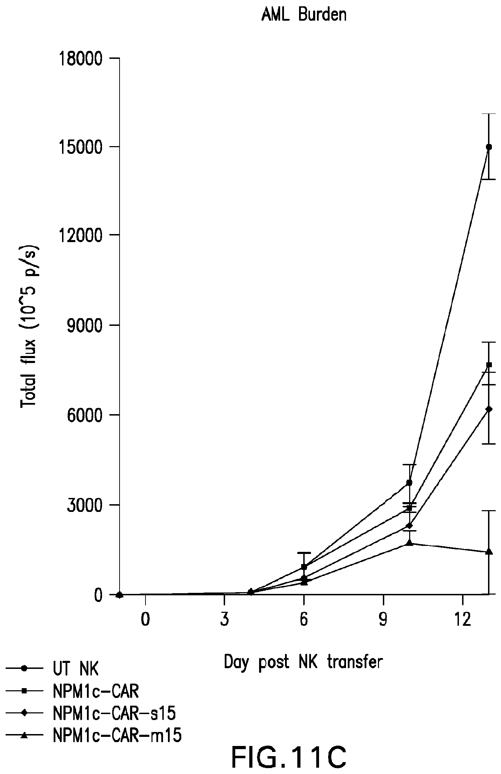

To examine anti-leukemia activity of anti-NPM1c CAR-ML NK cells *in vivo,* an AML mouse model was analyzed. As shown in **FIG. 11A,** NK cells were isolated from peripheral blood mononuclear cells of a healthy human donor. Following isolation, cells were cultured with cytokines as described in Example 3 to generate a ML-NK cells. On day -10, cells were transduced with anti-NPM1c-CAR (SEQ ID NO: 30) using the transduction method described in Example 3. While the cells were culturing, on day -7, mice were irradiated to eliminate immune cells, and on day -5 injected with $1\times10^6$ AML3-luc cells. On day 0, mice were injected with NK cells. The injection included $1\times10^6$ total cells with about 500k anti-NPM1c-CAR ML NK cells. AML burden was measured using imaging for the luciferase construct on day 3, day 10, and day 13 **(FIG. 11B)**. Anti-NPM1c CAR-ML NK (and secreted IL15 or membrane-bound IL15) treated mice demonstrated a reduction in the AML burden by day 12 **(FIG. 11C)**. These results demonstrate successful potency of anti-NPM1c CAR-CIML NK compared to untransduced NK cells in reducing overall AML burden.

### Example 8: In vivo efficacy against AML tumors

The in vivo efficacy and safety of CAR CIML NK cells are being validated against liquid and solid tumors. We will assess CIML NK cells bearing NPM1c-CAR (three constructs) and CAR-T cells transduced with the same constructs for in vivo anti-leukemia activity. Without wishing to be bound by theory, our unique CIML-NK CAR approach will enhance targeting of leukemic blasts without affecting normal hematopoietic stem cells and progenitors or causing severe toxicity, which has been one of the major challenges with T cell-based CAR approaches in AML. In brief, we will evaluate their activation, proliferation, persistence and efficacy in a xenograft mouse model using NSG mice previously injected with luc+ OCI-AML3. The animals will be sacrificed at 7, 14, 28 and 60 days after adoptive transfer to assess in vivo persistence, expansion and activity (based on bioluminescent tumor imaging and survival). We will also validate trafficking / homing of these cells into the key organs including bone marrow and spleen. Given that NK cell exhaustion is an active area of investigation as it negatively impacts their anti-tumor activity(Felices et al., 2018), we will characterize in vivo exhaustion of the cells via our NK-specific CyTOF panels and by their IFN-$\gamma$ secretion upon target re-stimulation (i.e. OCI-AML3 and K562). We will also compare the potency of IL-2, IL-15 and IL-15/IL-15R$\alpha$ to optimize expansion and persistence of the adoptively transferred CIML CAR-NK cells in NSG mice. This will include testing different dosing strategies to prevent NK cell exhaustion. After initial optimization in xenograft mice we will validate this strategy in NPM1c+ patient-derived xenograft (PDX) models available at DFCI.

We have also tried the classic VSVG-psedotyped lentivirus and the transduction efficiency was very low in a range of 1-3% in regular and CIML NK cells. For example, using the BaEV lentivirus we have also compared the transduction performance of NK cells with different cytokine pre-activation: IL-15, IL-15+IL-12, and IL-15+IL-12+IL18 and found that the latter (which generated CIML NK cells (Romee et al., 2016)) performed best.

### Example 9: Advantageous properties of CAR ML NK cells for use in a cancer immunotherapy

Differences between the CAR-memory-like NK cells as described herein and other NK cells engineered to express TCR include, for example:

memory-like ("CIML") primary NK cells versus other non-memory-like NK cells or NK cell lines (e.g. NK92 cell line) was used in majority of ongoing trials of engineered NK cell(Xie et al., 2020). See also, Mensali et al., 2019; Walseng et al., 2017.

affinity: our scFv-based CAR constructs have ~100 fold stronger affinity for peptide/MHC complex than their TCR-based counterparts for peptide/MHC complex. The stronger binding of CAR to target leads to stronger activation of NK cells and therefore stronger killing activity. In T cells, TCR interaction with peptide/MHC requires co-receptors such as CD4 or CD8 to compensate the weak binding affinity; in contrast, NK cells do not have the intact TCR signaling compartments to ideally support an optimized signaling and subsequent induction of robust activation and function.

To date T cells and NK cell lines (NK92 cells) have been transduced to express TCRs which are able to target intracellular antigens. While one can transduce primary NK cells and primary NK CIML cells with TCRs, there can be substantial drawbacks as to such transduction along with their much lower antigen/epitope affinities compared to the scFv-based CAR constructs. Using scFv based-CAR constructs we are able to achieve favorable transduction rates and target the intracellular antigens which has not been done with primary NK and memory-like ("CIML") NK cells. Besides, the shorter length of scFv compared to TCR also allows us to add additional modules like membrane or secretory version of the IL-15 into the construct while maintaining high transduction rates.

**Example 10**

***Engineered Memory-Like NK CARS targeting a neoepitope derived from intracellular NPM1C exhibit potent activity and specificity against acute myeloid leukemia***

*Introduction:* Acute myeloid leukemia (AML) continues to be a therapeutic challenge. There is an emerging need to develop less toxic and more effective targeted therapies. Natural Killer (NK) cells possess many of the key attributes critical for effective cancer therapies-"born to kill" but without apparent risk of graft versus host disease, cytokine release syndrome, or neurotoxicity. Furthermore, their intrinsic propensity to target myeloid blasts makes them attractive for AML. Despite promising clinical results in blood cancer, the development of NK cell-based therapy remains challenging mostly due to NK cells' short lifespan, inadequate proliferation and lack of specific tumor targeting. Here, we utilized a new approach to arm NK cells for adoptive immunotherapy based on innate cell memory. Chimeric antigen receptors (CARs) significantly enhance anti-tumor specificity and activity of immune effector cells. Our CAR-NK cells target a tumor-specific neoepitope in AML and harness potent function pathways in their design to enhance efficacy and minimize toxicity.

*Methods:*

1. Mutated NPM1c as a CAR Target in AML. Most CAR-T cell therapies target tumor-associated antigens (TAAs), which can lead to on-target/off-tumor toxicity as well as tumor resistance. One way to overcome these drawbacks is to target tumor-specific oncogenic driver mutations. The four-nucleotide duplication in nucleophosmin, referred to as NPM1c, is a driver oncogene mutation in about 35% of AML. The mutation creates a neoepitope that is presented by HLA-A2 allele. Using yeast surface display, we have isolated a human singlechain variable fragment (scFv) that specifically binds to the NPM1c epitope-HLA-A2 complex, but not HLA-A2 alone or HLA-A2 loaded with control peptides.

2. Cytokine-Induced Memory-Like (CIML) NK Cells as a CAR Platform. CIML NK cells can provide a unique platform for development of NK cell CARs based on the favorable safety profile, increased proliferation, prolonged persistence and enhanced anti-leukemia function that we have observed in pre-clinical models (Romee et al, Blood 2012) and in patients (Romee et al, Science Trans Med 2016) treated with un-modified CIML NK cells.

3. *Efficient Gene Editing in Primary NK Cells.* We have overcome the transduction block in primary human and mouse NK cells by utilizing an unconventional pseudotyped lentivirus based on a unique protein with high expression on CIML NK cells.

*Results:*

1. Engineered CAR-T cells with the isolated scFv exhibit potent cytotoxicity both *in vitro* and in vivo against NPM1c

HLA-A2 leukemia cells (OCI-AML3) and primary AML blasts, but not NPM1c HLA-A2 leukemia cells (OCI-AML2) or HLA-A2 tumor cells (PC-3).

2. The *in vivo* anti-leukemia efficacy of anti-NPM1c CAR-T cells was however transient (overall survival extended from 28 to 42 days, median survival extended from 21 to 37 days, compared with the control mice adoptively transferred with untraduced T cells), with unneglectable toxicity.

3. Utilizing an unconventional pseudotyped lentivirus to transduce CIML NK cells from healthy donor blood (n = 5 donors), we have successfully generated anti-NPM1c CAR-NK cells with high transduction efficiency (using MOI = 10: transduction rate mean 48%, range 32% to 65%; compared with 2%, range 0.8% to 4.5% for the conventional approach with VSVG pseudotyped lentivirus).

4. Harnessing key cytokine pathways in the CAR design substantially promoted CAR-NK cell survival (indicated by the enhanced cell viability from 29.7% to 75.2%) and proliferation (marked by the increased levels of ki-67 from 60.2% to 94.5%).

5. Anti-NPM1c CAR significantly promoted anti-tumor function (represented by CD107a, IFNgamma) and tumor-specific killing (measured by annexin V and 7-AAD) of CIML NK cells against AML with NPM1c oncogene (OCI-AML3).

6. Dual-armed CIML NK cells with CAR and cytokine signaling exhibited optimal specificity and sustainability against AML targets.

*Conclusion:* These results indicate that the innovative CAR-CIML NK cells can be developed as an efficient cellular immunotherapy for treating NPM1c+HLA-A2+ AML with potentially reduced on-target/off-tumor toxicity and tumor resistance. Our study will drive new conception and design of CAR-NK cell therapies against myeloid malignancies in the clinic.

We have developed new gene constructs to target a neoepitope derived from NPM1c, a driver oncogene mutation occurring in 30% of AML.

Utilizing an unconventional pseudotyped lentivirus and memory-like NK cells as a platform for CAR development, we have optimized transduction methods for in vitro generation of NPM1c-CAR-NK cells (with ~50% transduction rate).

CAR-NK cells exhibited potent and specific function against NPM1c+ AML cells.

Preliminary results using xenograft mouse models supported a robust anti-leukemia efficacy of CAR-NK cells in vivo (esp. for the construct with membrane-bound IL-15 ).

Safety:

Even mismatched natural killer (NK) cells do not cause severe GvHD

NK cell CARs are safe (no CRS/no neurotoxicity), response rate similar to CAR T cells in CLL (Liu et al, NEJM, 2020).

Efficacy:

Limited efficacy of CAR T cells in AML/MDS
Robust responses of memory-like NK cells against AML in phase 1 trial (Romee et al, STM, 2016)
Dual targeting of myeloid blasts by memory-like NK cells armed with CARs (antigen expression triggers CAR vs HLA loss triggers direct NK cell cytotoxicity

**SEQUENCES**

| SEQ ID NO: | Description | Sequence |
|---|---|---|
| 1 | Leukemia-specific neoantigen epitope of NPM1c | AIQDLCLAV |

(continued)

| SEQ ID NO: | Description | Sequence |
|---|---|---|
| 2 | anti-NPM1c scFv amino acid sequence | DIQMTQSPSSLSASVGDRVTITCRASQSISSYLNWYQQKPGKAPKL LIYAASSLQSGVPSRFSGSGSGTDFTLTISSLQPEDFATYYCQQSY STPLTFGQGTKVEIKSGILGTTAASGSSGGSSSGAEVQLVESGGGL VQPGGSLRLSCAASGFTFSSYAMSWVRQAPGKGLEWVSAISGSGGS TYYADSVKGRFTISRDNSKNTLYLQMNSLRAEDTAVYYCARLGYPT TTLLPFDYWGQGTLVTVSS |
| 3 | anti-NPM1c scFv VL amino acid sequence | DIQMTQSPSSLSASVGDRVTITCRASQSISSYLNWYQQKPGKAPKL LIYAASSLQSGVPSRFSGSGSGTDFTLTISSLQPEDFATYYCQQSY STPLTFGQGTKVEIKSGILGTTAA |
| 4 | anti-NPM1c scFv linker amino acid sequence | SGSSGGSSSG |
| 5 | anti-NPM1c scFv VH amino acid sequence | AEVQLVESGGGLVQPGGSLRLSCAASGFTFSSYAMSWVRQAPGKGL EWVSAISGSGGSTYYADSVKGRFTISRDNSKNTLYLQMNSLRAEDT AVYYCARLGYPTTTLLPFDYWGQGTLVTVSS |
| 6 | anti-NPM1c scFv VL CDR1 amino acid sequence (IMGT) | QSISSY |
| 7 | anti-NPM1c scFv VL CDR2 amino acid sequence (IMGT) | AAS |
| 8 | anti-NPM1c scFv VL CDR3 amino acid sequence (IMGT) | QQSYSTPLT |
| 9 | anti-NPM1c scFv VH CDR1 amino acid sequence (IMGT) | GFTFSSYA |
| 10 | anti-NPM1c scFv VH CDR2 amino acid sequence (IMGT) | ISGSGGST |
| 11 | anti-NPM1c scFv VH CDR3 amino acid sequence (IMGT) | ARLGYPTTTLLPFDY |

(continued)

| SEQ ID NO: | Description | Sequence |
|---|---|---|
| 12 | anti-NPM1c scFv nucleic acid sequence | GACATCCAGATGACCCAGTCTCCATCCTCCCTGTCTGCATCTGTAG GAGACAGAGTCACCATCACTTGCCGGGCAAGTCAGAGCATTAGCAG CTATTTAAATTGGTATCAGCAGAAACCAGGGAAAGCCCCTAAGCTC CTGATCTATGCTGCATCCAGTTTGCAAAGTGGGGTCCCATCAAGGT TCAGTGGCAGTGGATCTGGGACAGATTTCACTCTCACCATCAGCAG TCTGCAACCTGAAGATTTTGCAACTTACTACTGTCAACAGAGTTAC AGTACCCCGCTCACGTTCGGCCAAGGGACCAAGGTGGAAATCAAAT CCGGAATTCTAGGTACTACTGCCGCTAGTGGTAGTAGTGGTGGCAG TAGCAGTGGTGCCGAGGTGCAGCTGGTGGAGTCTGGGGGAGGCTTG GTACAGCCTGGGGGGTCCCTGAGACTCTCCTGTGCAGCCTCTGGAT TCACCTTTAGCAGCTATGCCATGAGCTGGGTCCGCCAGGCTCCAGG GAAGGGGCTGGAGTGGGTCTCAGCTATTAGTGGTAGTGGTGGTAGC ACATACTACGCAGACTCCGTGAAGGGCCGGTTCACCATCTCCAGAG ACAATTCCAAGAACACGCTGTATCTGCAAATGAACAGCCTGAGAGC CGAGGACACGGCCGTGTATTACTGTGCGAGGCTGGGTTACCCTACT ACTACCCTACTACCCTTTGATTACTGGGGCCAAGGTACCCTGGTCA CTGTCTCCAGT |
| 13 | anti-NPM1c scFv VL nucleic acid sequence | GACATCCAGATGACCCAGTCTCCATCCTCCCTGTCTGCATCTGTAG GAGACAGAGTCACCATCACTTGCCGGGCAAGTCAGAGCATTAGCAG CTATTTAAATTGGTATCAGCAGAAACCAGGGAAAGCCCCTAAGCTC CTGATCTATGCTGCATCCAGTTTGCAAAGTGGGGTCCCATCAAGGT TCAGTGGCAGTGGATCTGGGACAGATTTCACTCTCACCATCAGCAG TCTGCAACCTGAAGATTTTGCAACTTACTACTGTCAACAGAGTTAC AGTACCCCGCTCACGTTCGGCCAAGGGACCAAGGTGGAAATCAAAT CCGGAATTCTAGGTACTACTGCCGCT |
| 14 | anti-NPM1c scFv linker nucleic acid sequence | AGTGGTAGTAGTGGTGGCAGTAGCAGTGGT |
| 15 | anti-NPM1c scFv VH nucleic acid sequence | GCCGAGGTGCAGCTGGTGGAGTCTGGGGGAGGCTTGGTACAGCCTG GGGGGTCCCTGAGACTCTCCTGTGCAGCCTCTGGATTCACCTTTAG CAGCTATGCCATGAGCTGGGTCCGCCAGGCTCCAGGGAAGGGGCTG GAGTGGGTCTCAGCTATTAGTGGTAGTGGTGGTAGCACATACTACG CAGACTCCGTGAAGGGCCGGTTCACCATCTCCAGAGACAATTCCAA GAACACGCTGTATCTGCAAATGAACAGCCTGAGAGCCGAGGACACG GCCGTGTATTACTGTGCGAGGCTGGGTTACCCTACTACTACCCTAC TACCCTTTGATTACTGGGGCCAAGGTACCCTGGTCACTGTCTCCAG T |
| 16 | anti-NPM1c scFv VL CDR1 nucleic acid sequence (IMGT) | CAGAGCATTAGCAGCTAT |
| 17 | anti-NPM1c scFv VL CDR2 nucleic acid sequence (IMGT) | GCTGCATCC |

(continued)

| SEQ ID NO: | Description | Sequence |
|---|---|---|
| 18 | anti-NPM1c scFv VL CDR3 nucleic acid sequence (IMGT) | CAACAGAGTTACAGTACCCCGCTCACG |
| 19 | anti-NPM1c scFv VH CDR1 nucleic acid sequence (IMGT) | GGATTCACCTTTAGCAGCTATGCC |
| 20 | anti-NPM1c scFv VH CDR2 nucleic acid sequence (IMGT) | ATTAGTGGTAGTGGTGGTAGCACA |
| 21 | anti-NPM1c scFv VH CDR3 nucleic acid sequence (IMGT) | GCGAGGCTGGGTTACCCTACTACTACCCTACTACCCTTTGATTAC |
| 22 | anti-NPM1c CAR amino acid sequence | MALPVTALLLPLALLLHAARPDIQMTQSPSSLSASVGDRVTITCRA SQSISSYLNWYQQKPGKAPKLLIYAASSLQSGVPSRFSGSGSGTDF TLTISSLQPEDFATYYCQQSYSTPLTFGQGTKVEIKSGILGTTAAS GSSGGSSSGAEVQLVESGGGLVQPGGSLRLSCAASGFTFSSYAMSW VRQAPGKGLEWVSAISGSGGSTYYADSVKGRFTISRDNSKNTLYLQ MNSLRAEDTAVYYCARLGYPTTTLLPFDYWGQGTLVTVSSTTTPAP RPPTPAPTIASQPLSLRPEACRPAAGGAVHTRGLDFACDIYIWAPL AGTCGVLLLSLVITLYCKRGRKKLLYIFKQPFMRPVQTTQEEDGCS CRFPEEEEGGCELRVKFSRSADAPAYKQGQNQLYNELNLGRREEYD VLDKRRGRDPEMGGKPRRKNPQEGLYNELQKDKMAEAYSEIGMKGE RRRGKGHDGLYQGLSTATKDTYDALHMQALPPRATNFSLLKQAGDV EENPGPMVSKGEELFTGVVPILVELDGDVNGHKFSVSGEGEGDATY GKLTLKFICTTGKLPVPWPTLVTTLTYGVQCFSRYPDHMKQHDFFK SAMPEGYVQERTIFFKDDGNYKTRAEVKFEGDTLVNRIELKGIDFK EDGNILGHKLEYNYNSHNVYIMADKQKNGIKVNFKIRHNIEDGSVQ LADHYQQNTPIGDGPVLLPDNHYLSTQSALSKDPNEKRDHMVLLEF VTAAGITLGMDELYK |
| 23 | Amino acid sequence of the leading sequence in the anti-NPM1c CAR | MALPVTALLLPLALLLHAARP |
| 24 | Amino acid sequence of scFv in the anti-NPM1c CAR | DIQMTQSPSSLSASVGDRVTITCRASQSISSYLNWYQQKPGKAPKL LIYAASSLQSGVPSRFSGSGSGTDFTLTISSLQPEDFATYYCQQSY STPLTFGQGTKVEIKSGILGTTAASGSSGGSSSGAEVQLVESGGGL VQPGGSLRLSCAASGFTFSSYAMSWVRQAPGKGLEWVSAISGSGGS TYYADSVKGRFTISRDNSKNTLYLQMNSLRAEDTAVYYCARLGYPT TTLLPFDYWGQGTLVTVSS |

(continued)

| SEQ ID NO: | Description | Sequence |
|---|---|---|
| 25 | Amino acid sequence of the CD8 hinge and transmembrane regions in the anti-NPM1c CAR | TTTPAPRPPTPAPTIASQPLSLRPEACRPAAGGAVHTRGLDFACDI YIWAPLAGTCGVLLLSLVITLYC |
| 26 | Amino acid sequence of the 4-1BB signaling domain in the anti-NPM1c CAR | KRGRKKLLYIFKQPFMRPVQTTQEEDGCSCRFPEEEEGGCEL |
| 27 | Amino acid sequence of the CD3-zeta signaling domain in the anti-NPM1c CAR | RVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR |
| 28 | Amino acid sequence of the P2A self-cleaving peptide in the anti-NPM1c CAR | ATNFSLLKQAGDVEENPGP |
| 29 | Amino acid sequence of the EGFP region in the anti-NPM1c CAR | MVSKGEELFTGVVPILVELDGDVNGHKFSVSGEGEGDATYGKLTLK FICTTGKLPVPWPTLVTTLTYGVQCFSRYPDHMKQHDFFKSAMPEG YVQERTIFFKDDGNYKTRAEVKFEGDTLVNRIELKGIDFKEDGNIL GHKLEYNYNSHNVYIMADKQKNGIKVNFKIRHNIEDGSVQLADHYQ QNTPIGDGPVLLPDNHYLSTQSALSKDPNEKRDHMVLLEFVTAAGI TLGMDELYK |

(continued)

| SEQ ID NO: | Description | Sequence |
|---|---|---|
| 30 | anti-NPM1c CAR nucleic acid sequence | ATGGCCCTCCCTGTCACCGCCCTGCTGCTTCCGCTGGCTCTTCTGC TCCACGCCGCTCGGCCCGACATCCAGATGACCCAGTCTCCATCCTC CCTGTCTGCATCTGTAGGAGACAGAGTCACCATCACTTGCCGGGCA AGTCAGAGCATTAGCAGCTATTTAAATTGGTATCAGCAGAAACCAG GGAAAGCCCCTAAGCTCCTGATCTATGCTGCATCCAGTTTGCAAAG TGGGGTCCCATCAAGGTTCAGTGGCAGTGGATCTGGGACAGATTTC ACTCTCACCATCAGCAGTCTGCAACCTGAAGATTTTGCAACTTACT ACTGTCAACAGAGTTACAGTACCCCGCTCACGTTCGGCCAAGGGAC CAAGGTGGAAATCAAATCCGGAATTCTAGGTACTACTGCCGCTAGT GGTAGTAGTGGTGGCAGTAGCAGTGGTGCCGAGGTGCAGCTGGTGG AGTCTGGGGGAGGCTTGGTACAGCCTGGGGGGTCCCTGAGACTCTC CTGTGCAGCCTCTGGATTCACCTTTAGCAGCTATGCCATGAGCTGG GTCCGCCAGGCTCCAGGGAAGGGGCTGGAGTGGGTCTCAGCTATTA GTGGTAGTGGTGGTAGCACATACTACGCAGACTCCGTGAAGGGCCG GTTCACCATCTCCAGAGACAATTCCAAGAACACGCTGTATCTGCAA ATGAACAGCCTGAGAGCCGAGGACACGGCCGTGTATTACTGTGCGA GGCTGGGTTACCCTACTACTACCCTACTACCCTTTGATTACTGGGG CCAAGGTACCCTGGTCACTGTCTCCAGTACCACTACCCCAGCACCG AGGCCACCCACCCCGGCTCCTACCATCGCCTCCAGCCTCTGTCCC TGCGTCCGGAGGCATGTAGACCCGCAGCTGGTGGGGCCGTGCATAC |

(continued)

| SEQ ID NO: | Description | Sequence |
|---|---|---|
| | | CCGGGGTCTTGACTTCGCCTGCGATATCTACATTTGGGCCCCTCTG GCTGGTACTTGCGGGGTCCTGCTGCTTTCACTCGTGATCACTCTTT ACTGTAAGCGCGGTCGGAAGAAGCTGCTGTACATCTTTAAGCAACC CTTCATGAGGCCTGTGCAGACTACTCAAGAGGAGGACGGCTGTTCA TGCCGGTTCCCAGAGGAGGAGGAAGGCGGCTGCGAACTGCGCGTGA AATTCAGCCGCAGCGCAGATGCTCCAGCCTACAAGCAGGGGCAGAA CCAGCTCTACAACGAACTCAATCTTGGTCGGAGAGAGGAGTACGAC GTGCTGGACAAGCGGAGAGGACGGGACCCAGAAATGGGCGGGAAGC CGCGCAGAAAGAATCCCCAAGAGGGCCTGTACAACGAGCTCCAAAA GGATAAGATGGCAGAAGCCTATAGCGAGATTGGTATGAAAGGGGAA CGCAGAAGAGGCAAAGGCCACGACGGACTGTACCAGGGACTCAGCA CCGCCACCAAGGACACCTATGACGCTCTTCACATGCAGGCCCTGCC GCCTCGGGGATCCGGCGCAACAAACTTCTCTCTGCTGAAACAAGCC GGAGATGTCGAAGAGAATCCTGGACCGATGGTGAGCAAGGGCGAGG AGCTGTTCACCGGGGTGGTGCCCATCCTGGTCGAGCTGGACGGCGA CGTAAACGGCCACAAGTTCAGCGTGTCCGGCGAGGGCGAGGGCGAT GCCACCTACGGCAAGCTGACCCTGAAGTTCATCTGCACCACCGGCA AGCTGCCCGTGCCCTGGCCCACCCTCGTGACCACCCTGACCTACGG CGTGCAGTGCTTCAGCCGCTACCCCGACCACATGAAGCAGCACGAC TTCTTCAAGTCCGCCATGCCCGAAGGCTACGTCCAGGAGCGCACCA TCTTCTTCAAGGACGACGGCAACTACAAGACCCGCGCCGAGGTGAA GTTCGAGGGCGACACCCTGGTGAACCGCATCGAGCTGAAGGGCATC GACTTCAAGGAGGACGGCAACATCCTGGGGCACAAGCTGGAGTACA ACTACAACAGCCACAACGTCTATATCATGGCCGACAAGCAGAAGAA CGGCATCAAGGTGAACTTCAAGATCCGCCACAACATCGAGGACGGC AGCGTGCAGCTCGCCGACCACTACCAGCAGAACACCCCCATCGGCG ACGGCCCCGTGCTGCTGCCCGACAACCACTACCTGAGCACCCAGTC CGCCCTGAGCAAAGACCCCAACGAGAAGCGCGATCACATGGTCCTG CTGGAGTTCGTGACCGCCGCCGGGATCACTCTCGGCATGGACGAGC TGTACAAGTGA |
| 31 | Nucleic acid sequence of the leading sequence in the anti-NPM1c CAR | ATGGCCCTCCCTGTCACCGCCCTGCTGCTTCCGCTGGCTCTTCTGC TCCACGCCGCTCGGCCC |

(continued)

| SEQ ID NO: | Description | Sequence |
|---|---|---|
| 32 | Nucleic acid sequence of the scFv in the anti-NPM1c CAR | GACATCCAGATGACCCAGTCTCCATCCTCCCTGTCTGCATCTGTAG GAGACAGAGTCACCATCACTTGCCGGGCAAGTCAGAGCATTAGCAG CTATTTAAATTGGTATCAGCAGAAACCAGGGAAAGCCCCTAAGCTC CTGATCTATGCTGCATCCAGTTTGCAAAGTGGGGTCCCATCAAGGT TCAGTGGCAGTGGATCTGGGACAGATTTCACTCTCACCATCAGCAG TCTGCAACCTGAAGATTTTGCAACTTACTACTGTCAACAGAGTTAC AGTACCCCGCTCACGTTCGGCCAAGGGACCAAGGTGGAAATCAAAT CCGGAATTCTAGGTACTACTGCCGCTAGTGGTAGTAGTGGTGGCAG TAGCAGTGGTGCCGAGGTGCAGCTGGTGGAGTCTGGGGGAGGCTTG GTACAGCCTGGGGGGTCCCTGAGACTCTCCTGTGCAGCCTCTGGAT TCACCTTTAGCAGCTATGCCATGAGCTGGGTCCGCCAGGCTCCAGG GAAGGGGCTGGAGTGGGTCTCAGCTATTAGTGGTAGTGGTGGTAGC ACATACTACGCAGACTCCGTGAAGGGCCGGTTCACCATCTCCAGAG ACAATTCCAAGAACACGCTGTATCTGCAAATGAACAGCCTGAGAGC CGAGGACACGGCCGTGTATTACTGTGCGAGGCTGGGTTACCCTACT ACTACCCTACTACCCTTTGATTACTGGGGCCAAGGTACCCTGGTCA CTGTCTCCAGT |
| 33 | Nucleic acid sequence of the CD8 hinge and transmembrane regions in the anti-NPM1c CAR | ACCACTACCCCAGCACCGAGGCCACCCACCCCGGCTCCTACCATCG CCTCCCAGCCTCTGTCCCTGCGTCCGGAGGCATGTAGACCCGCAGC TGGTGGGGCCGTGCATACCCGGGGTCTTGACTTCGCCTGCGATATC TACATTTGGGCCCCTCTGGCTGGTACTTGCGGGGTCCTGCTGCTTT CACTCGTGATCACTCTTTACTGT |
| 34 | Nucleic acid sequence of the 4-1BB signaling domain in the anti-NPM 1c CAR | AAGCGCGGTCGGAAGAAGCTGCTGTACATCTTTAAGCAACCCTTCA TGAGGCCTGTGCAGACTACTCAAGAGGAGGACGGCTGTTCATGCCG GTTCCCAGAGGAGGAGGAAGGCGGCTGCGAACTG |
| 35 | Nucleic acid sequence of the CD3-zeta signaling domain in the anti-NPM1c c CAR | CGCGTGAAATTCAGCCGCAGCGCAGATGCTCCAGCCTACAAGCAGG GGCAGAACCAGCTCTACAACGAACTCAATCTTGGTCGGAGAGAGGA GTACGACGTGCTGGACAAGCGGAGAGGACGGGACCCAGAAATGGGC GGGAAGCCGCGCAGAAAGAATCCCCAAGAGGGCCTGTACAACGAGC TCCAAAAGGATAAGATGGCAGAAGCCTATAGCGAGATTGGTATGAA AGGGGAACGCAGAAGAGGCAAAGGCCACGACGGACTGTACCAGGGA CTCAGCACCGCCACCAAGGACACCTATGACGCTCTTCACATGCAGG CCCTGCCGCCTCGG |
| 36 | Nucleic acid sequence of the P2A self-cleaving peptide in the anti-NPM1c CAR | GCAACAAACTTCTCTCTGCTGAAACAAGCCGGAGATGTCGAAGAGA ATCCTGGACCG |

(continued)

| SEQ ID NO: | Description | Sequence |
|---|---|---|
| 37 | Nucleic acid sequence of the EGFP region in the anti-NPM1c CAR | ATGGTGAGCAAGGGCGAGGAGCTGTTCACCGGGGTGGTGCCCATCC TGGTCGAGCTGGACGGCGACGTAAACGGCCACAAGTTCAGCGTGTC CGGCGAGGGCGAGGGCGATGCCACCTACGGCAAGCTGACCCTGAAG TTCATCTGCACCACCGGCAAGCTGCCCGTGCCCTGGCCCACCCTCG TGACCACCCTGACCTACGGCGTGCAGTGCTTCAGCCGCTACCCCGA CCACATGAAGCAGCACGACTTCTTCAAGTCCGCCATGCCCGAAGGC TACGTCCAGGAGCGCACCATCTTCTTCAAGGACGACGGCAACTACA AGACCCGCGCCGAGGTGAAGTTCGAGGGCGACACCCTGGTGAACCG CATCGAGCTGAAGGGCATCGACTTCAAGGAGGACGGCAACATCCTG GGGCACAAGCTGGAGTACAACTACAACAGCCACAACGTCTATATCA TGGCCGACAAGCAGAAGAACGGCATCAAGGTGAACTTCAAGATCCG CCACAACATCGAGGACGGCAGCGTGCAGCTCGCCGACCACTACCAG CAGAACACCCCCATCGGCGACGGCCCCGTGCTGCTGCCCGACAACC ACTACCTGAGCACCCAGTCCGCCCTGAGCAAAGACCCCAACGAGAA GCGCGATCACATGGTCCTGCTGGAGTTCGTGACCGCCGCCGGGATC ACTCTCGGCATGGACGAGCTGTACAAGTGA |
| 38 | Human IgG1 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSRDELTKNQVSLTCLVKGFYPSDIAVEWESNGQPENNYKT TPPVLDSDGSFFLYSKLTVDKSRWQQGNVFSCSVMHEALHNHYTQK SLSLSPGK |
| 39 | Human IgG4 (terminal K absent) | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCPSCPAPEFLGGPSVFLFPPKPKDTLMISRTPE VTCVVVDVSQEDPEVQFNWYVDGVEVHNAKTKPREEQFNSTYRVVS VLTVLHQDWLNGKEYKCKVSNKGLPSSIEKTISKAKGQPREPQVYT LPPSQEEMTKNQVSLTCLVKGFYPSDIAVEWESNGQPENNYKTTPP VLDSDGSFFLYSRLTVDKSRWQEGNVFSCSVMHEALHNHYTQKSLS LSLG– |
| 40 | Human IgG4 single mutant (S228P) (terminal K absent) | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCP**P**CPAPEFLGGPSVFLFPPKPKDTLMISRTPE VTCVVVDVSQEDPEVQFNWYVDGVEVHNAKTKPREEQFNSTYRVVS VLTVLHQDWLNGKEYKCKVSNKGLPSSIEKTISKAKGQPREPQVYT LPPSQEEMTKNQVSLTCLVKGFYPSDIAVEWESNGQPENNYKTTPP VLDSDGSFFLYSRLTVDKSRWQEGNVFSCSVMHEALHNHYTQKSLS LSLG– |

(continued)

| SEQ ID NO: | Description | Sequence |
|---|---|---|
| 41 | Human IgG4 double mutant (S228P) (L235E) (terminal K absent) | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCP**P**CPAPEF**E**GGPSVFLFPPKPKDTLMISRTPE VTCVVVDVSQEDPEVQFNWYVDGVEVHNAKTKPREEQFNSTYRVVS VLTVLHQDWLNGKEYKCKVSNKGLPSSIEKTISKAKGQPREPQVYT LPPSQEEMTKNQVSLTCLVKGFYPSDIAVEWESNGQPENNYKTTPP VLDSDGSFFLYSRLTVDKSRWQEGNVFSCSVMHEALHNHYTQKSLS LSLG– |
| 42 | Human IgG4 double mutant (S228P) (L235A) (terminal K absent) | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCP**P**CPAPEF**A**GGPSVFLFPPKPKDTLMISRTPE VTCVVVDVSQEDPEVQFNWYVDGVEVHNAKTKPREEQFNSTYRVVS VLTVLHQDWLNGKEYKCKVSNKGLPSSIEKTISKAKGQPREPQVYT LPPSQEEMTKNQVSLTCLVKGFYPSDIAVEWESNGQPENNYKTTPP VLDSDGSFFLYSRLTVDKSRWQEGNVFSCSVMHEALHNHYTQKSLS LSLG– |
| 43 | CD20 amino acid sequence | MTTPRNSVNGTFPAEPMKGPIAMQSGPKPLFRRMSSLVGPTQSFFM RESKTLGAVQIMNGLFHIALGGLLMIPAGIYAPICVTVWYPLWGGI MYIISGSLLAATEKNSRKCLVKGKMIMNSLSLFAAISGMILSIMDI LNIKISHFLKMESLNFIRAHTPYINIYNCEPANPSEKNSPSTQYCY SIQSLFLGILSVMLIFAFFQELVIAGIVENEWKRTCSRPKSNIVLL SAEEKKEQTIEIKEEVVGLTETSSQPKNEEDIEIIPIQEEEEEETE TNFPEPPQDQESSPIENDSSP |
| 44 | FLAG | DYKDDDDK |
| 45 | polyhistidine (6-His) | HHHHHH |
| 46 | hemagglutinin (HA) | YPYDVPDYA |
| 47 | HCDR1.1 (anti-CD3) amino acid sequence | GYTFTRYTMH |
| 48 | HCDR1.2 (anti-CD3) amino acid sequence | RYTMH |
| 49 | HCDR2 (anti-CD3) amino acid sequence | YINPSRGYTNYNQKFKD |
| 50 | HCDR3 (anti-CD3) amino acid sequence | YYDDHYCLDY |
| 51 | LCDR1 (anti-CD3) amino acid sequence | RASSSVSYMN |

(continued)

| SEQ ID NO: | Description | Sequence |
|---|---|---|
| 52 | LCDR2 (anti-CD3) amino acid sequence | DTSKVAS |
| 53 | LCDR3 (anti-CD3) amino acid sequence | QQWSSNPLT |
| 54 | Human wild type nucleophosmin (amino acid sequence - Accession # NM_002520) | MEDSMDMDMSPLRPQNYLFGCELKADKDYHFKVDNDENEHQLSLRT VSLGAGAKDELHIVEAEAMNYEGSPIKVTLATLKMSVQPTVSLGGF EITPPVVLRLKCGSGPVHISGQHLVAVEEDAESEDEEEEDVKLLSI SGKRSAPGGGSKVPQKKVKLAADEDDDDDEEDDDEDDDDDDFDDE EAEEKAPVKKSIRDTPAKNAQKSNQNGKDSKPSSTPRSKGQESFKK QEKTPKTPKGPSSVEDIKAKMQASIEKGGSLPKVEAKFINYVKNCF RMTDQEAIQDLWQWRKSL |
| 55 | C-terminus of human wild type nucleophosmin (amino acid sequence) | MTDQEAIQDLWQWRKSL |
| 56 | Human nucleophosmin encoded by mutant *NPM1c* gene (amino acid sequence) | MEDSMDMDMSPLRPQNYLFGCELKADKDYHFKVDNDENEHQLSLRT VSLGAGAKDELHIVEAEAMNYEGSPIKVTLATLKMSVQPTVSLGGF EITPPVVLRLKCGSGPVHISGQHLVAVEEDAESEDEEEEDVKLLSI SGKRSAPGGGSKVPQKKVKLAADEDDDDDEEDDDEDDDDDDFDDE EAEEKAPVKKSIRDTPAKNAQKSNQNGKDSKPSSTPRSKGQESFKK QEKTPKTPKGPSSVEDIKAKMQASIEKGGSLPKVEAKFINYVKNCF RMTDQEAIQDLCLAVEEVSLRK |
| 57 | C-terminus of human nucleophosmin encoded by mutant *NPM1c* gene (amino acid sequence) | MTDQEAIQDLCLAVEEVSLRK |
| 58 | Linker | (Gly4Ser) 1 |
| 59 | Linker | (Gly4Ser) 2 |
| 60 | Linker | (Gly4Ser) 3 |
| 61 | Linker | (Gly4Ser) 4 |
| 62 | NY-ESO-1 neoepitope (SLL) | SLLMWITQC |
| 63 | influenza virus M1 protein neoepitope (GIL) | GILGFVFTL |
| 64 | Forward primer | GTCAGTAATTGCGGTTCTCACC |
| 65 | Reverse primer | GTACAGTGGGAACAAAGTCG |

(continued)

| SEQ ID NO: | Description | Sequence |
|---|---|---|
| 66 | Forward primer | CCGGGGTAGAACCTAAAAGTTCCG |
| 67 | Reverse primer | TTTGTTCTGCACGCGTGGATC |
| 68 | Forward primer | GGGTAATTAATCAGCGAAGCGATG |
| 69 | Forward primer | GTTAGGCCAGCTTGGCACTTGATGT |
| 70 | Reverse primer | AGGCACAATCAGCATTGGTAGCTG |
| 71 | NPM1c neoepitope | AIQDLCVAV |
| 72 | NPM1c neoepitope | CLAVEEVSL |
| 73 | NPM1c neoepitope | VEEVSLRK |
| 74 | NPM1c neoepitope | AVEEVSLR |
| 75 | NPM1c neoepitope | AVEEVSLRK |
| 76 | NPM1c neoepitope | CLAVEEVSLRK |
| 77 | AIQ $X_1$ substitution | VIQDLCLAV |
| 78 | AIQ $X_1$ substitution | LIQDLCLAV |
| 79 | AIQ $X_1$ substitution | IIQDLCLAV |
| 80 | AIQ $X_3$ substitution | ANQDLCLAV |
| 81 | AIQ $X_4$ substitution | AIQELCLAV |
| 82 | AIQ $X_5$ substitution | AIQDICLAV |
| 83 | AIQ $X_5$ substitution | AIQDVCLAV |
| 84 | AIQ $X_5$ substitution | AIQDMCLAV |
| 85 | AIQ $X_5$ substitution | AIQDACLAV |
| 86 | AIQ $X_5$ substitution | AIQDFCLAV |
| 87 | AIQ $X_6$ substitution | AIQDLSLAV |
| 88 | AIQ $X_6$ substitution | AIQDLALAV |
| 89 | AIQ $X_7$ substitution | AIQDLCIAV |

(continued)

| SEQ ID NO: | Description | Sequence |
|---|---|---|
| 90 | AIQ $X_7$ substitution | AIQDLCVAV |
| 91 | AIQ $X_7$ substitution | AIQDLCMAV |
| 92 | AIQ $X_7$ substitution | AIQDLCAAV |
| 93 | AIQ $X_7$ substitution | AIQDLCFAV |
| 94 | AIQ $X_8$ substitution | AIQDLCLVV |
| 95 | AIQ $X_8$ substitution | AIQDLCLLV |
| 96 | AIQ $X_8$ substitution | AIQDLCLIV |
| 97 | Mature IL-15 amino acid sequence | NWVNVISDLKKIEDLIQSMHIDATLYTESDVHPSCKVTAMKCFLLE LQVISLESGDASIHDTVENLIILANNSLSSNGNVTESGCKECEELE EKNIKEFLQSFVHIVQMFINTS |
| 98 | Mature IL-15 nucleotide sequence | AACTGGGTGAATGTAATAAGTGATTTGAAAAAAATTGAAGATCTTA TTCAATCTATGCATATTGATGCTACTTTATATACGGAAAGTGATGT TCACCCCAGTTGCAAAGTAACAGCAATGAAGTGCTTTCTCTTGGAG TTACAAGTTATTTCACTTGAGTCCGGAGATGCAAGTATTCATGATA CAGTAGAAAATCTGATCATCCTAGCAAACAACAGTTTGTCTTCTAA TGGGAATGTAACAGAATCTGGATGCAAAGAATGTGAGGAACTGGAG GAAAAAAATATTAAAGAATTTTTGCAGAGTTTTGTACATATTGTCC AAATGTTCATCAACACTTCT |

(continued)

| SEQ ID NO: | Description | Sequence |
|---|---|---|
| 99 | anti-NPM1c CAR with membrane-bound (mb) IL-15 (DNA) | atggccctccctgtcaccgccctgctgcttccgctggctcttctgctccacgccgctcggcccGACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCGCTCACGTTCGGCCAAGGGACCAAGGTGGAAATCAAATCCGGAATTCTAGGTACTACTGCCGCTAGTGGTAGTAGTGGTGGCAGTAGCAGTGGTGCCGAGGTGCAGCTGGTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAGACAATTCCAAGAACACGCTGTATCTGCAAATGAACAGCCTGAGAGCCGAGGACACGGCCGTGTATTACTGTGCGAGGCTGGGTTACCCTACTACTACCCTACTACCCTTTGATTACTGGGGCCAAGGTACCCTGGTCACTGTCTCCAGT*accactaccccagcaccgaggccacccaccccggctcctaccatcgcctcccagcctctgtccctgcgtccggaggcatgtagacccgcagctggtggggccgtgcataccggggtcttgacttcgcctgcgat*atctacatttgggcccctctg*ctggtacttgcggggtcctgctgctttcactcgtgatcactcttt*actgtaagcgcggtcggaagaagctgctgtacatctttaagcaacccttcatgaggcctgtgcagactactcaagaggaggacggctgttcatgccggttcccagaggaggaggaaggcggctgcgaactg****cgcgtgaaattcagccgcagcgcagatgctccagcctacaagcaggggcagaaccagctctacaacgaactcaatcttggtcggagagaggagtacgacgtgctggacaagcggagaggacgggacccagaaatgggcgggaagccgcgcagaaagaatccccaagagggcctgtacaacgagctccaaaaggataagatggcagaagcctatagcgagattggtatgaaaggggaacgcagaagaggcaaaggccacgacggactgtaccagggactcagcaccgccaccaaggacacctatgacgctcttcacatgcaggccctgcccgcctcgg*GGATCCGGCGCAACAAACTTCTCTCTGCTGAAACAAGCCGGAGATGTCGAAGAGAATCCTGGACCGatggccctccctgtcaccgccctgctgcttccgctggctcttctgctccacgccgctcggccc*aactgggtgaatgtaataagtgatttgaaaaaaattgaagatcttattcaatctatgcatattgatgctactttatatacggaaagtgatgttcaccccagttgcaaagtaacagcaatgaagtgctttctcttggagttacaagttatttcacttgagtccggagatgcaagtattcatgatacagtagaaatctgatcatcctagcaaacaacagtttgtcttctaatgggaatgtaacagaatctggatgcaaagaatgtgaggaactggaggaaaaaatattaaagaattttttgcagagttttgtacatattgtccaatgttcatcaacacttct*accactaccccagcaccgaggccacccaccccggctcctaccatcgcctcccagcctctgtccctgcgtccgga* |

| SEQ ID NO: | Description | Sequence |
|---|---|---|
| | | *ggcatgtagacccgcagctggtggggccgtgcatacccggggtctt gacttcgcctgcgat<u>atctacatttgggcccctctggctggtactt gcggggtcctgctgctttcactcgtgatcactctttactgtt</u>ga*<br><br>CD8 signal peptide **(bold)**; anti-NPM1c scFV **(bold underline)**; CD8 hinge (*italics*); CD8 transmembrande domain (*italics underline*); 4-1BB (underline); CD3z **(*bold italics*)**; P2A (double underline); Mature IL-15 (dashed underline) |
| 100 | anti-NPM1c CAR with mbIL-15 (amino acid) | **MALPVTALLLPLALLLHAARP**<u>**DIQMTQSPSSLSASVGDRVTITCRA SQSISSYLNWYQQKPGKAPKLLIYAASSLQSGVPSRFSGSGSGTDF TLTISSLQPEDFATYYCQQSYSTPLTFGQGTKVEIKSGILGTTAAS GSSGGSSSGAEVQLVESGGGLVQPGGSLRLSCAASGFTFSSYAMSW VRQAPGKGLEWVSAISGSGGSTYYADSVKGRFTISRDNSKNTLYLQ MNSLRAEDTAVYYCARLGYPTTTLLPFDYWGQGTLVTVSS**</u>*TTTPAP RPPTPAPTIASQPLSLRPEACRPAAGGAVHTRGLDFACD<u>IYIWAPL AGTCGVLLLSLVITLYC</u>*<u>KRGRKKLLYIFKQPFMRPVQTTQEEDGCS CRFPEEEEGGCEL</u>**_RVKFSRSADAPAYKQGQNQLYNELNLGRREEYD VLDKRRGRDPEMGGKPRRKNPQEGLYNELQKDKMAEAYSEIGMKGE RRRGKGHDGLYQGLSTATKDTYDALHMQALPPR_**<u><u>GSGATNFSLLKQA GDVEENPGP</u></u>**MALPVTALLLPLALLLHAARP**<span style="text-decoration: underline dashed">NWVNVISDLKKIEDLI QSMHIDATLYTESDVHPSCKVTAMKCFLLELQVISLESGDASIHDT VENLIILANNSLSSNGNVTESGCKECEELEEKNIKEFLQSFVHIVQ MFINTS</span>*TTTPAPRPPTPAPTIASQPLSLRPEACRPAAGGAVHTRGL DFACD*<u>*IYIWAPLAGTCGVLLLSLVITLYC*</u>*</u>

CD8 signal peptide **(bold)**; anti-NPM1c scFV **(bold underline)**; CD8 hinge (*italics*); CD8 transmembrande domain (*italics underline*); 4-1BB (underline); CD3z **(*bold italics*)**; P2A (double underline); Mature IL-15 (dashed underline) |
| 101 | anti-NPM1c CAR with secreted (s) IL-15 (DNA) | atggccctccctgtcaccgccctgctgcttccgctggctcttctgc tccacgccgctcggccc<u>GACATCCAGATGACCCAGTCTCCATCCTC CTGTCTGCATCTGTAGGAGACAGAGTCACCATCACTTGCCGGGCA AGTCAGAGCATTAGCAGCTATTTAAATTGGTATCAGCAGAAACCAG GGAAAGCCCCTAAGCTCCTGATCTATGCTGCATCCAGTTTGCAAAG TGGGGTCCCATCAAGGTTCAGTGGCAGTGGATCTGGGACAGATTTC ACTCTCACCATCAGCAGTCTGCAACCTGAAGATTTTGCAACTTACT ACTGTCAACAGAGTTACAGTACCCCGCTCACGTTCGGCCAAGGGAC CAAGGTGGAAATCAAATCCGGAATTCTAGGTACTACTGCCGCTAGT GGTAGTAGTGGTGGCAGTAGCAGTGGTGCCGAGGTGCAGCTGGTGG AGTCTGGGGGAGGCTTGGTACAGCCTGGGGGGTCCCTGAGACTCTC CTGTGCAGCCTCTGGATTCACCTTTAGCAGCTATGCCATGAGCTGG</u> |

| SEQ ID NO: | Description | Sequence |
|---|---|---|
|  |  | **GTCCGCCAGGCTCCAGGGAAGGGGCTGGAGTGGGTCTCAGCTATTA GTGGTAGTGGTGGTAGCACATACTACGCAGACTCCGTGAAGGGCCG GTTCACCATCTCCAGAGACAATTCCAAGAACACGCTGTATCTGCAA ATGAACAGCCTGAGAGCCGAGGACACGGCCGTGTATTACTGTGCGA GGCTGGGTTACCCTACTACTACCCTACTACCCTTTGATTACTGGGG CCAAGGTACCCTGGTCACTGTCTCCAGT**_accactaccccagcaccg aggccacccacccggctcctaccatcgcctcccagcctctgtccc tgcgtccggaggcatgtagacccgcagctggtggggccgtgcatac ccggggtcttgacttcgcctgcgat_<u>_atctacatttgggcccctctg gctggtacttgcgggggtcctgctgctttcactcgtgatcactcttt actgt_</u>_aagcgcggtcggaagaagctgctgtacatctttaagcaacc cttcatgaggcctgtgcagactactcaagaggaggacggctgttca tgccggttcccagaggaggaggaaggcggctgcgaactg_**cgcgtga aattcagccgcagcgcagatgctccagcctacaagcaggggcagaa ccagctctacaacgaactcaatcttggtcggagagaggagtacgac gtgctggacaagcggagaggacgggacccagaaatgggcgggaagc cgcgcagaaagaatccccaagagggcctgtacaacgagctccaaaa ggataagatggcagaagcctatagcgagattggtatgaaaggggaa cgcagaagaggcaaaggc**cacgacggactgtaccagggactcagca ccgccaccaaggacacctatgacgctcttcacatgcaggccctgcc gcctcgg<u>GGATCCGGC</u><u>GCAACAAACTTCTCTCTGCTGAAACAAGCC GGAGATGTCGAAGAGAATCCTGGACCG</u>**atggccctccctgtcaccg ccctgctgcttccgctggctcttctgctccacgccgctcggccc**<u>aa ctgggtgaatgtaataagtgatttgaaaaaaattgaagatcttatt caatctatgcatattgatgctactttatatacggaaagtgatgttc accccagttgcaaagtaacagcaatgaagtgctttctcttggagtt acaagttatttcacttgagtccggagatgcaagtattcatgataca gtagaaatctgatcatcctagcaaacaacagtttgtcttctaatg ggaatgtaacagaatctggatgcaaagaatgtgaggaactggagga aaaaaatattaaagaattttgcagagttttgtacatattgtccaa atgttcatcaacacttcttga</u> |

CD8 signal peptide **(bold);** anti-NPM1c scFV **(bold underline);** CD8 hinge (_italics_); CD8 transmembrande domain (_italics underline_)_;_ 4-1BB (<u>underline</u>); CD3z **(_bold italics_);** P2A (<u>double underline</u>); Mature IL-15 (<u>dashed underline</u>) |
| 102 | anti-NPM1c CAR with sIL-15 (amino acid) | <u>MALPVTALLLPLALLLHAARP</u>DIQMTQSPSSLSASVGDRVTITCRA SQSISSYLNWYQQKPGKAPKLLIYAASSLQSGVPSRFSGSGSGTDF TLTISSLQPEDFATYYCQQSYSTPLTFGQGTKVEIKSGILGTTAAS GSSGGSSSGAEVQLVESGGGLVQPGGSLRLSCAASGFTFSSYAMSW VRQAPGKGLEWVSAISGSGGSTYYADSVKGRFTISRDNSKNTLYLQ MNSLRAEDTAVYYCARLGYPTTTLLPFDYWGQGTLVTVSS_TTTPAP RPPTPAPTIASQPLSLRPEACRPAAGGAVHTRGLDFACD_<u>_IYIWAPL AGTCGVLLLSLVITLYC_</u>**KRGRKKLLYIFKQPFMRPVQTTQEEDGCS CRFPEEEEGGCEL**_RVKFSRSADAPAYKQGQNQLYNELNLGRREEYD_ |

(continued)

| SEQ ID NO: | Description | Sequence |
|---|---|---|
| | | *VLDKRRGRDPEMGGKPRRKNPQEGLYNELQKDKMAEAYSEIGMKGE* *RRRGKGHDGLYQGLSTATKDTYDALHMQALPPRGSG*<u>ATNFSLLKQA</u> <u>GDVEENPGP</u>MALPVTALLLPLALLLHAARPNWVNVISDLKKIEDLI QSMHIDATLYTESDVHPSCKVTAMKCFLLELQVISLESGDASIHDT VENLIILANNSLSSNGNVTESGCKECEELEEKNIKEFLQSFVHIVQ MFINTS* <br><br>CD8 signal peptide **(bold);** anti-NPM1c scFV **(bold underline);** CD8 hinge (*italics*); CD8 transmembrande domain (*italics underline*); 4-1BB (underline); CD3z (***bold italics***); P2A (double underline); Mature IL-15 (dashed underline) |
| 103 | CD8 hinge (DNA) | accactaccccagcaccgaggccacccacccggctcctaccatcg cctcccagcctctgtccctgcgtccggaggcatgtagacccgcagc tggtggggccgtgcatacccgggggtcttgacttcgcctgcgat |
| 104 | CD8 hinge (aa) | TTTPAPRPPTPAPTIASQPLSLRPEACRPAAGGAVHTRGLDFACD |
| 105 | CD8 transmembrane (DNA) | Atctacatttgggcccctctggctggtacttgcggggtcctgctgc tttcactcgtgatcactctttactgt |
| 106 | CD8 transmembrane (aa) | IYIWAPLAGTCGVLLLSLVITLYC |
| 107 | BaEV glycoprotein (amino acid) | MGFTTKIIFLYNLVLVYAGFDDPRKAIELVQKRYGRPCDCSGGQVS EPPSDRVSQVTCSGKTAYLMPDQRWKCKSIPKDTSPSGPLQECPCN SYQSSVHSSCYTSYQQCRSGNKTYYTATLLKTQTGGTSDVQVLGST NKLIQSPCNGIKGQSICWSTTAPIHVSDGGGPLDTTRIKSVQRKLE EIHKALYPELQYHPLAIPKVRDNLMVDAQTLNILNATYNLLLMSNT SLVDDCWLCLKLGPPTPLAIPNFLLSYVTRSSDNISCLIIPPLLVQ PMQFSNSSCLFSPSYNSTEEIDLGHVAFSNCTSITNVTGPICAVNG SVFLCGNNMAYTYLPTNWTGLCVLATLLPDIDIIPGDEPVPIPAID HFIYRPKRAIQFIPLLAGLGITAAFTTGATGLGVSVTQYTKLSNQL ISDVQILSSTIQDLQDQVDSLAEVVLQNRRGLDLLTAEQGGICLAL QEKCCFYVNKSGIVRDKIKTLQEELERRRKDLASNPLWTGLQGLLP YLLPFLGPLLTLLLLLTIGPCIFNRLTAFINDKLNIIHAM* |
| 108 | BaEV glycoprotein (DNA) | ATGGGTTTCACTACGAAAATTATCTTTCTGTATAATCTGGTACTCG TATATGCGGGTTTCGACGATCCCAGGAAAGCGATCGAACTTGTCCA GAAGAGATACGGGAGGCCCTGTGACTGCAGCGGAGGGCAAGTATCA GAACCCCCTCTGATCGGGTCAGCCAAGTTACTTGCAGCGGCAAAA CAGCTTACCTGATGCCGGATCAGAGATGGAAATGCAAATCCATACC CAAGGACACCAGTCCGAGTGGACCATTGCAGGAATGTCCGTGTAAT AGTTACCAATCAAGCGTCCATTCAAGTTGCTACACGTCATACCAGC AATGTCGCTCAGGAAATAAAACCTATTATACGGCGACACTGCTTAA AACCCAAACGGGTGGCACCTCTGATGTTCAGGTTCTCGGAAGTACG |

(continued)

| SEQ ID NO: | Description | Sequence |
|---|---|---|
|  |  | AATAAGTTGATTCAGAGTCCCTGCAACGGTATCAAAGGCCAGTCAA TTTGTTGGTCTACGACAGCGCCTATCCATGTGAGTGACGGCGGTGG GCCGTTGGATACAACACGAATAAAAAGTGTACAGCGGAAACTTGAG GAGATACACAAAGCCCTCTACCCCGAGCTTCAGTACCATCCCCTGG CCATCCCTAAGGTCAGGGACAATCTCATGGTAGACGCTCAAACCCT CAACATCCTCAATGCCACCTACAATCTCTTGTTGATGTCTAACACA AGCTTGGTAGATGACTGCTGGCTCTGTCTTAAATTGGGCCCTCCGA CTCCCCTCGCTATACCCAACTTCCTTCTGTCATACGTAACGCGCAG CTCCGACAACATATCATGTCTGATAATCCCGCCGTTGCTTGTGCAG CCCATGCAGTTCTCTAACAGCTCCTGCTTGTTCAGTCCATCTTATA ATTCAACAGAAGAAATTGATTTGGGCCATGTAGCTTTCAGTAACTG TACATCAATAACTAACGTCACTGGCCCCATCTGCGCCGTGAACGGT TCTGTCTTCCTCTGCGGCAACAATATGGCTTATACATACTTGCCAA CTAACTGGACCGGTCTGTGTGTATTGGCCACGCTGTTGCCTGACAT AGATATAATCCCTGGCGACGAACCCGTCCCTATCCCAGCCATCGAC CATTTTATTTATCGCCCCAAGCGCGCGATTCAGTTTATCCCTCTGC TCGCTGGGTTGGGCATTACGGCTGCTTTTACTACGGGGGCTACCGG CCTTGGAGTGTCCGTTACCCAATATACGAAACTGTCCAATCAATTG ATTTCAGACGTGCAAATCTTGAGCTCTACTATCCAGGATCTGCAGG ACCAGGTAGACTCTCTGGCGGAAGTCGTCTTGCAAAATCGGCGGGG GTTGGATCTGCTGACCGCCGAGCAGGGCGGCATCTGTCTTGCTCTT CAAGAAAAATGCTGTTTTTACGTGAACAAATCAGGTATTGTAAGAG ATAAAATAAAAACTTTGCAAGAAGAGCTCGAAAGGAGGCGGAAAGA CCTGGCGTCTAATCCTCTGTGGACTGGCCTGCAGGGGCTCCTCCCC TATTTGCTGCCCTTTCTTGGTCCGCTCCTGACTTTGTTGCTGCTCC TGACTATTGGGCCATGCATCTTCAATCGACTCACCGCGTTCATCAA TGATAAACTCAACATAATCCACGCTATGTGA |

**Additional References**

Greiner J, Ono Y, Hofmann S, Schmitt A, Mehring E, Götz M, Guillaume P, Döhner K, Mytilineos J, Döhner H, Schmitt M. Mutated regions of nucleophosmin 1 elicit both CD4+ and CD8+ T-cell responses in patients with acute myeloid leukemia. Blood. 2012;120(6):1282-1289.

Greiner J, Schneider V, Schmitt M, Götz M, Döhner K, Wiesneth M, Döhner H, Hofmann S. Immune responses against the mutated region of cytoplasmatic NPM1 might contribute to the favorable clinical outcome of AML patients with NPM1 mutations (NPM1mut). Blood. 2013;122(6):1087-8.

Felices M, Lenvik AJ, McElmurry R, Chu S, Hinderlie P, Bendzick L, Geller MA, Tolar J, Blazar BR, Miller JS. Continuous treatment with IL-15 exhausts human NK cells via a metabolic defect. JCI insight. 2018;3(3).

Kohn, D.B. & Hollis, R.P. Envelope, please. And the award goes to. Blood 124, 1203-1204 (2014).

Bald, T., Krummel, M. F., Smyth, M. J., & Barry, K. C. (2020). The NK cell-cancer cycle: advances and new challenges in NK cell-based immunotherapies. Nat Immunol, 21(8), 835-847. do1:10.1038/5s41590-020-0728-z

Chan, C. J., Smyth, M. J., & Martinet, L. (2014). Molecular mechanisms of natural killer cell activation in response to cellular stress. Cell Death Differ, 21(1), 5-14. doi: 10.1038/cdd.2013.26

Chiossone, L., Dumas, P. Y., Vienne, M., & Vivier, E. (2018). Natural killer cells and other innate lymphoid cells in cancer. Nat Rev Immunol, 18(11), 671-688. doi:10.1038/s41577-018-0061-z

Felices, M., Lenvik, A. J., McElmurry, R., Chu, S., Hinderlie, P., Bendzick, L., . . . Miller, J. S. (2018). Continuous treatment with IL-15 exhausts human NK cells via a metabolic defect. JCI Insight, 3(3). doi:10.1172/jci.insight.96219

Hashimoto, N., Tsuboi, A., Kagawa, N., Chiba, Y., Izumoto, S., Kinoshita, M., ... Sugiyama, H. (2015). Wilms tumor 1 peptide vaccination combined with temozolomide against newly diagnosed glioblastoma: safety and impact on immunological response. Cancer Immunol Immunother, 64(6), 707-716. doi:10.1007/s00262-015-1674-8

Romee, R., Rosario, M., Berrien-Elliott, M. M., Wagner, J. A., Jewell, B. A., Schappe, T., ... Fehniger, T. A. (2016). Cytokine-induced memory-like natural killer cells exhibit enhanced responses against myeloid leukemia. Sci Transl Med, 8(357), 357ra123. doi:10.1126/scitranslmed.aaf2341

Smith, C. C., Wang, Q., Chin, C. S., Salerno, S., Damon, L. E., Levis, M. J., ... Shah, N. P. (2012). Validation of ITD mutations in FLT3 as a therapeutic target in human acute myeloid leukaemia. Nature, 485(7397), 260-263. doi:10.1038/nature11016

Thomas, R., Al-Khadairi, G., Roelands, J., Hendrickx, W., Dermime, S., Bedognetti, D., & Decock, J. (2018). NY-ESO-1 Based Immunotherapy of Cancer: Current Perspectives. Front Immunol, 9, 947. doi:10.3389/fimmu.2018.0094

Wang, E., & Aifantis, I. (2020). RNA Splicing and Cancer. Trends Cancer, 6(8), 631-644. doi:10.1016/j.trecan.2020.04.011

Xie, G., Dong, H., Liang, Y., Ham, J. D., Rizwan, R., & Chen, J. (2020). CAR-NK cells: A promising cellular immunotherapy for cancer. EBioMedicine, 59, 102975. doi: 10.10 16/j .ebiom.2020.1 02975

Mensali, N., Dillard, P., Hebeisen, M., Lorenz, S., Theodossiou, T., Myhre, M. R., ... Walchli, S. (2019). NK cells specifically TCR-dressed to kill cancer cells. EBioMedicine, 40, 106-117. doi:10.1016/j.ebiom.2019.01.031

Walseng, E., Koksal, H., Sektioglu, I. M., Fane, A., Skorstad, G., Kvalheim, G., ... Walchli, S. (2017). A TCR-based Chimeric Antigen Receptor. Sci Rep, 7(1), 10713. doi:10.1038/s41598-017-11126-y

## Claims

1. An engineered cytokine-induced memory-like human NK cell or a population of said cells, wherein the engineered NK cell or population of said cells expresses a chimeric antigen receptor (CAR) polypeptide comprising an intracellular domain, a transmembrane domain and an extracellular binding domain, wherein the extracellular binding domain specifically binds to an antigen comprising an NPM1c neoepitope in complex with a class I major histocompatibility complex (MHC class I) protein.

2. The engineered NK cell or population of cells of claim 1, wherein the extracellular binding domain does not bind to, or substantially does not bind to: (a) the MHC class I protein alone, and/or (b) a control peptide in complex with the MHC class I protein, optionally wherein the control peptide is an NY-ESO-1 epitope or influenza virus M1 epitope.

3. The engineered NK cell or population of cells of claim 1 or 2, wherein the NPM1c neoepitope comprises an amino acid sequence selected from: AIQDLCLAV (SEQ ID NO:1) or AIQDLCVAV (SEQ ID NO: 71).

4. The engineered NK cell or population of cells of claim 1 or 2, wherein the NPM1c neoepitope comprises an amino acid sequence selected from: CLAVEEVSL (SEQ ID NO:72), VEEVSLRK (SEQ ID NO:73), AVEEVSLR (SEQ ID NO:74), AVEEVSLRK (SEQ ID NO:75), CLAVEEVSLRK (SEQ ID NO:76).

5. The engineered NK cell or population of cells of any one of claims 1-4, wherein the MHC class I protein is an HLA-A*02 protein and/or is encoded by the HLA-A*02 allele group.

6. The engineered NK cell or population of cells of any one of claims 1-5, wherein the extracellular domain comprises:

(i) a heavy chain variable region (VH) comprising VH complementarity determining region (CDR)1, VH CDR2 and VH CDR3, said VH CDR1, VH CDR2 and VH CDR3 being the CDRs of a VH that has an amino acid sequence of SEQ ID NO: 5, and/or
(ii) a light chain variable region (VL) comprising VL complementarity determining region (CDR)1, VL CDR2 and VL CDR3, said VL CDR1, VL CDR2 and VL CDR3 being the CDRs of a VL that has an amino acid sequence of SEQ ID NO: 3.

7. The engineered NK cell or population of cells of any one of claims 1-6, wherein the extracellular domain comprises a VH comprising VH CDR1, VH CDR2 and VH CDR3, wherein the VH CDR1 has the amino acid sequence GFTFSSYA (SEQ ID NO: 9), the VH CDR2 has the amino acid sequence ISGSGGST (SEQ ID NO: 10), and the VH CDR3 has the amino acid sequence ARLGYPTTTLLPFDY (SEQ ID NO: 11),wherein optionally the extracellular domain comprises a VL comprising VL CDR1, VL CDR2 and VL CDR3, wherein the VL CDR1 has the amino acid sequence QSISSY (SEQ ID NO: 6), the VL CD2 has the amino acid sequence AAS (SEQ ID NO: 7), and the VL CD3 has the amino acid sequence QQSYSTPLT (SEQ ID NO: 8).

8. The engineered NK cell or population of cells of any one of claims 1-7, wherein the extracellular domain comprises a scFV, wherein optionally the scFV comprises a Gly-Ser linker selected from the group consisting of (Gly4Ser) (SEQ ID NO:58), (Gly4Ser)2 (SEQ ID NO:59), (Gly4Ser)3 (SEQ ID NO:60), and (Gly4Ser)4 (SEQ ID NO:61).

9. The engineered NK cell or population of cells of claim 8, wherein the scFv comprises the amino acid sequence of SEQ ID NO: 2.

10. The engineered NK cell or population of cells, according to any one of claims 1-9, wherein the antigen is on the surface of a cancer cell and wherein optionally the cancer is Acute Myeloid Leukemia (AML).

11. The engineered NK cell or population of cells of any one of claims 1-10, wherein the transmembrane domain comprises the transmembrane domain of CD3-zeta, CD8, CD28, DAP12, 2B4, NKG2D, CD16, NKp44 or NKp46 and/or wherein the intracellular domain comprises one or more costimulatory domains of one or more costimulatory molecules selected from the group consisting of: CD27, CD28, 4-1BB, OX40, CD30, CD40, PD-1, ICOS, 2B4, DAP10, CD137 and DAP12.

12. The engineered NK cell or population of cells of any one of the claims 1-11, wherein the intracellular domain comprises a CD3-zeta signaling domain comprising the amino acid sequence set forth in SEQ ID NO: 27, and a 4-1BB costimulatory domain comprising the amino acid sequence set forth in SEQ ID NO: 26; wherein the CAR polypeptide comprises a CD8 transmembrane domain and a CD8 hinge region, wherein the CD8 transmembrane domain and the CD8 hinge region comprise the amino acid sequence set forth in SEQ ID NO: 25; and wherein the extracellular binding domain comprises the antibody, or antigen binding fragment thereof, and a leading sequence comprising the amino acid sequence set forth in SEQ ID NO: 23.

13. The engineered NK cell or population of cells of any one of the claims 1-12, wherein the intracellular domain further comprises a self-cleaving peptide sequence and a cytokine, wherein cleavage of the self-cleaving peptide releases the cytokine,
wherein optionally the cytokine is IL-12, IL-7, IL-13, IL-15, TNF-α, IFN-y, or CCL19.

14. The engineered NK cell or population of cells of any one of claims 1-13, which

(i) produces increased IFNγ in the presence of one or more cytokines and/or tumor targets relative to a control human NK cell or human NK cell line;
(ii) has enhanced antibody-dependent cellular cytotoxicity relative to a control human NK cell or human NK cell line; and/or
(iii) has enhanced anti-tumor efficacy relative to a control human NK cell or human NK cell line,

optionally wherein the control NK cell is a human NK cell activated in the presence of IL-15 alone, or a human NK cell line activated in the presence of IL-15 alone.

**15.** The engineered NK cell or population of cells of any one of claims 1-14, which expresses an IL-15 polypeptide, optionally a human IL-15 polypeptide,
wherein optionally the IL-15 polypeptide is a secreted IL-15 polypeptide or a membrane bound IL-15 polypeptide.

**Patentansprüche**

**1.** Manipulierte zytokininduzierte gedächtnisähnliche humane NK-Zelle oder eine Population der Zellen, wobei die manipulierte NK-Zelle oder Population der Zellen ein chimäres Antigenrezeptor-(CAR)-Polypeptid exprimiert, das eine intrazelluläre Domäne, eine Transmembrandomäne und eine extrazelluläre Bindungsdomäne umfasst, wobei die extrazelluläre Bindungsdomäne spezifisch an ein Antigen bindet, das ein NPM1c-Neoepitop im Komplex mit einem Protein eines Haupt-Histokompatibilitätskomplexes der Klasse I (MHC-Klasse I) umfasst.

**2.** Manipulierte NK-Zelle oder Population von Zellen nach Anspruch 1, wobei die extrazelluläre Bindungsdomäne nicht an Folgendes bindet, oder im Wesentlichen nicht an Folgendes bindet: (a) das MHC-Klasse-I-Protein allein und/oder (b) ein Kontrollpeptid im Komplex mit dem MHC-Klasse-I-Protein, wahlweise, wobei das Kontrollpeptid ein NY-ESO-1-Epitop oder Influenza-Virus-M1-Epitop ist.

**3.** Manipulierte NK-Zelle oder Population von Zellen nach Anspruch 1 oder 2, wobei das NPM1c-Neoepitop eine Aminosäuresequenz umfasst, ausgewählt aus: AIQDLCLAV (SEQ ID NO:1) oder AIQDLCVAV (SEQ ID NO: 71).

**4.** Manipulierte NK-Zelle oder Population von Zellen nach Anspruch 1 oder 2, wobei das NPM1c-Neoepitop eine Aminosäuresequenz umfasst, ausgewählt aus: CLAVEEVSL (SEQ ID NO:72), VEEVSLRK (SEQ ID NO:73), AVEEVSLR (SEQ ID NO:74), AVEEVSLRK (SEQ ID NO:75), CLAVEEVSLRK (SEQ ID NO:76).

**5.** Manipulierte NK-Zelle oder Population von Zellen nach einem der Ansprüche 1-4, wobei das MHC-Klasse-I-Protein ein HLA-A*02-Protein ist und/oder durch die HLA-A*02-Allelgruppe kodiert ist.

**6.** Manipulierte NK-Zelle oder Population von Zellen nach einem der Ansprüche 1-5, wobei die extrazelluläre Domäne Folgendes umfasst:

(i) eine Variableregion der schweren Kette (VH), umfassend VH Komplementaritätsbestimmende Region (CDR) 1, VH CDR2 und VH CDR3, wobei die VH CDR1, VH CDR2 und VH CDR3 die CDRs eines VH sind, das eine Aminosäuresequenz von SEQ ID NO: 5 aufweist, und/oder
(ii) eine Variableregion der leichten Kette (VL), umfassend VL Komplementaritätsbestimmende Region (CDR)1, VL CDR2 und VL CDR3, wobei die VL CDR1, VL CDR2 und VL CDR3 die CDRs einer VL sind, das eine Aminosäuresequenz von SEQ ID NO: 3 aufweist.

**7.** Manipulierte NK-Zelle oder Population von Zellen nach einem der Ansprüche 1-6, wobei die extrazelluläre Domäne ein VH umfasst, umfassend VH CDR1, VH CDR2 und VH CDR3, wobei die VH CDR1 die Aminosäuresequenz GETESSYA (SEQ ID NO: 9) aufweist, die VH CDR2 die Aminosäuresequenz ISGSGGST (SEQ ID NO: 10) aufweist und die VH CDR3 die Aminosäuresequenz ARLGYPTTTLLPFDY (SEQ ID NO: 11) aufweist, wobei wahlweise die extrazelluläre Domäne eine VL umfasst, umfassend VL CDR1, VL CDR2 und VL CDR3, wobei die VL CDR1 die Aminosäuresequenz QSISSY (SEQ ID NO: 6) aufweist, die VL CDR2 die Aminosäuresequenz AAS (SEQ ID NO: 7) aufweist und die VL CDR3 die Aminosäuresequenz QQSYSTPLT (SEQ ID NO: 8) aufweist.

**8.** Manipulierte NK-Zelle oder Population von Zellen nach einem der Ansprüche 1-7, wobei die extrazelluläre Domäne ein scFV umfasst, wobei wahlweise das scFV einen Gly-Ser-Linker umfasst, ausgewählt aus der Gruppe bestehend aus (Gly4Ser) (SEQ ID NO:58), (Gly4Ser)2 (SEQ ID NO:59), (Gly4Ser)3 (SEQ ID NO:60) und (Gly4Ser)4 (SEQ ID NO:61).

**9.** Manipulierte NK-Zelle oder Population von Zellen nach Anspruch 8, wobei das scFv die Aminosäuresequenz von SEQ ID NO: 2 umfasst.

**10.** Manipulierte NK-Zelle oder Population von Zellen, nach einem der Ansprüche 1-9, wobei das Antigen auf der Oberfläche einer Krebszelle ist, und wobei wahlweise der Krebs akute myeloische Leukämie (AML) ist.

**11.** Manipulierte NK-Zelle oder Population von Zellen nach einem der Ansprüche 1-10, wobei die Transmembrandomäne die Transmembrandomäne von CD3-zeta, CD8, CD28, DAP 12, 2B4, NKG2D, CD16, NKp44 oder NKp46 umfasst, und/oder wobei die intrazelluläre Domäne eine oder mehrere kostimulatorische Domänen eines oder mehrerer kostimulatorischer Moleküle umfasst, ausgewählt aus der Gruppe bestehend aus: CD27, CD28, 4-1BB, OX40, CD30, CD40, PD-1, ICOS, 2B4, DAP10, CD137 und DAP12.

12. Manipulierte NK-Zelle oder Population von Zellen nach einem der Ansprüche 1-11, wobei die intrazelluläre Domäne eine CD3-zeta-Signaldomäne umfasst, umfassend die in SEQ ID NO: 27 dargelegte Aminosäuresequenz, und eine 4-1BB kostimulatorische Domäne umfasst, umfassend die in SEQ ID NO: 26 dargelegte Aminosäuresequenz; wobei das CAR-Polypeptid eine CD8 Transmembrandomäne und eine CD 8 Scharnierregion umfasst, wobei die CD 8 Transmembrandomäne und die CD8 Scharnierregion die in SEQ ID NO: 25 dargelegte Aminosäuresequenz umfassen; und wobei die extrazelluläre Bindungsdomäne den Antikörper, oder ein Antigenbindungsfragment davon und eine Leadersequenz umfasst, umfassend die in SEQ ID NO: 23 dargelegte Aminosäuresequenz.

13. Manipulierte NK-Zelle oder Population von Zellen nach einem der Ansprüche 1-12, wobei die intrazelluläre Domäne weiter eine selbstspaltende Peptidsequenz und ein Zytokin umfasst, wobei die Spaltung des selbstspaltenden Peptids das Zytokin freisetzt,
wobei wahlweise das Zytokin IL-12, IL-7, IL-13, IL-15, TNF-$\alpha$, IFN-$\gamma$ oder CCL19 ist.

14. Manipulierte NK-Zelle oder Population von Zellen nach einem der Ansprüche 1-13, welche

(i) erhöhtes IFN$\gamma$ in Gegenwart eines oder mehrerer Zytokine und/oder Tumorziele in Bezug auf eine humane Kontroll-NK-Zelle oder humane Kontroll-NK-Zelllinie produziert;
(ii) eine verstärkte antikörperabhängige zellvermittelte Zytotoxizität in Bezug auf eine humane Kontroll-NK-Zelle oder humane Kontroll-NK-Zelllinie aufweist; und/oder
(iii) eine verstärkte Anti-Tumor-Wirksamkeit in Bezug auf eine humane Kontroll-NK-Zelle oder humane Kontroll-NK-Zelllinie aufweist,

wahlweise, wobei die Kontroll-NK-Zelle eine humane NK-Zelle ist, die in Gegenwart von IL-15 allein aktiviert wird, oder eine humane NK-Zelllinie ist, die in Gegenwart von IL-15 allein aktiviert wird.

15. Manipulierte NK-Zelle oder Population von Zellen nach einem der Ansprüche 1-14, welche ein IL-15-Polypeptid exprimiert, wahlweise ein humanes IL-15-Polypeptid,
wobei wahlweise das IL-15-Polypeptid ein sekretiertes IL-15-Polypeptid oder ein membrangebundenes IL-15-Polypeptid ist.

**Revendications**

1. Cellule NK humaine de type mémoire induite par des cytokines, modifiée, ou une population desdites cellules, dans laquelle la cellule NK modifiée ou la population desdites cellules exprime un polypeptide de récepteur antigénique chimérique (CAR) comprenant un domaine intracellulaire, un domaine transmembranaire et un domaine de liaison extracellulaire, dans laquelle le domaine de liaison extracellulaire se lie spécifiquement à un antigène comprenant un néo-épitope de NPM1c en complexe avec une protéine de complexe majeur d'histocompatibilité (CMH de classe I).

2. Cellule NK modifiée ou population de cellules selon la revendication 1, dans laquelle le domaine de liaison extracellulaire ne se lie pas à, ou ne se lie sensiblement pas à : (a) la protéine CMH de classe I seule, et/ou (b) un peptide témoin en complexe avec la protéine CMH de classe I, éventuellement dans laquelle le peptide témoin est un épitope de NY-ESO-1 ou un épitope M1 du virus de la grippe.

3. Cellule NK modifiée ou population de cellules selon la revendication 1 ou la revendication 2, dans laquelle le néo-épitope de NPM1c comprend une séquence d'acides aminés choisie parmi : AIQDLCLAV (SEQ ID NO:1) ou AIQDLCVAV (SEQ ID NO: 71).

4. Cellule NK modifiée ou population de cellules selon la revendication 1 ou la revendication 2, dans laquelle le néo-épitope de NPM1c comprend une séquence d'acides aminés choisie parmi : CLAVEEVSL (SEQ ID NO :72), VEEVSLRK (SEQ ID NO :73), AVEEVSLR (SEQ ID NO :74), AVEEVSLRK (SEQ ID NO :75), CLAVEEVSLRK (SEQ ID NO :76).

**5.** Cellule NK modifiée ou population de cellules selon l'une quelconque des revendications 1 à 4, dans laquelle la protéine CMH de classe I est une protéine HLA-A*02 et/ou est codée par le groupe allélique HLA-A*02.

**6.** Cellule NK modifiée ou population de cellules selon l'une quelconque des revendications 1 à 5, dans laquelle le domaine extracellulaire comprend :

(i) une région variable de chaîne lourde (VH) comprenant une région déterminant la complémentarité (CDR)1 de VH, une VH CDR2 et une VH CDR3, lesdites VH CDR1, VH CDR2 et VH CDR3 étant les CDR d'une VH qui présente une séquence d'acides aminés de SEQ ID NO :5, et/ou
(ii) une région variable de chaîne légère (VL) comprenant une région déterminant la complémentarité (CDR)1 de VL, une VL CDR2 et une VL CDR3, lesdites VL CDR1, VL CDR2 et VL CDR3 étant les CDR d'une VL qui présente une séquence d'acides aminés de SEQ ID NO :3.

**7.** Cellule NK modifiée ou population de cellules selon l'une quelconque des revendications 1 à 6, dans laquelle le domaine extracellulaire comprend une VH comprenant VH CDR1, VH CDR2 et VH CDR3, dans laquelle la VH CDR1 présente la séquence d'acides aminés GETESSYA (SEQ ID NO :9), la VH CDR2 présente la séquence d'acides aminés ISGSGGST (SEQ ID NO :10), et la VH CDR3 présente la séquence d'acides aminés ARLGYPTTTLLPFDY (SEQ ID NO :11), dans laquelle éventuellement le domaine extracellulaire comprend une VL comprenant VL CDR1, VL CDR2 et VL CDR3, dans laquelle la VL CDR1 présente la séquence d'acides aminés QSISSY (SEQ ID NO :6), la VL CD2 présente la séquence d'acides aminés AAS (SEQ ID NO :7), et la VL CD3 présente la séquence d'acides aminés QQSYSTPLT (SEQ ID NO :8).

**8.** Cellule NK modifiée ou population de cellules selon l'une quelconque des revendications 1 à 7, dans laquelle le domaine extracellulaire comprend un scFV, dans laquelle éventuellement le scFV comprend un lieur Gly-Ser choisi dans le groupe consistant en (Gly4Ser) (SEQ ID NO :58), (Gly4Ser)2 (SEQ ID NO :59), (Gly4Ser)3 (SEQ ID NO :60), et (Gly4Ser)4 (SEQ ID NO :61).

**9.** Cellule NK modifiée ou population de cellules selon la revendication 8, dans laquelle le scFv comprend la séquence d'acides aminés de SEQ ID NO :2.

**10.** Cellule NK modifiée ou population de cellules, selon l'une quelconque des revendications 1 à 9, dans laquelle l'antigène est à la surface d'une cellule cancéreuse et dans laquelle éventuellement le cancer est une leucémie myéloïde aiguë (AML).

**11.** Cellule NK modifiée ou population de cellules selon l'une quelconque des revendications 1 à 10, dans laquelle le domaine transmembranaire comprend le domaine transmembranaire de CD3-zêta, CD8, CD28, DAP 12, 2B4, NKG2D, CD 16, NKp44 ou NKp46 et/ou dans laquelle le domaine intracellulaire comprend un ou plusieurs domaines costimulateurs d'une ou de plusieurs molécules costimulatrices choisies dans le groupe consistant en : CD27, CD28, 4-1BB, 0X40, CD30, CD40, PD-1, ICOS, 2B4, DAP10, CD137 et DAP12.

**12.** Cellule NK modifiée ou population de cellules selon l'une quelconque des revendications 1 à 11, dans laquelle le domaine intracellulaire comprend un domaine de signalisation CD3-zêta comprenant la séquence d'acides aminés présentée dans SEQ ID NO :27, et un domaine costimulateur 4-1BB comprenant la séquence d'acides aminés présentée dans SEQ ID NO :26 ; dans laquelle le polypeptide CAR comprend un domaine transmembranaire CD8 et une région charnière CD8, dans laquelle le domaine transmembranaire CD8 et la région charnière CD8 comprennent la séquence d'acides aminés présentée dans SEQ ID NO :25 ; et dans laquelle le domaine de liaison extracellulaire comprend l'anticorps, ou un fragment de liaison à l'antigène de celui-ci, et une séquence principale comprenant la séquence d'acides aminés présentée dans SEQ ID NO :23.

**13.** Cellule NK modifiée ou population de cellules selon l'une quelconque des revendications 1 à 12, dans laquelle le domaine intracellulaire comprend en outre une séquence peptidique auto-clivante et une cytokine, dans laquelle le clivage du peptide auto-clivant libère la cytokine,
dans laquelle éventuellement la cytokine est IL-12, IL-7, IL-13, IL-15, TNF-$\alpha$, IFN-$\gamma$, ou CCL19.

**14.** Cellule NK modifiée ou population de cellules selon l'une quelconque des revendications 1 à 13, qui

(i) produit une quantité accrue d'IFN$\gamma$ en présence d'une ou de plusieurs cytokines et/ou de cibles tumorales par rapport à une cellule NK humaine témoin ou à une lignée cellulaire NK humaine témoin ;

(ii) présente une cytotoxicité cellulaire dépendante des anticorps améliorée par rapport à une cellule NK humaine témoin ou à une lignée cellulaire NK humaine témoin ;
et/ou
(iii) présente une efficacité antitumorale améliorée par rapport à une cellule NK humaine témoin ou à une lignée cellulaire NK humaine témoin,

éventuellement dans laquelle la cellule NK témoin est une cellule NK humaine activée en présence d'IL-15 seule, ou une lignée cellulaire NK humaine activée en présence d'IL-15 seule.

15. Cellule NK modifiée ou population de cellules selon l'une quelconque des revendications 1 à 14, qui exprime un polypeptide IL-15, éventuellement un polypeptide IL-15 humain, dans laquelle éventuellement le polypeptide IL-15 est un polypeptide IL-15 sécrété ou un polypeptide IL-15 lié à la membrane.

FIG.1

cNK: Conventional NK cells
CIML NK: Cytokine-induced memory-like NK cells

%LDL-R in hNK

FIG.2A

%ASCT2 in hNK

FIG.2B

FIG.2C

NPM1c CAR-NK

CD3ζ

4-1BB

CD8α hinge&TM

ScFv

AIQ

AIQ-HLA-A2 complex

NPM1c⁺ AML cell

ScFv    CD8 Hinge&TM  4-1BB  CD3ζ  P2A  EGFP

CAR

# FIG.3A

Untransduced T                    NPM1c CAR-T

FIG.3B

CAR targeting a neoepitope derived from NPM1c protein

FIG.4

EP 4 117 716 B1

UT

NPM1c CAR

FIG.5

Human PBMC

NK → 16 hrs
IL-15
IL-12
IL-18
→ Wash → → CAR introduction via unconventional lentiviral transduction → 72 hrs → CAR CIML NK

## FIG.6A

%ASCT2Hi in hNK

| IL-15 | + | + | + | + |
| IL-12 | − | − | + | + |
| IL-18 | − | + | − | + |

## FIG.6B

FIG.6C

FIG.6D

FIG.6E

FIG.6F

FIG.7A

EP 4 117 716 B1

FIG.7B

Human NK cell development and maturation

| CD56 bright | CD56 dim | CD56 dim | CD56 dim |
|---|---|---|---|
| CD16 low/− | CD16 + | CD16 + | CD16 + |
| NKG2A + | NKG2A +/− | NKG2A +/− | NKG2A +/− |
| KIRs − | KIRs − | KIRs + | KIRs + |
| CD57 − | CD57 − | CD57 − | CD57 + |

maturation

## FIG.7C

live
62.6

$10^5$
$10^4$
$10^3$
0
$-10^3$

Comp-FL8-A :: CD56 Y763-PC7-A

CD56+ CD3−
6.28

0    $10^4$    $10^5$

Comp-FL10-A :: CD3 R712-APCA700-A

## FIG.7D

FIG.7D on sheet 12/51

FIG.7D cont.

**FIG.7E**

**FIG.7F**

FIG.8A

IFNg

GzmB

CD107a

FIG.8B

EP 4 117 716 B1

FIG.8C

FIG.8D

FIG.8E

FIG.8G

FIG.8F

FIG.9A

CAR surface expression during culture

IN ORDER:

Untransduced

CAR construct 1
anti-NPM1c CAR s15

CAR construct 2
anti-NPM1c CAR m15

CAR construct 3
anti-NPM1c CAR

% protein L binding+ in viable NK

CIML NK with indicated CAR transduction

FIG.9B

FIG.9C

Targets:
OCI–AML3
(NPM1c+,
HLA–A2+,
Luc+)

Targets:
OCI–AML2
(NPM1c–,
HLA–A2+,
Luc+)

FIG.10A

FIG.10B

Target: OCI-AML3 (NPM1c+)

Control Target: OCI-AML2 (NPM1c−)

FIG.10C

UT
CAR construct1 anti-NPM1c CAR s15
CAR construct2 anti-NPM1c CAR m15
CAR construct3 anti-NPM1c CAR

EP 4 117 716 B1

CIML NK transduction

Untransduced

CAR-transduced

GFP (transduction reporter)

FIG.10D

EP 4 117 716 B1

CAR CIML NK cell expansion

# NK cells

2.5x10^6
2.0x10^6
1.5x10^6
1.0x10^6
5.0x10^5
0.0

Days post transduction of human primary NK

● NPM1c-CAR
■ NPM1c-CAR-mIL15

## FIG.1OE

CAR CIML NK cell function
IFN-gamma

Untransduced
NPM1c-CAR
NPM1c-CAR-mIL15

CD107a

**FIG.10F**

AML target killing

Untransduced
NPM1c-CAR
NPM1c-CAR-mIL15

**FIG.10G**

FIG.11A

EP 4 117 716 B1

FIG.11B

AML Burden

FIG.11C

FIG.12

"Missing Self"

Inhibitory
KIR

MHC
Class 1

Virus

Activating/
NKG2D

Activating
ligands
MIC-A/MIC-B

Malignant
transformation

FIG.13

EP 4 117 716 B1

Conventional NK cells have a life span of around 12-14 days
Limited expansion and persistence *in vivo*
Modest success mostly restricted to AML and MDS patients (~5-25%)
Primary NK cells are resistant to genetic manipulation

FIG.14

FIG.15

FIG.16

EP 4 117 716 B1

FIG.17

FIG.18

FIG.18 cont.

FIG.18 cont.

| UPN | Dose Level | Gender | Age (years) | WHO Dx | BM Blast (%) | KIR Ligand Mismatch | IWG Response | DLT | GVHD | |
|-----|-----------|--------|-------------|--------|--------------|---------------------|--------------|-----|------|---|
| 001 | 1 | M | 73 | M2 | 16 | Yes | TF–PD | No | No | >50% CR Rat |
| 006 | 1 | M | 70 | M0 | 28 | Yes | TF–PD | No | No | |
| 007 | 1 | M | 77 | M0 | 47 | Yes | CR | No | No | 2 Long term survivors |
| 008 | 2 | M | 76 | t–AML | 17 | Yes | TF–PD | No | No | |
| 009 | 2 | F | 73 | M1 | 80 | No | MLFS | No | No | |
| 012 | 2 | F | 71 | M5 | 15 | Yes | CR | No | No | |
| 017 | 3 | M | 64 | t–AML | 69 | Yes | TF–PD | No | No | 1 Died in CR |
| 019 | 3 | F | 71 | M5 | 15 | Yes | CR | No | No | |
| 020 | 3 | M | 60 | MDS–AML | 13 | Yes | CRp | No | No | |
| 023 | 3 | M | 83 | M1 | 86 | No | CRp | No | No | |
| 025 | 3 | M | 72 | t–AML | 1 | No | CR | No | No | |
| 027 | 3 | M | 76 | M1 | In CR | No | CRp | No | No | |
| 028 | 3 | F | 69 | HMA failed MDS | 6 | No | Marrow CR | No | No | |

FIG.19

Relapse remains most common cause of death.

Phase 1 a/b study in relapsed patients

Donor lymphocyte infusion (DLI) with suboptimal outcomes (15–20% response)

Potential for more GVHD due to HLA mismatch

*Unmet need*

Donor

NK cell purification (CD3-/CD56+)

Non-mobilized apheresis

rhIL-12, rhIL-15+ rhIL-18 (14–16 hours)

Wash

CIML NK cell infusion

AML/MDS/MPN Patients relapsed after haplo-HCT    Days:

Flu/Cy

IL-2($1\times10^6$IU/$m^2$) SC QOD x 7

-7 -6 -5 -4 -3 -2 -1  0   1 2 3 4 5 6 7 ...          30

FIG.20

First 4 patients cleared disease, 2nd patient *(TP53 mutated)* MRD− by flow & RHP

*Massive expansion & prolonged persistence*

FIG.21

FIG.22

EP 4 117 716 B1

66M, diagnosed in 5/2019, achieves CR with tagraxofusp (SL-401)
Underwent RIC Haplo HCT on 9/2019 but relapsed 5 months after Haplo HCT

Underwent memory-like NK cell immunotherapy in 8/2020                    In CR!

Pre-NK cell infusion                              Day 30 post NK cell infusion

Mature NK cells constituted 75% of the 1350/uL peripheral blood lymphocytes at
day +28 after infusion

In vitro BPDCN targeting with ML-NK cells

# FIG.23

EP 4 117 716 B1

Anti-CTLA-4 antibody leads to Treg depletion to facilitate NK cell activity
*NK cell infiltration associated with favorable outcomes in H&N ca patients*
**N-803 is an IL-15 super-agonist** which enhances NK cell activation in vivo

NK cell enrichment
CD3-/CD56+ selection
rhIL-12/15/18
CIML NK cell infusion

Key Eligibility:
platinum-refractory disease

Ipilimumab (3mg/kg)
Flu/Cy
N-803 Q21 days

-21 -7-6-5-4-3-2-1 0 1 2 3 4 5 6 7 8 9 10 11... 30

NK cells 90.0    T cells 0.41
CD56
CD3

First patient treated on 9/15 (platinum/cetux/pembro failure)

>2500 mature NK cells/uL on day 21

IL-15 injection site rash

FIG.24

EP 4 117 716 B1

FIG.25

FIG.26

NK cell CARs are safe (no CRS/no neurotoxicity), response rate similar to CAR T cells

Limited efficacy of CAR T cells in AML/MDS

Dual targeting of myeloid blasts by Mem-NK cells armed with CARs *(antigen expression triggers CAR vs HLA loss triggers direct NK cell cytotoxicity)*

Primary Peripheral blood NK cells are resistant to transduction

FIG.27

EP 4 117 716 B1

FIG.28

pHIV-CAR-CD19hscFv-P2A-GFP (8606 bp)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180057609
- US 20180037625 A
- US 20170362295 A
- US 20170137783 A
- US 20160152723 A
- US 20160206656 A
- US 20160199412 A
- US 20160208018 A
- US 20150232880 A
- US 20150225480 A
- US 20150224143 A
- US 20150224142 A
- US 20150190428 A
- US 20150196599 A
- US 20150152181 A
- US 20150140023 A
- US 20150118202 A
- US 20150110760 A
- US 20150099299 A
- US 20150093822 A
- US 20150093401 A
- US 20150051266 A
- US 20150050729 A
- US 20150024482 A
- US 20150023937 A
- US 20150017141 A
- US 20150017136 A
- US 20150017120 A
- US 20140370045 A
- US 20140370017 A
- US 20140369977 A
- US 20140349402 A
- US 20140328812 A
- US 20140322275 A
- US 20140322216 A
- US 20140322212 A
- US 20140322183 A
- US 20140314795 A
- US 20140308259 A
- US 20140301993 A
- US 20140296492 A
- US 20140294784 A
- US 20140286973 A
- US 20140274909 A
- US 20140274801 A
- US 20140271635 A
- US 20140271582 A
- US 20140271581 A
- US 20140271579 A
- US 20140255363 A
- US 20140242701 A
- US 20140242049 A
- US 20140227272 A
- US 20140219975 A
- US 20140170114 A
- US 20140134720 A
- US 20140134142 A
- US 20140120622 A
- US 20140120136 A
- US 20140106449 A
- US 20140099340 A
- US 20140086828 A
- US 20140065629 A
- US 20140050708 A
- US 20140024809 A
- US 20130344039 A
- US 20130323214 A
- US 20130315884 A
- US 20130309258 A
- US 20130288368 A
- US 20130287752 A
- US 20130287748 A
- US 20130280221 A
- US 20130280220 A
- US 20130266551 A
- US 20130216528 A
- US 20130202622 A
- US 20130071414 A
- US 20120321667 A
- US 20120302466 A
- US 20120301448 A
- US 20120301447 A
- US 20120060230 A
- US 20110213288 A
- US 20110158957 A
- US 20110104128 A
- US 20110038836 A
- US 20070036773 A
- US 20040043401 A
- WO 2016168595 A
- WO 12079000 A
- WO 20150141347 A
- WO 20150031624 A
- WO 20150030597 A
- WO 20140378389 A
- WO 20140219978 A
- WO 20140206620 A
- WO 20140037628 A

- WO 20130274203 A
- WO 20130225668 A
- WO 20130116167 A
- WO 20120230962 A
- WO 20120213783 A
- WO 20120093842 A
- WO 20120071420 A
- WO 20120015888 A
- WO 20110268754 A
- WO 20100297093 A
- WO 20100158881 A
- WO 20100034834 A
- WO 20100015113 A
- WO 20090304657 A
- WO 20040043401 A
- WO 20140322253 A
- WO 20150118208 A
- WO 20150038684 A
- WO 20140024601 A

- WO 20120148552 A
- WO 20110223129 A
- WO 20090257994 A
- WO 20080160607 A
- WO 20080003683 A
- WO 20130121960 A
- WO 20110052554 A
- WO 20100178276 A
- US 7709226 B
- US 6995259 B
- US 6001329 A
- US 6933368 B
- US 5994136 A
- US 6013516 A
- WO 9404678 A
- WO 9425591 A
- US 6005079 A
- US 62987612

**Non-patent literature cited in the description**

- **ANDREESEN, R. et al.** *Cancer Res*, 1990, vol. 50, 7450-7456
- **RUGGERI, L. et al.** *Science*, 2002, vol. 295, 2097-2100
- **REZVANI, K.** *Bone Marrow Transplantation*, 2019, vol. 54, 785-788
- **COULIE et al.** *NAT REV CANCER*, 2014, vol. 14, 135
- **SRIVASTAVA ; RIDDELL.** *J IMMUNOL*, 2018, vol. 200, 459
- **XIE et al.** *Nat Biomed Eng*, 2021, vol. 5, 124
- **ROMEE et al.** *Blood*, 2012
- **ROMEE et al.** *Science TM*, 2016
- **KAKARLA et al.** *Cancer J.*, 2014, vol. 20, 151-155
- **SRIVASTAVA et al.** *Trends Immunol.*, 2015, vol. 36, 494-502
- **NISHIO et al.** *Oncoimmunology*, 2015, vol. 4 (2), e988098
- **GHORASHIAN et al.** *Br. J. Haematol.*, 2015, vol. 169, 463-478
- **LEVINE.** *Cancer Gene Ther.*, 2015, vol. 22, 79-84
- **JENSEN et al.** *Curr. Opin. Immunol.*, 2015, vol. 33, 9-15
- **SINGH et al.** *Cancer Gene Ther.*, 2015, vol. 22, 95-100
- **LI et al.** *Zhongguo Shi Yan Xue Ye Xue Za Zhi*, 2014, vol. 22, 1753-1756
- **GILL et al.** *Immunol. Rev.*, 2015, vol. 263, 68-89
- **MAGEE et al.** *Discov. Med.*, 2014, vol. 18, 265-271
- **GARGETT et al.** *Front. Pharmacol.*, 2014, vol. 5, 235

- **YUAN et al.** *Zhongguo Shi Yan Xue Ye Xue Za Zhi*, 2014, vol. 22, 1137-1141
- **PEDGRAM et al.** *Cancer J.*, 2014, vol. 20, 127-133
- **ESHHAR et al.** *Cancer J.*, 2014, vol. 20, 123-126
- **RAMOS et al.** *Cancer J.*, 2014, vol. 20, 112-118
- **MAUS et al.** *Blood*, 2014, vol. 123, 2625-2635
- **JENA et al.** *Curr. Hematol. Malig. Rep.*, 2014, vol. 9, 50-56
- **MAHER et al.** *Curr. Gene Ther.*, 2014, vol. 14, 35-43
- **RICHES et al.** *Discov. Med.*, 2013, vol. 16, 295-302
- **CHEADLE et al.** *Immunol. Rev.*, 2014, vol. 257, 83-90
- **DAVILA et al.** *Int. J. Hematol.*, 2014, vol. 99, 361-371
- **XU et al.** *Cancer Lett.*, 2014, vol. 343, 172-178
- **KOCHENDERFER et al.** *Nat. Rev. Clin. Oncol.*, 2013, vol. 10, 267-276
- **HOSING et al.** *Curr. Hematol. Malig. Rep.*, 2013, vol. 8, 60-70
- **HOMBACH et al.** *Curr. Mol. Med.*, 2013, vol. 13, 1079-1088
- **XU et al.** *Leuk. Lymphoma*, 2013, vol. 54, 255-260
- **GILHAM et al.** *Trends Mol. Med.*, 2012, vol. 18, 377-384
- **LIPOWSKA-BHALLA et al.** *Cancer Immunol. Immunother.*, 2012, vol. 61, 953-962
- **CHMIELEWSKI et al.** *Cancer Immunol. Immunother.*, 2013, vol. 61, 1269-1277
- **JENA et al.** *Blood*, 2010, vol. 116, 1035-1044

- **DOTTI et al.** *Immunology Reviews*, 2013, vol. 257 (1), 107-126
- **DAI et al.** *Journal of the National Cancer Institute*, 2016, vol. 108 (7), 439
- **WANG** ; **RIVIERE**. *Molecular Therapy-Oncolytics*, 2016, vol. 3, 16015
- **GUO et al.** *Nature*, 1992, vol. 360, 364
- **SILVER et al.** *Nature*, 1992, vol. 360, 367
- **GORGA et al.** *Proteins*, 1992, vol. 12, 87
- **MADDEN**. *Annu Rev Immunol*, 1995, vol. 13, 587
- **MADDEN et al.** *Cell*, 1993, vol. 75, 693
- **MADDEN et al.** *Cell*, 1992, vol. 70, 1035
- **BJORKMAN et al.** *Nature*, 1987, vol. 329, 512
- **SAPER et al.** *J Mol Biol*, 1991, vol. 219, 277
- **SIDNEY et al.** *BMC Immunology*, 2008, vol. 9, 1
- **LEY et al.** *N Engl J Med*, 2013, vol. 368, 2059
- **ALEXANDROV et al.** *NATURE*, 2013, vol. 500, 415
- **KANDOTH et al.** *NATURE*, 2013, vol. 502, 333
- **PAPAEMMANUIL et al.** *N Engl J Med*, 2016, vol. 374, 2209
- **LEE et al.** *J CLIN INVEST*, 2019, vol. 129, 774
- **FALINI et al.** *N Engl J Med*, 2005, vol. 352, 254
- **GREINER et al.** *BLOOD*, 2012, vol. 120, 1282
- **GREINER**. *Blood*, 2012, vol. 120, 1282
- **VAN DER LEE**. *J Clin Invest*, 2019, vol. 129, 774
- **KABAT EA** ; **WU TT**. *Ann NY Acad Sci*, 1971, vol. 190, 382-391
- **KABAT EA et al.** Sequences of Proteins of Immunological Interest. U.S. Department of Health and Human Services, 1991
- **CHOTHIA C** ; **LESK AM**. *J Mol Biol*, 1987, vol. 196, 901-917
- **AL-LAZIKANI B et al.** *J Mol Biol*, 1997, vol. 273, 927-948
- **CHOTHIA C et al.** *J Mol Biol*, 1992, vol. 227, 799-817
- **TRAMONTANO A et al.** *J Mol Biol*, 1990, vol. 215 (1), 175-82
- **CHOTHIA** ; **LESK**. *J. Mol. Biol.*, 1987, vol. 196, 901-917
- Protein Sequence and Structure Analysis of Antibody Variable Domains. **MARTIN, A.** Antibody Engineering. Springer- Verlag, 2001, vol. 31, 422-439
- **LEFRANC, M.-P.** *The Immunologist*, 1999, vol. 7, 132-136
- **LEFRANC, M.-P. et al.** *Nucleic Acids Res.*, 1999, vol. 27, 209-212
- **MACCALLUM RM et al.** *J Mol Biol*, 1996, vol. 5, 732-745
- Protein Sequence and Structure Analysis of Antibody Variable Domains. **MARTIN A.** Antibody Engineering. Springer- Verlag, 2001, 422-439
- **ADACHI et al.** *Nature Biotechnology*, 2018
- **LIU et al.** *J. Immunol.*, 2019
- **KRENCIUTE et al.** *Cancer Immunol. Res.*, 2017, vol. 597, 571-581
- **LIU et al.** *Leukemia*, 2018, vol. 32, 520-531
- **AHMAD et al.** *Clinical and Developmental Immunology*, 2012
- **WANG et al.** *Anal. Chem.*, 2006, vol. 78, 997-1004
- **PANSRI et al.** *BMC Biotechnology*, 2009, vol. 9, 6
- **CHAO et al.** *Nature Protocols*, 2006, vol. 1 (2), 755-768
- **RIECHMANN** ; **MUYLDERMANS**. *J Immunol*, 1999, vol. 231, 25-38
- **NUTTALL et al.** *Curr Pharm Biotechnol*, 2000, vol. 1 (3), 253-263
- **MUYLDERMANS**. *J Biotechnol*, 2001, vol. 74 (4), 277-302
- **KONTERMAN**. *MAbs*, 2012, vol. 4, 182-197
- **GRAMER et al.** *MAbs*, 2013, vol. 5, 962-973
- **SHIRAISHI et al.** *Nucleic Acids Symposium Series*, 2007, vol. 51 (1), 129-130
- **MULLIGAN** ; **BERG**. *Proc Natl Acad Sci USA*, 1981, vol. 78, 2072
- **SOUTHERN** ; **BERG**. *Mol Appl Genet*, 1982, vol. 1, 327
- **WIGLER et al.** *Cell*, 1979, vol. 16, 77
- **SARVER et al.** *Proc Natl Acad Sci USA*, 1982, vol. 79, 7147
- **DEANS et al.** *Proc Natl Acad Sci USA*, 1984, vol. 81, 1292
- **LUSKY** ; **BOTCHAN**. *Nature*, 1981, vol. 293, 79
- **HOU et al.** *Cytokine*, 1998, vol. 10, 319-30
- Current Protocols in Molecular Biology. Wiley & Sons
- Molecular Cloning--A Laboratory Manual. Cold Spring Harbor Laboratory Press, 2001
- **KASZUBSKA et al.** *Protein Expression and Purification*, 2000, vol. 18, 213-220
- **SCOPES**. Protein Purification. Springer-Verlag, 1994
- **ROGERS et al.** *J Nucl Med*, 1997, vol. 38, 1221-1229
- **WELCH** ; **REDVANLY**. Handbook of Radiopharmaceuticals: Radiochemistry and Applications. John Wiley and Sons, 2003
- **WRIGHT et al.** *EMBO J*, 1991, vol. 10 (10), 2717-2723
- **CO et al.** *Mol Immunol*, 1993, vol. 30, 1361
- Eukaryotic Viral Vectors. CSH Laboratory Press
- **CRONIN et al.** *CURR. GENE THER.*, 2005, vol. 5, 387-398
- **BERRIEN-ELLIOTT, M. M. et al.** *Cancer Discovery*, December 2020
- **ROMEE. R. et al.** *Sci Transl Med*, 21 September 2016, vol. 8 (357)

- ELISA: Theory and Practice: Methods in Molecular Biology. Human Press, 1995, vol. 42
- **E. DIAMANDIS** ; **T. CHRISTOPOULUS**. Immunoassay. Academic Press, Inc., 1996
- **P. TIJSSEN**. Practice and Theory of Enzyme Immunoassays. Elsevier Science Publishers, 1985
- **ROMEE, RIZWAN et al.** Cytokine activation induces human memory-like NK cells. *Blood*, 2012, vol. 120 (24), 4751-4760
- **SUN et al.** *Cytokine*, October 2015, vol. 75 (2), 249-255
- **HE, QINGHUA et al.** TCR-like antibodies in cancer immunotherapy. *Journal of hematology & oncology*, 2019, vol. 12 (1), 99
- **ENBLAD et al.** *Human Gene Therapy*, 2015, vol. 26 (8), 498-505
- **ROMEE R et al.** *Blood*, 2012, vol. 120 (24)
- **ROMEE R.** *Sci. Transl. Med.*, 2016, vol. 21, 357-123
- **BARI, R. et al.** *Frontiers in Immuno.*, 2019, vol. 10
- **TOMÁS, H. et al.** *Mol Ther Methods Clin Dev.*, 2019, vol. 15, 1-8
- Remington's Pharmaceutical Sciences. Mack Publishing Co., 1990
- **A. R. GENNARO**. Remington: The Science and Practice of Pharmacy. Lippincott, Williams & Wilkins, 2006
- **VAN DER LEE et al.** *J. Clin. Invest.*, 2019, vol. 129 (2), 774-785
- **VAN DER LEE et al.** *JCI*, 2019, vol. 129 (2), 774-785
- **VERHAAK, R. et al.** *Blood*, 2005, vol. 106, 3747
- **ALTSCHUL et al.** *Nucleic Acids Res.*, 1997, vol. 25 (17), 3389-3402
- **NEEDLEMAN** ; **WUNSCH**. *J. Mol. Biol.*, 1970 (48), 444-453
- **MUYLDERMANS et al.** *Trends Biochem Sci*, 2001, vol. 26, 230-235
- **NUTTALL et al.** *Curr Pharm Biotech*, 2000, vol. 1, 253-263
- **REICHMANN et al.** *J Immunol Meth*, 1999, vol. 231, 25-38
- **TODOROVSKA et al.** *J Immunol Methods*, 2001, vol. 248 (1), 47-66
- **HUDSON** ; **KORTT**. *J Immunol Methods*, 1999, vol. 231 (1), 177-189
- **POLJAK**. *Structure*, 1994, vol. 2 (12), 1121-1123
- **RONDON** ; **MARASCO**. *Annual Review of Microbiology*, 1997, vol. 51, 257-283
- **KYTE et al.** *J Mol Biol*, 1982, vol. 157, 105-131
- **FALINI B et al.** *Discov. Med.*, 2010, vol. 10 (53), 281-92
- **VENSTROM JM et al.** *N. Engl. J. Med.*, 2012, vol. 367 (9), 805-816
- **COOLEY S**. *Blood*, 2010, vol. 116 (14), 2411-9
- **REZVANI et al.** *ASH*, 2018
- **SUTLU et al.** *Cytotherapy*, 2010
- **SUTLU T et al.** *Cytotherapy*, 2010, vol. 12 (8), 1044-55
- **DENMAN CJ et al.** *PLoS One*, 2012, vol. 7 (1), e30264
- **ROMEE, R. et al.** *Blood*, 2012, vol. 120, 4751-4760
- **ROMEE, R. et al.** *Sci Transl Med*, 2016, vol. 8, 357-123
- **ROMEE, R. et al.** *Scientifica (Cairo)*, 2014, vol. 2014, 205796
- **LIU et al.** *NEJM*, 2020, vol. 382, 545-53
- **ROMEE R et al.** *Blood*, 2012, vol. 120 (24), 4751-4760
- **ROMEE R et al.** *Sci Transl Med*, 2016, vol. 8 (357), 357-123
- **ZHENG et al.** *J. Transl Med.*, 2012, vol. 10, 29
- **STRAUSS-ALBEE**. *Sci Transl Med*, 2015, vol. 7, 297-115
- **AMIR et al.** *Nat Biotech*, 2013, vol. 31, 545
- **ROMEE et al.** *Sci Transl Med*, 2016, vol. 8, 357-123
- **DIGGINS et al.** *Methods*, 2015, vol. 82, 55
- **ROMEE et al.** *Science Trans Med*, 2016
- **LIU et al.** *NEJM*, 2020
- **ROMEE et al.** *STM*, 2016
- **GREINER J** ; **ONO Y** ; **HOFMANN S** ; **SCHMITT A** ; **MEHRING E** ; **GÖTZ M** ; **GUILLAUME P** ; **DÖHNER K** ; **MYTILINEOS J** ; **DÖHNER H**. Mutated regions of nucleophosmin 1 elicit both CD4+ and CD8+ T-cell responses in patients with acute myeloid leukemia. *Blood*, 2012, vol. 120 (6), 1282-1289
- **GREINER J** ; **SCHNEIDER V** ; **SCHMITT M** ; **GÖTZ M** ; **DÖHNER K** ; **WIESNETH M** ; **DÖHNER H** ; **HOFMANN S**. Immune responses against the mutated region of cytoplasmatic NPM1 might contribute to the favorable clinical outcome of AML patients with NPM1 mutations (NPM1mut). *Blood*, 2013, vol. 122 (6), 1087-8
- **FELICES M** ; **LENVIK AJ** ; **MCELMURRY R** ; **CHU S** ; **HINDERLIE P** ; **BENDZICK L** ; **GELLER MA** ; **TOLAR J** ; **BLAZAR BR** ; **MILLER JS**. Continuous treatment with IL-15 exhausts human NK cells via a metabolic defect. *JCI insight*, 2018, vol. 3 (3)
- **KOHN, D.B.** ; **HOLLIS, R.P.** Envelope, please. And the award goes to. *Blood*, 2014, vol. 124, 1203-1204
- **BALD, T.** ; **KRUMMEL, M. F.** ; **SMYTH, M. J.** ; **BARRY, K. C.** The NK cell-cancer cycle: advances and new challenges in NK cell-based immunotherapies. *Nat Immunol*, 2020, vol. 21 (8), 835-847

- **CHAN, C. J.** ; **SMYTH, M. J.** ; **MARTINET, L.** Molecular mechanisms of natural killer cell activation in response to cellular stress. *Cell Death Differ*, 2014, vol. 21 (1), 5-14
- **CHIOSSONE, L.** ; **DUMAS, P. Y.** ; **VIENNE, M.** ; **VIVIER, E.** Natural killer cells and other innate lymphoid cells in cancer. *Nat Rev Immunol*, 2018, vol. 18 (11), 671-688
- **FELICES, M.** ; **LENVIK, A. J.** ; **MCELMURRY, R.** ; **CHU, S.** ; **HINDERLIE, P.** ; **BENDZICK, L.** ; **MILLER, J. S.** Continuous treatment with IL-15 exhausts human NK cells via a metabolic defect. *JCI Insight*, 2018, vol. 3 (3)
- **HASHIMOTO, N.** ; **TSUBOI, A.** ; **KAGAWA, N.** ; **CHIBA, Y.** ; **IZUMOTO, S.** ; **KINOSHITA, M.** Wilms tumor 1 peptide vaccination combined with temozolomide against newly diagnosed glioblastoma: safety and impact on immunological response. *Cancer Immunol Immunother*, 2015, vol. 64 (6), 707-716
- **ROMEE, R.** ; **ROSARIO, M.** ; **BERRIEN-ELLIOTT, M. M.** ; **WAGNER, J. A.** ; **JEWELL, B. A.** ; **SCHAPPE, T.** ; **FEHNIGER, T. A.** Cytokine-induced memory-like natural killer cells exhibit enhanced responses against myeloid leukemia. *Sci Transl Med*, 2016, vol. 8 (357), 357-123
- **SMITH, C. C.** ; **WANG, Q.** ; **CHIN, C. S.** ; **SALERNO, S.** ; **DAMON, L. E.** ; **LEVIS, M. J.** ; **SHAH, N. P.** Validation of ITD mutations in FLT3 as a therapeutic target in human acute myeloid leukaemia. *Nature*, 2012, vol. 485 (7397), 260-263
- **THOMAS, R.** ; **AL-KHADAIRI, G.** ; **ROELANDS, J.** ; **HENDRICKX, W.** ; **DERMIME, S.** ; **BEDOGNETTI, D.** ; **DECOCK, J.** NY-ESO-1 Based Immunotherapy of Cancer: Current Perspectives. *Front Immunol*, 2018, vol. 9, 947
- **WANG, E.** ; **AIFANTIS, I.** RNA Splicing and Cancer. *Trends Cancer*, 2020, vol. 6 (8), 631-644
- **XIE, G.** ; **DONG, H.** ; **LIANG, Y.** ; **HAM, J. D.** ; **RIZWAN, R.** ; **CHEN, J.** CAR-NK cells: A promising cellular immunotherapy for cancer. *EBioMedicine*, 2020, vol. 59, 102975
- **MENSALI, N.** ; **DILLARD, P.** ; **HEBEISEN, M.** ; **LORENZ, S.** ; **THEODOSSIOU, T.** ; **MYHRE, M. R.** ; **WALCHLI, S.** NK cells specifically TCR-dressed to kill cancer cells. *EBioMedicine*, 2019, vol. 40, 106-117
- **WALSENG, E.** ; **KOKSAL, H.** ; **SEKTIOGLU, I. M.** ; **FANE, A.** ; **SKORSTAD, G.** ; **KVALHEIM, G.** ; **WALCHLI, S.** A TCR-based Chimeric Antigen Receptor. *Sci Rep*, 2017, vol. 7 (1), 10713